(19) Europäisches Patentamt
European Patent Office
Office européen des brevets

(11) EP 4 534 543 A1

(12) EUROPEAN PATENT APPLICATION
published in accordance with Art. 153(4) EPC

(43) Date of publication:
09.04.2025 Bulletin 2025/15

(21) Application number: 23815226.8

(22) Date of filing: 30.05.2023

(51) International Patent Classification (IPC):
*C07H 15/203* (2006.01) *A61K 31/706* (2006.01)
*A61K 38/14* (2006.01) *A61P 35/00* (2006.01)

(52) Cooperative Patent Classification (CPC):
A61K 31/706; A61K 38/14; A61P 35/00;
C07H 15/203

(86) International application number:
PCT/CN2023/097277

(87) International publication number:
WO 2023/232048 (07.12.2023 Gazette 2023/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 30.05.2022 CN 202210602187

(71) Applicant: Tianjin Hemay Oncology Pharmaceutical Co., Ltd
Tianjin 300385 (CN)

(72) Inventors:
- HONG, Zhangyong
Tianjin 300385 (CN)
- ZHANG, Hesheng
Tianjin 300385 (CN)
- ZENG, Guanghuai
Tianjin 300385 (CN)

(74) Representative: Müller-Boré & Partner
Patentanwälte PartG mbB
Friedenheimer Brücke 21
80639 München (DE)

# (54) CAMPTOTHECIN PRODRUG AND PHARMACEUTICAL COMPOSITION THEREOF

(57) Disclosed are a compound of Formula (I), a stereoisomer, and a cis-trans isomer thereof:

EP 4 534 543 A1

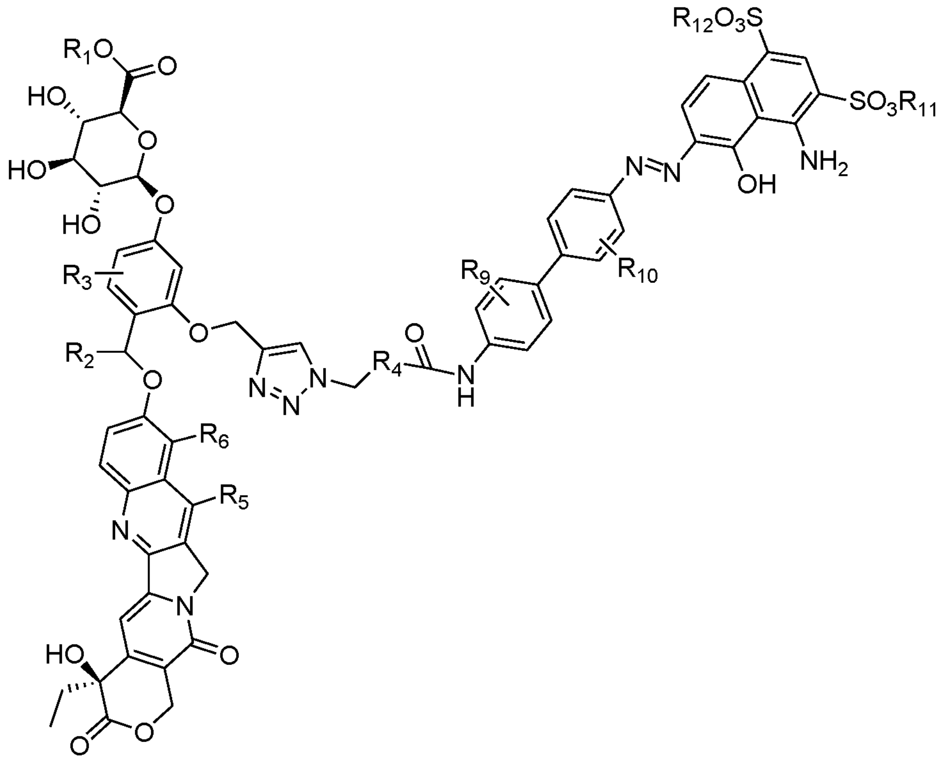

Formula (I)

wherein $R_1$, $R_2$, $R_3$, $R_4$, $R_5$, $R_6$, $R_7$, $R_8$, $R_9$, $R_{10}$, $R_{11}$, $R_{12}$, $R_{13}$, $R_{14}$, $R_{15}$ are as defined in the present disclosure.

## Description

### REFERENCE TO RELATED APPLICATION

The present disclosure claims all the benefits of the Chinese patent application No. 202210602187.7, entitled "Camptothecin Prodrug and Pharmaceutical Composition Thereof", filed on May 30, 2022 before the National Intellectual Property Administration of the People's Republic of China, which is incorporated herein in its entirety by reference.

### FIELD

The present disclosure generally relates to the field of organic chemistry and pharmaceutical chemistry.

### BACKGROUND

Cancer is a major killer of human health. According to the latest worldwide cancer burden data for 2020 released by the World Health Organization International Cancer Research Institute (IARC), there are 19.29 million new cancer cases worldwide in 2020, of which 4.57 million are new cancer cases in China, and 9.96 million are cancer deaths worldwide in 2020, of which 3 million are cancer deaths in China, and both the new cancer cases and the deaths in China are among the highest in the world. Chemotherapy is still one of the main treatments for cancer, but traditional chemotherapeutic drugs lack tumor cell specificity and are also highly toxic to normal cells while killing tumor cells, which can lead to serious systemic toxic and side effects. In addition, many chemotherapeutic drugs have problems such as poor water solubility, low bioavailability, and the like, which limits their clinical application. Carbohydrates, as one of the basic substances of life, are widely used in the design of prodrugs. The glycosyl modification not only can greatly improve the water solubility of the drug, but also can achieve tumor cell targeting of the chemotherapeutic drug through the sugar transporter, the glycosidase, and the like. Prodrug modification is an effective strategy to improve tumor targeting of chemotherapeutic drugs, reduce drug side effects, and improve antitumor activity.

Camptothecin is an alkaloid antineoplastic drug extracted from the bark and fruit of Camptotheca involucrata by American chemist Wall et al., in 1966, camptothecin exerts antineoplastic activity at a nanomolar concentration, and has a good therapeutic effect on various malignancies such as digestive tract tumors, leukemias, and bladder carcinomas. However, camptothecin has a very poor water solubility (2.5μg/ml). In clinic, the treatment of camptothecin causes serious toxic side effects such as myelosuppression, diarrhea, vomiting, and urine blood, but its clinical use faces problems such as high toxicity, low selectivity, and low water solubility. The development of camptothecin derivatives and their prodrugs has been ongoing.

Prijovich et al., using 5,6-dihydro -4H- benzo [de] quinoline camptothecin (BQC) as a lead compound, coupled glucuronic acid at its 10-hydroxy group, prepared a saccharide conjugate BQC-G. The water solubility of BQC-G is greatly improved at physiological pH compared to BQC.

9ACG is a carbohydrate conjugate of a 9-amino camptothecin (9AC) lead compound. Toxicity studies have shown that a dose of 50 mg/kg has reached the lethal dose of 9ACG to mice.

The use of glycosidases to modify prodrugs of camptothecin and its derivatives has continued to improve.

### SUMMARY

In one aspect, the present disclosure relates to a compound of Formula (I), a stereoisomer and a cis-trans isomer thereof:

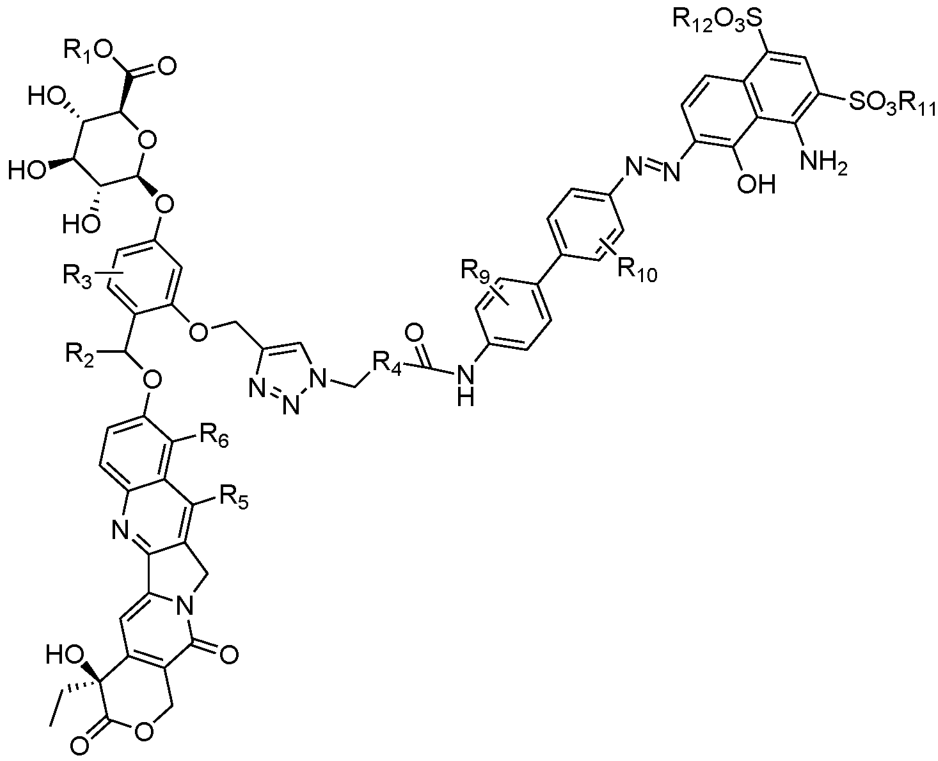

Formula (I)

wherein

$R_1$ is selected from the group consisting of hydrogen, $C_1$-$C_8$ hydrocarbyl, -Na, -Li, -K, -Cs, $-NH_4^+$, $-NH_2^+R_{13}R_{14}$, $-NH^+R_{13}R_{14}R_{15}$, $-NH^+_3R_{13}$, and natural or unnatural amino acids;

$R_2$ is selected from the group consisting of hydrogen, and $C_1$-$C_8$ hydrocarbyl;

$R_3$ is one or more substituents at any substitution position selected from the group consisting of hydrogen, halogen, $C_1$-$C_8$ hydrocarbyl, $C_1$-$C_8$ hydrocarbyloxy, cyano, nitro, amino, aryl, arylhydrocarbyl, and $C_1$-$C_4$ hydrocarbyl substituted amino;

$R_4$ is selected from the group consisting of $C_1$-$C_9$ alkylidene, $C_1$-$C_8$ alkylideneoxy, $C_1$-$C_8$ hydrocarbyl substituted or unsubstituted $C_3$-$C_8$ heteroaryl, $C_1$-$C_8$ hydrocarbyl substituted or unsubstituted $C_3$-$C_{15}$ aryl, $-(CH_2R_7)_n$, $-(R_8)_m$, $C_1$-$C_9$ alkylidene-arylidene, $C_1$-$C_9$ alkylidene-arylidene-$C_1$-$C_9$ alkyldiene, $C_1$-$C_9$ alkylidene-arylidene-arylidene, $C_1$-$C_9$ alkylidene-heteroaryliden-$C_1$-$C_9$ alkylidene, and amino acid peptide chain; wherein the amino acid peptide chain is composed of a combination of natural or unnatural amino acids, and the peptide chain length is 1 to100 peptides, preferably 1 to 50 peptides, and more preferably 1 to 20 peptides;

$R_5$ is selected from the group consisting of hydrogen, and $C_1$-$C_8$ hydrocarbyl;

$R_6$ is selected from the group consisting of hydrogen, $C_1$-$C_8$ hydrocarbyl, $C_1$-$C_8$ hydrocarbylidene amino ($C_1$-$C_4$ hydrocarbyl)$_2$, $C_1$-$C_8$ hydrocarbylidene thio $C_1$-$C_8$ hydrocarbyl, and $C_1$-$C_9$ alkylidene-oxy-$C_1$-$C_9$ alkyl;

$R_5$ and $R_6$ may form a ring, and if $R_5$ and $R_6$ form a ring, the ring may be a five-membered ring or a six-membered ring;

$R_7$ is selected from hydrogen, $C_1$-$C_8$ hydrocarbylideneoxy, amino $C_1$-$C_8$ hydrocarbylidene, thio $C_1$-$C_8$ hydrocarbylidene, and oxy $C_1$-$C_8$ hydrocarbylidene;

n is selected from the group consisting of 0, 1, 2, 3, 4, 5, 6, 7, 8, and 9; if n is 0, $(CH_2R_7)_n$ represents a covalent bond;

$R_8$ is carbonylamino, wherein amino may be substituted with $C_1$-$C_8$ hydrocarbyl or arylhydrocarbyl, $C_1$-$C_8$ hydrocarbylidene amino $C_1$-$C_8$ hydrocarbylidene, $C_1$-$C_8$ hydrocarbylidene thio $C_1$-$C_8$ hydrocarbylidene, or $C_1$-$C_8$ hydrocarbylidene oxy $C_1$-$C_8$ hydrocarbylidene;

m is selected from the group consisting of 0, 1, 2, 3, 4, 5, 6, 7, 8, and 9; if m is 0, $-(R_8)_m$ represents a covalent bond;

$R_9$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{10}$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{13}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{14}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{15}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl; and

the natural or unnatural amino acids in $R_1$, $R_{11}$, and $R_{12}$ are selected from the group consisting of glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine.

In another aspect, the present disclosure relates to a pharmaceutical composition, comprising a compound of Formula (I), or a stereoisomer thereof, and a pharmaceutically acceptable carrier:

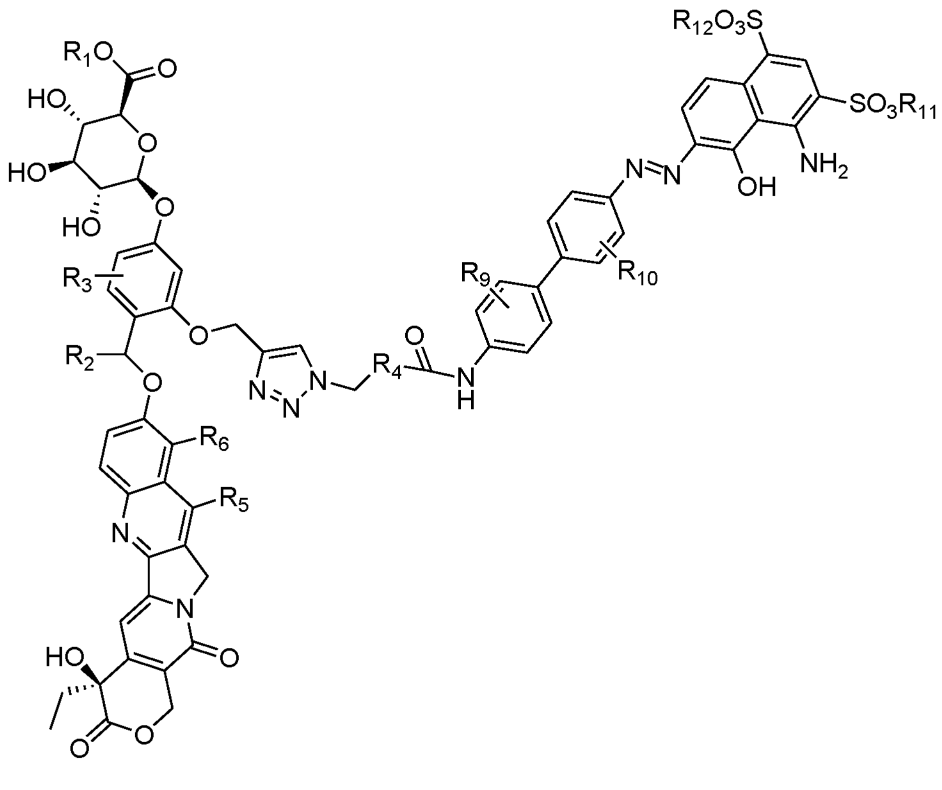

Formula (I)

wherein

$R_1$ is selected from the group consisting of hydrogen, $C_1$-$C_8$ hydrocarbyl, -Na, -Li, -K, -Cs, $-NH_4^+$, $-NH_2^+R_{13}R_{14}$, $-NH^+R_{13}R_{14}R_{15}$, $-NH^+{}_3R_{13}$, and natural or unnatural amino acids;

$R_2$ is selected from the group consisting of hydrogen, and $C_1$-$C_8$ hydrocarbyl;

$R_3$ is one or more substituents at any substitution position selected from the group consisting of hydrogen, halogen, $C_1$-$C_8$ hydrocarbyl, $C_1$-$C_8$ hydrocarbyloxy, cyano, nitro, amino, aryl, arylhydrocarbyl, and $C_1$-$C_4$ hydrocarbyl substituted amino;

$R_4$ is selected from the group consisting of $C_1$-$C_9$ alkylidene, $C_1$-$C_8$ alkylideneoxy, $C_1$-$C_8$ hydrocarbyl substituted or unsubstituted $C_3$-$C_8$ heteroaryl, $C_1$-$C_8$ hydrocarbyl substituted or unsubstituted $C_3$-$C_{15}$ aryl, $-(CH_2R_7)_n$, $-(R_8)_m$, $C_1$-$C_9$ alkylidene-arylidene, $C_1$-$C_9$ alkylidene-arylidene-$C_1$-$C_9$ alkyldiene, $C_1$-$C_9$ alkylidene-arylidene-arylidene, $C_1$-$C_9$ alkylidene-heteroaryliden-$C_1$-$C_9$ alkylidene, and amino acid peptide chain; wherein the amino acid peptide chain is composed of a combination of natural or unnatural amino acids, and the peptide chain length is 1 to100 peptides, preferably 1 to 50 peptides, and more preferably 1 to 20 peptides;

$R_5$ is selected from the group consisting of hydrogen, and $C_1$-$C_8$ hydrocarbyl;

$R_6$ is selected from the group consisting of hydrogen, $C_1$-$C_8$ hydrocarbyl, $C_1$-$C_8$ hydrocarbylidene amino ($C_1$-$C_4$ hydrocarbyl)$_2$, $C_1$-$C_8$ hydrocarbylidene thio $C_1$-$C_8$ hydrocarbyl, and $C_1$-$C_9$ alkylidene-oxy-$C_1$-$C_9$ alkyl;

$R_5$ and $R_6$ may form a ring, and if $R_5$ and $R_6$ form a ring, the ring may be a five-membered ring or a six-membered ring;

$R_7$ is selected from hydrogen, $C_1$-$C_8$ hydrocarbylideneoxy, amino $C_1$-$C_8$ hydrocarbylidene, thio $C_1$-$C_8$ hydrocarbylidene, and oxy $C_1$-$C_8$ hydrocarbylidene;

n is selected from the group consisting of 0, 1, 2, 3, 4, 5, 6, 7, 8, and 9; if n is 0, $(CH_2R_7)_n$ represents a covalent bond;

$R_8$ is carbonylamino, wherein amino may be substituted with $C_1$-$C_8$ hydrocarbyl or arylhydrocarbyl, $C_1$-$C_8$ hydrocarbylidene amino $C_1$-$C_8$ hydrocarbylidene, $C_1$-$C_8$ hydrocarbylidene thio $C_1$-$C_8$ hydrocarbylidene, or $C_1$-$C_8$ hydrocarbylidene oxy $C_1$-$C_8$ hydrocarbylidene;

m is selected from the group consisting of 0, 1, 2, 3, 4, 5, 6, 7, 8, and 9; if m is 0, $-(R_8)_m$ represents a covalent bond;

$R_9$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{10}$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{13}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{14}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{15}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl; and

the natural or unnatural amino acids in $R_1$, $R_{11}$, and $R_{12}$ are selected from the group consisting of glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine.

In a further aspect, the present disclosure relates to use of a compound of Formula (I), or a stereoisomer thereof, or a pharmaceutical composition comprising a compound of Formula (I), or a stereoisomer thereof, and a pharmaceutically acceptable carrier in the manufacture of a medicament for the treatment of a tumor or cancer:

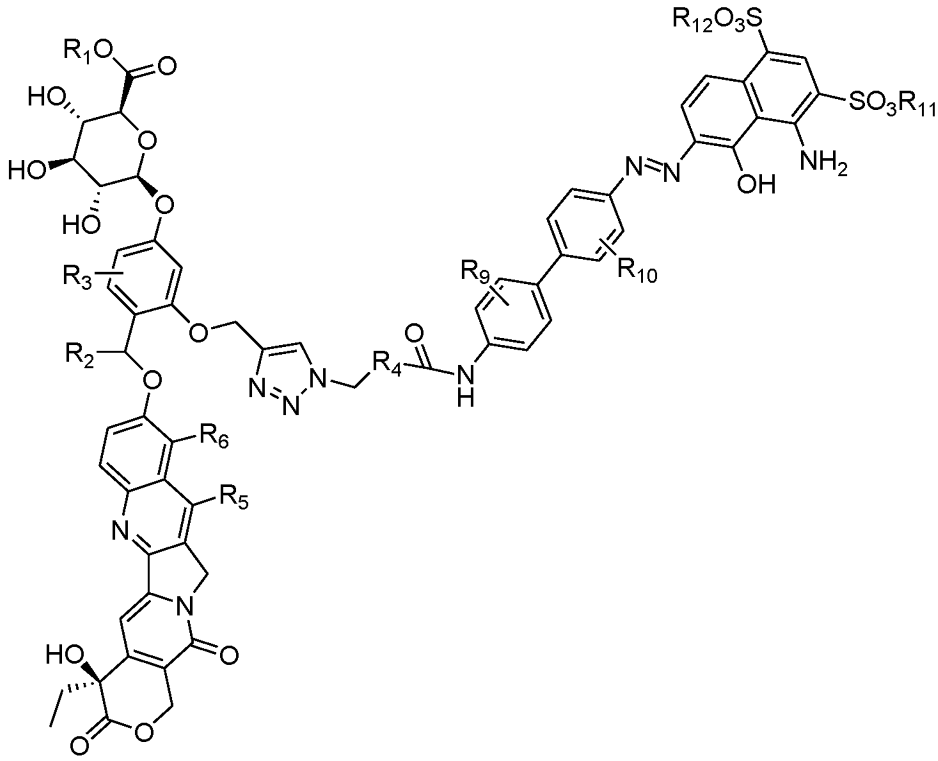

Formula (I)

wherein

$R_1$ is selected from the group consisting of hydrogen, $C_1$-$C_8$ hydrocarbyl, -Na, -Li, -K, -Cs, $-NH_4^+$, $-NH_2^+R_{13}R_{14}$, $-NH^+R_{13}R_{14}R_{15}$, $-NH^+{}_3R_{13}$, and natural or unnatural amino acids;

$R_2$ is selected from the group consisting of hydrogen, and $C_1$-$C_8$ hydrocarbyl;

$R_3$ is one or more substituents at any substitution position selected from the group consisting of hydrogen, halogen, $C_1$-$C_8$ hydrocarbyl, $C_1$-$C_8$ hydrocarbyloxy, cyano, nitro, amino, aryl, arylhydrocarbyl, and $C_1$-$C_4$ hydrocarbyl substituted amino;

$R_4$ is selected from the group consisting of $C_1$-$C_9$ alkylidene, $C_1$-$C_8$ alkylideneoxy, $C_1$-$C_8$ hydrocarbyl substituted or unsubstituted $C_3$-$C_8$ heteroaryl, $C_1$-$C_8$ hydrocarbyl substituted or unsubstituted $C_3$-$C_{15}$ aryl, $-(CH_2R_7)_n$, $-(R_8)_m$, $C_1$-$C_9$ alkylidene-arylidene, $C_1$-$C_9$ alkylidene-arylidene-$C_1$-$C_9$ alkyldiene, $C_1$-$C_9$ alkylidene-arylidene-arylidene, $C_1$-$C_9$ alkylidene-heteroaryliden-$C_1$-$C_9$ alkylidene, and amino acid peptide chain; wherein the amino acid peptide chain is composed of a combination of natural or unnatural amino acids, and the peptide chain length is 1 to100 peptides, preferably 1 to 50 peptides, and more preferably 1 to 20 peptides;

$R_5$ is selected from the group consisting of hydrogen, and $C_1$-$C_8$ hydrocarbyl;

$R_6$ is selected from the group consisting of hydrogen, $C_1$-$C_8$ hydrocarbyl, $C_1$-$C_8$ hydrocarbylidene amino ($C_1$-$C_4$ hydrocarbyl$)_2$, $C_1$-$C_8$ hydrocarbylidene thio $C_1$-$C_8$ hydrocarbyl, and $C_1$-$C_9$ alkylidene-oxy-$C_1$-$C_9$ alkyl;

$R_5$ and $R_6$ may form a ring, and if $R_5$ and $R_6$ form a ring, the ring may be a five-membered ring or a six-membered ring;

$R_7$ is selected from hydrogen, $C_1$-$C_8$ hydrocarbylideneoxy, amino $C_1$-$C_8$ hydrocarbylidene, thio $C_1$-$C_8$ hydrocarbylidene, and oxy $C_1$-$C_8$ hydrocarbylidene;

n is selected from the group consisting of 0, 1, 2, 3, 4, 5, 6, 7, 8, and 9; if n is 0, $(CH_2R_7)_n$ represents a covalent bond;

$R_8$ is carbonylamino, wherein amino may be substituted with $C_1$-$C_8$ hydrocarbyl or arylhydrocarbyl, $C_1$-$C_8$ hydrocarbylidene amino $C_1$-$C_8$ hydrocarbylidene, $C_1$-$C_8$ hydrocarbylidene thio $C_1$-$C_8$ hydrocarbylidene, or $C_1$-$C_8$ hydrocarbylidene oxy $C_1$-$C_8$ hydrocarbylidene;

m is selected from the group consisting of 0, 1, 2, 3, 4, 5, 6, 7, 8, and 9; if m is 0, $-(R_8)_m$ represents a covalent bond;

$R_9$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{10}$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{13}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{14}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{15}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl; and

the natural or unnatural amino acids in $R_1$, $R_{11}$, and $R_{12}$ are selected from the group consisting of glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine.

In some embodiments, exemplary examples of the tumor or cancer include, but are not limited to, colorectal, lung, cervical, ovarian, gastric, esophageal, breast, pancreatic, bladder, liver, gastric, colon, head and neck, uterine, urothelial, osteosarcoma, sarcoma, renal, melanoma, prostate, glioma, glioma, leukemia.

In still another aspect, the present disclosure relates to a method for treating a tumor or cancer, comprising administering to a subject in need thereof a therapeutically effective amount of a compound of Formula (I), or a stereoisomer thereof, or a pharmaceutical composition comprising a compound of Formula (I), or a stereoisomer thereof, and a pharmaceutically acceptable carrier:

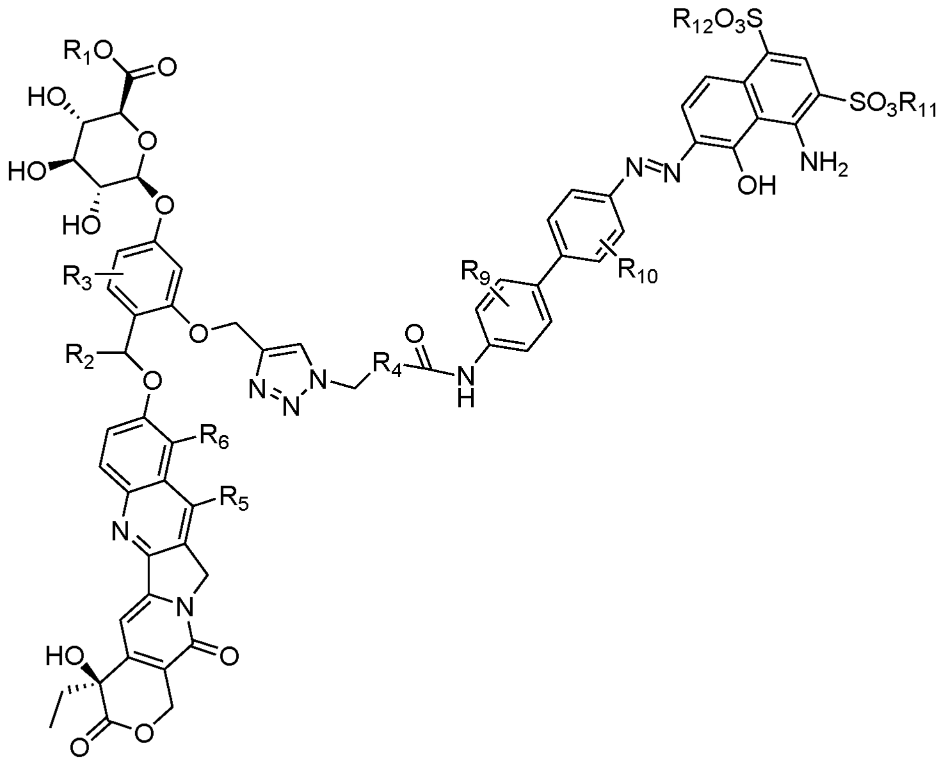

Formula (I)

wherein

$R_1$ is selected from the group consisting of hydrogen, $C_1$-$C_8$ hydrocarbyl, -Na, -Li, -K, -Cs, $-NH_4^+$, $-NH_2^+R_{13}R_{14}$, $-NH^+R_{13}R_{14}R_{15}$, $-NH^+{}_3R_{13}$, and natural or unnatural amino acids;

$R_2$ is selected from the group consisting of hydrogen, and $C_1$-$C_8$ hydrocarbyl;

$R_3$ is one or more substituents at any substitution position selected from the group consisting of hydrogen, halogen, $C_1$-$C_8$ hydrocarbyl, $C_1$-$C_8$ hydrocarbyloxy, cyano, nitro, amino, aryl, arylhydrocarbyl, and $C_1$-$C_4$ hydrocarbyl substituted amino;

$R_4$ is selected from the group consisting of $C_1$-$C_9$ alkylidene, $C_1$-$C_8$ alkylideneoxy, $C_1$-$C_8$ hydrocarbyl substituted or unsubstituted $C_3$-$C_8$ heteroaryl, $C_1$-$C_8$ hydrocarbyl substituted or unsubstituted $C_3$-$C_{15}$ aryl, $-(CH_2R_7)_n$, $-(R_8)_m$, $C_1$-$C_9$

alkylidene-arylidene, $C_1$-$C_9$ alkylidene-arylidene-$C_1$-$C_9$ alkyldiene, $C_1$-$C_9$ alkylidene-arylidene-arylidene, $C_1$-$C_9$ alkylidene-heteroaryliden-$C_1$-$C_9$ alkylidene, and amino acid peptide chain; wherein the amino acid peptide chain is composed of a combination of natural or unnatural amino acids, and the peptide chain length is 1 to100 peptides, preferably 1 to 50 peptides, and more preferably 1 to 20 peptides;

$R_5$ is selected from the group consisting of hydrogen, and $C_1$-$C_8$ hydrocarbyl;

$R_6$ is selected from the group consisting of hydrogen, $C_1$-$C_8$ hydrocarbyl, $C_1$-$C_8$ hydrocarbylidene amino ($C_1$-$C_4$ hydrocarbyl)$_2$, $C_1$-$C_8$ hydrocarbylidene thio $C_1$-$C_8$ hydrocarbyl, and $C_1$-$C_9$ alkylidene-oxy-$C_1$-$C_9$ alkyl;

$R_5$ and $R_6$ may form a ring, and if $R_5$ and $R_6$ form a ring, the ring may be a five-membered ring or a six-membered ring;

$R_7$ is selected from hydrogen, $C_1$-$C_8$ hydrocarbylideneoxy, amino $C_1$-$C_8$ hydrocarbylidene, thio $C_1$-$C_8$ hydrocarbylidene, and oxy $C_1$-$C_8$ hydrocarbylidene;

n is selected from the group consisting of 0, 1, 2, 3, 4, 5, 6, 7, 8, and 9; if n is 0, $(CH_2R_7)_n$ represents a covalent bond;

$R_8$ is carbonylamino, wherein amino may be substituted with $C_1$-$C_8$ hydrocarbyl or arylhydrocarbyl, $C_1$-$C_8$ hydrocarbylidene amino $C_1$-$C_8$ hydrocarbylidene, $C_1$-$C_8$ hydrocarbylidene thio $C_1$-$C_8$ hydrocarbylidene, or $C_1$-$C_8$ hydrocarbylidene oxy $C_1$-$C_8$ hydrocarbylidene;

m is selected from the group consisting of 0, 1, 2, 3, 4, 5, 6, 7, 8, and 9; if m is 0, $-(R_8)_m$ represents a covalent bond;

$R_9$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{10}$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{13}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{14}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{15}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl; and

the natural or unnatural amino acids in $R_1$, $R_{11}$, and $R_{12}$ are selected from the group consisting of glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine.

In some embodiments, exemplary examples of the tumor or cancer include, but are not limited to, colorectal, lung, cervical, ovarian, gastric, esophageal, breast, pancreatic, bladder, liver, gastric, colon, head and neck, uterine, urothelial, osteosarcoma, sarcoma, renal, melanoma, prostate, glioma, glioma, land eukemia.

In yet another aspect, the present disclosure relates to a process for preparing a compound of Formula (I):

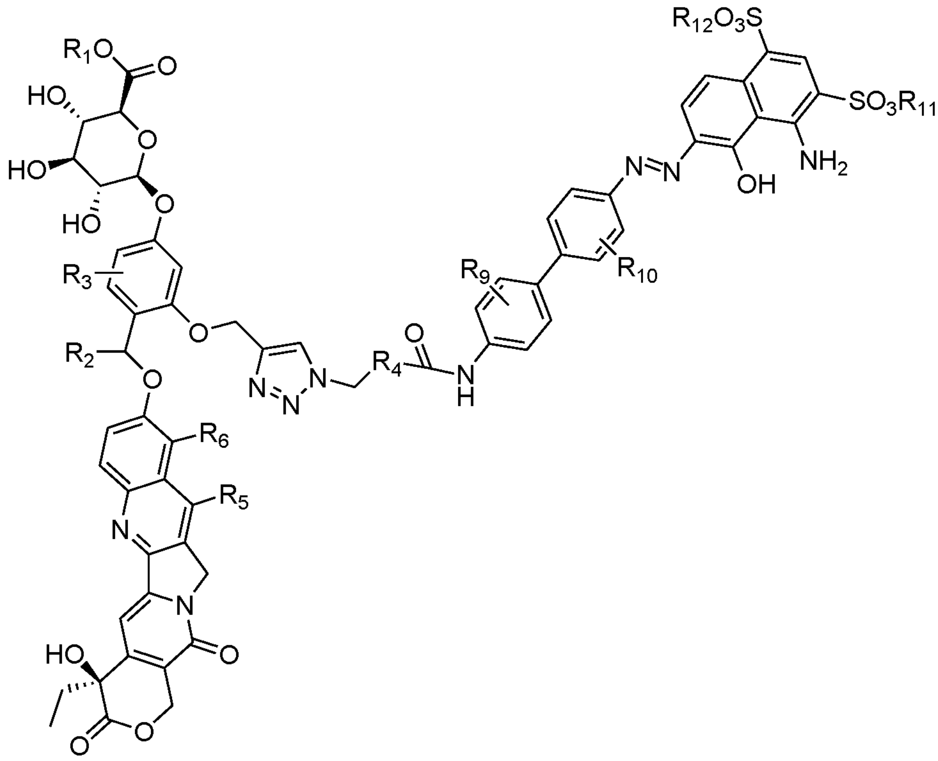

Formula (I)

wherein

$R_1$ is selected from the group consisting of hydrogen, $C_1$-$C_8$ hydrocarbyl, -Na, -Li, -K, -Cs, $-NH_4^+$, $-NH_2^+R_{13}R_{14}$, $-NH^+R_{13}R_{14}R_{15}$, $-NH^+{}_3R_{13}$, and natural or unnatural amino acids;
$R_2$ is selected from the group consisting of hydrogen, and $C_1$-$C_8$ hydrocarbyl;
$R_3$ is one or more substituents at any substitution position selected from the group consisting of hydrogen, halogen, $C_1$-$C_8$ hydrocarbyl, $C_1$-$C_8$ hydrocarbyloxy, cyano, nitro, amino, aryl, arylhydrocarbyl, and $C_1$-$C_4$ hydrocarbyl substituted amino;
$R_4$ is selected from the group consisting of $C_1$-$C_9$ alkylidene, $C_1$-$C_8$ alkylideneoxy, $C_1$-$C_8$ hydrocarbyl substituted or unsubstituted $C_3$-$C_8$ heteroaryl, $C_1$-$C_8$ hydrocarbyl substituted or unsubstituted $C_3$-$C_{15}$ aryl, $-(CH_2R_7)_n$, $-(R_8)_m$, $C_1$-$C_9$ alkylidene-arylidene, $C_1$-$C_9$ alkylidene-arylidene-$C_1$-$C_9$ alkyldiene, $C_1$-$C_9$ alkylidene-arylidene-arylidene, $C_1$-$C_9$ alkylidene-heteroaryliden-$C_1$-$C_9$ alkylidene, and amino acid peptide chain; wherein the amino acid peptide chain is composed of a combination of natural or unnatural amino acids, and the peptide chain length is 1 to100 peptides, preferably 1 to 50 peptides, and more preferably 1 to 20 peptides;
$R_5$ is selected from the group consisting of hydrogen, and $C_1$-$C_8$ hydrocarbyl;
$R_6$ is selected from the group consisting of hydrogen, $C_1$-$C_8$ hydrocarbyl, $C_1$-$C_8$ hydrocarbylidene amino ($C_1$-$C_4$ hydrocarbyl$)_2$, $C_1$-$C_8$ hydrocarbylidene thio $C_1$-$C_8$ hydrocarbyl, and $C_1$-$C_9$ alkylidene-oxy-$C_1$-$C_9$ alkyl;
$R_5$ and $R_6$ may form a ring, and if $R_5$ and $R_6$ form a ring, the ring may be a five-membered ring or a six-membered ring;
$R_7$ is selected from hydrogen, $C_1$-$C_8$ hydrocarbylideneoxy, amino $C_1$-$C_8$ hydrocarbylidene, thio $C_1$-$C_8$ hydrocarbylidene, and oxy $C_1$-$C_8$ hydrocarbylidene;
n is selected from the group consisting of 0, 1, 2, 3, 4, 5, 6, 7, 8, and 9; if n is 0, $(CH_2R_7)_n$ represents a covalent bond;
$R_8$ is carbonylamino, wherein amino may be substituted with $C_1$-$C_8$ hydrocarbyl or arylhydrocarbyl, $C_1$-$C_8$ hydrocarbylidene amino $C_1$-$C_8$ hydrocarbylidene, $C_1$-$C_8$ hydrocarbylidene thio $C_1$-$C_8$ hydrocarbylidene, or $C_1$-$C_8$ hydrocarbylidene oxy $C_1$-$C_8$ hydrocarbylidene;
m is selected from the group consisting of 0, 1, 2, 3, 4, 5, 6, 7, 8, and 9; if m is 0, $-(R_8)_m$ represents a covalent bond;
$R_9$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;
$R_{10}$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;
$R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;
$R_{13}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;
$R_{14}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;
$R_{15}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl; and
the natural or unnatural amino acids in $R_1$, $R_{11}$, and $R_{12}$ are selected from the group consisting of glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine;
wherein the process comprises:

(1) reacting the compound of Formula (A-I) with a compound of Formula (A-II) to obtain a compound of Formula (A-III),

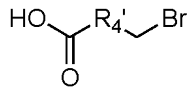

Formula (A-I)

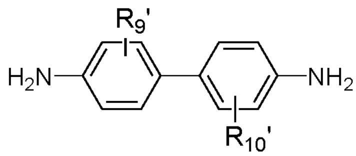

Formula (A-II)

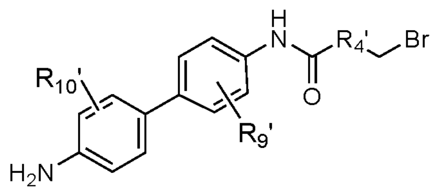

Formula (A-III)

wherein the groups represented by $R_4$', $R_9$', and $R_{10}$' in the Formula (A-I) and the Formula (A-II) are as defined for $R_4$, $R_9$ and $R_{10}$ in the Formula (I);
(2) reacting the compound of Formula (A-III) with sodium azide to obtain a compound of Formula (A-IV),

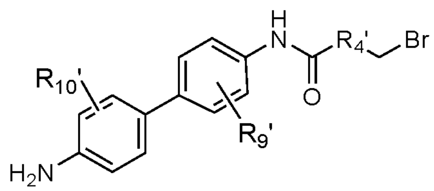

Formula (A-III)

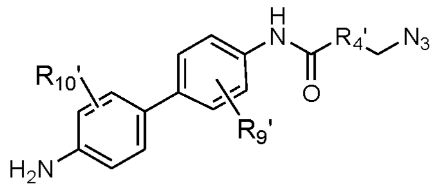

Formula (A-IV)

wherein the groups represented by $R_4$', $R_9$', and $R_{10}$' in the Formula (A-III) and the Formula (A-IV) are as defined for $R_4$, $R_9$ and $R_{10}$ in the Formula (I);

(3) reacting the compound of Formula (A-IV) with 4-amino-5-hydroxy-1,3-naphthalene disulfonic acid monosodium to obtain a compound of Formula (A-V),

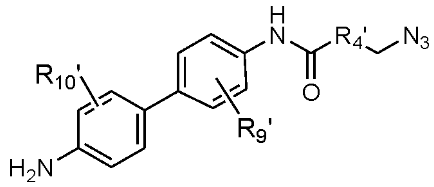

Formula (A-IV)

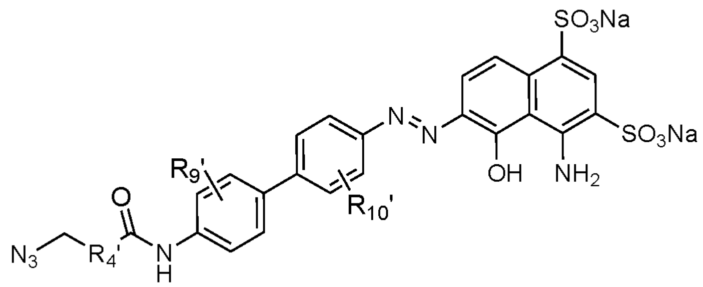

;

Formula (A-V)

wherein the groups represented by $R_4$', $R_9$', and $R_{10}$' in the Formula (A-IV) and the Formula (A-V) are as defined for $R_4$, $R_9$ and $R_{10}$ in the Formula (I);

(4) reacting the compound of Formula (B-I) with carbon tetrabromide to obtain a compound of Formula (B-II),

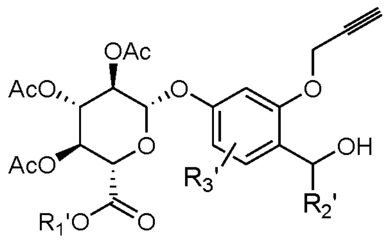

Formula (B-I)

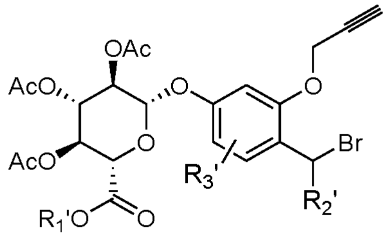

Formula (B-II)

wherein the groups represented by $R_1$', $R_2$', and $R_3$' in the Formula (B-I) and the Formula (B-II) are as defined for $R_1$, $R_2$ and $R_3$ in the Formula (I);

(5) reacting the compound of Formula (B-II) with a compound of Formula (B-III) to obtain a compound of Formula (B-IV)

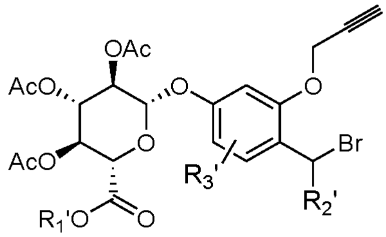

Formula (B-II)

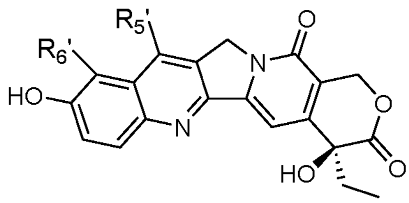

Formula (B-III)

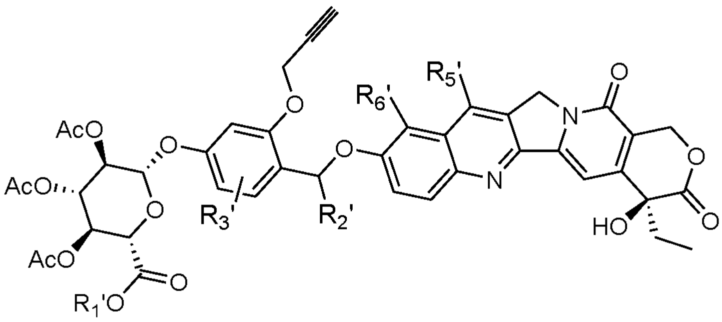

Formula (B-IV)

wherein the groups represented by $R_1$', $R_2$', $R_3$', $R_5$', and $R_6$' in the Formula (B-II), the Formula (B-III), and the Formula (B-IV) are as defined for $R_1$, $R_2$, $R_3$, $R_5$, and $R_6$ in the Formula (I);

(6) reacting the compound of Formula (B-IV) under basic conditions to obtain a compound of Formula (B-V),

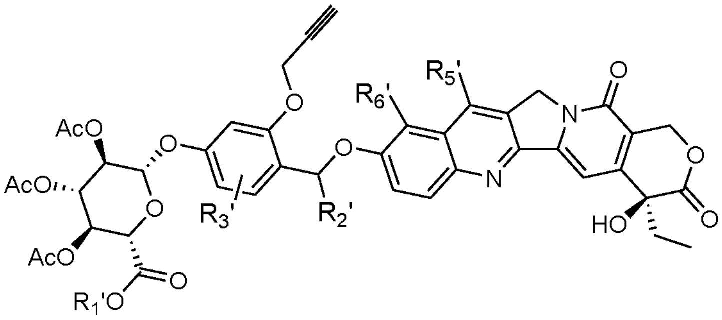

Formula (B-IV)

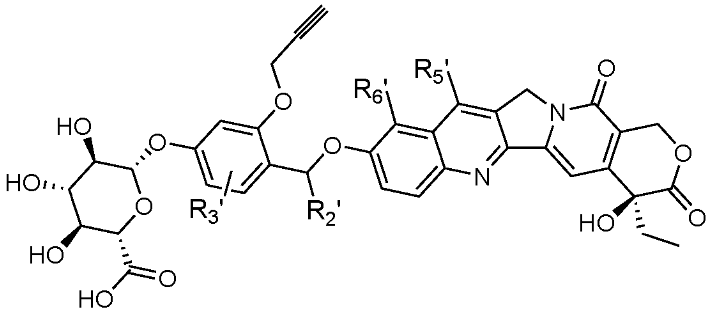

Formula (B-V)

wherein the groups represented by $R_1$', $R_2$', $R_3$', $R_5$', and $R_6$' in the Formula (B-IV) and the Formula (B-V) are as defined for $R_1$, $R_2$, $R_3$, $R_5$ and $R_6$ in the Formula (I);

(7) reacting the compound of Formula (A-V) with the compound of Formula (B-V) to obtain the compound of Formula (I), or reacting a product obtained by reacting the compound of Formula (A-V) with the compound of Formula (B-V) with a corresponding acid or base to form a pharmaceutically acceptable salt thereof

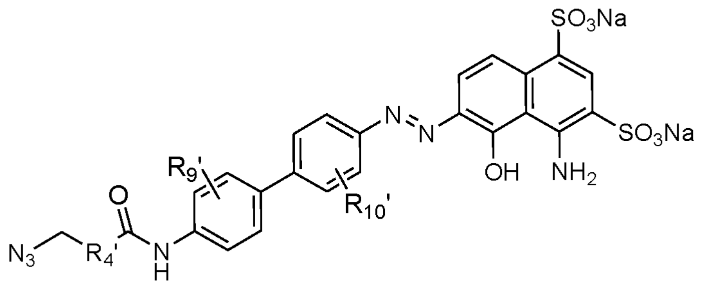

Formula (A-V)

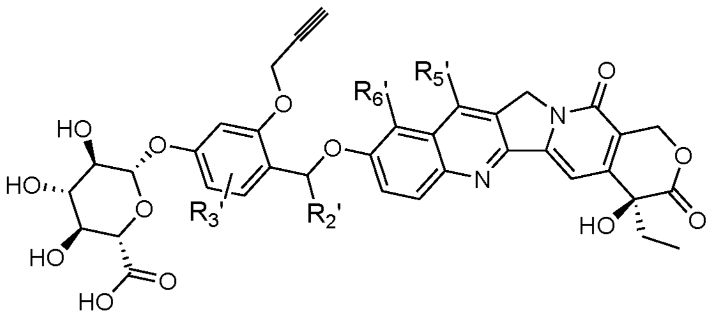

Formula (B-V)

wherein the groups represented by $R_2'$, $R_3'$, $R_4'$, $R_5'$, $R_6'$, $R_9'$, and $R_{10}'$ in the Formula (A-V) and the Formula (B-V) are defined as $R_2$, $R_3$, $R_4$, $R_5$, $R_6$, $R_9$, and $R_{10}$ in the Formula (I).

In still yet another aspect, the present disclosure relates to the following compounds and stereoisomers thereof:

| No. | Structure | Chemical Name |
|---|---|---|
| 1 | 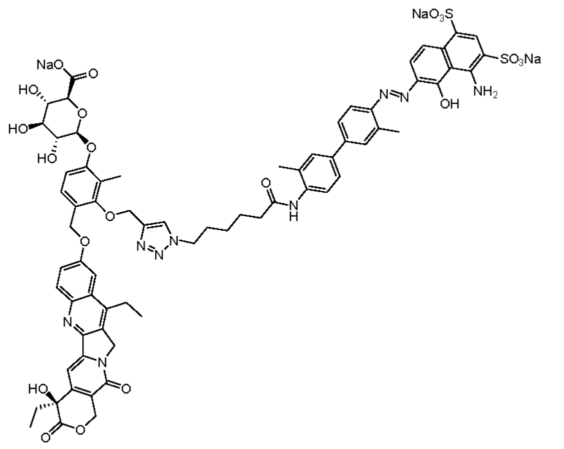 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolazino[1,2-b]quino lin-9-yl)oxy) methyl)2-methylphe noxy)-3,4,5-trihydroxytetrahydro -2H-pyran-2-carboxylate trisodium |
| 2 | 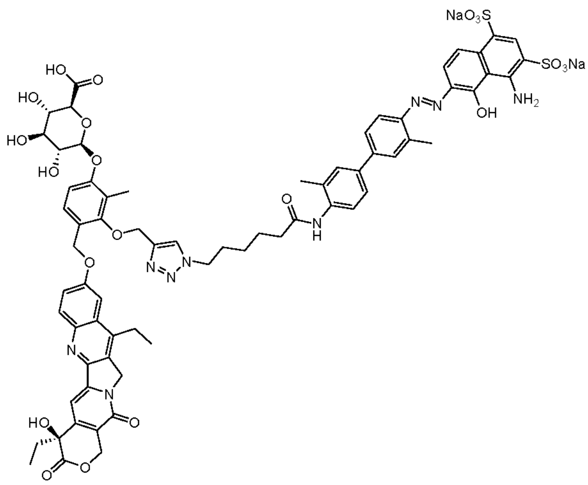 | 4-amino-6-((E)-(4'-(6-(4-((3-(((2 S,3R,4S,5S,6S)-6-carboxy-3,4,5-trihydroxytetrahydro-2H-pyran-2 -yl)oxy)-6-((((S)-4,11-diethyl-4-hydroxy-3,14-dioxy-3,4,12,14-tet rahydro-1H-pyrano[3',4':6,7]ind olazino[1,2-b] quinolin-9-yl)oxy) methyl)-2-tolyloxy) methyl)-1H-1 ,2,3-triazol-1-yl)hexanamido)-3,3 '-dimethyl-[1,1'-biphenyl]-4-yl)d iazenyl)-5-hydroxynaphthalene-1 -3-disulfonate disodium |
| 3 | 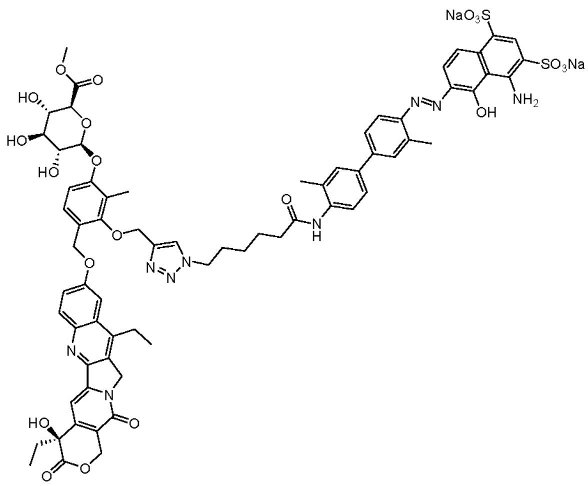 | 4-amino-6-((E)-(4'-(6-(4-((6-(((S )-4,11-diethyl-4-hydroxy-3,14-di oxy-3,4,12,14-tetrahydro-1H-pyr ano[3',4':6,7]indolazo[1,2-b]qui nolin-9-yl)oxy)methyl)-2-methyl -3-((((2S,3R,4S,5S,6S)-3,4,5-trih ydroxy-6-(methoxycarbonyl)tetra hydro-2H-pyran-2-yl)oxy)pheno xy)methyl)-1H-1,2,3-triazol-1-yl ) hexanamido)-3,3'-dimethyl-[1,1 '-biphenyl]-4-yl)diazenyl)-5-hyd roxynaphthalene-1,3-disulfonate disodium |

(continued)

| No. | Structure | Chemical Name |
|---|---|---|
| 4 | 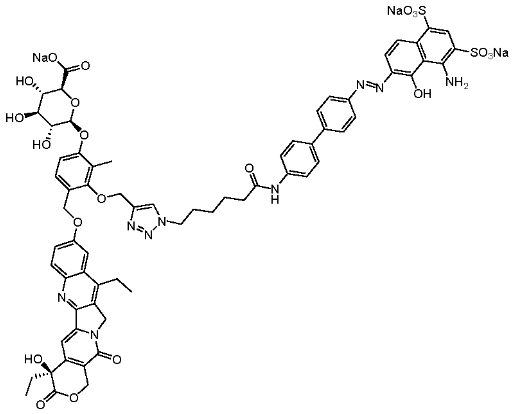 | (2S,3S,4S,5R,6S)-6-(3-((1-(6-((4 '-((E)-(8-amino-1-hydroxy-5,7-di sulfonicnaphthalen-2-yl) diazenyl )-[1,1'-biphenyl]-4-yl)amino)-6-oxohexyl)-1H-1,2,3-triazol-4-yl) methoxy)-4-((((S)-4,11-diethyl-4 -hydroxy-3,14-dioxo-3,4,12,14-t etrahydro-1H-pyrano[3',4':6,7] in dolazino[1,2-b]quinolin-9-yl)oxy )methyl)-2-methylphenoxy)-3,4, 5-trihydroxytetrahydro-2H-pyran -2-carboxylate trisodium |
| 5 | 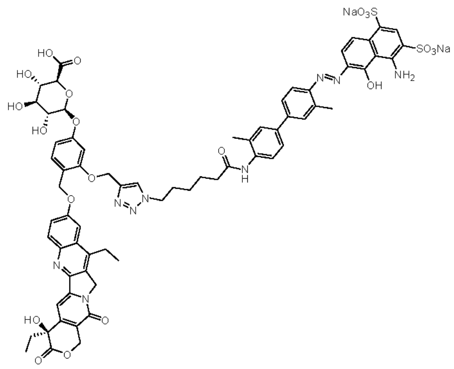 | 4-amino-6-((E)-(4'-(6-(4-((5-(((2 S,3R,4S,5S,6S)-6-carboxy-3,4,5-trihydroxytetrahydro-2H-pyran-2 -yl)oxy)-2-((((S)-4,11-diethyl-4-hydroxy-3,14-dioxy-3,4,12,14-tet rahydro-1H-pyrano[3',4':6,7]ind olazino[1,2-b] quinolin-9-yl)oxy) methyl)phenoxy) methyl)-1H-1,2, 3-triazol-1-yl)hexanamido)-3,3'-dimethyl-[1,1'-biphenyl]-4-yl)dia zenyl)-5-hydroxynaphthalene-1,3 -disulfonate disodium |
| 6 | 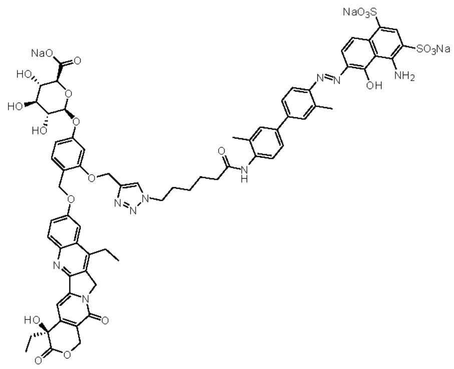 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolazino[1,2-b]quinolin-9-yl)oxy) methyl)phenoxy)-3, 4,5-trihydroxytetrahydro-2H-pyr an-2-carboxylate trisodium |
| 7 | 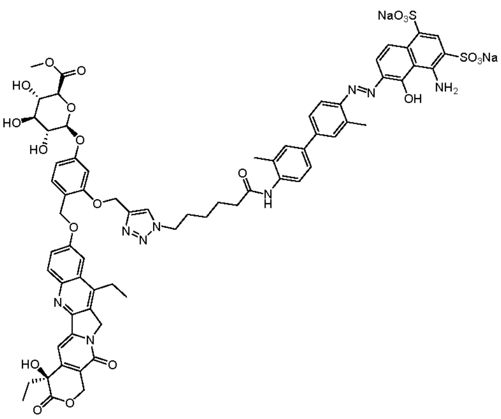 | 4-amino-6-((E)-(4'-(6-(4-((2-(((S )-4,11-diethyl-4-hydroxy-3,14-di oxy-3,4,12,14-tetrahydro-1H-pyr ano[3',4':6,7]indolazo[1,2-b]qui nolin-9-yl)oxy)methyl)-5-(((2S,3 R,4S,5S,6S)-3,4,5-trihydroxy-6-( methoxycarbonyl)tetrahydro-2H-pyran-2-yl)oxy)phenoxy) methyl) -1H-1,2,3-triazol-1-yl)hexanamid o)-3,3'-dimethyl-[1,1'-biphenyl]-4-yl)diazenyl)-5-hydroxynaphtha lene-1,3-disulfonate disodium |

(continued)

| No. | Structure | Chemical Name |
|---|---|---|
| 8 | 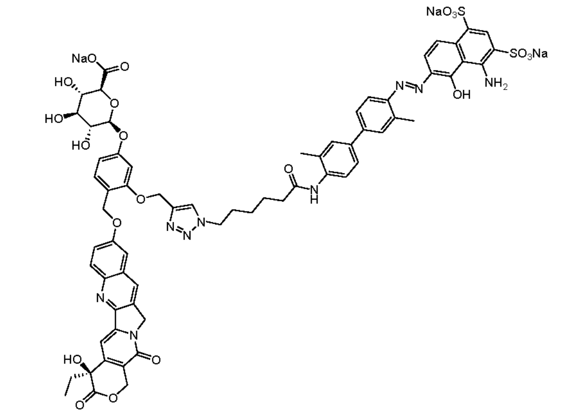 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-(((S)-4-ethyl-4-hydroxy-3,14-dioxy-3,4, 12,14-tetrahydro-1H-pyrano[3',4 ':6,7] indolazino[1,2-b]quinolin-9 -yl)oxy)methyl)phenoxy)-3,4,5-tr ihydroxytetrahydro-2H-pyran-2-carboxylate trisodium |
| 9 | 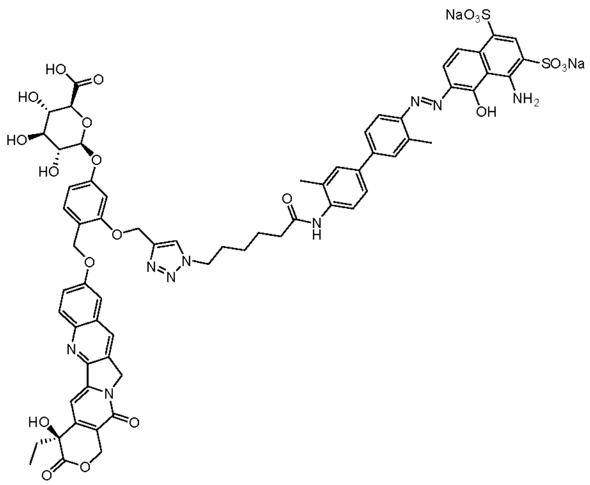 | 4-amino-6-((E)-(4'-(6-(4-((5-(((2 S,3R,4S,5S,6S)-6-carboxy-3,4,5-trihydroxytetrahydro-2H-pyran-2 -yl)oxy)-2-((((S)-4-ethyl-4-hydro xy-3,14-dioxo-3,4,12,14-tetrahyd ro-1H-pyrano[3',4':6,7]indolazin o[1,2-b]quinolin-9-yl)oxy)methyl )phenoxy)methyl)-1H-1,2,3-triaz ol-1-ylhexanamido)-3,3'-dimethy 1-[1,1'-biphenyl]-4-yl)diazenyl)-5-hydroxynaphthalene-1,3-disulf onate disodium |
| 10 | 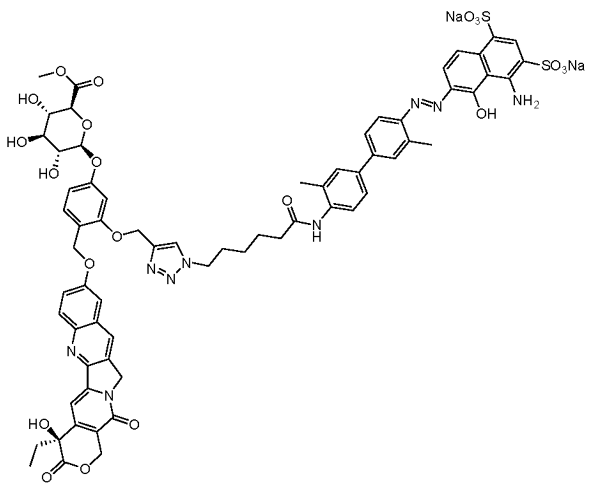 | 4-amino-6-((E)-(4'-(6-(4-((2-(((S )-4-ethyl-4-hydroxy-3,14-dioxo-3,4,12,14-tetrahydro-1H-pyrano[ 3',4':6,7]indolizino[1,2-b]quinoli n-9-yl) oxy)methyl)-5-(((2S,3R,4 S,5S,6S)-3,4,5-trihydroxy-6-(met hoxycarbonyl)tetrahydro-2H-pyr an-2-yl)oxy)phenoxy)methyl)-1 H-1,2,3-triazol-1-yl)hexanamido) -3,3'-dimethyl-[1,1'-biphenyl]-4-yl)diazenyl)-5-hydroxynaphthale ne-1,3-disulfonate disodium |

(continued)

| No. | Structure | Chemical Name |
|---|---|---|
| 11 | 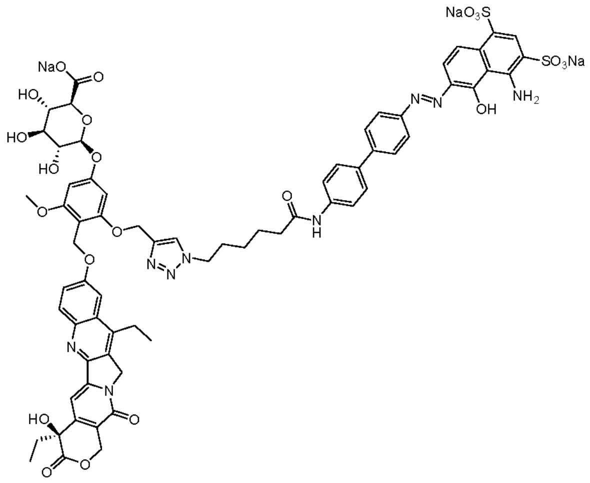 | (2S,3S,4S,5R,6S)-6-(3-((1-(6-((4 '-((E)-(8-amino-1-hydroxy-5,7-di sulfonicnaphthalen-2-yl) diazenyl )-[1,1'-biphenyl]-4-yl)amino)-6-oxohexyl)-1H-1,2,3-triazol-4-yl) methoxy)-4-((((S)-4,11-diethyl-4 -hydroxy-3,14-dioxo-3,4,12,14-t etrahydro-1H-pyrano[3',4':6,7] in dolazino[1,2-b]quinolin-9-yl)oxy )methyl)-5-methoxyphenoxy)-3,4 ,5-trihydroxytetrahydro-2H-pyra n-2-carboxylate trisodium |
| 12 | 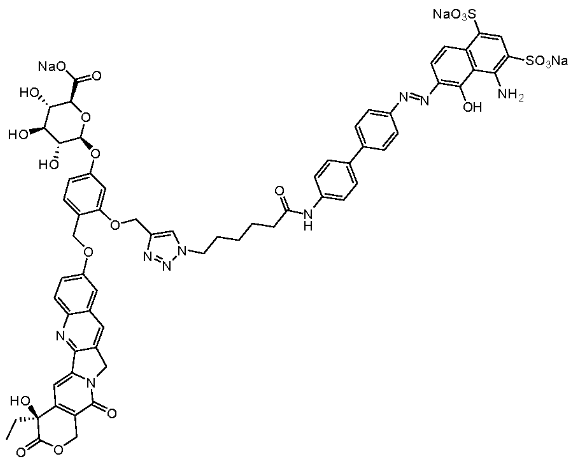 | (2S,3S,4S,5R,6S)-6-(3-((1-(6-((4 '-((E)-(8-amino-1-hydroxy-5,7-di sulfonicnaphthalen-2-yl) diazenyl )-[1,1'-biphenyl]-4-yl)amino)-6-oxohexyl)-1H-1,2,3-triazol-4-yl) methoxy)-4-((((S)-4-ethyl-4-hydr oxy-3,14-dioxo-3,4,12, 14-tetrahy dro-1H-pyrano[3',4':6,7] indolazi no[1,2-b]quinolin-9-yl)oxy)meth yl) phenoxy)-3,4,5-trihydroxytetr ahydro-2H-pyran-2-carboxylate trisodium |
| 13 | 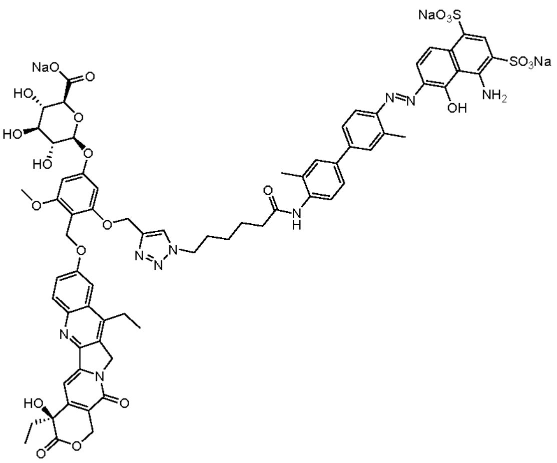 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolazino[1,2-b]quino lin-9-yl)oxy) methyl)-5-methoxyp henoxy)-3,4,5-trihydroxytetrahyd ro-2H-pyran-2-carboxylate trisodium |

(continued)

| No. | Structure | Chemical Name |
|---|---|---|
| 14 | 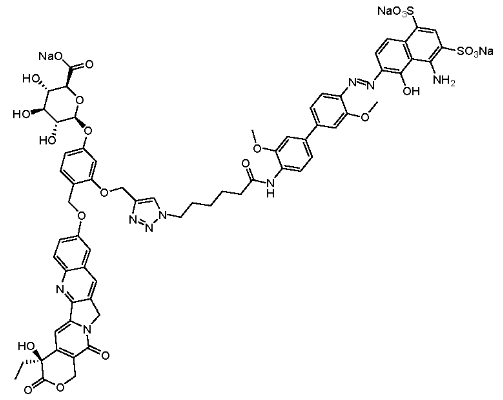 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethoxy-[1,1'-biphenyl] -4-yl) amino)-6-oxohexyl)-1H-1,2 ,3-triazol-4-yl) methoxy)-4-(((S)-4-ethyl-4-hydroxy-3,14-dioxy-3, 4,12,14-tetrahydro-1H-pyrano[3' ,4':6,7] indolazino[1,2-b]quinolin -9-yl)oxy)methyl)phenoxy)-3,4,5 -trihydroxytetrahydro-2H-pyran-2-carboxylate trisodium |
| 15 | 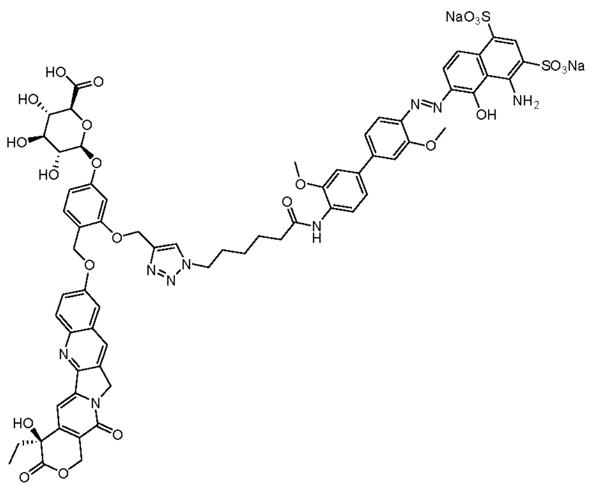 | 4-amino-6-((E)-(4'-(6-(4-((5-(((2 S,3R,4S,5S,6S)-6-carboxy-3,4,5-trihydroxytetrahydro-2H-pyran-2 -yl)oxy)-2-((((S)-4-ethyl-4-hydro xy-3,14-dioxo-3,4,12,14-tetrahyd ro-1H-pyrano[3',4':6,7]indolazin o[1,2-b]quinolin-9-yl)oxy)methyl )phenoxy)methyl)-1H-1,2,3-triaz ol-1-yl)hexanamido)-3,3'-dimeth oxy-[1,1'-biphenyl]-4-yl)diazeny l)-5-hydroxynaphthalene-1,3-dis ulfonate disodium |
| 16 | 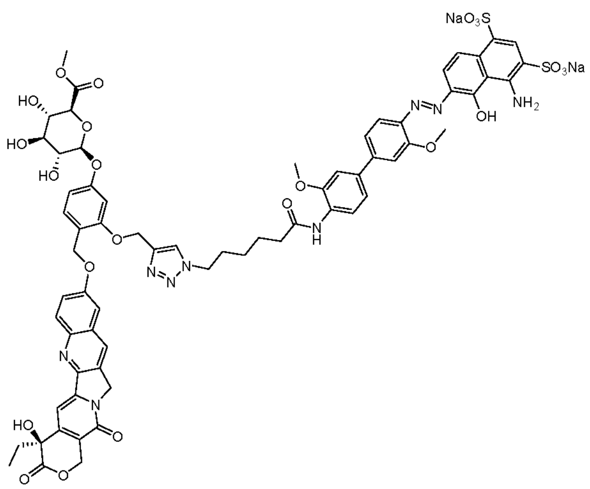 | 4-amino-6-((E)-(4'-(6-(4-((2-(((S )-4-ethyl-4-hydroxy-3,14-dioxo-3,4,12,14-tetrahydro-1H-pyrano[ 3',4':6,7]indolizino[1,2-b]quinoli n-9-yl) oxy)methyl)-5-(((2S,3R,4 S,5S,6S)-3,4,5-trihydroxy-6-(met hoxycarbonyl)tetrahydro-2H-pyr an-2-yl)oxy)phenoxy)methyl)-1 H-1,2,3-triazol-1-yl)hexanamido) -3,3'-dimethoxy-[1,1'-biphenyl]-4-yl)diazenyl)-5-hydroxynaphtha lene-1,3-disulfonate disodium |

(continued)

| No. | Structure | Chemical Name |
|---|---|---|
| 17 | 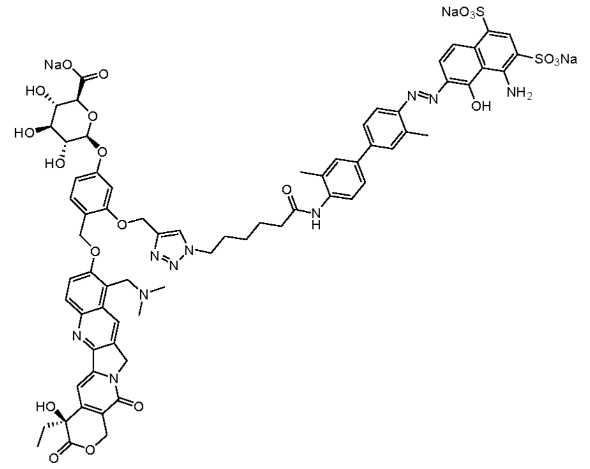 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-(((S)-10 -((dimethylamino) methyl)-4-ethy 1-4-hydroxy-3,14-dioxy-3,4,12,1 4-tetrahydro-1H-pyrano[3',4':6,7 ] indolazino[1,2-b]quinolin-9-yl)o xy)methyl)phenoxy)-3,4,5-trihyd roxytetrahydro-2H-pyran-2-carb oxylate trisodium |
| 18 | 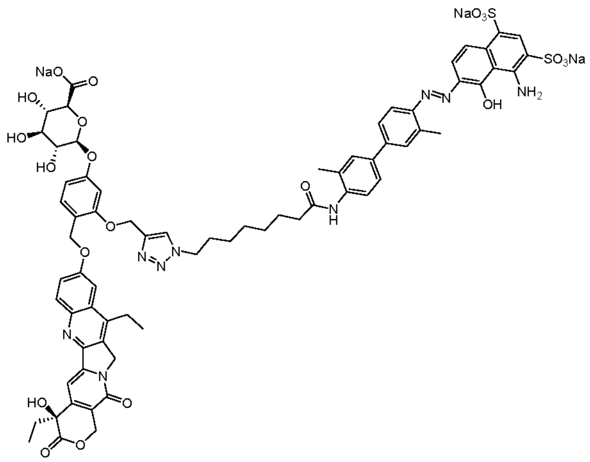 | (2S,3S,4S,5R,6S)-6-(3-((1-(8-((4 '-((E)-(8-amino-1-hydroxy-5,7-di sulfonicnaphthalen-2-yl) diazenyl )-3,3'-dimethyl-[1,1'-biphenyl]-4 -yl) amino)-8-oxooctyl)-1H-1,2,3 -triazol-4-yl)methoxy)-4-((((S)-4, 11-diethyl-4-hydroxy-3,14-dioxy -3,4,12,14-tetrahydro-1H-pyrano [3',4':6,7] indolizino[1,2-b]quinol in-9-yl)oxy)methyl)phenoxy)-3,4 ,5-trihydroxytetrahydro-2H-pyra n-2-carboxylate trisodium |
| 19 | 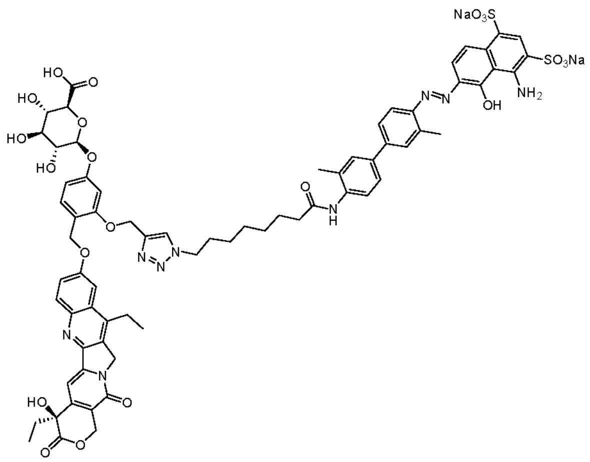 | 4-amino-6-((E)-(4'-(8-(4-((5-(((2 S,3R,4S,5S,6S)-6-carboxy-3,4,5-trihydroxytetrahydro-2H-pyran-2 -yl)oxy)-2-((((S)-4,11-diethyl-4-hydroxy-3,14-dioxy-3,4,12,14-tet rahydro-1H-pyrano[3',4':6,7]ind olazino[1,2-b] quinolin-9-yl)oxy) methyl)phenoxy) methyl)-1H-1,2, 3-triazol-1-yl)octanamido)-3,3'-d imethyl-[1,1'-biphenyl]-4-yl)diaz enyl)-5-hydroxynaphthalene-1,3-disulfonate disodium |

(continued)

| No. | Structure | Chemical Name |
|---|---|---|
| 20 | 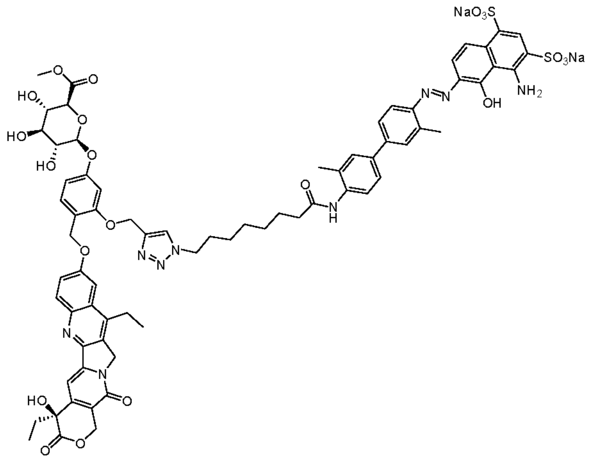 | 4-amino-6-((E)-(4'-(8-(4-((2-(((S )-4,11-diethyl-4-hydroxy-3,14-di oxo-3,4,12,14-tetrahydro-1H-pyr ano[3',4':6,7]indolazo[1,2-b]qui nolin-9-yl)oxy)methyl)-5-(((2S,3 R,4S,5S,6S)-3,4,5-trihydroxy-6-( methoxycarbonyl)tetrahydro-2H-pyran-2-yl)oxy)phenoxy) methyl) -1H-1,2,3-triazol-1-yl)octanamid o)-3,3'-dimethyl-[1,1'-biphenyl]-4-yl)diazenyl)-5-hydroxynaphtha lene-1,3-disulfonate disodium |
| 21 | 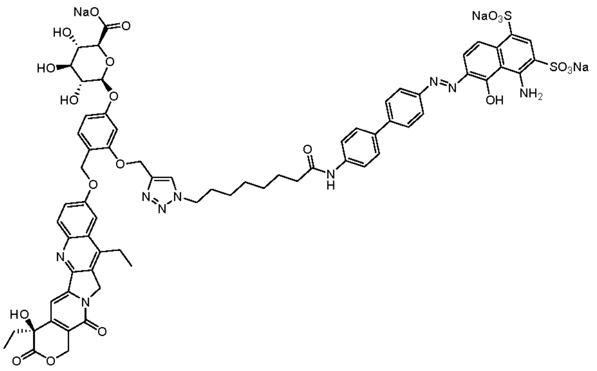 | (2S,3S,4S,5R,6S)-6-(3-((1-(8-((4 '-((E)-(8-amino-1-hydroxy-5,7-di sulfonicnaphthalen-2-yl) diazenyl )-[1,1'-biphenyl]-4-yl)amino)-8-oxooctyl)-1H-1,2,3-triazol-4-yl) methoxy)-4-((((S)-4,11-diethyl-4 -hydroxy-3,14-dioxo-3,4,12,14-t etrahydro-1H-pyrano[3',4':6,7] in dolazino[1,2-b]quinolin-9-yl)oxy)methyl)phenoxy)-3,4,5-trihydro xytetrahydro-2H-pyran-2-carbox ylate trisodium |
| 22 | 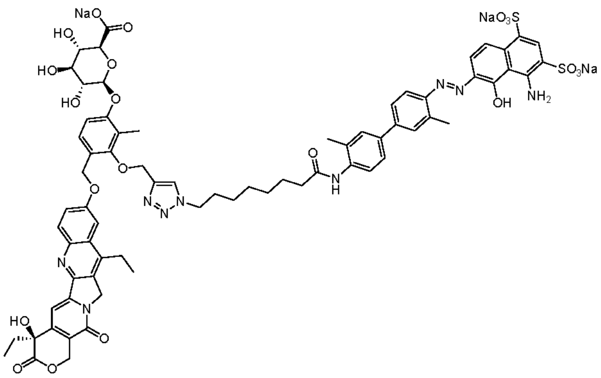 | (2S,3S,4S,5R,6S)-6-(3-((1-(8-((4 '-((E)-(8-amino-1-hydroxy-5,7-di sulfonicnaphthalen-2-yl) diazenyl )-3,3'-dimethyl-[1,1'-biphenyl]-4 -yl) amino)-8-oxooctyl)-1H-1,2,3 -triazol-4-yl)methoxy)-4-((((S)-4, 11-diethyl-4-hydroxy-3,14-dioxy -3,4,12,14-tetrahydro-1H-pyrano [3',4':6,7] indolazino[1,2-b]quino lin-9-yl)oxy)methyl)-2-methylph enoxy)-3,4,5-trihydroxytetrahydr o-2H-pyran-2-carboxylate trisodium |
| 23 | 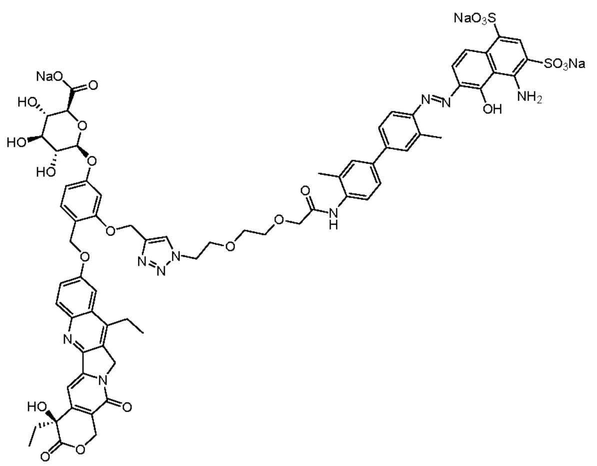 | (2S,3S,4S,5R,6S)-6-(3-((1-(2-(2-(2-((4'-((-E)-(8-amino-1-hydroxy-5,7-disulfonicnaphthalen-2-yl)dia zenyl)-3,3'-dimethyl-[1,1'-biphe nyl]-4-yl)amino)-2-oxoethoxy)et hoxy) ethyl)-1H-1,2,3-triazol-4-yl )methoxy)-4-(((S)-4,11-diethyl-4 -hydroxy-3,14-dioxo-3,4,12,14-t etrahydro-1H-pyrano[3',4':6,7] in dolazino[1,2-b]quinolin-9-yl)oxy )methyl) phenoxy)-3,4,5-trihydro xytetrahydro-2H-pyran-2-carbox ylate trisodium |

(continued)

| No. | Structure | Chemical Name |
|---|---|---|
| 24 | 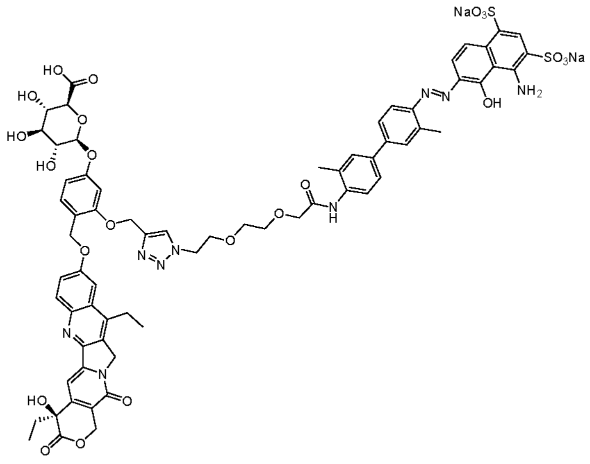 | 4-amino-6-((E)-(4'-(2-(2-(2-(2-(( 5-(((2S,3R,4S,5S,6S)-6-carboxy-3,4,5-trihydroxytetrahydro-2H-pyran-2-yl)oxy)-2-((((S)-4,11-diet hyl-4-hydroxy-3,14-dioxy-3,4,12 ,14-tetrahydro-1H-pyrano [3',4':6 ,7]indolazino[1,2-b]quinolin-9-yl )oxy) methyl)phenoxy)methyl)-1 H-1,2,3-triazol-1-yl) ethoxy)aceta mido)-3,3'-dimethyl-[1,1'-biphenyl]-4-yl)diazenyl)-5-hydroxyna phthalene-1,3-disulfonate disodium |
| 25 | 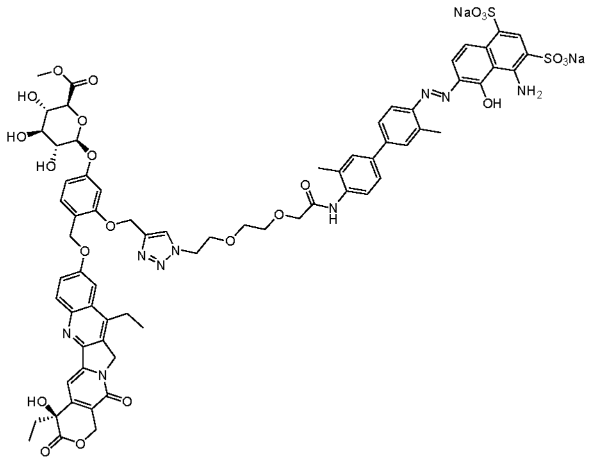 | 4-amino-6-((E)-(4'-(2-(2-(2-((2-( ((S)-4,11-diethyl-4-hydroxy-3,14 -dioxy-3,4,12,14-tetrahydro-1H-pyrano[3',4':6,7]indolazo[1,2-b] quinolin-9-yl)oxy)methyl)-5-(((2 S,3R,4S,5 S,6S)-3,4,5-trihydroxy-6-(methoxycarbonyl) tetrahydro-2 H-pyran-2-yl)oxy)phenoxy)meth yl)-1H-1,2,3-triazol-1-yl)ethoxy) acetamido)-3,3'-dimethyl-[1,1'-b iphenyl]-4-yl)diazenyl)-5-hydrox ynaphthalene-1,3-disulfonate disodium |
| 26 | 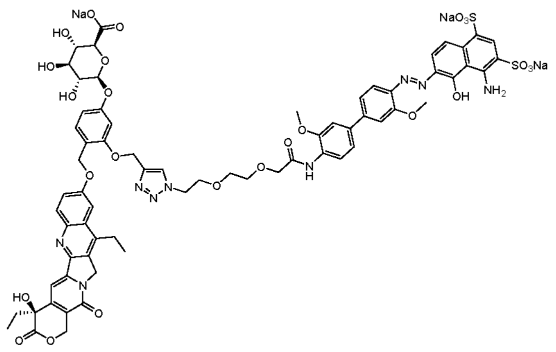 | (2S,3S,4S,5R,6S)-6-(3-((1-(2-(2-(2-((4'-((-E)-(8-amino-1-hydroxy-5,7-disulfonicnaphthalen-2-yl)dia zenyl)-3,3'-dimethoxy-[1,1'-biph enyl]-4-yl)amino)-2-oxoethoxy)e thoxy) ethyl)-1H-1,2,3-triazol-4-y l)methoxy)-4-(((S)-4,11-diethyl-4-hydroxy-3,14-dioxo-3,4,12,14-tetrahydro-1H-pyrano[3',4' :6,7]i ndolazino[1,2-b]quinolin-9-yl)ox y)methyl)phenoxy)-3,4,5-trihydr oxytetrahydro-2H-pyran-2-carbo xylate trisodium |
| 27 | 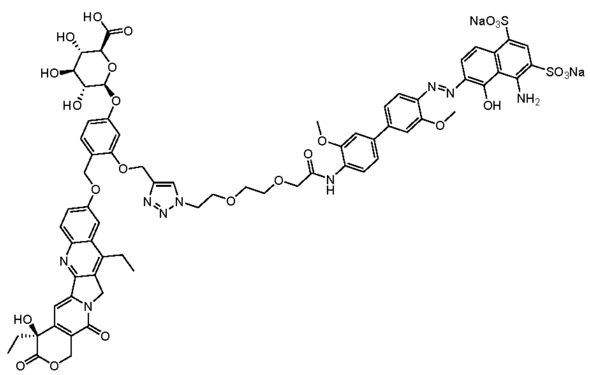 | 4-amino-6-((E)-(4'-(2-(2-(2-(2-(4 -((5-(((2S,3R,4S,5S,6S)-6-carbox y-3,4,5-trihydroxytetrahydro-2H-pyran-2-yl) oxy)-2-((((S)-4,11-die thyl-4-hydroxy-3,14-dioxy-3,4,1 2,14-tetrahydro-1H-pyrano[3',4': 6,7] indolazino[1,2-b]quinolin-9-yl)oxy)methyl)phenoxy)methyl)-1H-1,2,3-triazol-1-yl)ethoxy)eth oxy)acetamido)-3,3'-dimethoxy-[ 1,1'-biphenyl]-4-yl)diazenyl)-5-h ydroxynaphthalene-1,3-disulfona te disodium |

(continued)

| No. | Structure | Chemical Name |
|---|---|---|
| 28 | 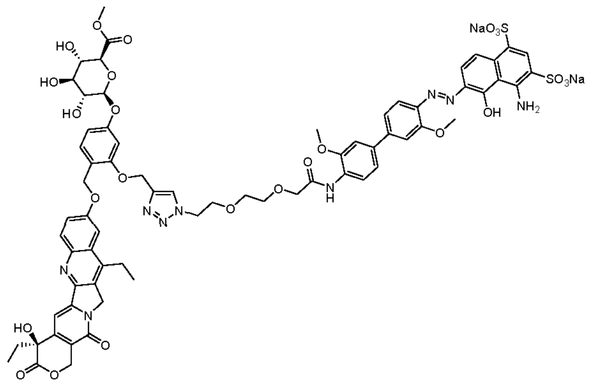 | 4-amino-6-((E)-(4'-(2-(2-(2-((2-( ((S)-4,11-diethyl-4-hydroxy-3,14 -dioxy-3,4,12,14-tetrahydro-1H-pyrano[3',4':6,7]indolazo[1,2-b] quinolin-9-yl)oxy)methyl)-5-(((2 S,3R,4S,5S,6S)-3,4,5-trihydroxy-6-(methoxycarbonyl)tetrahydro-2 H-pyran-2-yl)oxy)phenoxy)meth yl)-1H-1,2,3-triazol-1-yl)ethoxy) acetamido)-3,3'-dimethoxy-[1,1' -biphenyl]-4-yl)diazenyl)-5-hydr oxynaphthalene-1,3-disul-fonate disodium |
| 29 | 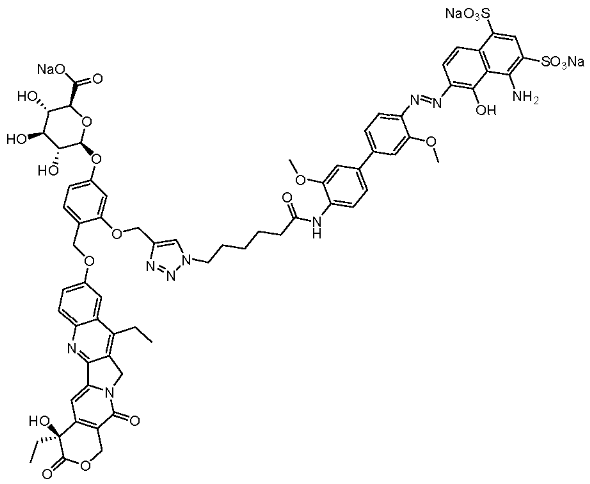 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethoxy-[1,1'-biphenyl] -4-yl) amino)-6-oxohexyl)-1H-1,2 ,3-triazol-4-yl) methoxy)-4-(((S)-4,11-diethyl-4-hydroxy-3,14-dio xy-3,4,12,14-tetrahydro-1H-pyra no [3',4':6,7]indolizino[1,2-b]qui nolin-9-yl)oxy) methyl)phenoxy)-3,4,5-trihydroxytetrahydro-2H-p yran-2-carboxylate trisodium |
| 30 | 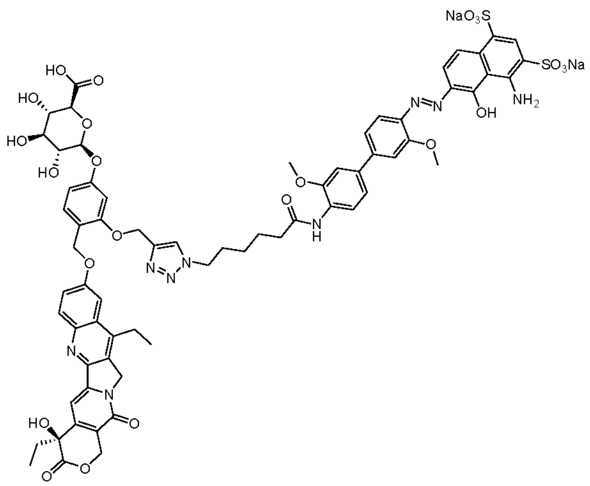 | 4-amino-6-((E)-(4'-(6-(4-((5-(((2 S,3R,4S,5S,6S)-6-carboxy-3,4,5-trihydroxytetrahydro-2H-pyran-2 -yl)oxy)-2-((((S)-4,11-diethyl-4-hydroxy-3,14-dioxy-3,4,12,14-tet rahydro-1H-pyrano[3',4':6,7]ind olazino[1,2-b] quinolin-9-yl)oxy) methyl)phenoxy) methyl)-1H-1,2, 3-triazol-1-yl)hexanamido)-3,3'-dimethoxy-[1,1'-biphenyl]-4-yl)d iazenyl)-5-hydroxynaphthalene-1 ,3-disulfonate disodium |

(continued)

| No. | Structure | Chemical Name |
|---|---|---|
| 31 | 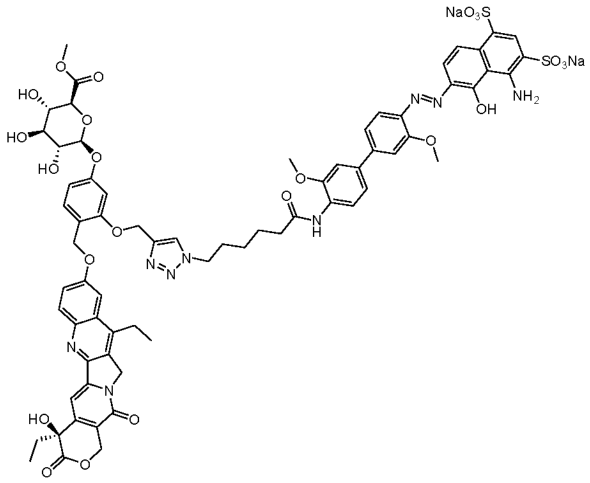 | 4-amino-6-((E)-(4'-(6-(4-((2-(((S )-4,11-diethyl-4-hydroxy-3,14-di oxo-3,4,12,14-tetrahydro-1H-pyr ano[3',4':6,7]indolazo[1,2-b]qui nolin-9-yl)oxy)methyl)-5-(((2S,3 R,4S,5S,6S)-3,4,5-trihydroxy-6-( methoxycarbonyl)tetrahydro-2H-pyran-2-yl)oxy)phenoxy) methyl) -1H-1,2,3-triazol-1-yl)hexanamid o)-3,3'-dimethoxy-[1,1'-biphenyl ]-4-yl)diazenyl)-5-hydroxynapht halene-1,3-disulfonate disodium |
| 32 | 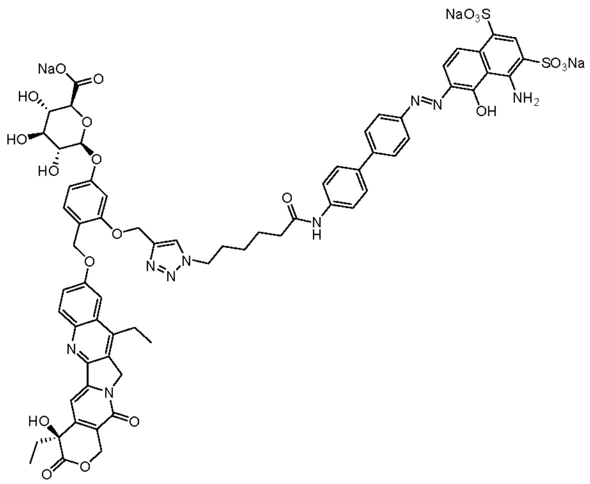 | (2S,3S,4S,5R,6S)-6-(3-((1-(6-((4 '-((E)-(8-amino-1-hydroxy-5,7-di sulfonicnaphthalen-2-yl) diazenyl )-[1,1'-biphenyl]-4-yl)amino)-6-oxohexyl)-1H-1,2,3-triazol-4-yl) methoxy)-4-((((S)-4,11-diethyl-4 -hydroxy-3,14-dioxo-3,4,12,14-t etrahydro-1H-pyrano[3',4':6,7] in dolazino[1,2-b]quinolin-9-yl)oxy )methyl) phenoxy)-3,4,5-trihydro xytetrahydro-2H-pyran-2-carbox ylate trisodium |
| 33 | 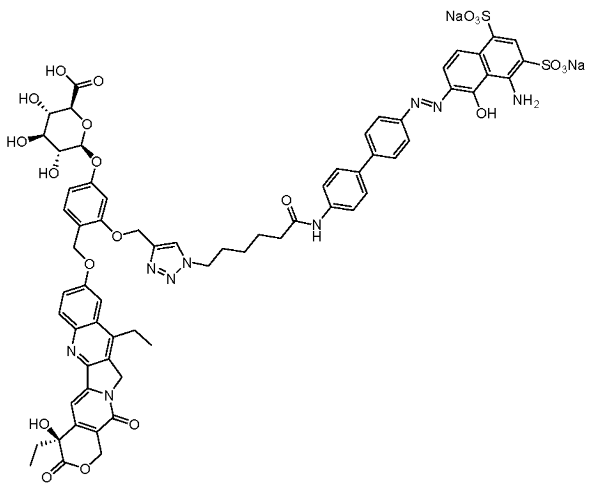 | 4-amino-6-((E)-(4'-(6-(4-((5-(((2 S,3R,4S,5S,6S)-6-carboxy-3,4,5-trihydroxytetrahydro-2H-pyran-2 -yl)oxy)-2-((((S)-4,11-diethyl-4-hydroxy-3,14-dioxy-3,4,12,14-tet rahydro-1H-pyrano[3',4':6,7]ind olazino[1,2-b] quinolin-9-yl)oxy) methyl)phenoxy) methyl)-1H-1,2, 3-triazol-1-yl)hexanamido)-[1,1'-biphenyl]-4-yl)diazenyl)-5-hydro xynaphthalene-1,3-disulfonate disodium |

(continued)

| No. | Structure | Chemical Name |
|---|---|---|
| 34 | 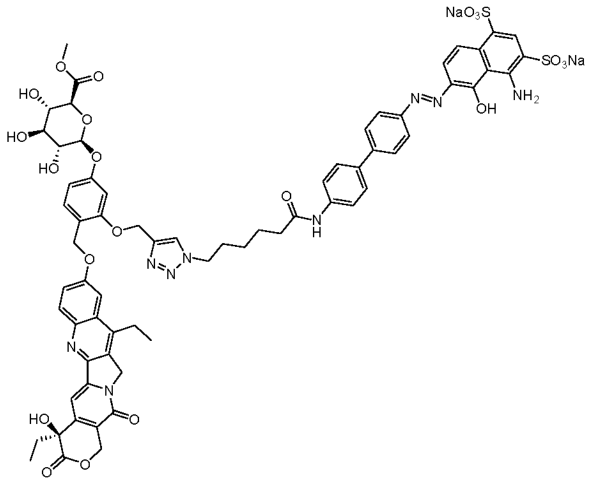 | 4-amino-6-((E)-(4'-(6-(4-((2-(((S )-4,11-diethyl-4-hydroxy-3,14-di oxy-3,4,12,14-tetrahydro-1H-pyr ano[3',4':6,7]indolazino[1,2-b]q uinolin-9-yl)oxy) methyl)-5-(((2S ,3R,4S,5S,6S)-3,4,5-trihydroxy-6 -(methoxycarbonyl)tetrahydro-2 H-pyran-2-yl)oxy)phenoxy)meth yl)-1H-1,2,3-triazol-1-yl)hexana mido)-[1,1'-biphenyl]-4-yl) diaze nyl)-5-hydroxynaphthalene-1,3-d isulfo-nate disodium |
| 35 | 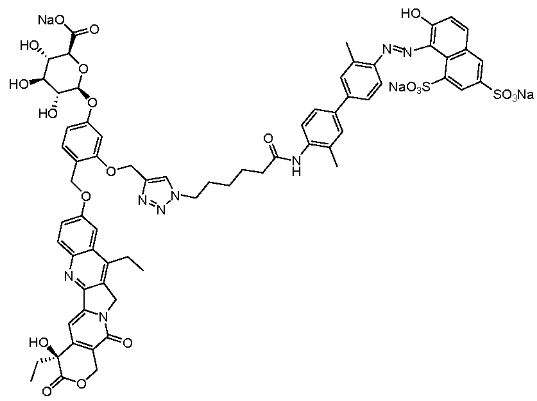 | (2S,3S,4S,5R,6S)-6-(4-(((S)-4,11 -diethyl-4-hydroxy-3,14-dioxo-3, 4,12,14-tetrahydro-1H-pyrano[3' ,4':6,7]indolazino[1,2-b]quinolin -9-yl)oxy)methyl)-3-((1-(6-((4'-( (E)-(2-hydroxy-6,8-disulfonicnap hthalen-1-yl)diazenyl)-3,3'-dimet hyl-[1,1'-biphenyl]-4-yl)amino)-6-oxohexyl)-1H-1,2,3-triazol-4-y l)methoxy)phenoxy)-3,4,5-trihyd roxytetrahydro-2H-pyran-2-carb oxylate trisodium |
| 36 | 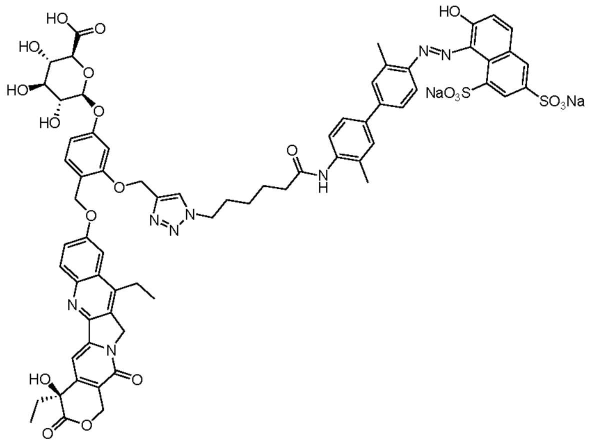 | 8-((E)-(4'-(6-(4-((5-(((2S,3R,4S, 5S,6S)-6-carboxy-3,4,5-trihydrox ytetrahydro-2H-pyran-2-yl)oxy)-2-((((S)-4,11-diethyl-4-hydroxy-3,14-dioxy-3,4,12,14-tetrahydro-1H-pyrano[3',4':6,7] indolazino[ 1,2-b]quinolin-9-yl)oxy)methyl)p henoxy)methyl)-1H-1,2,3-triazol -1-yl)hexanamido)-3,3'-dimethyl -[1,1'-biphenyl]-4-yl)diazenyl)-7 -hydroxynaphthalene-1,3-disulfo nate disodium |

(continued)

| No. | Structure | Chemical Name |
|---|---|---|
| 37 | 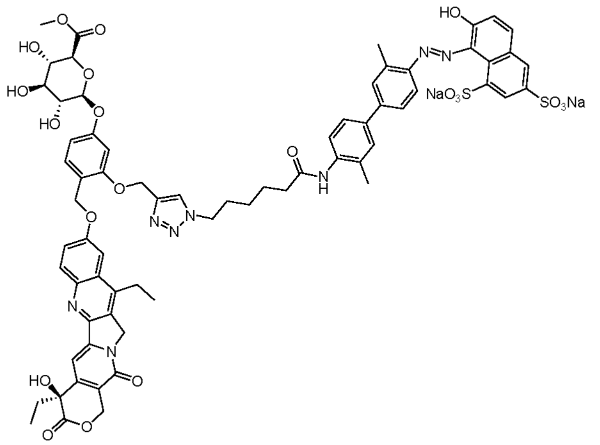 | 8-((E)-(4'-(6-(4-((2-((((S)-4,11-d iethyl-4-hydroxy-3,14-dioxy-3,4, 12,14-tetrahydro-1H-pyrano [3',4 ':6,7]indolizino[1,2-b]quinolin-9 -yl)oxy) methyl)-5-(((2S,3R,4S,5 S,6S)-3,4,5-trihydroxy-6-(metho xycarbonyl)tetrahydro-2H-pyran-2-yl)oxy)phenoxy)methyl)-1H-1, 2,3-triazol-1-yl) hexanamido)-3,3 '-dimethyl-[1,1'-biphenyl]-4-yl)d iazenyl)-7-hydroxynaphthalene-1 ,3-disulfonate disodium |
| 38 | 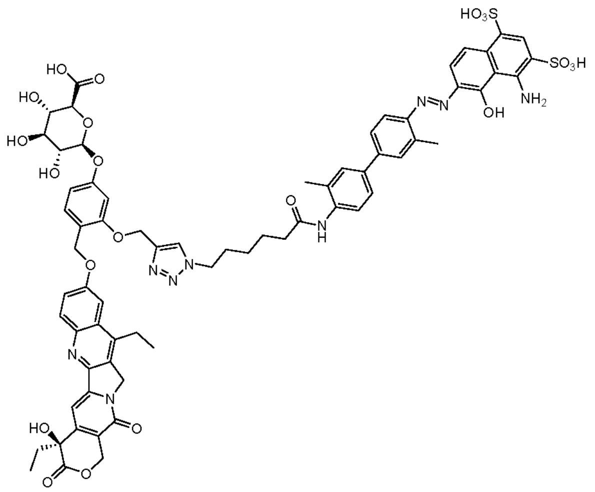 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-(((S)-4, 11-diethyl-4-hydroxy-3,14-dioxy -3,4,12,14-tetrahydro-1H-pyrano [3',4':6,7]indolazino[1,2-b]quino lin-9-yl)oxy) methyl)phenoxy)-3, 4,5-trihydroxytetrahydro-2H-pyr an-2-carboxylic acid |
| 39 | 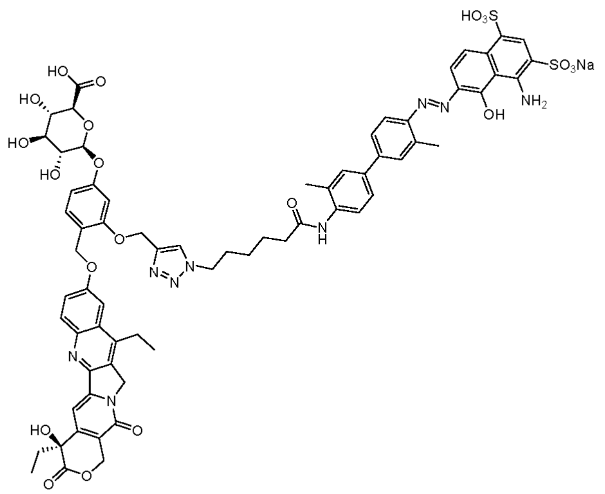 | 1-amino-7-((E)-(4'-(6-(4-((5-(((2 S,3R,4S,5S,6S)-6-carboxy-3,4,5-trihydroxytetrahydro-2H-pyran-2 -yl)oxy)-2-((((S)-4,11-diethyl-4-hydroxy-3,14-dioxy-3,4,12,14-tet rahydro-1H-pyrano[3',4':6,7]ind olazino[1,2-b] quinolin-9-yl)oxy) methyl)phenoxy) methyl)-1H-1,2, 3-triazol-1-yl)hexanamido)-3,3'-dimethyl-[1,1'-biphenyl]-4-yl)dia zenyl)-8-hydroxy-4-sulfonicnaph thalene-2-sulfonate sodium |

(continued)

| No. | Structure | Chemical Name |
|---|---|---|
| 40 | 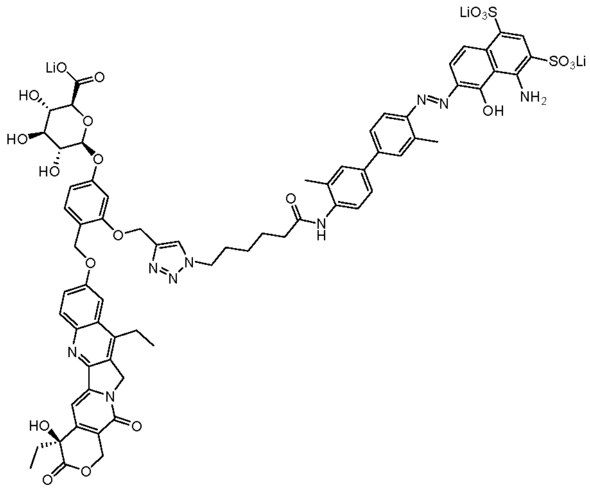 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolizino[1,2-b]quinol in-9-yl)oxy) methyl)phenoxy)-3,4 ,5-trihydroxytetrahydro-2H-pyra n-2-carboxylate trilithium |
| 41 | 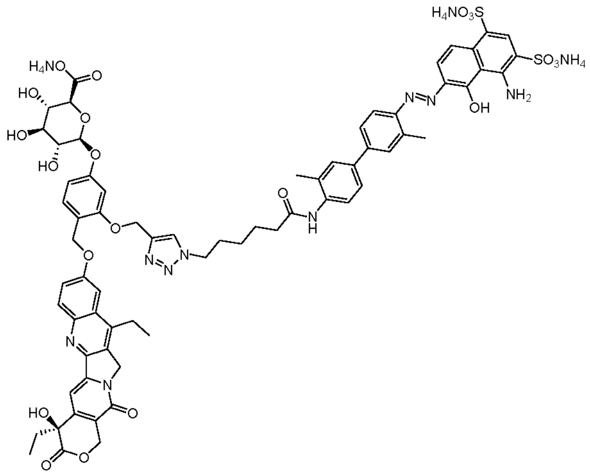 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolazino[1,2-b]inolin -9-yl)oxy) methyl)phenoxy)-3,4,5 -trihydroxytetrahydro-2H-pyran-2-carboxylate triammonium |
| 42 | 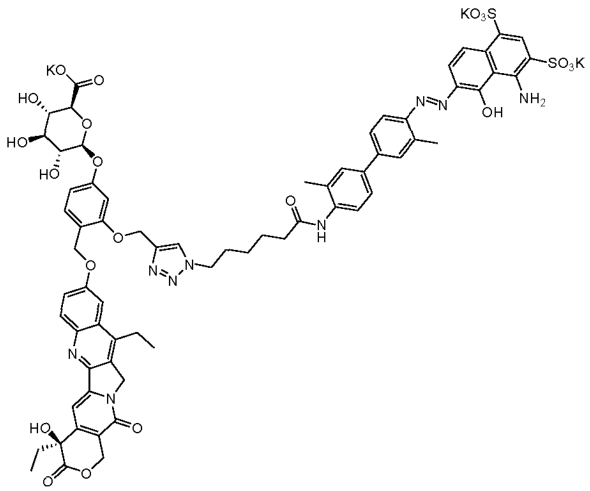 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolazino[1,2-b]quino lin-9-yl)oxy) methyl)phenoxy)-3, 4,5-trihydroxytetrahydro-2H-pyr an-2-carboxylate tripotassium |

(continued)

| No. | Structure | Chemical Name |
|---|---|---|
| 43 | 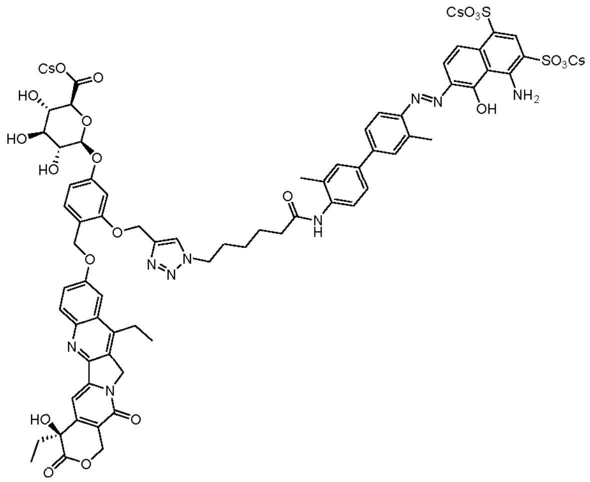 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolazino[1,2-b]quino lin-9-yl)oxy) methyl)phenoxy)-3, 4,5-trihydroxytetrahydro-2H-pyr an-2-carboxylate tricesium |
| 44 | 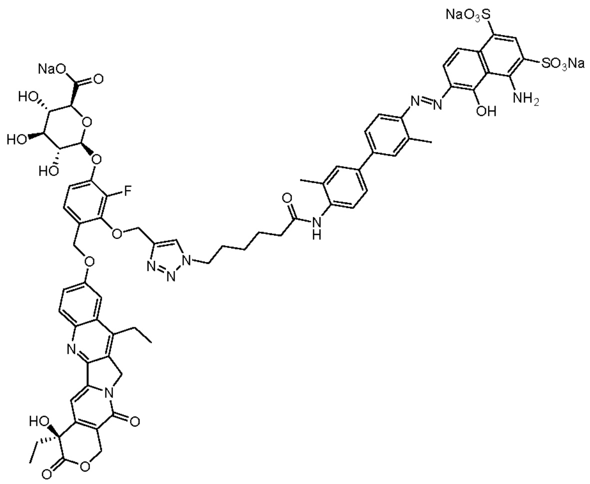 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolizino[1,2-b]quinol in-9-yl)oxy) methyl)-2-fluorophe noxy)-3,4,5-trihydroxytetrahydro -2H-pyran-2-carboxylate trisodium |
| 45 | 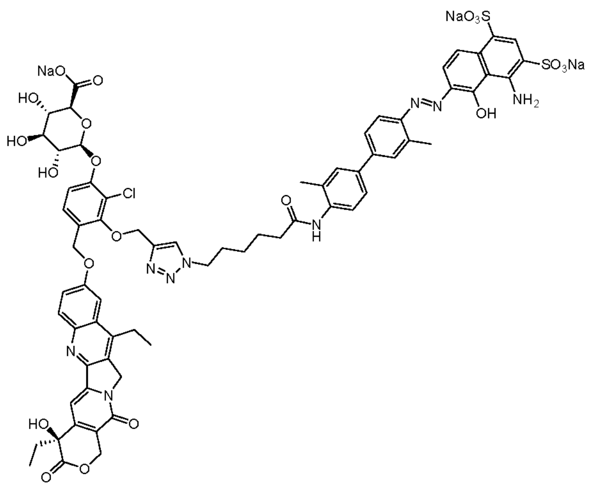 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-2-chloro-4-((((S)-4,11-diethyl-4-hydroxy-3,14-dioxy-3,4,12,14-tetrahydro-1H-pyrano[3',4':6,7]indolazino[ 1,2-b]quinolin-9-yl) oxy)methyl)p henoxy)-3,4,5-trihydroxytetrahyd ro-2H-pyran-2-carboxylate trisodium |

(continued)

| No. | Structure | Chemical Name |
|---|---|---|
| 46 | 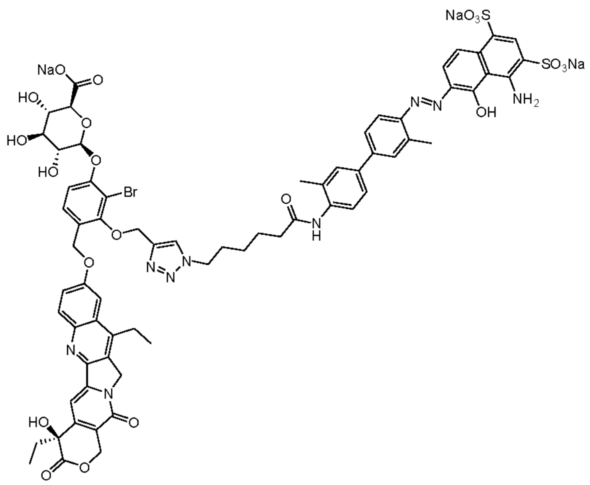 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-2-bromo-4-((((S)-4,11-diethyl-4-hydroxy-3,14-dioxy-3,4,12,14-tetrahydro-1H-pyrano[3',4':6,7]indolazino[ 1,2-b]quinolin-9-yl) oxy)methyl)p henoxy)-3,4,5-trihydroxytetrahyd ro-2H-pyran-2-carboxylate trisodium |
| 47 | 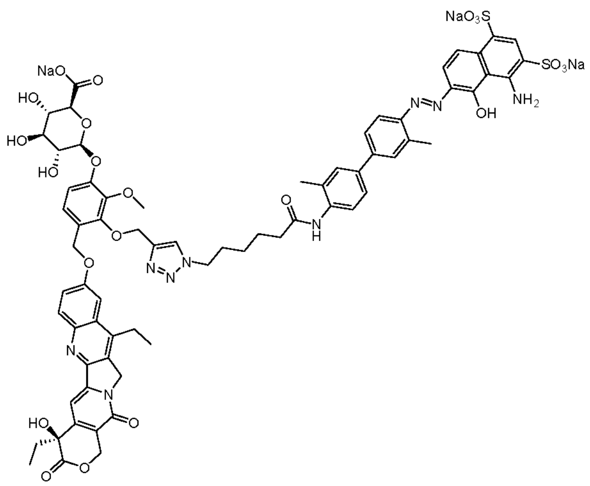 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolizino[1,2-b]quinol in-9-yl)oxy) methyl)-2-methoxyp henoxy)-3,4,5-trihydroxytetrahyd ro-2H-pyran-2-carboxylate trisodium |
| 48 | 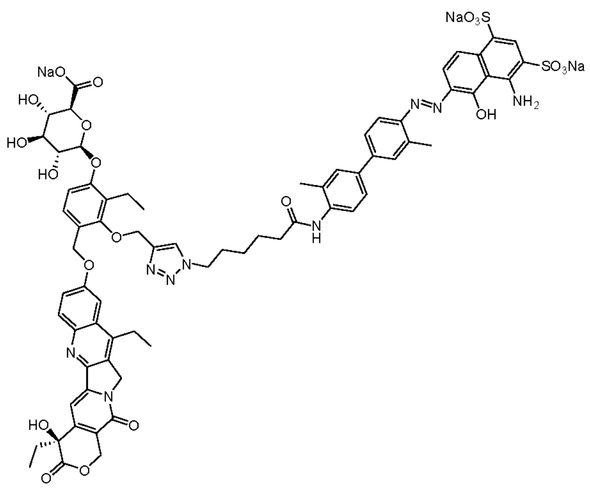 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolazino[1,2-b]quino lin-9-yl)oxy) methyl)-2-ethylphen oxy)-3,4,5-trihydroxytetrahydro-2H-pyran-2-carboxylate trisodium |

(continued)

| No. | Structure | Chemical Name |
|---|---|---|
| 49 | 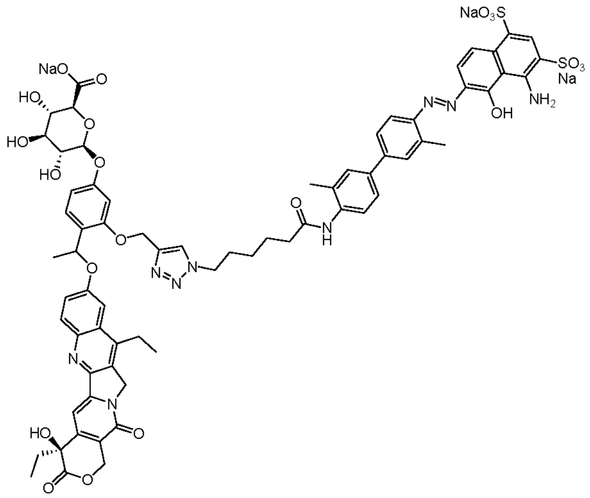 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-(1-(((S) -4,11-diethyl-4-hydroxy-3,14-dio xy-3,4,12,14-tetrahydro-1H-pyra no[3',4':6,7]indolazino[1,2-b]qui nolin-9-yl)oxy) ethyl)phenoxy)-3, 4,5-trihydroxytetrahydro-2H-pyr an-2-carboxylate trisodium |
| 50 | 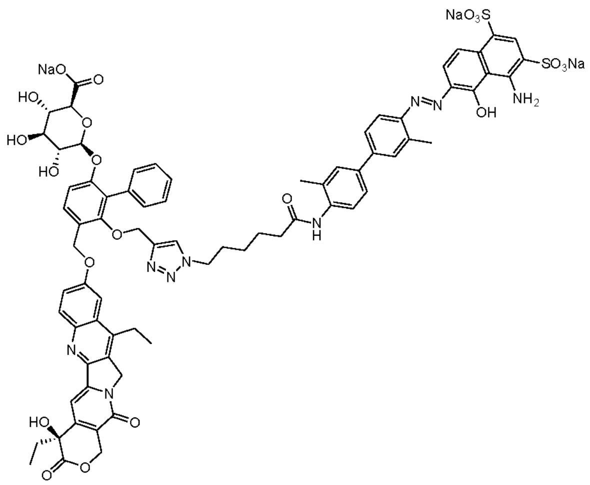 | (2S,3S,4S,5R,6S)-6-((6-((1-((4'-( (E)-(8-amino-1-hydroxy-5,7-disu lfonicnaphthalen-2-yl) diazenyl)-3,3'-dimethyl-[1,1'-biphenyl]-4-yl) amino)-6-oxohexyl)-1H-1,2,3-triazol-4-yl)methoxy)-5-((((S)-4, 11-diethyl-4-hydroxy-3,14-dioxy -3,4,12,14-tetrahydro-1H-pyrano [3',4':6,7] indolizino[1,2-b]quinol in-9-yl)oxy) methyl)-[1,1'-biphen yl]-2-yl)oxy)-3,4,5-trihydroxytetr ahydro-2H-pyran-2-carboxylate trisodium |
| 51 | 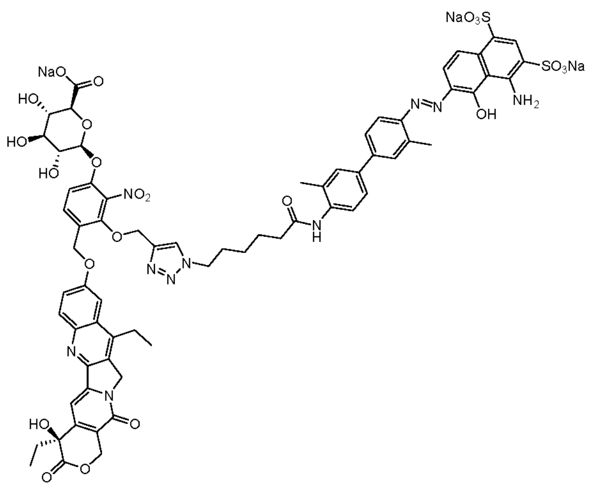 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-(((S)-4, 11-diethyl-4-hydroxy-3,14-dioxy -3,4,12,14-tetrahydro-1H-pyrano [3',4':6,7]indolazino[1,2-b]quino lin-9-yl)oxy) methyl)-2-nitrophen oxy)-3,4,5-trihydroxytetrahydro-2H-pyran-2-carboxylate trisodium |

(continued)

| No. | Structure | Chemical Name |
|---|---|---|
| 52 | 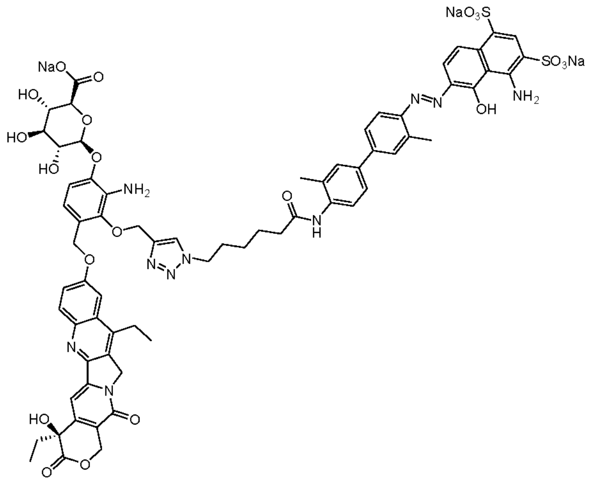 | (2S,3S,4S,5R,6S)-6-(2-amino-3-( (1-(6-((4'-((E)-(8-amino-1-hydro xy-5,7-disulfonicnaphthalen-2-yl )diazenyl)-3,3'-dimethyl-[1,1-bi phenyl]-4-yl)amino)-6-oxohexyl) -1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[ 1,2-b] quinolin-9-yl)oxy)methyl)p henoxy)-3,4,5-trihydroxytetrahyd ro-2H-pyran-2-carboxylate trisodium |
| 53 | 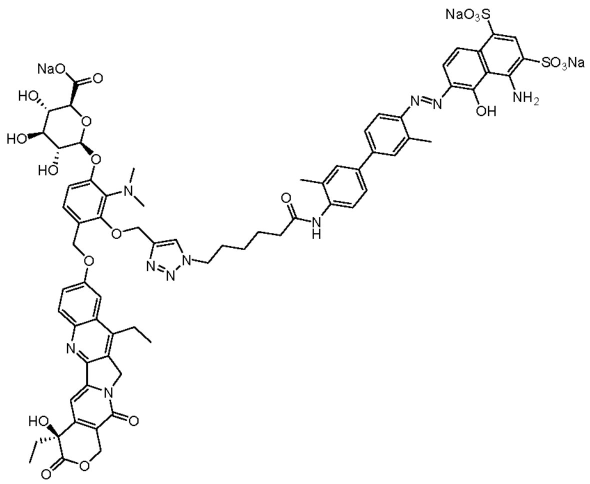 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolazino[1,2-b]quino lin-9-yl)oxy) methyl)-2-(dimethyl amino)phenoxy)-3,4,5-trihydrox ytetrahydro-2H-pyran-2-carboxyl ate trisodium |
| 54 | 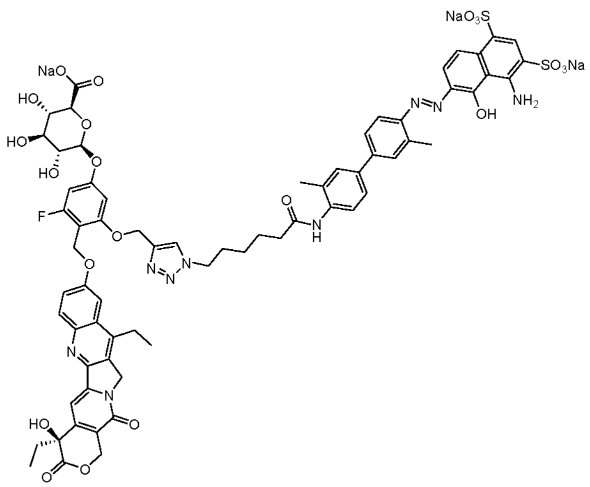 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-(((S)-4, 11-diethyl-4-hydroxy-3,14-dioxy -3,4,12,14-tetrahydro-1H-pyrano [3',4':6,7]indolazino[1,2-b]quino lin-9-yl)oxy) methyl)-5-fluorophe noxy)-3,4,5-trihydroxytetrahydro -2H-pyran-2-carboxylate trisodium |

(continued)

| No. | Structure | Chemical Name |
|---|---|---|
| 55 | 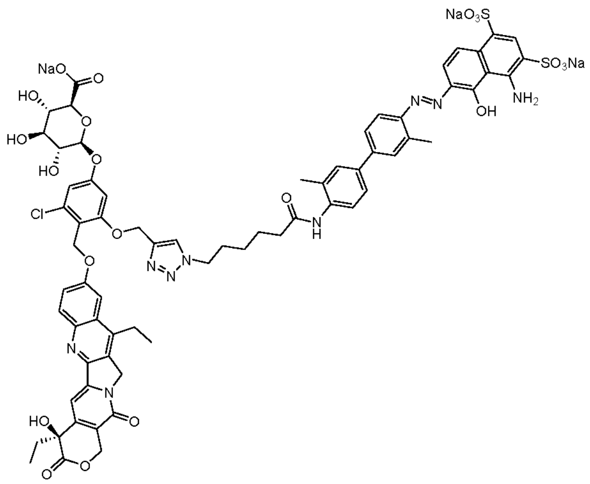 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-5-chloro-4-((((S)-4,11-diethyl-4-hydroxy-3,14-dioxy-3,4,12,14-tetrahydro-1H-pyrano[3',4':6,7]indolazino[ 1,2-b]quinolin-9-yl) oxy)methyl)p henoxy)-3,4,5-trihydroxytetrahyd ro-2H-pyran-2-carboxylate trisodium |
| 56 | 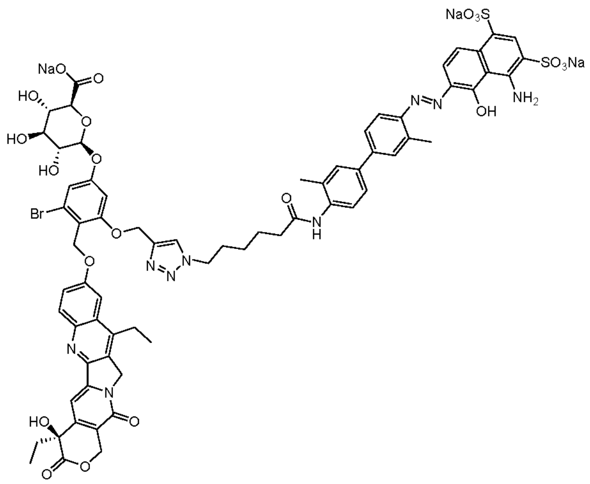 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-5-bromo-4-((((S)-4,11-diethyl-4-hydroxy-3,14-dioxy-3,4,12,14-tetrahydro-1H-pyrano[3',4':6,7]indolazino[ 1,2-b]quinolin-9-yl) oxy)methyl)p henoxy)-3,4,5-trihydroxytetrahyd ro-2H-pyran-2-carboxylate trisodium |
| 57 | 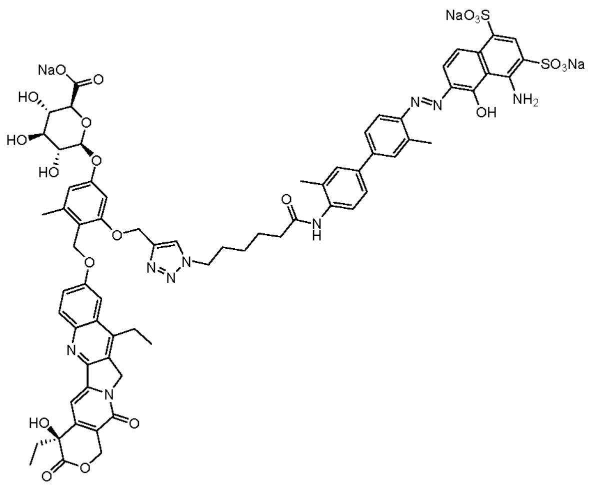 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3,4:6,7]indolazino[1,2-b]quinoli n-9-yl)oxy) methyl)-5-methylphe noxy)-3,4,5-trihydroxytetrahydro -2H-pyran-2-carboxylate trisodium |

(continued)

| No. | Structure | Chemical Name |
|---|---|---|
| 58 | 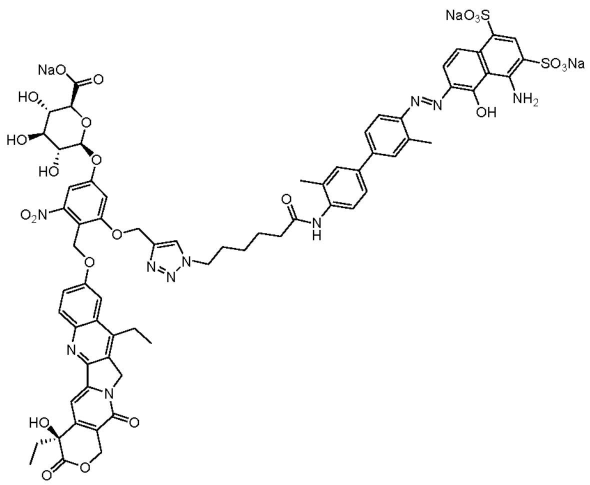 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolazino[1,2-b]quino lin-9-yl)oxy) methyl)-5-nitrophen oxy)-3,4,5-trihydroxytetrahydro-2H-pyran-2-carboxylate trisodium |
| 59 | 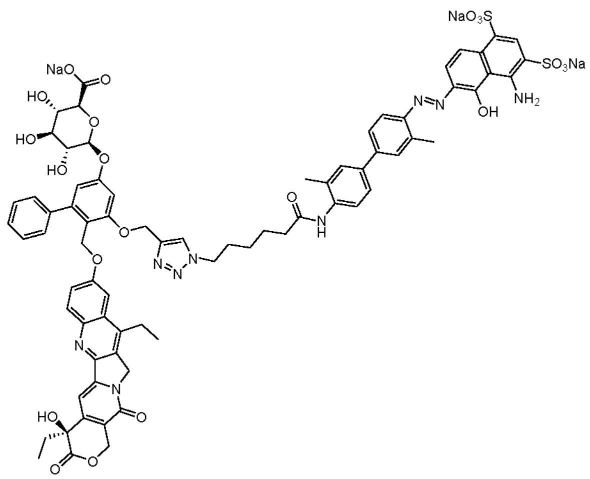 | (2S,3S,4S,5R,6S)-6-((5-((1-((4'-( (E)-(8-amino-1-hydroxy-5,7-disu lfonicnaphthalen-2-yl) diazenyl)-3,3'-dimethyl-[1,1'-biphenyl]-4-yl) amino)-6-oxohexyl)-1H-1,2,3-triazol-4-yl)methoxy)-6-((((S)-4, 11-diethyl-4-hydroxy-3,14-dioxy -3,4,12,14-tetrahydro-1H-pyrano [3',4':6,7] indolizino[1,2-b]quinol in-9-yl)oxy) methyl)-[1,1'-biphen yl]-3-yl)oxy)-3,4,5-trihydroxytetr ahydro-2H-pyran-2-carboxylate trisodium |
| 60 | 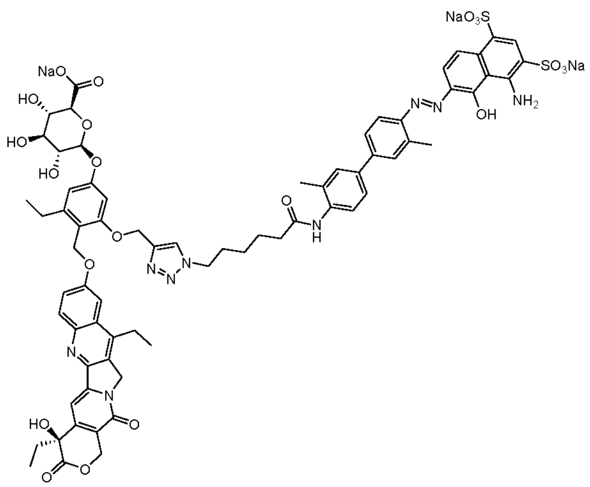 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolizino[1,2-b]quinol in-9-yl)oxy) methyl)-5-ethylphen oxy)-3,4,5-trihydroxytetrahydro-2H-pyran-2-carboxylate trisodium |

(continued)

| No. | Structure | Chemical Name |
|---|---|---|
| 61 | 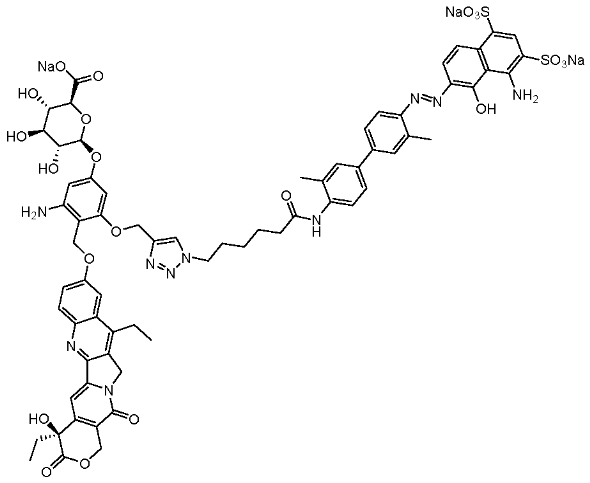 | (2S,3S,4S,5R,6S)-6-(3-amino-5-( (1-(6-((4'-((E)-(8-amino-1-hydro xy-5,7-disulfonicnaphthalen-2-yl )diazenyl)-3,3'-dimethyl-[1,1-bi phenyl]-4-yl)amino)-6-oxohexyl) -1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[ 1,2-b] quinolin-9-yl)oxy)methyl)p henoxy)-3,4,5-trihydroxytetrahyd ro-2H-pyran-2-carboxylate trisodium |
| 62 | 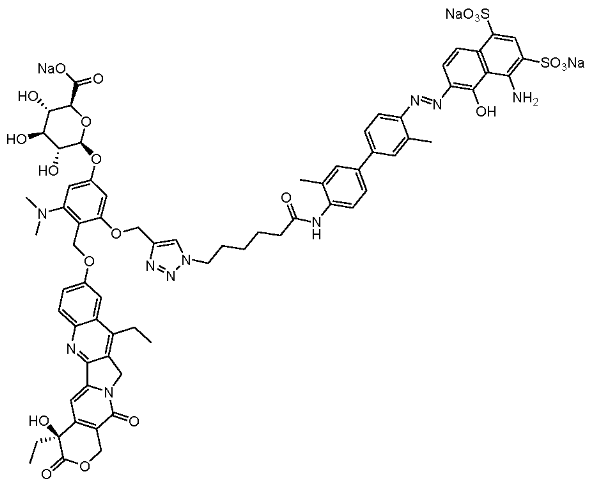 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolazino[1,2-b]quino lin-9-yl)oxy) methyl)-5-(dimethyl amino)phenoxy)-3,4,5-trihydrox ytetrahydro-2H-pyran-2-carboxyl ate trisodium |
| 63 | 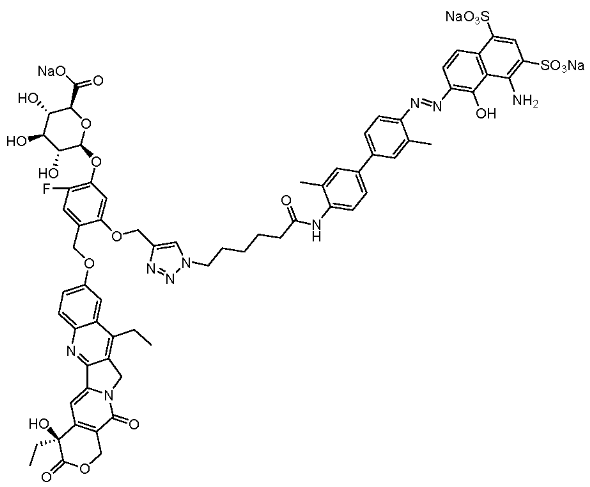 | (2S,3S,4S,5R,6S)-6-(5-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolizino[1,2-b]quinol in-9-yl)oxy) methyl)-2-fluorophe noxy)-3,4,5-trihydroxytetrahydro -2H-pyran-2-carboxylate trisodium |

(continued)

| No. | Structure | Chemical Name |
|---|---|---|
| 64 | 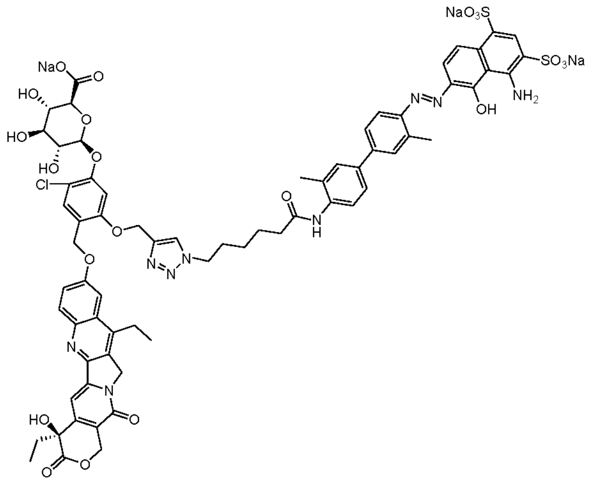 | (2S,3S,4S,5R,6S)-6-(5-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-2-chloro-4-((((S)-4,11-diethyl-4-hydroxy-3,14-dioxy-3,4,12,14-tetrahydro-1H-pyrano[3',4':6,7]indolazino[ 1,2-b]quinolin-9-yl) oxy)methyl)p henoxy)-3,4,5-trihydroxytetrahyd ro-2H-pyran-2-carboxylate trisodium |
| 65 | 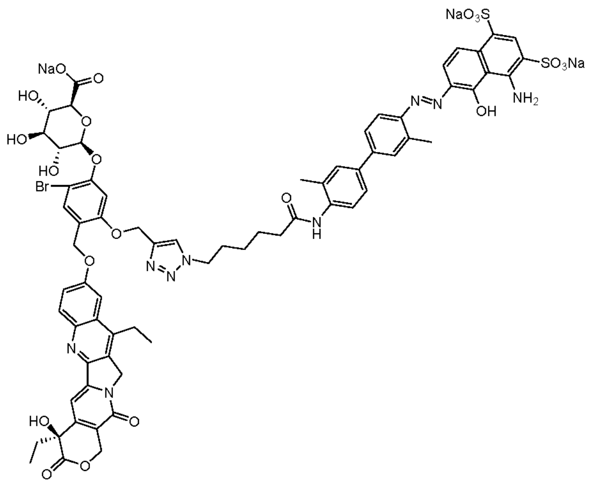 | (2S,3S,4S,5R,6S)-6-(5-((1-(6-((4 '-((E)-(8-amino-1-hydroxy-5,7-di sulfoniconaphthalen-2-yl) diazen yl)-3,3'-dimethyl-[1,1'-biphenyl] -4-yl) amino)-6-oxohexyl)-1H-1,2 ,3-triazol-4-yl) methoxy)-2-brom o-4-((((S)-4,11-diethyl-4-hydrox y-3,14-dioxy-3,4,12,14-tetrahydr o-1H-pyrano[3',4':6,7]indolazin o[1,2-b]quinolin-9-yl) oxy)methyl )phenoxy)-3,4,5-trihydroxytetrah ydro-2H-pyran-2-carboxylate trisodium |
| 66 | 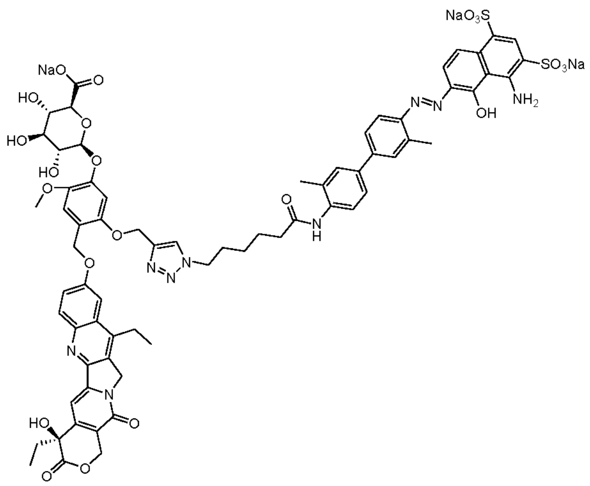 | (2S,3S,4S,5R,6S)-6-(5-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolizino[1,2-b]quinol in-9-yl)oxy) methyl)-2-methoxyp henoxy)-3,4,5-trihydroxytetrahyd ro-2H-pyran-2-carboxylate trisodium |

(continued)

| No. | Structure | Chemical Name |
|---|---|---|
| 67 | 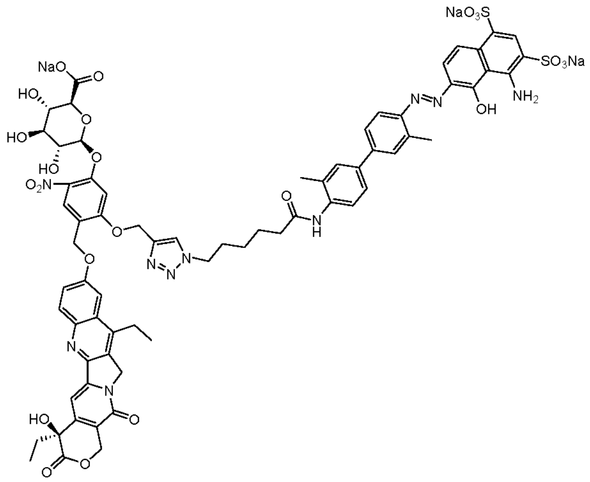 | (2S,3S,4S,5R,6S)-6-(5-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolizino[1,2-b]quinol in-9-yl)oxy) methyl)-2-nitrophen oxy)-3,4,5-trihydroxytetrahydro-2H-pyran-2-carboxylate trisodium |
| 68 | 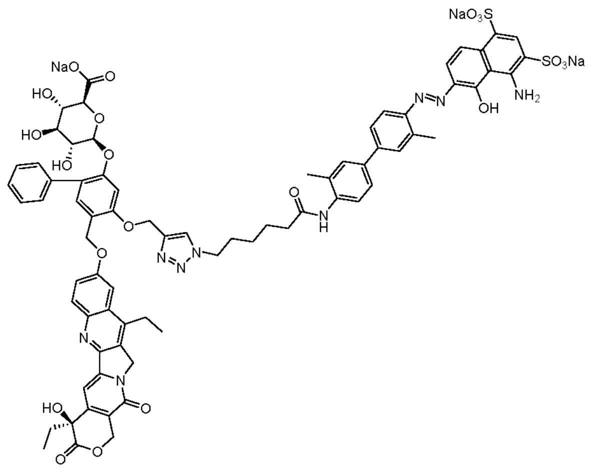 | (2S,3S,4S,5R,6S)-6-((4-((1-(6-(( 4'-((E)-(8-amino-1-hydroxy-5,7-disulfonicnaphthalen-2-yl)diazen yl)-3,3'-dimethyl-[1,1'-biphenyl] -4-yl) amino)-6-oxohexyl)-1H-1,2 ,3-triazol-4-yl) methoxy)-5-((((S)-4,11-diethyl-4-hydroxy-3,14-dio xy-3,4,12,14-tetrahydro-1H-pyra no [3',4':6,7]indolizino[1,2-b]qui nolin-9-yl)oxy) methyl)-[1,1'-bip henyl]-2-yl)oxy)-3,4,5-trihydrox ytetrahydro-2H-pyran-2-carboxyl ate trisodium |
| 69 | 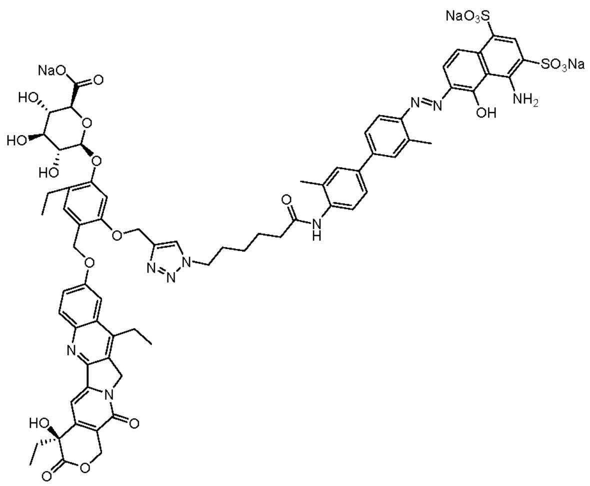 | (2S,3S,4S,5R,6S)-6-(5-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolazino[1,2-b]quino lin-9-yl)oxy) methyl)-2-ethylphen oxy)-3,4,5-trihydroxytetrahydro-2H-pyran-2-carboxylate trisodium |

(continued)

| No. | Structure | Chemical Name |
|---|---|---|
| 70 | 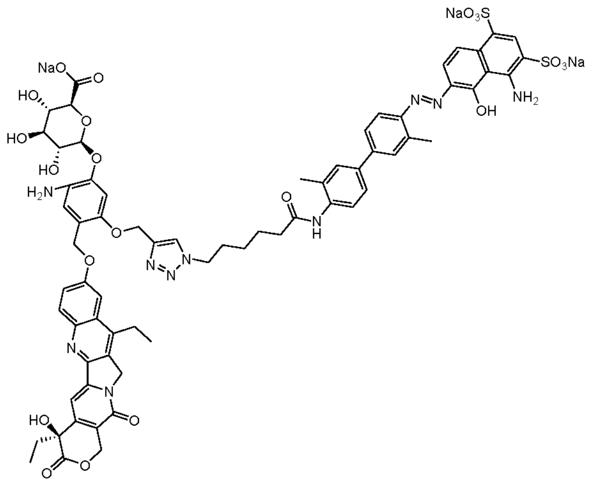 | (2S,3S,4S,5R,6S)-6-(2-amino-5-( (1-(6-((4'-((E)-(8-amino-1-hydro xy-5,7-disulfonicnaphthalen-2-yl )diazenyl)-3,3'-dimethyl-[1,1-bi phenyl]-4-yl)amino)-6-oxohexyl) -1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[ 1,2-b] quinolin-9-yl)oxy)methyl)p henoxy)-3,4,5-trihydroxytetrahyd ro-2H-pyran-2-carboxylate trisodium |
| 71 | 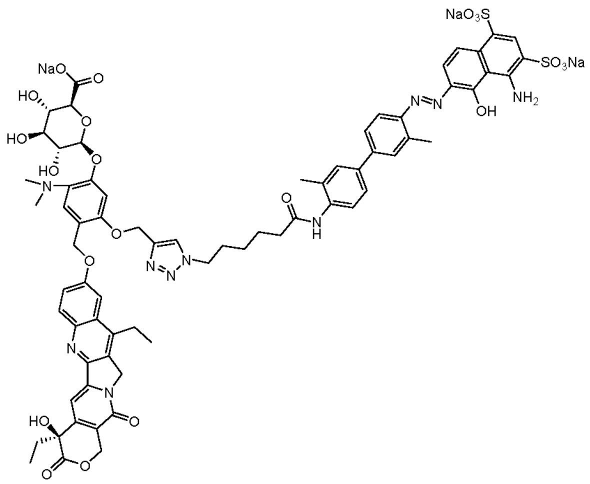 | (2S,3S,4S,5R,6S)-6-(5-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolizino[1,2-b]quinol in-9-yl)oxy) methyl)-2-(dimethyl amino)phenoxy)-3,4,5-trihydrox ytetrahydro-2H-pyran-2-carboxyl ate trisodium |
| 72 | 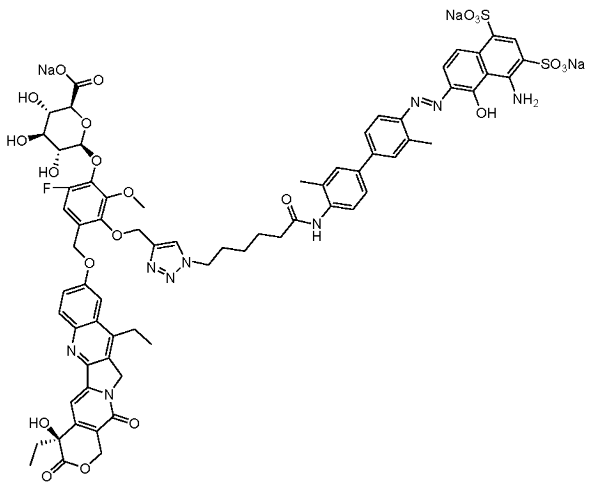 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolizino[1,2-b]quinol in-9-yl)oxy) methyl)-6-fluoro-2-methoxyphenoxy)-3,4,5-trihydro xytetrahydro-2H-pyran-2-carbox ylate trisodium |

(continued)

| No. | Structure | Chemical Name |
|---|---|---|
| 73 | 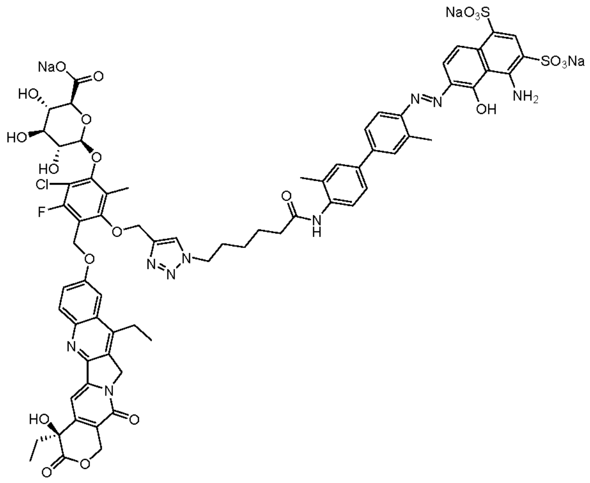 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-6-chloro-4-((((S)-4,11-diethyl-4-hydroxy-3,14-dioxy-3,4,12,14-tetrahydro-1H-pyrano[3',4':6,7]indolazino[ 1,2-b]quinolin-9-yl) oxy)methyl)-5-fluoro-2-methylphenoxy)-3,4,5 -trihydroxytetrahydro-2H-pyran-2-carboxylate trisodium |
| 74 | 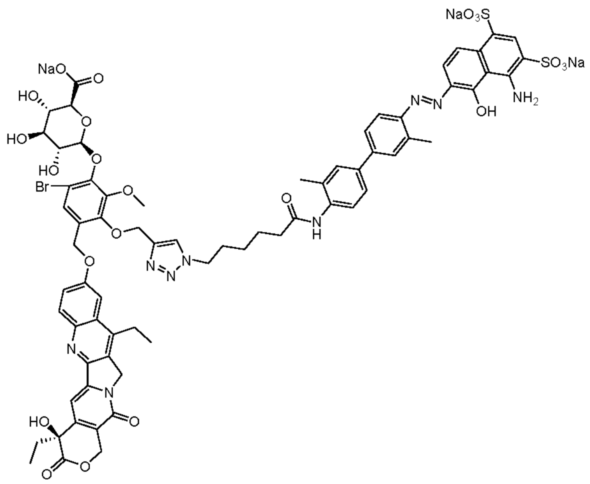 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-6-bromo-4-((((S)-4,11-diethyl-4-hydroxy-3,14-dioxy-3,4,12,14-tetrahydro-1H-pyrano[3',4':6,7]indolazino[ 1,2-b]quinolin-9-yl) oxy)methyl)-2-methoxyphenoxy)-3,4,5-trihyd roxytetrahydro-2H-pyran-2-carb oxylate trisodium |
| 75 | 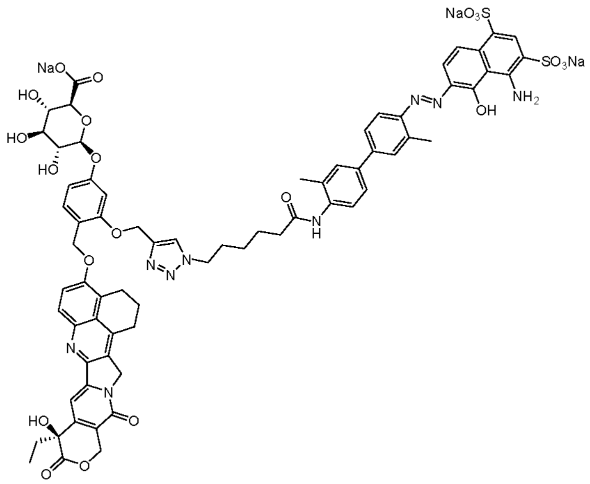 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((S)-9 -ethyl-9-hydroxy-10,13-dioxy-2, 3,9,10,13,15-tetrahydro-1H,12H-benzo [de]pyrano[3',4':6,7]indoli zino[1,2-b]quinolin-4-yl)oxy)met hyl)phenoxy)-3,4,5-trihydroxytet rahydro-2H-pyran-2-carboxylate trisodium |

(continued)

| No. | Structure | Chemical Name |
|---|---|---|
| 76 | 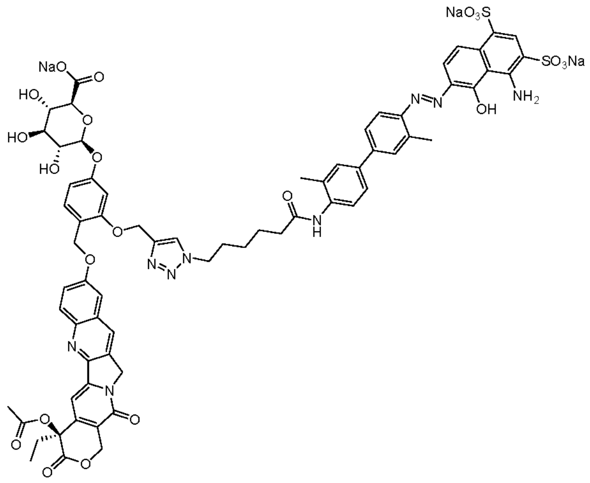 | (2S,3S,4S,5R,6S)-6-(4-(((S)-4-ac etoxy-4-ethyl-3,14-dioxo-3,4,12, 14-tetrahydro-1H-pyrano[3',4':6, 7]indolazino[1,2-b]quinolin-9-yl) oxy)methyl)-3-((1-(6-((4'-((E)-(8 -amino-1-hydroxy-5,7-disulfonic naphthalen-2-yl)diazenyl)-3,3'-di methyl-[1,1'-biphenyl]-4-yl)amin o)-6-oxohexyl)-1H-1,2,3-triazol-4-yl)methoxy) phenoxy)-3,4,5-tri hydroxytetrahydro-2H-pyran-2-c arboxylate trisodium |
| 77 | 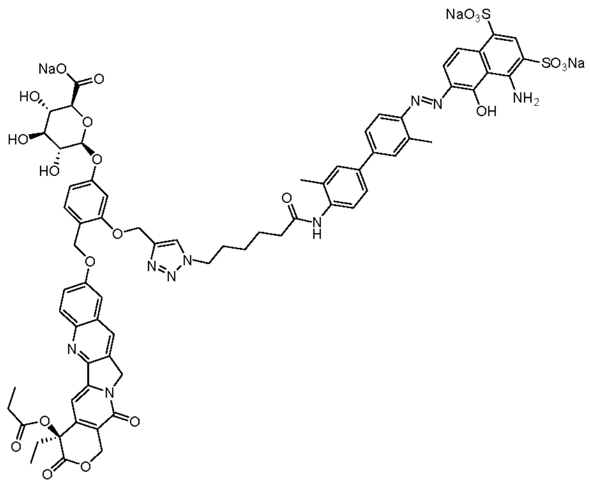 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-(((S)-4-ethyl-3,14-dioxo-4-(propionylox y)-3,4,12,14-tetrahydro-1H-pyra no [3',4':6,7]indolizino[1,2-b]qui nolin-9-yl)oxy) methyl)phenoxy)-3,4,5-trihydroxytetrahydro-2H-p yran-2-carboxylate trisodium |
| 78 | 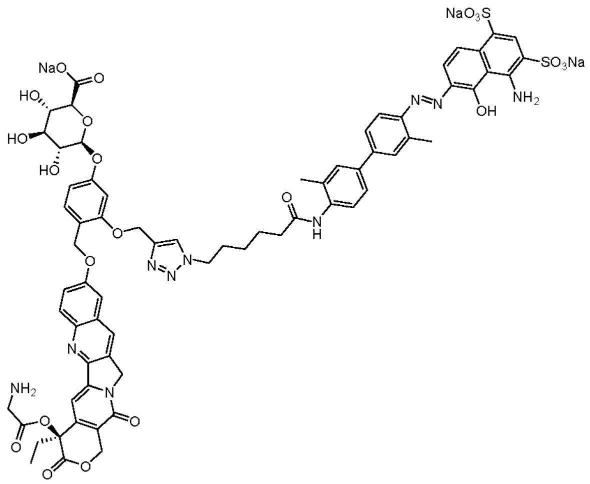 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-(((S)-4-ethyl-4-(glycyloxy)-3,14-dioxy-3 ,4,12,14-tetrahydro-1H-pyrano[3 ',4':6,7] indolizino[1,2-b]quinolin -9-yl)oxy)methyl)phenoxy)-3,4,5 -trihydroxytetrahydro-2H-pyran-2-carboxylate trisodium |

(continued)

| No. | Structure | Chemical Name |
|---|---|---|
| 79 | 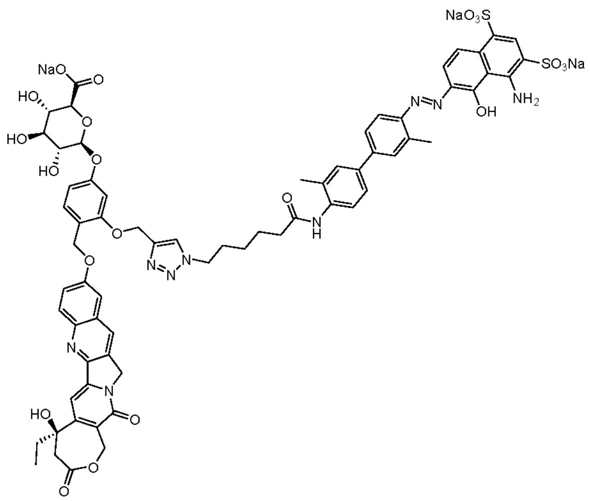 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-((((R)-5 -ethyl-5-hydroxy-3,15-dioxo-4,5, 13,15-tetrahydro-1H,3H-oxazepi no [3',4':6,7]indolazino[1,2-b]qui nolin-10-yl)oxy) methyl)phenoxy )-3,4,5-trihydroxytetrahydro-2H-pyran-2-carboxylate trisodium |
| 80 | 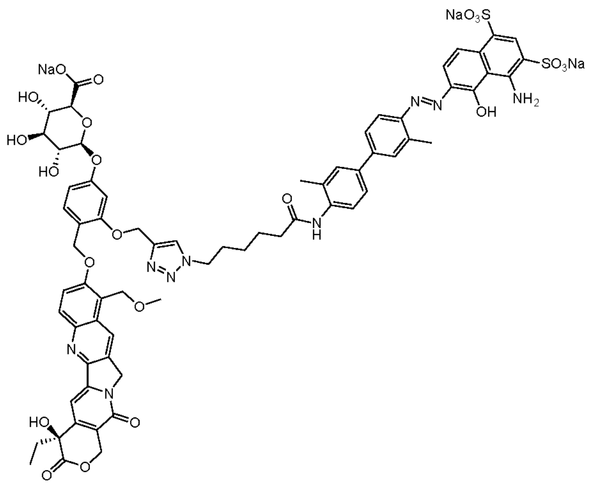 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-(((S)-4-ethyl-4-hydroxy-10-(methoxyme thyl)-3,14-dioxy-3,4,12,14-tetrah ydro-1H-pyrano[3',4':6,7]indola zino[1,2-b]quinolin-9-yl)oxy)met hyl)phenoxy)-3,4,5-trihydroxytet rahydro-2H-pyran-2-carboxylate trisodium |
| 81 | 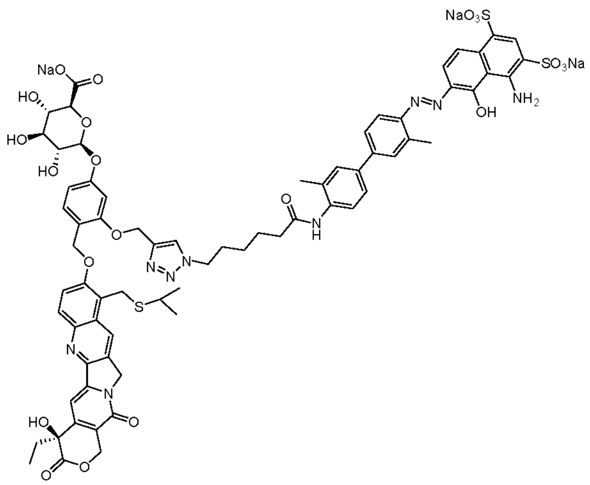 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-(((S)-4-ethyl-4-hydroxy-10-((isopropylth io)methyl)-3,14-dioxy-3,4,12,14-tetrahydro-1H-pyrano[3',4':6,7]i ndolazino[1,2-b] quinolin-9-yl)ox y)methyl)phenoxy)-3,4,5-trihydr oxytetrahydro-2H-pyran-2-carbo xylate trisodium |

(continued)

| No. | Structure | Chemical Name |
|---|---|---|
| 82 | 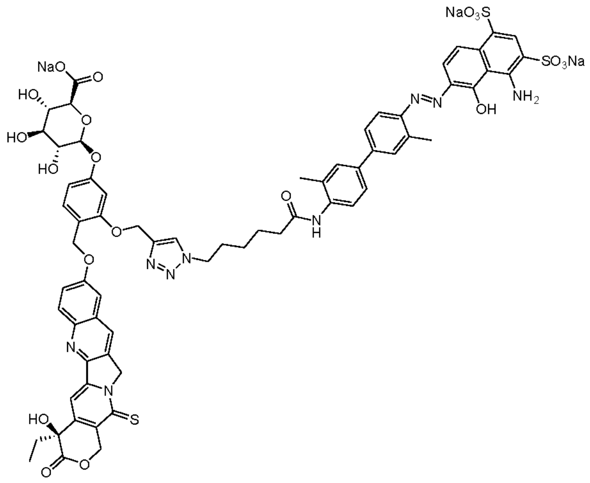 | (2S,3S,4S,5R,6S)-6-(3-((1-(6-((4 '-((E)-(8-amino-1-hydroxy-5,7-di sulfonicnaphthalen-2-yl) diazenyl )-3,3'-dimethyl-[1,1'-biphenyl]-4 -yl) amino)-6-oxohexyl)-1H-1,2,3 -triazol-4-yl) methoxy)-4-(((S)-4-ethyl-4-hydroxy-3-oxy-14-thio-3, 4,12,14-tetrahydro-1H-pyrano[3' ,4':6,7] indolazo[1,2-b]quinolin-9 -yl)oxy)methyl)phenoxy)-3,4,5-tr ihydroxytetrahydro-2H-pyran-2-carboxylate trisodium |
| 83 | 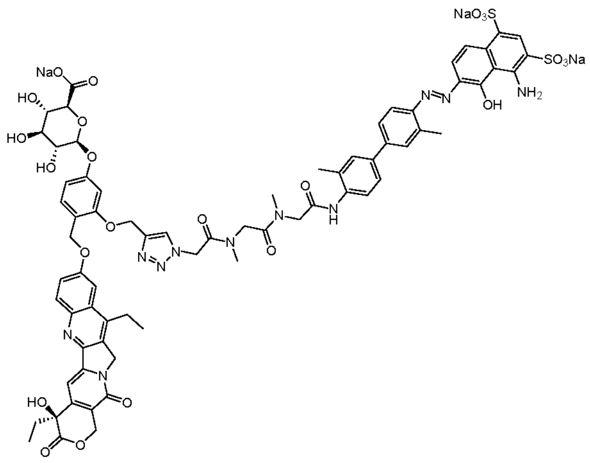 | (2S,3S,4S,5R,6S)-6-(3-((1-((2-(( 2-((2-((4'-(-E)-(8-amino-1-hydrox y-5,7-disulfonicnaphthalen-2-yl) diazenyl)-3,3'-dimethyl-[1,1'-bip henyl]-4-yl)amino)-2-oxoethyl)( methyl)amino)-2-oxoethyl)(meth yl)amino)-2-oxoethyl)-1H-1,2,3-t riazol-4-yl)methoxy)-4-(((S)-4,1 1-diethyl-4-hydroxy-3,14-dioxo-3,4,12,14-tetrahydro-1H-pyrano[ 3',4':6,7] indolazino[1,2-b]quinol in-9-yl)oxy)methyl)phenoxy)-3,4 ,5-trihydroxytetrahydro-2H-pyra n-2-carboxylate trisodium |
| 84 | 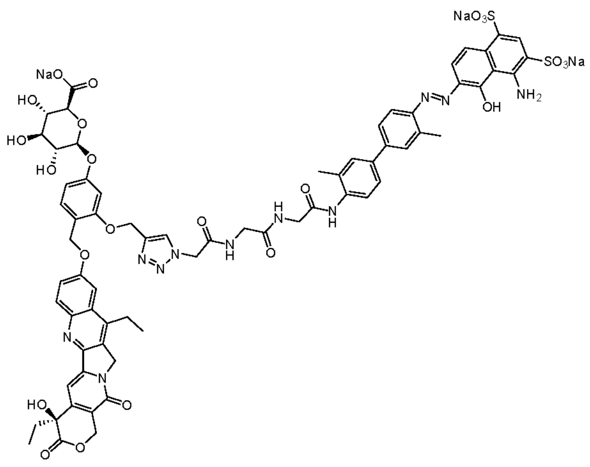 | (2S,3S,4S,5R,6S)-6-(3-((1-((2-(( 2-((2-((4'-(-E)-(8-amino-1-hydrox y-5,7-disulfonicnaphthalen-2-yl) diazenyl)-3,3'-dimethyl-[1,1'-bip henyl]-4-yl)amino)-2-oxoethyl)a mino)-2-oxoethyl)amino)-2-oxoe thyl)-1H-1,2,3-triazol-4-yl)metho xy)-4-((((S)-4,11-diethyl-4-hydro xy-3,14-dioxo-3,4,12,14-tetrahyd ro-1H-pyrano [3',4':6,7]indolazin o[1,2-b]quinolin-9-yl)oxy) methyl )phenoxy)-3,4,5-trihydroxytetrah ydro-2H-pyran-2-carboxylate trisodium |

(continued)

| No. | Structure | Chemical Name |
|---|---|---|
| 85 | 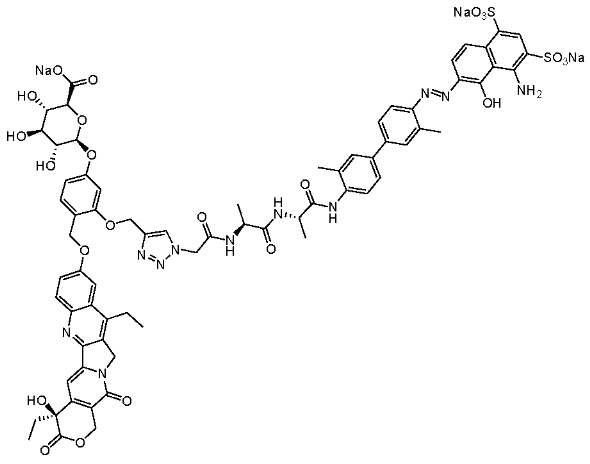 | (2S,3S,4S,5R,6S)-6-(3-((1-(2-((S )-1-((-S)-1-((4'-((E)-(8-amino-1-h ydroxy-5,7-disulfonicnaphthalen-2-yl)diazenyl)-3,3'-dimethyl-[1,1 '-biphenyl]-4-yl)amino)-1-oxopr opan-2-yl)amino)-1-oxopropan-2 -yl)amino)-2-oxoethyl)-1H-1,2,3 -triazol-4-yl)methoxy)-4-((((S)-4, 11-diethyl-4-hydroxy-3,14-dioxo -3,4,12,14-tetrahydro-1H-pyrano [3',4':6,7] indolazino[1,2-b]quino lin-9-yl)oxy)methyl)phenoxy)-3, 4,5-trihydroxytetrahydro-2H-pyr an-2-carboxylate trisodium |
| 86 | 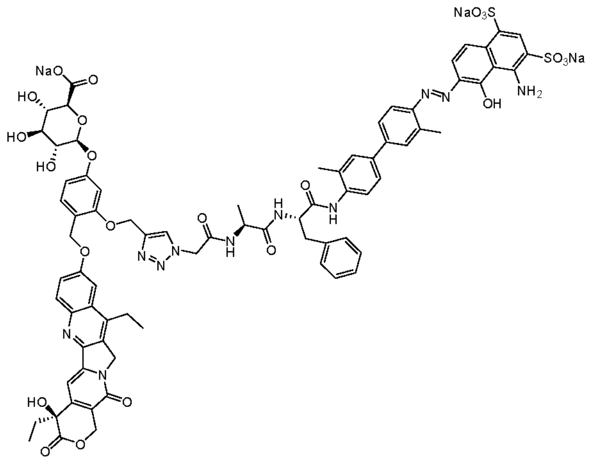 | (2S,3S,4S,5R,6S)-6-(3-((1-(2-((S )-1-((-S)-1-((4'-((E)-(8-amino-1-h ydroxy-5,7-disulfonicnaphthalen-2-yl)diazenyl)-3,3'-dimethyl-[1,1 '-biphenyl]-4-yl)amino)-1-oxo-3-phenylpropan-2-yl)amino)-1-oxo propan-2-yl)amino)-2-oxoethyl)-1H-1,2,3-triazol-4-yl)methoxy)-4 -((((S)-4,11-diethyl-4-hydroxy-3, 14-dioxy-3,4,12,14-tetrahydro-1 H-pyrano[3',4':6,7] indolazino[1, 2-b]quinolin-9-yl)oxy)methyl)ph enoxy)-3,4,5-trihydroxytetrahydr o-2H-pyran-2-carboxylate trisodium |
| 87 | 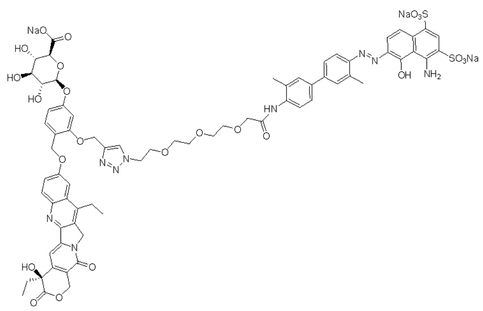 | (2S,3S,4S,5R,6S)-6-(3-((1-(2-(2-(2-(2-((4'-((-E)-(8-amino-1-hydro xy-5,7-disulfonicnaphthalen-2-yl )diazenyl)-3,3'-dimethyl-[1,1-bi phenyl]-4-yl)amino)-2-oxoethox y)ethoxy)ethoxy) ethyl)-1H-1,2,3 -triazol-4-yl)methoxy)-4-((((S)-4, 11-diethyl-4-hydroxy-3,14-dioxy -3,4,12,14-tetrahydro-1H-pyrano [3',4':6,7] indolazino[1,2-b]quino lin-9-yl)oxy)methyl)phenoxy)-3, 4,5-trihydroxytetrahydro-2H-pyr an-2-carboxylate trisodium |
| 88 | 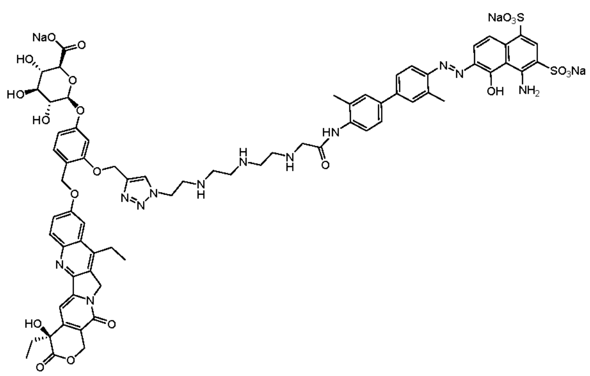 | (2S,3S,4S,5R,6S)-6-(3-((1-((2-((-2-((2-((2-((4'-((E)-(8-amino-1-hy droxy-5,7-disulfonicnaphthalen-2 -yl)diazenyl)-3,3'-dimethyl-[1,1' -biphenyl]-4-yl)amino)-2-oxoeth yl) amino)ethyl)amino)ethyl)amin o) ethyl)-1H-1,2,3-triazol-4-yl)me thoxy)-4-((((S)-4,11-diethyl-4-hy droxy-3,14-dioxy-3,4,12,14-tetra hydro-1H-pyrano[3',4':6,7] indol azino[1,2-b]quinolin-9-yl)oxy)m ethyl) phenoxy)-3,4,5-trihydroxyt etrahydro-2H-pyran-2-carboxylat e trisodium |

(continued)

| No. | Structure | Chemical Name |
|---|---|---|
| 89 | 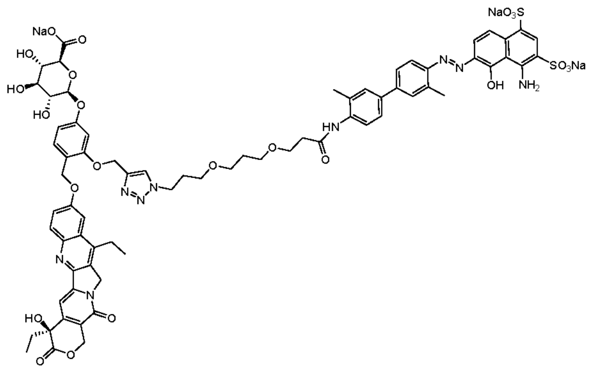 | (2S,3S,4S,5R,6S)-6-(3-((1-(3-(3-(3-((4'-((-E)-(8-amino-1-hydroxy-5,7-disulfonicnaphthalen-2-yl)dia zenyl)-3,3'-dimethyl-[1,1'-biphe nyl]-4-yl)amino)-3-oxopropoxy) propoxy)propyl)-1H-1,2,3-triazol -4-yl)methoxy)-4-(((S)-4,11-diet hyl-4-hydroxy-3,14-dioxy-3,4,12 ,14-tetrahydro-1H-pyrano[3',4':6 ,7] indolazino[1,2-b]quinolin-9-yl )oxy)methyl)phenoxy)-3,4,5-trih ydroxytetrahydro-2H-pyran-2-ca rboxylate trisodium |
| 90 | 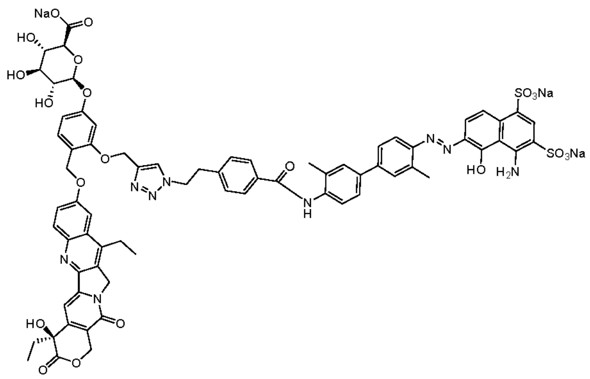 | (2S,3S,4S,5R,6S)-6-(3-((1-(4-((4 '-((E)-(8-amino-1-hydroxy-5,7-di sulfonicnaphthalen-2-yl) diazenyl )-3,3'-dimethyl-[1,1'-biphenyl]-4 -yl) carbamoyl)phenethyl)-1H-1,2 ,3-triazol-4-yl) methoxy)-4-((((S)-4,11-diethyl-4-hydroxy-3,14-dio xy-3,4,12,14-tetrahydro-1H-pyra no [3',4':6,7]indolazino[1,2-b]qui nolin-9-yl)oxy) methyl)phenoxy)-3,4,5-trihydroxytetrahydro-2H-p yran-2-carboxylate trisodium |
| 91 | 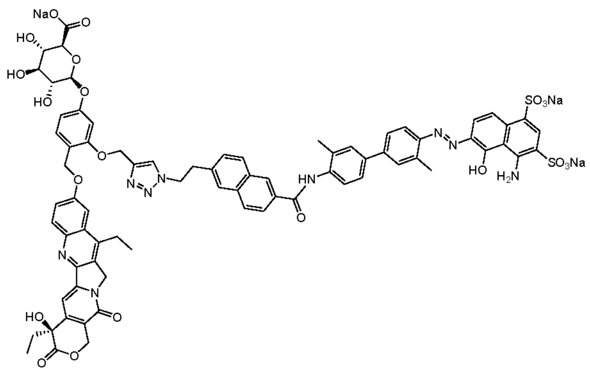 | (2S,3S,4S,5R,6S)-6-(3-((1-(2-(2-((4'-((E)-(8-amino-1-hydroxy-5, 7-disulfonicnaphthalen-2-yl)diaz enyl)-3,3'-dimethyl-[1,1'-biphen yl]-4-yl) carbamoyl)naphthalen-2 -yl)ethyl)-1H-1,2,3-triazol-4-yl) methoxy)-4-((((S)-4,11-diethyl-4 -hydroxy-3,14-dioxy-3,4,12,14-t etrahydro-1H-pyrano[3',4':6,7]in dolazino[1,2-b]quinolin-9-yl) oxy )methyl)phenoxy)-3,4,5-trihydro xytetrahydro-2H-pyran-2-carbox ylate trisodium |
| 92 | 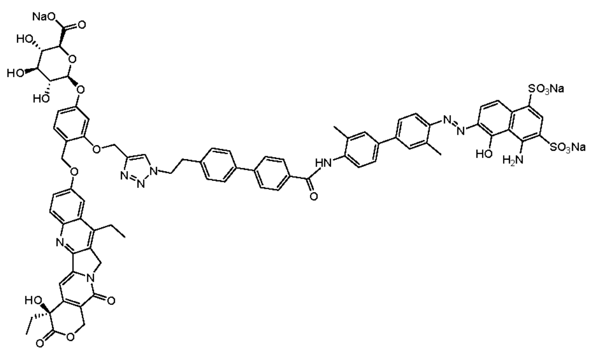 | (2S,3S,4S,5R,6S)-6-(3-((1-(2-(4' -((4'-((E)-(8-amino-1-hydroxy-5, 7-disulfonicnaphthalen-2-yl)diaz enyl)-3,3'-dimethyl-[1,1'-biphen yl]-4-yl) carbamoyl)-[1,1'-biphen yl]-4-yl) ethyl)-1H-1,2,3-triazol-4 -yl)methoxy)-4-((((S)-4,11-dieth yl-4-hydroxy-3,14-dioxy-3,4,12, 14-tetrahydro-1H-pyrano[3',4' :6, 7] indolazino[1,2-b]quinolin-9-yl) oxy)methyl)phenoxy)-3,4,5-trihy droxytetrahydro-2H-pyran-2-car boxylate trisodium |

(continued)

| No. | Structure | Chemical Name |
|---|---|---|
| 93 | 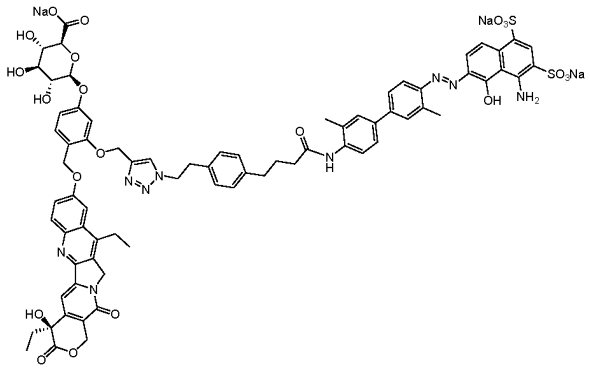 | (2S,3S,4S,5R,6S)-6-(3-((1-(4-(4-((4'-((E)-(8-amino-1-hydroxy-5, 7-disulfonicnaphthalen-2-yl)diaz enyl)-3,3'-dimethyl-[1,1'-biphen yl]-4-yl) amino)-4-oxobutyl)phen ethyl)-1H-1,2,3-triazol-4-yl)meth oxy)-4-((((S)-4,11-diethyl-4-hydr oxy-3,14-dioxy-3,4,12,14-tetrahy dro-1H-pyrano[3',4':6,7]indolazi no[1,2-b]quinolin-9-yl)oxy) meth yl)phenoxy)-3,4,5-trihydroxytetr ahydro-2H-pyran-2-carboxylate trisodium |
| 94 | 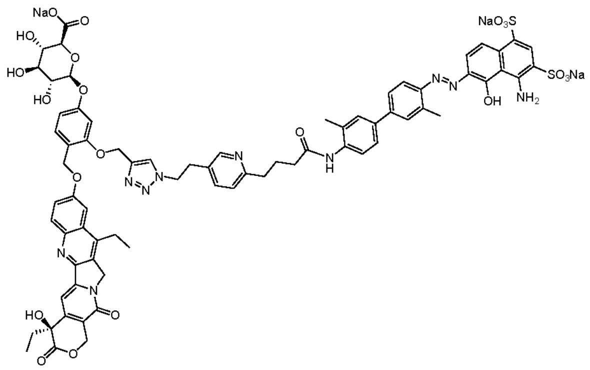 | (2S,3S,4S,5R,6S)-6-(3-((1-(2-(2-(6-(4-((4'-((-E)-(8-amino-1-hydro xy-5,7-disulfonicnaphthalen-2-yl )diazenyl)-3,3'-dimethyl-[1,1-bi phenyl]-4-yl)amino)-4-oxobutyl) pyridin-3-yl) ethyl)-1H-1,2,3-tria zol-4-yl)methoxy)-4-((((S)-4,11-diethyl-4-hydroxy-3,14-dioxy-3, 4,12,14-tetrahydro-1H-pyrano[3' ,4':6,7] indolazino[1,2-b]quinolin -9-yl)oxy)methyl)phenoxy)-3,4,5 -trihydroxytetrahydro-2H-pyran-2-carboxylate trisodium |
| 95 | 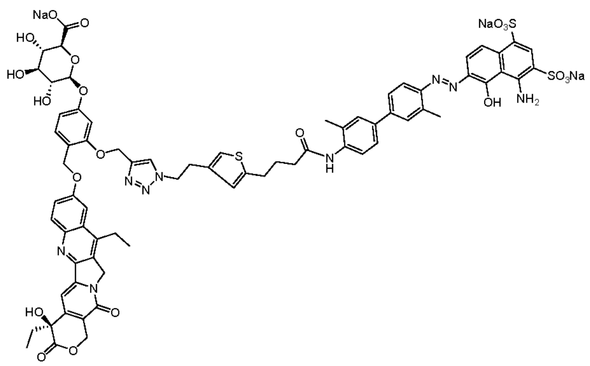 | (2S,3S,4S,5R,6S)-6-(3-((1-(2-(2-(5-(4-((4'-((-E)-(8-amino-1-hydro xy-5,7-disulfonicnaphthalen-2-yl )diazenyl)-3,3'-dimethyl-[1,1-bi phenyl]-4-yl)amino)-4-oxobutyl) thiophen-3-yl) ethyl)-1H-1,2,3-tri azol-4-yl)methoxy)-4-((((S)-4,11 -diethyl-4-hydroxy-3,14-dioxy-3, 4,12,14-tetrahydro-1H-pyrano[3' ,4':6,7] indolazino[1,2-b]quinolin -9-yl)oxy)methyl)phenoxy)-3,4,5 -trihydroxytetrahydro-2H-pyran-2-carboxylate trisodium |
| 96 | 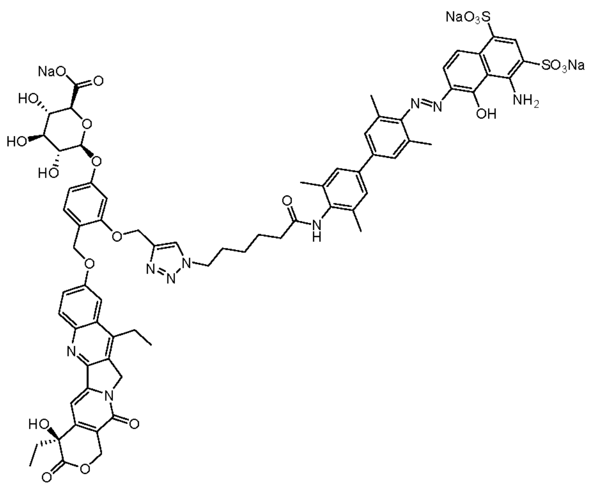 | (2S,3S,4S,5R,6S)-6-(3-((1-(6-((4 '-((E)-(8-amino-1-hydroxy-5,7-di sulfonicnaphthalen-2-yl) diazenyl )-3,3',5,5'-tetramethyl-[1,1'-biph enyl]-4-yl)amino)-6-oxohexyl)-1 H-1,2,3-triazol-4-yl)methoxy)-4-((((S)-4,11-diethyl-4-hydroxy-3, 14-dioxy-3,4,12,14-tetrahydro-1 H-pyrano[3',4':6,7]indolazino[1, 2-b]quinolin-9-yl) oxy)methyl)ph enoxy)-3,4,5-trihydroxytetrahydr o-2H-pyran-2-carboxylate trisodium |

(continued)

| No. | Structure | Chemical Name |
|---|---|---|
| 97 | 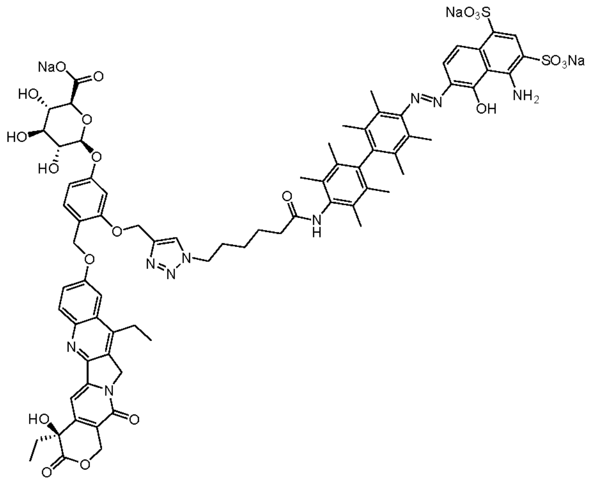 | (2S,3S,4S,5R,6S)-6-(3-((1-(6-((4 '-((E)-(8-amino-1-hydroxy-5,7-di sulfonicnaphthalen-2-yl) diazenyl )-2,2',3,3',5,5',6,6'-octamethyl-[ 1,1'-biphenyl]-4-yl)amino)-6-ox ohexyl)-1H-1,2,3-triazol-4-yl)me thoxy)-4-((((S)-4,11-diethyl-4-hy droxy-3,14-dioxy-3,4,12,14-tetra hydro-1H-pyrano[3',4':6,7]indol azino[1,2-b]quinolin-9-yl) oxy)m ethyl)phenoxy)-3,4,5-trihydroxyt etrahydro-2H-pyran-2-carboxylat e trisodium |
| 98 | 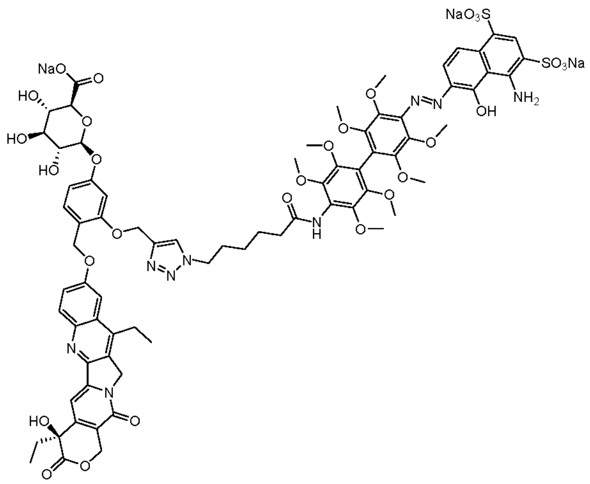 | (2S,3S,4S,5R,6S)-6-(3-((1-(6-((4 '-((E)-(8-amino-1-hydroxy-5,7-di sulfonicnaphthalen-2-yl) diazenyl )-2,2',3,3',5,5',6,6'-octamethoxy -[1,1'-biphenyl]-4-yl)amino)-6-o xohexyl)-1H-1,2,3-triazol-4-yl)m ethoxy)-4-((((S)-4,11-diethyl-4-h ydroxy-3,14-dioxy-3,4,12,14-tetr ahydro-1H-pyrano [3',4':6,7]indo lazino[1,2-b]quinolin-9-yl)oxy)m ethyl)phenoxy)-3,4,5-trihydroxyt etrahydro-2H-pyran-2-carboxylat e trisodium |
| 99 | 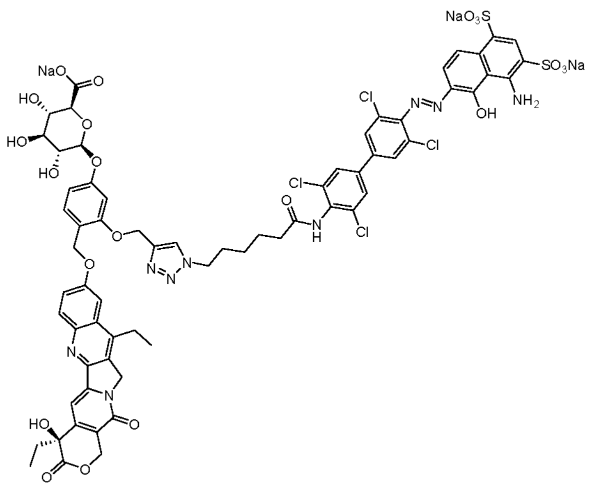 | (2S,3S,4S,5R,6S)-6-(3-((1-(6-((4 '-((E)-(8-amino-1-hydroxy-5,7-di sulfonicnaphthalen-2-yl) diazenyl )-3,3',5,5'-tetrachloro-[1,1'-biph enyl]-4-yl)amino)-6-oxohexyl)-1 H-1,2,3-triazol-4-yl)methoxy)-4-((((S)-4,11-diethyl-4-hydroxy-3, 14-dioxy-3,4,12,14-tetrahydro-1 H-pyrano[3,4':6,7]indolazino[1,2 -b]quinolin-9-yl) oxy)methyl)phe noxy)-3,4,5-trihydroxytetrahydro -2H-pyran-2-carboxylate trisodium |

(continued)

| No. | Structure | Chemical Name |
|---|---|---|
| 100 | 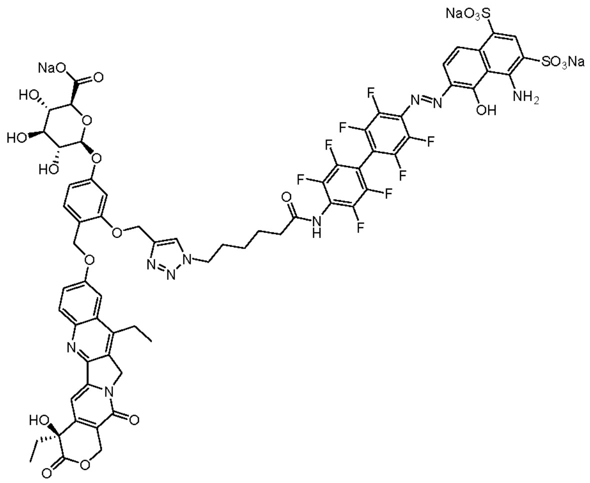 | (2S,3S,4S,5R,6S)-6-(3-((1-(6-((4 '-((E)-(8-amino-1-hydroxy-5,7-di sulfonicnaphthalen-2-yl) diazenyl )-2,2',3,3',5,5',6,6'-octafluoro-[1 ,1'-biphenyl]-4-yl)amino)-6-oxo hexyl)-1H-1,2,3-triazol-4-yl)met hoxy)-4-((((S)-4,11-diethyl-4-hy droxy-3,14-dioxy-3,4,12,14-tetra hydro-1H-pyrano[3',4':6,7]indol azino[1,2-b]quinolin-9-yl) oxy)m ethyl)phenoxy)-3,4,5-trihydroxyt etrahydro-2H-pyran-2-carboxylat e trisodium |
| 101 | 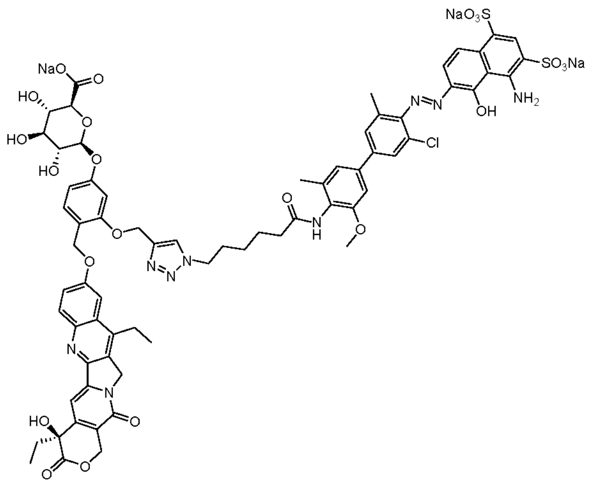 | (2S,3S,4S,5R,6S)-6-(3-((1-(6-((4 '-((E)-(8-amino-1-hydroxy-5,7-di sulfonicnaphthalen-2-yl) diazenyl )-3'-chloro-3-methoxy-5,5'-dime thyl-[1,1'-biphenyl]-4-yl)amino)-6-oxohexyl)-1H-1,2,3-triazol-4-y l)methoxy)-4-((((S)-4,11-diethyl-4-hydroxy-3,14-dioxy-3,4,12,14-tetrahydro-1H-pyrano[3',4' :6,7]i ndolazino[1,2-b]quinolin-9-yl)ox y)methyl)phenoxy)-3,4,5-trihydr oxytetrahydro-2H-pyran-2-carbo xylate trisodium |
| 102 | 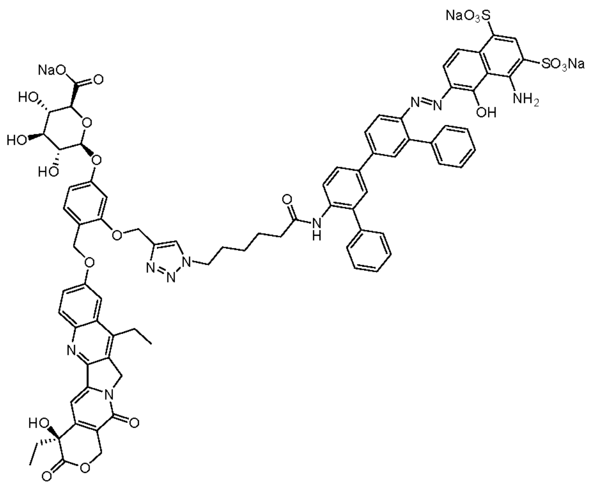 | (2S,3S,4S,5R,6S)-6-(3-((1-(6-((6 '-((E)-(8-amino-1-hydroxy-5,7-di sulfonicnaphthalen-2-yl) diazenyl )-[1,1':3',1":3",1'''-quaterphen yl]-4'-yl)-amino)-6-oxohexyl)-1 H-1,2,3-triazol-4-yl) methoxy)-4-(((((S)-4,11-diethyl-4-hydroxy-3, 14-dioxy-3,4,12,14-tetrahydro-1 H-pyrano [3',4':6,7]indolizino[1, 2-b]quinolin-9-yl)oxy) methyl)ph enoxy)-3,4,5-trihydroxytetrahydr o-2H-pyran-2-carboxylate trisodium |

(continued)

| No. | Structure | Chemical Name |
|---|---|---|
| 103 | 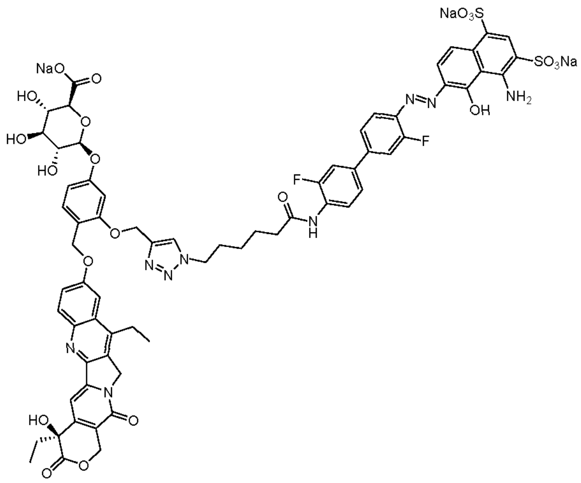 | (2S,3S,4S,5R,6S)-6-(3-((1-(6-((4 '-((E)-(8-amino-1-hydroxy-5,7-di sulfonicnaphthalen-2-yl) diazenyl )-3,3'-difluoro-[1,1'-biphenyl]-4-yl) amino)-6-oxohexyl)-1H-1,2,3-triazol-4-yl)methoxy)-4-(((S)-4,1 1-diethyl-4-hydroxy-3,14-dioxy-3,4,12,14-tetrahydro-1H-pyrano[ 3',4':6,7] indolizino[1,2-b]quinoli n-9-yl)oxy)methyl)phenoxy)-3,4, 5-trihydroxytetrahydro-2H-pyran -2-carboxylate trisodium |
| 104 | 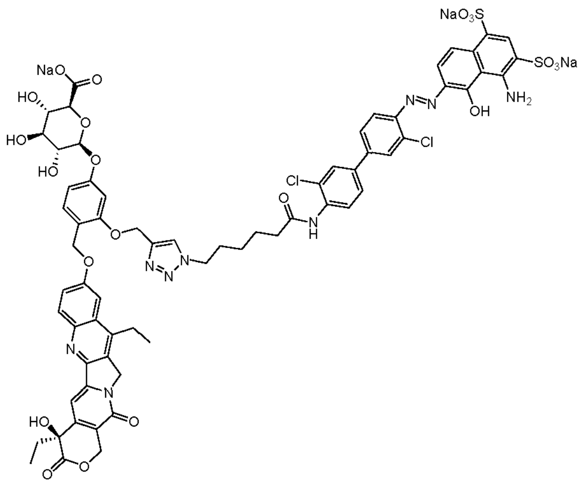 | (2S,3S,4S,5R,6S)-6-(3-((1-(6-((4 '-((E)-(8-amino-1-hydroxy-5,7-di sulfonicnaphthalen-2-yl) diazenyl )-3,3'-dichloro-[1,1'-biphenyl]-4 -yl) amino)-6-oxohexyl)-1H-1,2,3 -triazol-4-yl) methoxy)-4-(((S)-4, 11-diethyl-4-hydroxy-3,14-dioxy -3,4,12,14-tetrahydro-1H-pyrano [3',4':6,7]indolizino[1,2-b]quinol in-9-yl)oxy) methyl)phenoxy)-3,4 ,5-trihydroxytetrahydro-2H-pyra n-2-carboxylate trisodium |
| 105 | 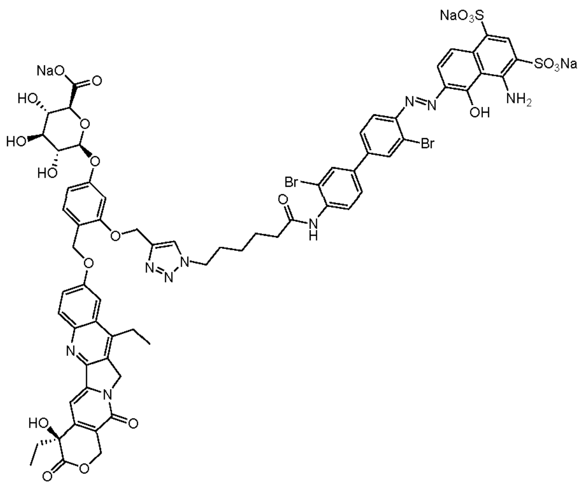 | (2S,3S,4S,5R,6S)-6-(3-((1-(6-((4 '-((E)-(8-amino-1-hydroxy-5,7-di sulfonicnaphthalen-2-yl) diazenyl )-3,3'-dibromo-[1,1'-biphenyl]-4 -yl) amino)-6-oxohexyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolazino[1,2-b]quinolin-9-yl)oxy) methyl)phenoxy)-3, 4,5-trihydroxytetrahydro-2H-pyr an-2-carboxylate trisodium |

(continued)

| No. | Structure | Chemical Name |
|---|---|---|
| 106 | 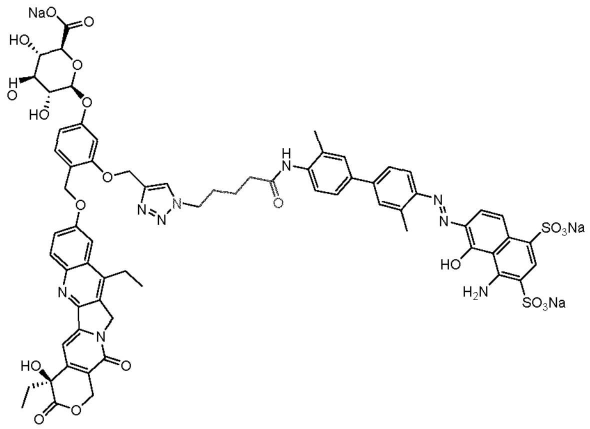 | (2S,3S,4S,5R,6S)-6-(3-((1-(5-((4 '-((E)-(8-amino-1-hydroxy-5,7-di sulfonicnaphthalen-2-yl) diazenyl )-3,3'-dimethyl-[1,1'-biphenyl]-4 -yl) amino)-5-oxopentyl)-1H-1,2, 3-triazol-4-yl) methoxy)-4-((((S)-4,11-diethyl-4-hydroxy-3,14-dio xy-3,4,12,14-tetrahydro-1H-pyra no [3',4':6,7]indolazino[1,2-b]qui nolin-9-yl)oxy) methyl)phenoxy)-3,4,5-trihydroxytetrahydro-2H-p yran-2-carboxylate trisodium |
| 107 | 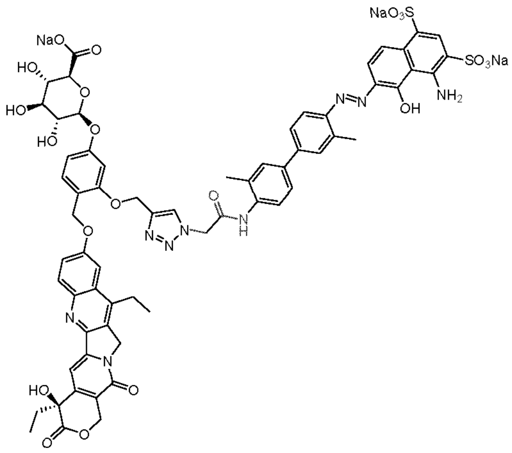 | (2S,3S,4S,5R,6S)-6-(3-((1-(2-((4 '-((E)-(8-amino-1-hydroxy-5,7-di sulfonicnaphthalen-2-yl) diazenyl )-3,3'-dimethyl-[1,1'-biphenyl]-4 -yl) amino)-2-oxoethyl)-1H-1,2,3 -triazol-4-yl) methoxy)-4-((((S)-4, 11-diethyl-4-hydroxy-3,14-dioxy -3,4,12,14-tetrahydro-1H-pyrano [3',4':6,7]indolazino[1,2-b]quino lin-9-yl)oxy) methyl)phenoxy)-3, 4,5-trihydroxytetrahydro-2H-pyr an-2-carboxylate trisodium |
| 108 | 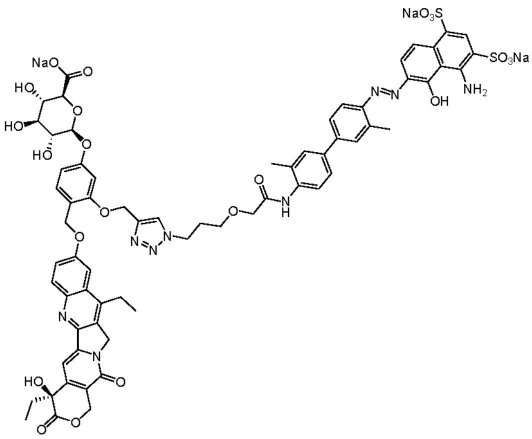 | (2S,3S,4S,5R,6S)-6-(3-((1-(3-(2-((4'-(E)-(8-amino-1-hydroxy-5,7 -disulfonicnaphthalen-2-yl)diaze nyl)-3,3'-dimethyl-[1,1'-bipheny l]-4-yl) amino)-2-oxoethoxy)prop yl)-1H-1,2,3-triazol-4-yl)methox y)-4-((((S)-4,11-diethyl-4-hydrox y-3,14-dioxy-3,4,12,14-tetrahydr o-1H-pyrano[3',4':6,7]indolazin o[1,2-b]quinolin-9-yl) oxy)methyl )phenoxy)-3,4,5-trihydroxytetrah ydro-2H-pyran-2-carboxylate trisodium |

(continued)

| No. | Structure | Chemical Name |
|---|---|---|
| 109 | 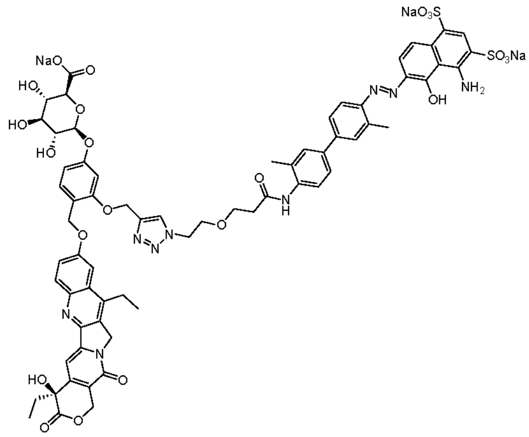 | (2S,3S,4S,5R,6S)-6-(3-((1-(2-(2-((4'-(E)-(8-amino-1-hydroxy-5,7 -disulfonicnaphthalen-2-yl)diaze nyl)-3,3'-dimethyl-[1,1'-bipheny l]-4-yl) amino)-3-oxopropoxy)eth yl)-1H-1,2,3-triazol-4-yl)methox y)-4-((((S)-4,11-diethyl-4-hydrox y-3,14-dioxy-3,4,12,14-tetrahydr o-1H-pyrano[3',4':6,7]indolazin o[1,2-b]quinolin-9-yl) oxy)methyl )phenoxy)-3,4,5-trihydroxytetrah ydro-2H-pyran-2-carboxylate trisodium |

In some embodiments, the compounds of the present disclosure have good water solubility.

In some embodiments, the compounds of the present disclosure have good stability.

The compounds of the present disclosure have good storage stability.

The compounds of the present disclosure have good stability in the circulation of blood in an organism.

In some embodiments, the compounds of the present disclosure have good anticancer activity.

In some embodiments, the compounds of the present disclosure have good safety.

In some embodiments, the compounds of the present disclosure are well tolerated.

In some embodiments, the compounds of the present disclosure have good anticancer activity against large tumors.

In some embodiments, the compounds of the present disclosure have good anticancer activity against tumors, of which the previous therapeutic effects with irinotecan are not good.

## BRIEF DESCRIPTION OF THE DRAWINGS

Fig. 1 shows tumor volume versus time profiles after administration of irinotecan prior to grouping.

## DETAILED DESCRIPTION

In the following description, certain specific details are included to provide a thorough understanding for various disclosed embodiments. One skilled in the relevant art, however, will recognize that the embodiments may be practiced without one or more these specific details, or with other methods, components, materials, etc.

Unless the context required otherwise, throughout the specification and claims which follows, the term "comprise" and variations thereof, such as "comprises" and "comprising" are to be construed in an open, inclusive sense, which is as "include, but not limited to".

Reference throughout this specification to "one embodiment", or "an embodiment", or "in another embodiment", or "in some embodiments" means that a particular referent feature, structure or characteristic described in connection with the embodiments is included in at least one embodiment. Therefore, the appearance of the phrases "in one embodiment", or "in the embodiment", or "in another embodiment", or "in some embodiments" in various places throughout this specification are not necessarily all referring to the same embodiment. Moreover, the particular features, structures or characteristics may be combined in any suitable manner in one or more embodiments.

It should be noted that, as used in this specification and the appended claims, the singular forms "a", "an" and "the" comprise plural referents unless the context clearly stated otherwise.

## Definition

Accordingly, as used in the specification and appended claims, unless specified to the contrary, the following terms have the meanings indicated:

Certain chemical groups named herein are preceded by a shorthand notation indicating the total number of carbon atoms that are to be found in the indicated chemical group. For example, $C_1$-$C_4$ alkyl describes an alkyl group, as defined below,

having a total of 1 to 4 carbon atoms, and $C_3$-$C_{10}$ cycloalkyl describes a cycloalkyl group having a total of 3 to 10 carbon atoms as defined below. The total number of carbon atoms in the shorthand notation does not include the carbons that can exist in the substituents of the groups described.

As used herein, the term "halogen" refers to fluoro, chloro, bromo or iodo.

As used herein, the term "hydroxy: refers to -OH group.

As used herein, the term "amino" refers to $-NH_2$ group.

As used herein, the term "carboxy" refers to -COOH group.

As used herein, the term "cyano" refers to -CN group.

As used herein, the term "nitro" refers to $-NO_2$ group.

As used herein, the term "hydrocarbyl" refers to an aliphatic hydrocarbon group. The hydrocarbyl moiety may be a "saturated hydrocarbyl" group, which means that it does not contain any alkene or alkyne moieties. The hydrocarbyl moiety may also be an "unsaturated hydrocarbyl" moiety, which means that it contains at least one alkene or alkyne moiety. An "alkene" moiety refers to a straight or branched hydrocarbon chain group consisting of from two to eight carbon atoms and at least one carbon-carbon double bond, which is attached to the rest of the molecule by a single bond, e.g., ethenyl, prop-1-enyl, but-1-enyl, pent-1-enyl, pent-1,4-dienyl, and the like. An "alkyne" moiety refers to a straight or branched hydrocarbon chain group consisting of from two to eight carbon atoms and at least one carbon-carbon triple bond, which is attached to the rest of the molecule by a single bond. The alkyl moiety, whether saturated or unsaturated, may be branched chain or straight chain.

The hydrocarbyl group may have 1 to 8 carbon atoms (whenever it appears herein, a numerical range such as "1 to 8" refers to each integer in the given number range; e.g., "1 to 8 carbon atoms" means that the hydrocarbyl group may consist of 1 carbon atom, 2 carbon atoms, 3 carbon atoms, etc., up to and including 8 carbon atoms, although the present definition also covers the occurrence of term "hydrocarbyl" where no numerical range is designated).

The hydrocarbyl group may be optionally substituted, *i.e.* substituted or unsubstituted. When substituted, the substituted group(s) is(are) individually and independently selected from the group consisting of cyclohydrocarbyl, aryl, heteroaryl, heteroalicyclyl, hydroxy, hydrocarbyloxy, aryloxy, mercapto, allkylthio, arylthio, cyano, halo, carbonyl, thiocarbonyl, O-carbamyl, N-carbamyl, O-thiocarbamyl, N-thiocarbamyl, C-amido, N-amido, S-sulfinamido, N-sulfinamido, C-carboxyl, O-carboxyl, isocyanato, thiocyano, isothiocyanato, nitro, silyl, trihalomethanesulfonyl, -NR'R''(R' and R'' are hydrocarbyl group defined herein) or amino comprising mono- and bi-substituted amino group, and the protected derivatives thereof. Typical hydrocarbyl groups comprise, but are not limited to, methyl, ethyl, propyl, isopropyl, butyl, isobutyl, t-butyl, pentyl, hexyl, ethenyl, propenyl, butenyl, ethynyl, propynyl and butynyl. Whenever a substituent is described as being "optionally substituted", that substituent may be substituted with one of the above substituents.

In some embodiments, "$C_1$-$C_4$ hydrocarbyl" refers to a hydrocarbyl group as defined above containing one to four carbon atoms. The $C_1$-$C_4$ hydrocarbyl group may be optionally substituted as defined for a hydrocarbyl group.

In some embodiments, "$C_1$-$C_6$ hydrocarbyl" refers to a hydrocarbyl group as defined above containing one to six carbon atoms. The $C_1$-$C_6$ hydrocarbyl group may be optionally substituted as defined for a hydrocarbyl group.

In some embodiments, "$C_1$-$C_{12}$ hydrocarbyl" refers to a hydrocarbyl group as defined above containing one to twelve carbon atoms. The $C_1$-$C_{12}$ hydrocarbyl group may be optionally substituted as defined for a hydrocarbyl group.

In some embodiments, "$C_2$-$C_6$ hydrocarbyl" refers to a hydrocarbyl group as defined above containing two to six carbon atoms. The $_{C2}$-$C_6$ hydrocarbyl group may be optionally substituted as defined for a hydrocarbyl group.

In some embodiments, "$C_3$-$C_6$ hydrocarbyl" refers to a hydrocarbyl group as defined above containing three to six carbon atoms. The $C_3$-$C_6$ hydrocarbylgroup may be optionally substituted as defined for a hydrocarbyl group.

In some embodiments, "$C_3$-$C_{12}$ hydrocarbyl" refers to a hydrocarbyl group as defined above containing three to twelve carbon atoms. The $C_3$-$C_{12}$ hydrocarbyl group may be optionally substituted as defined for a hydrocarbyl group.

In some embodiments, "$C_6$-$C_{12}$ hydrocarbyl" refers to a hydrocarbyl group as defined above containing six to twelve carbon atoms. The $C_6$-$C_{12}$ hydrocarbyl group may be optionally substituted as defined for a hydrocarbyl group.

In some embodiments, "$C_7$-$C_{12}$ hydrocarbyl" refers to a hydrocarbyl group as defined above containing seven to twelve carbon atoms. The $C_7$-$C_{12}$ hydrocarbyl group may be optionally substituted as defined for a hydrocarbyl group.

As used herein, "hydrocarbyloxy" refers to the formula -O-hydrocarbyl, wherein R is a hydrocarbyl group defined as above, e.g., methoxy, ethoxy, n-propoxy, 1-methyl ethoxy (isopropoxy), n-butoxy, iso-butoxy, sec-butoxy, t-butoxy, amoxy, and t-amoxy.

As used herein, the term "aryl" refers to a carbocycle (full carbon) or two or more fused rings (rings sharing with two adjacent carbon atoms), having completely delocalized Pi electron system. Examples of aryl group include, but are not limited to, fluorenyl, phenyl and naphthyl. The aryl group may have, for example, five to twelve carbon atoms. The aryl group of the present disclosure may be substituted or unsubstituted. Where substituted, hydrogen atom(s) is(are) substituted with one or more substituents independently selected from the group consisting of hydrocarbyl, cyclohydrocarbyl, aryl, heteroaryl, heteroalicyclyl, hydroxy, protected hydroxy, hydrocarbyloxy, aryloxy, mercapto, hydrocarbylthio, arylthio, cyano, halo, carbonyl, thiocarbonyl, O-carbamyl, N-carbamyl, O-thiocarbamyl, N-thiocarbamyl, C-amido, N-amido, S-sulfinamido, N-sulfinamido, C-carboxyl, protected C-carboxyl, O-carboxyl, isocyanato, thiocyano, isothiocyanato, nitro, silyl, trihalomethanesulfonyl, -NR'R'' (R' and R'' are hydrocarbyl groups defined herein) and protected amino. Whenever a substituent is described as being "optionally substituted", that substituent may be substituted with one of the above substituents.

As used herein, the term "arylidene" refers to a carbocycle (full carbon) or two or more fused rings (rings sharing with two adjacent carbon atoms), having completely delocalized Pi electron system. Examples of arylidene group include, but are not limited to, fluorenyliden, phenylidene and naphthylidene. The arylidene group may have, for example, five to twelve carbon atoms. The arylidene group of the present disclosure may be substituted or unsubstituted. Where substituted, hydrogen atom(s) is(are) substituted with one or more substituents independently selected from the group consisting of hydrocarbyl, cyclohydrocarbyl, aryl, heteroaryl, heteroalicyclyl, hydroxy, protected hydroxy, hydrocarbyloxy, aryloxy, mercapto, hydrocarbylthio, arylthio, cyano, halo, carbonyl, thiocarbonyl, O-carbamyl, N-carbamyl, O-thiocarbamyl, N-thiocarbamyl, C-amido, N-amido, S-sulfinamido, N-sulfinamido, C-carboxyl, protected C-carboxyl, O-carboxyl, isocyanato, thiocyano, isothiocyanato, nitro, silyl, trihalomethanesulfonyl, -NR'R'' (R' and R" are hydrocarbyl groups defined herein) and protected amino. Whenever a substituent is described as being "optionally substituted", that substituent may be substituted with one of the above substituents. The arylidene may be attached to the rest of the molecule and to the residue group through one carbon in the group or through any two carbons in the group.

As used herein, the term "aromatic ring group" refers to a carbocycle (full carbon) or two or more fused rings (rings sharing with two adjacent carbon atoms), having completely delocalized Pi electron system. Examples of aryl group include, but are not limited to, fluorenyl, phenyl and naphthyl. The aryl group may have, for example, five to twelve carbon atoms. The aromatic ring group of the present disclosure may be substituted or unsubstituted. Where substituted, hydrogen atom(s) is(are) substituted with one or more substituents independently selected from the group consisting of hydrocarbyl, cyclohydrocarbyl, aryl, heteroaryl, heteroalicyclyl, hydroxy, protected hydroxy, hydrocarbyloxy, aryloxy, mercapto, hydrocarbylthio, arylthio, cyano, halo, carbonyl, thiocarbonyl, O-carbamyl, N-carbamyl, O-thiocarbamyl, N-thiocarbamyl, C-amido, N-amido, S-sulfinamido, N-sulfinamido, C-carboxyl, protected C-carboxyl, O-carboxyl, isocyanato, thiocyano, isothiocyanato, nitro, silyl, trihalomethanesulfonyl, -NR'R'' (R' and R" are hydrocarbyl groups defined herein) and protected amino. Whenever a substituent is described as being "optionally substituted", that substituent may be substituted with one of the above substituents.

As used herein, the term "arylhydrocarbyloxy" refers to the formula -O-hydrocarbyl aryl, wherein hydrocarbyl and aryl are respectively as defined in the present disclosure. Exemplary examples of arylhydrocarbyloxy include, but are not limited to, benzyloxy, phenethyloxy, and phenylpropyloxy.

In some embodiments, "$C_6$-$C_{16}$ arylhydrocarbyloxy" refers to an arylhydrocarbyloxy group as defined above containing six to sixteen carbon atoms. The $C_6$-$C_{16}$ arylhydrocarbyloxy group may be optionally substituted as defined for a hydrocarbyl group and an aryl group, respectively.

In some embodiments, "$C_6$-$C_{18}$ arylhydrocarbyloxy" refers to an arylhydrocarbyloxy group as defined above containing six to eighteen carbon atoms. The $C_6$-$C_{18}$ arylhydrocarbyloxy group may be optionally substituted as defined for a hydrocarbyl group and an aryl group, respectively.

In some embodiments, "$C_7$-$C_{18}$ arylhydrocarbyloxy" refers to an arylhydrocarbyloxy group as defined above containing seven to eighteen carbon atoms. The $C_7$-$C_{18}$ arylhydrocarbyloxy group may be optionally substituted as defined for a hydrocarbyl group and an aryl group, respectively.

In some embodiments, "$C_8$-$C_{18}$ arylhydrocarbyloxy" refers to an arylhydrocarbyloxy group as defined above containing eight to eighteen carbon atoms. The $C_8$-$C_{18}$ arylhydrocarbyloxy group may be optionally substituted as defined for a hydrocarbyl group and an aryl group, respectively.

In some embodiments, "$C_8$-$C_{24}$ arylhydrocarbyloxy" refers to an arylhydrocarbyloxy group as defined above containing eight to twenty-four carbon atoms. The $C_8$-$C_{24}$ arylhydrocarbyloxy group may be optionally substituted as defined for a hydrocarbyl group and an aryl group, respectively.

In some embodiments, "$C_{11}$-$C_{24}$ arylhydrocarbyloxy" refers to an arylhydrocarbyloxy group as defined above containing eleven to twenty-four carbon atoms. The $C_{11}$-$C_{24}$ arylhydrocarbyloxy group may be optionally substituted as defined for a hydrocarbyl group and an aryl group, respectively.

In some embodiments, "$C_{12}$-$C_{24}$ arylhydrocarbyloxy" refers to an arylhydrocarbyloxy group as defined above containing twelve to twenty-four carbon atoms. The $C_{12}$-$C_{24}$ arylhydrocarbyloxy group may be optionally substituted as defined for a hydrocarbyl group and an aryl group, respectively.

In some embodiments, "$C_6$-$C_{24}$ arylhydrocarbyloxy" refers to an arylhydrocarbyloxy group as defined above containing six to twenty-four carbon atoms. The $C_6$-$C_{24}$ arylhydrocarbyloxy group may be optionally substituted as defined for a hydrocarbyl group and an aryl group, respectively.

As used herein, the term "heteroaryl" refers to a five- to eighteen-membered aromatic ring group, containing one to seventeen carbon atoms and one to ten heteroatoms selected from the group consisting of nitrogen, oxygen and sulphur. In some embodiments, the heteroaryl may be monocyclic, bicyclic, tricyclic or tetracyclic ring system, which may comprise fused or bridged ring system. Moreover, nitrogen, carbon or sulphur atom in the heteroaryl group may be optionally oxidized, and the nitrogen atom may be optionally quaternized. Exemplary examples of heteroaryl include, but

are not limited to, azepinyl, acridinyl, benzimidazolyl, benzothiazolyl, benzoindolyl, benzodioxolanyl, benzofuranyl, benzoxazolyl, benzothiazolyl, benzothiadiazolyl, benzo[b][1,4]dioxepanyl, 1,4-benzodioxanyl, benzonaphthofuranyl, benzodioxolanyl, benzodioxadienyl, benzopyranyl, benzopyronyl, benzofuranyl, benzofuranonyl, benzothienyl, benzotriazolyl, benzo[4,6]imidazo[1,2-a]pyridyl, carbazolyl, cinnolinyl, dibenzofuranyl, dibenzothienyl, furanyl, furanonyl, isothiazolyl, imidazolyl, indazolyl, indolyl, indazolyl, isoindolyl, indolinyl, isoindolinyl, isoquinolyl, indolizinyl, isoxazolyl, naphthyl, naphthyridinyl, oxadiazolyl, 2-oxoazepinyl, oxazolyl, oxiranyl, 1-phenyl-1H-pyrrolyl, phenazinyl, phenothiazinyl, phenoxazinyl, phthalazinyl, pteridinyl, purinyl, pyrrolyl, pyrazolyl, pyridinyl, pyrazinyl, pyrimidinyl, pyridazinyl, pyrrolyl, quinazolinyl, quinoxalinyl, quinolyl, quinuclidinyl, isoquinolyl, tetrahydroquinolyl, thiazolyl, thiadiazolyl, triazolyl, tetrazolyl, triazinyl and thiophenyl. The heteroaryl group of the present disclosure may be substituted or unsubstituted. Where substituted, hydrogen atom(s) is(are) substituted with one or more substituents independently selected from the group consisting of hydrocarbyl, cyclohydrocarbyl, aryl, heteroaryl, heteroalicyclyl, hydroxy, protected hydroxy, hydrocarbyloxy, aryloxy, mercapto, hydrocarbylthio, arylthio, cyano, halo, carbonyl, thiocarbonyl, O-carbamyl, N-carbamyl, O-thiocarbamyl, N-thiocarbamyl, C-amido, N-amido, S-sulfinamido, N-sulfinamido, C-carboxyl, protected C-carboxyl, O-carboxyl, isocyanato, thiocyano, isothiocyanato, nitro, silyl, trihalomethanesulfonyl, -NR'R'' (R' and R'' are hydrocarbyl groups defined herein) and protected amino. Whenever a substituent is described as being "optionally substituted", that substituent may be substituted with one of the above substituents.

As used herein, the term "heteroarylidene" refers to a five- to eighteen-membered arylidene group, containing one to seventeen carbon atoms and one to ten heteroatoms selected from the group consisting of nitrogen, oxygen and sulphur. In some embodiments, the heteroarylidene may be monocyclic, bicyclic, tricyclic or tetracyclic ring system, which may comprise fused or bridged ring system. Moreover, nitrogen, carbon or sulphur atom in the heteroarylidene group may be optionally oxidized, and the nitrogen atom may be optionally quaternized. Exemplary examples of heteroarylidene include, but are not limited to, azepinylidene, acridinylidene, benzimidazolylidene, benzothiazolylidene, benzoindolylidene, benzodioxolanylidene, benzofuranylidene, benzoxazolylidene, benzothiazolylidene, benzothiadiazolylidene, benzo[b][1,4]dioxepanylidene, 1,4-benzodioxanylidene, benzonaphthofuranylidene, benzodioxolanylidene, benzodioxadienylidene, benzopyranylidene, benzopyronylidene, benzofuranylidene, benzofuranonylidene, benzothienylidene, benzotriazolylidene, benzo[4,6]imidazo[1,2-a]pyridylidene, carbazolylidene, cinnolinylidene, dibenzofuranylidene, dibenzothienylidene, furanylidene, furanonylidene, isothiazolylidene, imidazolylidene, indazolylidene, indolylidene, indazolylidene, isoindolylidene, indolinylidene, isoindolinylidene, isoquinolylidene, indolizinylidene, isoxazolylidene, naphthylidene, naphthyridinylidene, oxadiazolylidene, 2-oxoazepinylidene, oxazolylidene, oxiranylidene, 1-phenyl-1H-pyrrolylidene, phenazinylidene, phenothiazinylidene, phenoxazinylidene, phthalazinylidene, pteridinylidene, purinylidene, pyrrolylidene, pyrazolylidene, pyridinylidene, pyrazinylidene, pyrimidinylidene, pyridazinylidene, pyrrolylidene, quinazolinylidene, quinoxalinylidene, quinolylidene, quinuclidinylidene, isoquinolylidene, tetrahydroquinolylidene, thiazolylidene, thiadiazolylidene, triazolylidene, tetrazolylidene, triazinylidene and thiophenylidene. The heteroarylidene group of the present disclosure may be substituted or unsubstituted. Where substituted, hydrogen atom(s) is(are) substituted with one or more substituents independently selected from the group consisting of hydrocarbyl, cyclohydrocarbyl, aryl, heteroaryl, heteroalicyclyl, hydroxy, protected hydroxy, hydrocarbyloxy, aryloxy, mercapto, hydrocarbylthio, arylthio, cyano, halo, carbonyl, thiocarbonyl, O-carbamyl, N-carbamyl, O-thiocarbamyl, N-thiocarbamyl, C-amido, N-amido, S-sulfinamido, N-sulfinamido, C-carboxyl, protected C-carboxyl, O-carboxyl, isocyanato, thiocyano, isothiocyanato, nitro, silyl, trihalomethanesulfonyl, -NR'R'' (R' and R'' are hydrocarbyl groups defined herein) and protected amino. Whenever a substituent is described as being "optionally substituted", that substituent may be substituted with one of the above substituents.

As used herein, the term "hetero aromatic ring group" refers to a five- to eighteen-membered aromatic ring group, containing one to seventeen carbon atoms and one to ten heteroatoms selected from the group consisting of nitrogen, oxygen and sulphur. In some embodiments, the hetero aromatic ring group may be monocyclic, bicyclic, tricyclic or tetracyclic ring system, which may comprise fused or bridged ring system. Moreover, nitrogen, carbon or sulphur atom in the heteroaryl group may be optionally oxidized, and the nitrogen atom may be optionally quaternized. Exemplary examples of hetero aromatic ring group include, but are not limited to, azepinyl, acridinyl, benzimidazolyl, benzothiazolyl, benzoindolyl, benzodioxolanyl, benzofuranyl, benzoxazolyl, benzothiazolyl, benzothiadiazolyl, benzo[b][1,4]dioxepanyl, 1,4-benzodioxanyl, benzonaphthofuranyl, benzodioxolanyl, benzodioxadienyl, benzopyranyl, benzopyronyl, benzofuranyl, benzofuranonyl, benzothienyl, benzotriazolyl, benzo[4,6]imidazo[1,2-a]pyridyl, carbazolyl, cinnolinyl, dibenzofuranyl, dibenzothienyl, furanyl, furanonyl, isothiazolyl, imidazolyl, indazolyl, indolyl, indazolyl, isoindolyl, indolinyl, isoindolinyl, isoquinolyl, indolizinyl, isoxazolyl, naphthyl, naphthyridinyl, oxadiazolyl, 2-oxoazepinyl, oxazolyl, oxiranyl, 1-phenyl-1H-pyrrolyl, phenazinyl, phenothiazinyl, phenoxazinyl, phthalazinyl, pteridinyl, purinyl, pyrrolyl, pyrazolyl, pyridinyl, pyrazinyl, pyrimidinyl, pyridazinyl, pyrrolyl, quinazolinyl, quinoxalinyl, quinolyl, quinuclidinyl, isoquinolyl, tetrahydroquinolyl, thiazolyl, thiadiazolyl, triazolyl, tetrazolyl, triazinyl and thiophenyl. The hetero aromatic ring group group of the present disclosure may be substituted or unsubstituted. Where substituted, hydrogen atom(s) is(are) substituted with one or more substituents independently selected from the group consisting of hydrocarbyl, cyclohydrocarbyl, aryl, heteroaryl, heteroalicyclyl, hydroxy, protected hydroxy, hydrocarbyloxy, aryloxy, mercapto, hydrocarbylthio, arylthio, cyano, halo,

carbonyl, thiocarbonyl, O-carbamyl, N-carbamyl, O-thiocarbamyl, N-thiocarbamyl, C-amido, N-amido, S-sulfinamido, N-sulfinamido, C-carboxyl, protected C-carboxyl, O-carboxyl, isocyanato, thiocyano, isothiocyanato, nitro, silyl, trihalomethanesulfonyl, -NR'R'' (R' and R'' are hydrocarbyl groups defined herein) and protected amino. Whenever a substituent is described as being "optionally substituted", that substituent may be substituted with one of the above substituents.

As used herein, the term "cyclohydrocarbyl" refers to stable non-aromatic monocyclic or bicyclohydrocarbyls composed of only carbon and hydrogen atoms, having three to fifteen carbon atoms, and in some embodiments having three to twelve carbon atoms, which are saturated or unsaturated and connected to the rest of the molecule through a single bond, such as cyclopropyl, cyclobutyl, cyclopentyl, cyclohexyl, cyclodecyl, *etc.* Unless stated otherwise specifically in the specification, the term "cyclohydrocarbyl" is meant to include the cyclohydrocarbyl groups as defined above, which may be optionally substituted with one or more substituents selected from the group consisting of cyclohydrocarbyl, aryl, heteroaryl, heteroalicyclyl, hydroxy, hydrocarbyloxy, aryloxy, mercapto, hydrocarbylthio, arylthio, cyano, halo, carbonyl, thiocarbonyl, O-carbamyl, N-carbamyl, O-thiocarbamyl, N-thiocarbamyl, C-amido, N-amido, S-sulfinamido, N-sulfinamido, C-carboxyl, O-carboxyl, isocyanato, thiocyanato, isothiocyanato, nitro, silyl, trihalomethanesulfonyl, -NR'R'' (R' and R'' are hydrocarbyl groups defined in the present disclosure) or amino comprising mono- and di-substituted amino group, and the protected derivatives thereof.

In some embodiments, "$C_3$-$C_6$ cyclohydrocarbyl" refers to a cyclohydrocarbyl group as defined above containing three to six carbon atoms. The $C_3$-$C_6$ cyclohydrocarbyl group may be optionally substituted as defined above for a cyclohydrocarbyl group.

In some embodiments, "$C_3$-$C_{10}$ cyclohydrocarbyl" refers to a cyclohydrocarbyl group as defined above containing three to ten carbon atoms. The $C_3$-$C_{10}$ cyclohydrocarbyl group may be optionally substituted as defined above for a cyclohydrocarbyl group.

In some embodiments, "$C_3$-$C_{12}$ cyclohydrocarbyl" refers to a cyclohydrocarbyl group as defined above containing three to twelve carbon atoms. The $C_3$-$C_{12}$ cyclohydrocarbyl group may be optionally substituted as defined above for a cyclohydrocarbyl group.

As used herein, the term "alkylidene" or "hydrocarbyldiene" refers to a divalent hydrocarbon chain consisting solely of carbon and hydrogen, which may be linear, branched, or cyclic alkyl. That is, a linear or branched divalent hydrocarbon chain bonded to other part of the molecule and a residue, such as methylidene, ethylidene, propylidene, n-butylidene, vinylene, propenylene, and n-butenylene. The chain of the alkylidene group is linked to the rest of the molecule and the residue by one carbon contained in the chain or any two carbons contained in the chain.

As used herein, the term "$C_1$-$C_9$ alkylidene" refers to a bifunctional linear, branched, or cyclic alkyl group containing one to nine carbon atoms. That is, "$C_1$-$C_9$ alkylidene" refers to a straight or branched divalent hydrocarbon chain consisting solely of carbon and hydrogen and containing one to nine carbon atoms bonded to other part of the molecule and a residue, such as methylidene, ethylidene, propylidene, n-butylidene, vinylene, propenylene, and n-butenylene. The chain of the alkylidene group is linked to the rest of the molecule and the residue by one carbon contained in the chain or any two carbons contained in the chain.

As used herein, the term "alkylideneoxy" or "hydrocarbyldieneoxy" refers to the formula alkylidene-O-, wherein alkylidene or hydrocarbylidene is as defined in the present disclosure. Exemplary examples of alkylideneoxy include, but are not limited to, methylideneoxy, ethylideneoxy, and n-propylideneoxy.

As used herein, the term "$C_1$-$C_9$ alkylideneoxy" or "$C_1$-$C_9$ hydrocarbyldieneoxy" refers to a bifunctional linear, branched, or cyclic alkoxy group containing one to nine carbon atoms.

As used herein, the term "$C_1$-$C_8$ alkylideneoxy" or "$C_1$-$C_8$ hydrocarbyldieneoxy" refers to a bifunctional linear, branched, or cyclic alkoxy group containing one to eight carbon atoms.

As used herein, the term "cyclohydrocarbyloxy" refers to -O-cyclohydrocarbyl, wherein cyclohydrocarbyl is as defined in the present disclosure. Exemplary examples of cyclohydrocarbyloxy include, but are not limited to, cyclopropyloxy, cyclobutyloxy, cyclopentyloxy, cyclohexyloxy, and cyclodecyloxy.

As used herein, the term "hydrocarbylcarbonylamino" refers to -NHC(=O)-hydrocarbyl group, wherein hydrocarbyl is as defined in the present disclosure. Exemplary examples of hydrocarbylcarbonylamino include, but are not limited to, acetamido, propionamido, butyramido, and valeramido.

As used herein, the term "hydrocarbylcarbonyloxy" refers to -OC(=O)-hydrocarbyl group, wherein hydrocarbyl is as defined in the present disclosure. Exemplary examples of hydrocarbylcarbonyloxy include, but are not limited to, acetoxy, propionyloxy, butyryloxy, and valeryloxy.

As used herein, the term "hydrocarbyloxycarbonylamino" refers to -NHC(=O)O-hydrocarbyl group, wherein hydrocarbyl is as defined in the present disclosure. Exemplary examples of hydrocarbyloxycarbonylamino include, but are not limited to, methoxycarbonylamino, ethoxycarbonylamino, propoxycarbonylamino, and tert-butoxycarbonylamino.

As used herein, the term "hydrocarbyloxycarbonyl" refers to -C(=O)O-hydrocarbyl, wherein hydrocarbyl is as defined in the present disclosure. Exemplary examples of hydrocarbyloxycarbonyl include, but are not limited to, methoxycarbonyl, ethoxycarbonyl, propoxycarbonyl, isopropoxycarbonyl, and butoxycarbonyl.

As used herein, the term "carbonylamino" refers to -C(=O)-NH-.

As used herein, the term "a compound of the present disclosure, a stereoisomer thereof, or a pharmaceutically acceptable salt thereof" refers to a compound of formula (I), a stereoisomer thereof of the present disclosure.

As used herein, the term "mammal" means animals including, for example, dogs, cats, cows, sheep, horses, and humans. In some embodiments, mammals include humans.

As used herein, the term "patient" means an animal, such as a human, a companion animal, such as a dog, cat and horse, and livestock, such as cattle, swine and sheep. In some embodiments, patients are mammals, including both males and females. In some embodiments, patients are humans.

As used herein, the term "pharmaceutically acceptable" as used herein means the carrier, vehicle, diluent, excipient and/or salt must be compatible with the other ingredients of the formulation, and not deleterious to the recipient thereof.

As used herein, the term "optional" or "optionally" means that the subsequently described event of circumstances may or may not occur, and that the description includes instances where said event or circumstance occurs and instances in which it does not.

As used herein, the term "pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isosmotic agent, solvent, or emulsifier, etc, which has been approved by the United States Food and Drug Administration as being acceptable for use in humans or animals and have no side effects on preparing a pharmaceutical composition.

As used herein, The term "carrier" defines a compound that facilitates the incorporation of a compound into cells or tissues. For example, dimethylsulfoxide (DMSO) is generally used as a carrier, as it facilitates the uptake of many organic compounds into cells or tissues of an organism.

As used herein, the term "pharmaceutically acceptable salts" include both "pharmaceutically acceptable acid addition salts" and "pharmaceutically acceptable base addition salts".

As used herein, the term "pharmaceutically acceptable acid addition salt" refers to those salts which retain the biological effectiveness and properties of the free bases, which are not biologically or otherwise undesirable, and which are formed with inorganic acids such as, but not limited to hydrochloric acid, hydrobromic acid, sulfuric acid, nitric acid, phosphoric acid and the like, and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphanic acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleinic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecylenic acid and the like.

As used herein, the term "Pharmaceutically acceptable base addition salt" refers to those salts which retain the biological effectiveness and properties of the free acids, which are not biologically or otherwise undesirable. These salts are prepared from addition of an inorganic or an organic base to the free acid. Salts derived from inorganic bases include, but are not limited, to sodium, potassium, lithium, ammonium, calcium, magnesium, iron, zinc, copper, manganese, aluminium salts and the like. In some embodiments, inorganic salts are the ammonium, sodium, potassium, calcium, and magnesium salts. Salts derived from organic bases include, but are not limited to, salts of primary, secondary and tertiary amines, substituted amines including naturally occurring substituted amines, cyclic amines and s basic ion exchange resins, such as ammonia, isopropylamine, trimethylamine, diethylamine, triethylamine, tripropylamine, diethanolamine, ethanol amine, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucosamine, theobromine, triethanolamine, trometamol, purine, piperazine, piperidine, N-ethyl piperidine, polyamine resins and the like. In some embodiments, organic bases are isopropylamine, diethylamine, ethanolamine, trimethylamine, dicyclohexylamine, choline and caffeine.

As used herein, the term "a solvent or a solvent mixture" refers to any and all solvents. In some embodiments, a solvent or a solvent mixture is organic solvents and water, which include, but are not limited to, water, methanol, ethanol, 2-propanol, n-butanol, iso-butanol, acetone, methylethylketone, ethylacetate, 1,4-dioxane, diethyl ether, MTBE, THF, acetonitrile, dichloromethane, chloroform, DMF, cyclohexane, cyclopentane, n-hexane, n-heptane, n-pentane, toluene, o-xylene, p-xylene, DMSO, pyridine, acetic acid, anisole, butylacetate, cumene, ethylformate, formic acid, iso-butylacetate, iso-propylacetate, methylacetate, 3-methyl-1-butanol, methylisobutylketone, 2-methyl-1-propanol, 1-pentanol, propylacetate, ethylenglycole, and 1-methyl-2-pyrrolidone, as well as any and all mixtures of two or more such solvents. In some embodiments, a solvent or a solvent mixture is single solvent and binary mixtures. In some embodiments, a solvent or a solvent mixture is water and single solvent of organic solvent and binary mixtures of water and organic solvent.

As used herein, the term "pharmaceutical composition" refers to a formulation of a compound of the present disclosure and a medium generally acceptable in the art for the delivery of the biologically active compound to mammals, e.g., humans. Such a medium includes all pharmaceutically acceptable carriers, diluents or excipients therefor.

As used herein, the term"therapeutically effective amount" refers to an amount of a compound or combination of compounds that ameliorates, attenuates or eliminates a particular disease or condition or a symptom of a particular disease or condition, or prevents or delays the onset of a particular disease or condition or a symptom of a particular disease or condition. The amount of a compound of the present disclosure which constitutes a "therapeutically effective amount" will vary depending on the compound, the condition and its severity, and the age of the mammal to be treated, but can be determined routinely by one of ordinary skill in the art having regard to his own knowledge and to this disclosure.

"Treating" or "treatment" as used herein covers the treatment of the disease or condition of interest in a mammal, such as a human, having the disease or disorder of interest, and includes:

(i) preventing the disease or condition from occurring in a mammal, in particular, when such mammal is predisposed to the condition but has not yet been diagnosed as having it;
(ii) inhibiting the disease or condition, i.e., arresting its development; or
(iii) relieving the disease or condition, i.e., causing regression of the disease or condition.

As used herein, the terms "disease" and "condition" may be used interchangeably or may be different in that the particular malady or condition may not have a known causative agent (so that etiology has not yet been worked out) and it is therefore not yet recognized as a disease but only as an undesirable condition or syndrome, wherein a more or less specific set of symptoms have been identified by clinicians.

As used herein, The term "physiologically acceptable" defines a carrier or a diluent that does not abrogate the biological activities and properties of a compound.

The compounds of the present disclosure or their pharmaceutically acceptable salt may contain one or more asymmetric centers and may thus give rise to enantiomers, diastereoisomers, and other stereoismeric forms that may be defined, in terms of absolute stereochemistry, as (R)- or (S)-, or (D)- or (L)- for amino acids. The present disclosure is meant to include all such possible isomers, as well as their racemic and optically pure forms. Optically active (+) and (-), (R)- and (S)-, or (D)- and (L)-isomers may be prepared using chiral synthons or chiral reagents, or resolved using conventional techniques, such as HPLC using a chiral column. When the compounds described herein contain olefinic double bonds or other centers of geometric asymmetry, and unless specified otherwise, it is intended that the compounds include both E and Z geometric isomers. Likewise, all tautomeric forms are also intended to be included.

As used herein, the term "stereoisomer" refers to a compound made up of the same atoms bonded by the same bonds but having different three-dimensional structures, which are not interchangeable. The present disclosure contemplates various stereoisomers and mixtures thereof.

Specific Embodiments

In one aspect, the present disclosure relates to a compound of Formula (I), a stereoisomer, and a cis-trans isomer thereof:

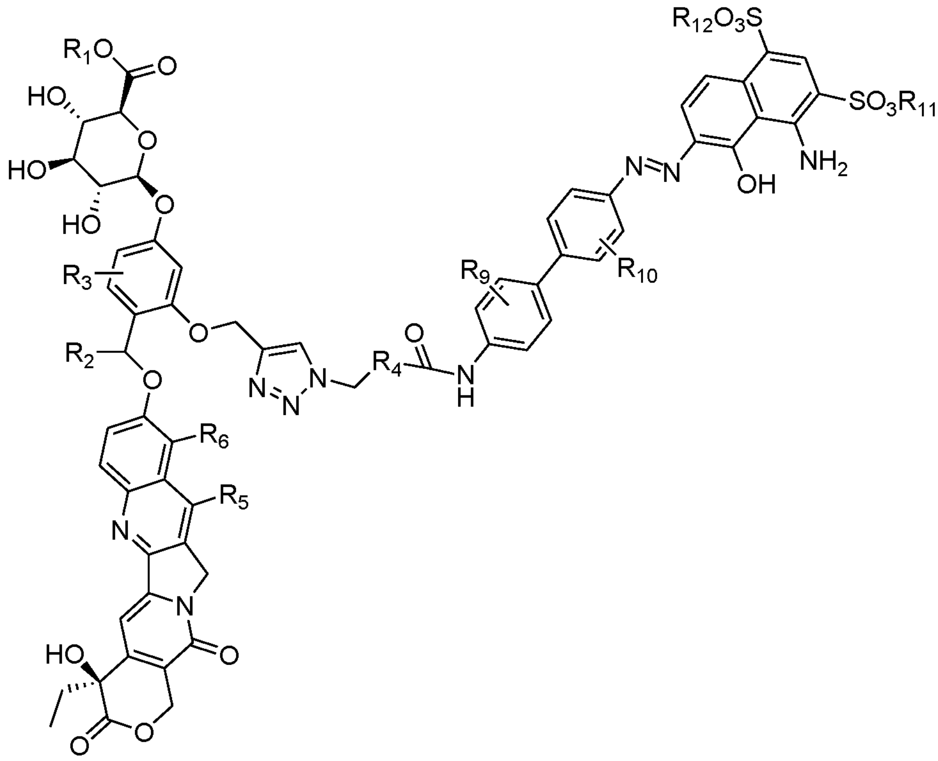

Formula (I)

wherein

$R_1$ is selected from the group consisting of hydrogen, $C_1$-$C_8$ hydrocarbyl, -Na, -Li, -K, -Cs, $-NH_4^+$, $-NH_2^+R_{13}R_{14}$, $-NH^+R_{13}R_{14}R_{15}$, $-NH^+{}_3R_{13}$, and natural or unnatural amino acids;

$R_2$ is selected from the group consisting of hydrogen, and $C_1$-$C_8$ hydrocarbyl;

$R_3$ is one or more substituents at any substitution position selected from the group consisting of hydrogen, halogen, $C_1$-$C_8$ hydrocarbyl, $C_1$-$C_8$ hydrocarbyloxy, cyano, nitro, amino, aryl, arylhydrocarbyl, and $C_1$-$C_4$ hydrocarbyl substituted amino;

$R_4$ is selected from the group consisting of $C_1$-$C_9$ alkylidene, $C_1$-$C_8$ alkylideneoxy, $C_1$-$C_8$ hydrocarbyl substituted or unsubstituted $C_3$-$C_8$ heteroaryl, $C_1$-$C_8$ hydrocarbyl substituted or unsubstituted $C_3$-$C_{15}$ aryl, $-(CH_2R_7)_n$, $-(R_8)_m$, $C_1$-$C_9$ alkylidene-arylidene, $C_1$-$C_9$ alkylidene-arylidene-$C_1$-$C_9$ alkyldiene, $C_1$-$C_9$ alkylidene-arylidene-arylidene, $C_1$-$C_9$ alkylidene-heteroaryliden-$C_1$-$C_9$ alkylidene, and amino acid peptide chain; wherein the amino acid peptide chain is composed of a combination of natural or unnatural amino acids, and the peptide chain length is 1 to100 peptides, preferably 1 to 50 peptides, and more preferably 1 to 20 peptides;

$R_5$ is selected from the group consisting of hydrogen, and $C_1$-$C_8$ hydrocarbyl;

$R_6$ is selected from the group consisting of hydrogen, $C_1$-$C_8$ hydrocarbyl, $C_1$-$C_8$ hydrocarbylidene amino ($C_1$-$C_4$ hydrocarbyl$)_2$, $C_1$-$C_8$ hydrocarbylidene thio $C_1$-$C_8$ hydrocarbyl, and $C_1$-$C_9$ alkylidene-oxy-$C_1$-$C_9$ alkyl;

$R_5$ and $R_6$ may form a ring, and if $R_5$ and $R_6$ form a ring, the ring may be a five-membered ring or a six-membered ring;

$R_7$ is selected from hydrogen, $C_1$-$C_8$ hydrocarbylideneoxy, amino $C_1$-$C_8$ hydrocarbylidene, thio $C_1$-$C_8$ hydrocarbylidene, and oxy $C_1$-$C_8$ hydrocarbylidene;

n is selected from the group consisting of 0, 1, 2, 3, 4, 5, 6, 7, 8, and 9; if n is 0, $(CH_2R_7)_n$ represents a covalent bond;

$R_8$ is carbonylamino, wherein amino may be substituted with $C_1$-$C_8$ hydrocarbyl or arylhydrocarbyl, $C_1$-$C_8$ hydrocarbylidene amino $C_1$-$C_8$ hydrocarbylidene, $C_1$-$C_8$ hydrocarbylidene thio $C_1$-$C_8$ hydrocarbylidene, or $C_1$-$C_8$ hydrocarbylidene oxy $C_1$-$C_8$ hydrocarbylidene;

m is selected from the group consisting of 0, 1, 2, 3, 4, 5, 6, 7, 8, and 9; if m is 0, $-(R_8)_m$ represents a covalent bond;

$R_9$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{10}$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{13}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{14}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{15}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl; and

the natural or unnatural amino acids in $R_1$, $R_{11}$, and $R_{12}$ are selected from the group consisting of glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine.

In another aspect, the present disclosure relates to a compound of Formula (I), a stereoisomer, and a cis-trans isomer thereof:

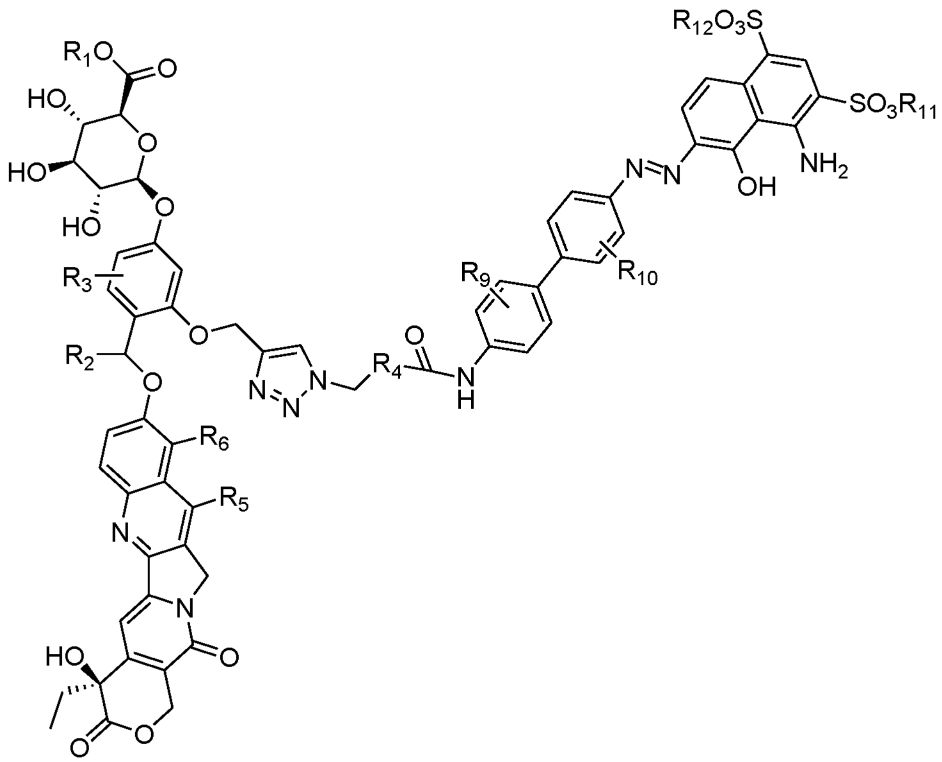

Formula (I)

wherein

$R_1$ is selected from the group consisting of hydrogen, $C_1$-$C_4$ hydrocarbyl, -Na, -Li, -K, -Cs, $-NH_4^+$, $-NH_2^+R_{13}R_{14}$, $-NH^+R_{13}R_{14}R_{15}$, $-NH^+{}_3R_{13}$, and natural or unnatural amino acids;

$R_2$ is selected from the group consisting of hydrogen, and $C_1$-$C_4$ hydrocarbyl;

$R_3$ is one or more substituents at any position selected from the group consisting of hydrogen, halogen, $C_1$-$C_4$ hydrocarbyl, $C_1$-$C_4$ hydrocarbyloxy, cyano, nitro, amino, aryl, arylhydrocarbyl, and $C_1$-$C_4$ hydrocarbyl substituted amino;

$R_4$ is selected from the group consisting of $C_1$-$C_6$ alkylidene, $C_1$-$C_6$ alkylideneoxy, $C_1$-$C_6$ hydrocarbyl substituted or unsubstituted $C_3$-$C_8$ heteroaryl, $C_1$-$C_8$ hydrocarbyl substituted or unsubstituted $C_3$-$C_{15}$ aryl, $-(CH_2R_7)_n$, $-(R_8)_m$, $C_1$-$C_4$ alkylidene-arylidene, $C_1$-$C_4$ alkylidene-aryliden-$C_1$-$C_4$ alkylidene, $C_1$-$C_4$ alkylidene-arylidene-arylidene, $C_1$-$C_4$ alkylidene-heteroarylidene-$C_1$-$C_4$ alkylidene, and amino acid peptide chain; wherein the amino acid peptide chain is composed of a combination of natural or unnatural amino acids, and the peptide chain length is 1 to 100 peptides, preferably 1 to 50 peptides, and more preferably 1 to 20 peptides;

$R_5$ is selected from the group consisting of hydrogen, and $C_1$-$C_4$ hydrocarbyl;

$R_6$ is selected from the group consisting of hydrogen, $C_1$-$C_4$ hydrocarbyl, $C_1$-$C_4$ hydrocarbylidene amino ($C_1$-$C_4$ hydrocarbyl)$_2$, $C_1$-$C_4$ hydrocarbylidene thio $C_1$-$C_4$ hydrocarbyl, and $C_1$-$C_4$ alkylidene-oxy-$C_1$-$C_4$ alkyl;

$R_5$ and $R_6$ may form a ring, and if $R_5$ and $R_6$ form a ring, the entire ring may be a five-membered ring or a six-membered ring;

$R_7$ is selected from the group consisting of hydrogen, $C_1$-$C_4$ alkylideneoxy, amino $C_1$-$C_4$-alkylidene, thio $C_1$-$C_4$-alkylidene, and oxy $C_1$-$C_4$ alkylidene;

n is selected from the group consisting of 0, 1, 2, 3, 4, 5, 6, 7, 8, and 9; if n is 0, $-(CH_2R_7)_n$ represents a covalent bond;

$R_8$ is carbonylamino, wherein amino may be substituted with $C_1$-$C_4$ hydrocarbyl or arylhydrocarbyl, $C_1$-$C_4$ hydrocarbylidene amino $C_1$-$C_4$ hydrocarbylidene, $C_1$-$C_4$ hydrocarbylidene thio $C_1$-$C_4$ hydrocarbylidene, or $C_1$-$C_4$ hydrocarbylidene oxy $C_1$-$C_4$ hydrocarbylidene;

m is selected from the group consisting of 0, 1, 2, 3, 4, 5, 6, 7, 8, and 9; if m is 0, $-(R_8)_m$ represents a covalent bond;

$R_9$ is one or more substituents at any position selected from the group consisting of hydrogen, halogen, cyano, $C_1$-$C_4$ hydrocarbyl, $C_1$-$C_4$ alkoxy, and $C_5$-$C_{12}$ aryl;

$R_{10}$ is one or more substituents at any position selected from the group consisting of hydrogen, halogen, cyano, $C_1$-$C_4$ hydrocarbyl, $C_1$-$C_4$ alkoxy, and $C_5$-$C_{12}$ aryl;

$R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{13}$ is selected from the group consisting of hydrogen, $C_1$-$C_4$ alkyl, $C_1$-$C_8$ cycloalkyl, $C_5$-$C_{18}$ aryl, and $C_5$-$C_{18}$ heteroaryl;

$R_{14}$ is selected from the group consisting of hydrogen, $C_1$-$C_4$ alkyl, $C_1$-$C_8$ cycloalkyl, $C_5$-$C_{18}$ aryl, and $C_5$-$C_{18}$ heteroaryl;

$R_{15}$ is selected from the group consisting of hydrogen, $C_1$-$C_4$ alkyl, $C_1$-$C_8$ cycloalkyl, $C_5$-$C_{18}$ aryl, and $C_5$-$C_{18}$ heteroaryl; and

the natural or unnatural amino acid in $R_1$, $R_{11}$, and $R_{12}$ are selected from the group consisting of glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine.

In some embodiments, $R_1$ is selected from the group consisting of hydrogen, $-CH_3$, $-CH_2CH_3$, -Na, -Li, -K, -Cs, $-NH_4^+$, $-NH_2^+R_{13}R_{14}$, $-NH^+R_{13}R_{14}R_{15}$, $-NH^+{}_3R_{13}$, and natural or unnatural amino acids.

In some embodiments, $R_2$ is selected from the group consisting of hydrogen, $-CH_3$, and $-CH_2CH_3$.

In some embodiments, $R_3$ is selected from one or more substituents substituted at any position selected from hydrogen, -F-, -Cl, -Br, -CN, $-CH_3$, $-CH_2CH_3$, $-CH_2CH_2CH_3$, $-CH_2CH_2CH_2CH_3$, $-OCH_3$, $-OCH_2CH_3$, $-OCH_2CH_2CH_3$, $-OCH_2CH_2CH_2CH_3$, phenyl, benzyl, $-NO_2$, $-NH_2$, and $-N(CH_3)_2$.

In some embodiments, $R_4$ is selected from the group consisting of $-CH_2-$, $-CH_2CH_2-$, $-CH_2CH_2CH_2-$, $-CH_2CH_2CH_2CH_2-$, $-CH_2CH_2CH_2CH_2CH_2-$, $-CH_2CH_2CH_2CH_2CH_2CH_2-$, $-CH_2CH_2CH_2CH_2CH_2CH_2CH_2-$, $-CH_2CH_2CH_2CH_2CH_2CH_2CH_2CH_2-$, $-CH_2CH_2CH_2CH_2CH_2CH_2CH_2CH_2CH_2-$, $-CH_2OCH_2-$, $-CH_2OCH_2CH_2-$, $-CH_2OCH_2CH_2OCH_2-$, $-CH_2OCH_2CH_2OCH_2CH_2OCH_2-$, $-CH_2OCH_2CH_2OCH_2CH_2OCH_2CH_2OCH_2-$, $-CH_2CH_2OCH_2CH_2CH_2OCH_2-$, $-CH_2CH_2OCH_2CH_2CH_2OCH_2CH_2CH_2OCH_2-$, $-CH_2CH_2OCH_2CH_2CH_2OCH_2CH_2CH_2OCH_2CH_2CH_2OCH_2-$, $-CH_2CH_2OCH_2-$, $-CH_2CH_2OCH_2CH_2CH_2OCH_2CH_2-$, $-CH_2CH_2OCH_2CH_2CH_2OCH_2-$, $-CH_2NHCH_2CH_2NHCH_2CH_2NHCH_2-$,

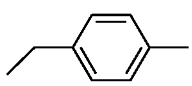,
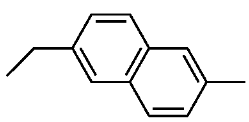,

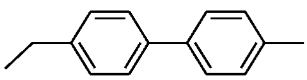,
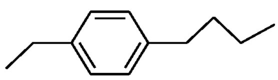,
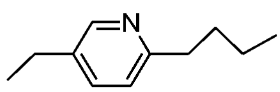,

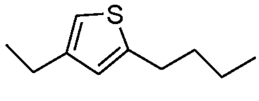,

and peptides, wherein the peptides are composed of a combination of natural or unnatural amino acids, such as glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, homoalanine, or any combination thereof; the peptide chain length is 1 to 100 peptides, preferably 1 to 50 peptides, and more preferably 1 to 20 peptides.

In some embodiments, $R_5$ is selected from the group consisting of hydrogen, $-CH_3$, and $-CH_2CH_3$.

In some embodiments, $R_6$ is selected from the group consisting of hydrogen, $-CH_3$, $-CH_2OCH_3$, $-CH_2N(CH_3)_2$, and $-CH_2SCH(CH_3)_2$.

In some embodiments, if $R_5$ and $R_6$ form a ring, the ring may be a five-membered ring or a six-membered ring.

In some embodiments, if $R_5$ and $R_6$ form a ring, the ring may be selected from the group consisting of cyclopentyl and cyclohexyl.

In some embodiments, $R_7$ is selected from the group consisting of -H, $-OCH_2-$, $-OCH_2CH_2-$, $-OCH_2CH_2CH_2-$, $-OCH_2CH_2CH_2CH_2-$, $-NHCH_2-$, $-NHCH_2CH_2-$, $-NHCH_2CH_2CH_2-$, $-NHCH_2CH_2CH_2CH_2-$, $-SCH_2-$, $-SCH_2CH_2-$, $-SCH_2CH_2CH_2-$, $-SCH_2CH_2CH_2CH_2-$, $-CH_2O-$, $-CH_2CH_2O-$, $-CH_2CH_2CH_2O-$, and $-CH_2CH_2CH_2CH_2O-$.

In sonme embodiments, n is selected from the group consisting of 0, 1, 2, 3, 4, 5, 6, 7, 8, and 9.

In certain embodiments, if n is 0, $-(CH_2R_7)_n$ represents a covalent bond.

In some embodiments, $R_8$ is selected from the group consisting of $-CH_2OCH_2CH_2-$, $-CH_2CH_2OCH_2-$, $-CH_2CH_2OCH_2CH_2-$, $-CH_2CH_2OCH_2CH_2CH_2-$, $-CH_2CH_2CH_2OCH_2CH_2-$, $-CH_2CH_2CH_2OCH_2CH_2CH_2-$, $-CH_2SCH_2CH_2-$, $-CH_2CH_2SCH_2-$, $-CH_2CH_2SCH_2CH_2-$, $-CH_2CH_2SCH_2CH_2CH_2-$, $-CH_2CH_2CH_2SCH_2CH_2-$, $-CH_2CH_2CH_2SCH_2CH_2CH_2-$, $-CH_2NHCH_2CH_2-$, $-CH_2CH_2NHCH_2-$, $-CH_2CH_2NHCH_2CH_2-$, $-CH_2CH_2NHCH_2CH_2CH_2-$, $-CH_2CH_2CH_2NHCH_2CH_2-$, $-CH_2CH_2CH_2NHCH_2CH_2CH_2-$, $-CONHCH_2-$, $-CONHCH_2CH_2-$, $-CONHCH_2CH_2CH_2-$, $-CON(CH_3)CH_2-$, $-CON(CH_3)CH_2CH_2-$, $-CON(CH_3)CH_2CH_2CH_2-$, $-CONHCH(CH_3)-$, $-CONHCH(CH_3)CH_2-$, $-CONHCH(CH_3)CH_2CH_2-$, $-CONHCH_2CH(CH_3)CH_2-$, $-CONHCH(CH_3)CONHCH(CH_3)CH_2C_6H_5-$.

In some embodiments, m is selected from the group consisting of 0, 1, 2, 3, 4, 5, 6, 7, 8, and 9.

In some embodiments, if m is 0, $-(R_8)_m$ represents a covalent bond.

In some embodiments, $R_9$ is one or more substituents at any position selected from the group consisting of hydrogen, -F-, -Cl, -Br, -CN, $-CH_3$, $-CH_2CH_3$, $-OCH_3$, $-OCH_2CH_3$, and phenyl.

In some embodiments, $R_{10}$ is one or more substituents at any position selected from the group consisting of hydrogen, -F-, -Cl, -Br, -CN, $-CH_3$, $-CH_2CH_3$, $-OCH_3$, $-OCH_2CH_3$, and phenyl.

In some embodiments, $R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine.

In some embodiments, $R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine.

In some embodiments, $R_{13}$ is selected from the group consisting of hydrogen, $-CH_3$, $-CH_2CH_3$, $-CH(CH_3)_2$, $-CH_2CH_2CH_3$, $-CH_2CH_2CH_2CH_2CH_3$, cyclohexyl, phenyl, pyridyl, thienyl, imidazolyl, indolyl, quinolinyl, pyridoimidazolyl, pyridoindolyl, imidazoquinolinyl, imidazoquinoxalinyl, imidazopyridyl, and benzoxazinyl.

In some embodiments, $R_{14}$ is selected from the group consisting of hydrogen, $-CH_3$, $-CH_2CH_3$, $-CH(CH_3)_2$, $-CH_2CH_2CH_3$, $-CH_2CH_2CH_2CH_2CH_3$, cyclohexyl, phenyl, pyridyl, thienyl, imidazolyl, indolyl, quinolinyl, pyridoimidazolyl, pyridoindolyl, imidazoquinolinyl, imidazoquinoxalinyl, imidazopyridyl, and benzoxazinyl.

In some embodiments, $R_{15}$ is selected from the group consisting of hydrogen, $-CH_3$, $-CH_2CH_3$, $-CH(CH_3)_2$, $-CH_2CH_2CH_3$, $-CH_2CH_2CH_2CH_2CH_3$, cyclohexyl, phenyl, pyridyl, thienyl, imidazolyl, indolyl, quinolinyl, pyridoimidazolyl, pyridoindolyl, imidazoquinolinyl, imidazoquinoxalinyl, imidazopyridyl, and benzoxazinyl.

In yet another aspect, the present disclosure relates to the following compounds, stereoisomers, cis-trans isomers, or pharmaceutically acceptable salts thereof:

(2S,3S,4S,SR,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)2-methylp henoxy)-3,4,5-trihydroxytetrahydro-2H-pyran-2-carboxylate trisodium;

4-amino-6-((E)-(4'-(6-(4-((3-(((2S,3R,4S,5S,6S)-6-carboxy-3,4,5-trihydr oxytetrahydro-2H-pyran-2-yl)oxy)-6-((((S)-4,11-diethyl-4-hydroxy-3,14-dioxy-3,4,12 ,14-tetrahydro-1H-pyrano[3',4':6,7]indolazino[1,2-b]quinolin-9-yl)oxy)methyl)-2-tol yloxy)methyl)-1H-1,2,3-triazol-1-yl)hexanamido)-3,3'-dimethyl-[1,1'-biphenyl]-4-yl) diazenyl)-5-hydroxynaphthalene-1,3-disulfonate disodium;

4-amino-6-((E)-(4'-(6-(4-((6-(((S)-4,11-diethyl-4-hydroxy-3,14-dioxy-3, 4,12,14-tetrahydro-1H-pyrano[3',4':6,7]indolazo[1,2-b]quinolin-9-yl)oxy)methyl)-2-methyl-3-(((2S,3R,4S,5S,6S)-3,4,5-trihydroxy-6-(methoxycarbonyl)tetrahydro-2H-py ran-2-yl)oxy)phenoxy)methyl)-1H-1,2,3-triazol-1-yl)hexanamido)-3,3'-dimethyl-[1,1' -biphenyl]-4-yl)diazenyl)-5-hydroxynaphthalene-1,3-disulfonate disodium;
(2S,3S,4S,5R,6S)-6-(3-((1-(6-((4'-((E)-(8-amino-1-hydroxy-5,7-disulfoni cnaphthalen-2-yl)diazenyl)-[1,1'-biphenyl]-4-yl)amino)-6-oxohexyl)-1H-1,2,3-triazol -4-yl)methoxy)-4-((((S)-4,11-diethyl-4-hydroxy-3,14-dioxo-3,4,12,14-tetrahydro-1H-pyrano[3',4':6,7]indolazino[1,2-b]quinolin-9-yl)oxy)methyl)-2-methylphenoxy)-3,4,5 -trihydroxytetrahydro-2H-pyran-2-carboxylate trisodium;
4-amino-6-((E)-(4'-(6-(4-((5-(((2S,3R,4S,5S,6S)-6-carboxy-3,4,5-trihydr oxytetrahydro-2H-pyran-2-yl)oxy)-2-((((S)-4,11-diethyl-4-hydroxy-3,14-dioxy-3,4,12 ,14-tetrahydro-1H-pyrano[3',4':6,7]indolazino[1,2-b]quinolin-9-yl)oxy)methyl)pheno xy)methyl)-1H-1,2,3-triazol-1-yl)hexanamido)-3,3'-dimethyl-[1,1'-biphenyl]-4-yl)dia zenyl)-5-hydroxynaphthalene-1,3-disulfonate disodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;
4-amino-6-((E)-(4'-(6-(4-((2-((((S)-4,11-diethyl-4-hydroxy-3,14-dioxy-3, 4,12,14-tetrahydro-1H-pyrano[3',4':6,7]indolazino[1,2-b]quinolin-9-yl)oxy)methyl)-5 -(((2S,3R,4S,5S,6S)-3,4,5-trihydroxy-6-(methoxycarbonyl)tetrahydro-2H-pyran-2-yl) oxy)phenoxy)methyl)-1H-1,2,3-triazol-1-yl)hexanamido)-3,3'-dimethyl-[1,1'-bipheny l]-4-yl)diazenyl)-5-hydroxynaphthalene-1,3-disulfonate disodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-(((S)-4-ethyl-4-hydroxy-3,14-dioxy-3,4,12,14-tetrah ydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;
4-amino-6-((E)-(4'-(6-(4-((5-(((2S,3R,4S,5S,6S)-6-carboxy-3,4,5-trihydr oxytetrahydro-2H-pyran-2-yl)oxy)-2-((((S)-4-ethyl-4-hydroxy-3,14-dioxo-3,4,12,14-t etrahydro-1H-pyrano[3',4':6,7]indolazino[1,2-b]quinolin-9-yl)oxy)methyl)phenoxy) methyl)-1H-1,2,3-triazol-1-yl)hexanamido)-3,3'-dimethyl-[1,1'-biphenyl]-4-yl)diazen yl)-5-hydroxynaphthalene-1,3-disulfonate disodium;
4-amino-6-((E)-(4'-(6-(4-((2-(((S)-4-ethyl-4-hydroxy-3,14-dioxo-3,4,12,1 4-tetrahydro-1H-pyrano[3',4':6,7]indolizino[1,2-b]quinolin-9-yl)oxy)methyl)-5-(((2S, 3R,4S,5S,6S)-3,4,5-trihydroxy-6-(methoxycarbonyl)tetrahydro-2H-pyran-2-yl)oxy)ph enoxy)methyl)-1H-1,2,3-triazol-1-yl)hexanamido)-3,3'-dimethyl-[1,1'-biphenyl]-4-yl )diazenyl)-5-hydroxynaphthalene-1,3-disulfonate disodium;
(2S,3S,4S,5R,6S)-6-(3-((1-(6-((4'-((E)-(8-amino-1-hydroxy-5,7-disulfoni cnaphthalen-2-yl)diazenyl)-[1,1'-biphenyl]-4-yl)amino)-6-oxohexyl)-1H-1,2,3-triazol -4-yl)methoxy)-4-((((S)-4,11-diethyl-4-hydroxy-3,14-dioxo-3,4,12,14-tetrahydro-1H-pyrano[3',4':6,7]indolazino[1,2-b]quinolin-9-yl)oxy)methyl)-5-methoxyphenoxy)-3,4 ,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-[1,1'-biphenyl]-4-yl)amino)-6-oxohexyl)-1H-1,2,3-triazol -4-yl)methoxy)-4-((((S)-4-ethyl-4-hydroxy-3,14-dioxy-3,4,12,14-tetrahydro-1H-pyran o[3',4':6,7]indolazino[1,2-b]quinolin-9-yl)oxy)methyl)phenoxy)-3,4,5-trihydroxytetra hydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-5-methox yphenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethoxy-[1,1'-biphenyl]-4-yl)amino)-6-oxohexyl)-1H-1,2,3-triazol-4-yl)methoxy)-4-(((S)-4-ethyl-4-hydroxy-3,14-dioxy-3,4,12,14-tetra hydro-1H-pyrano[3',4':6,7]indolazino[1,2-b]quinolin-9-yl)oxy)methyl)phenoxy)-3,4, 5-trihydroxytetrahydro-2H-pyran-2-carboxylate trisodium;
4-amino-6-((E)-(4'-(4-((5-(((2S,3R,4S,5S,6S)-6-carboxy-3,4,5-trihydroxy tetrahydro-2H-pyran-2-yl)oxy)-2-((((S)-4-ethyl-4-hydroxy-3,14-dioxo-3,4,12,14-tetra hydro-1H-pyrano[3',4':6,7]indolazino[1,2-b]quinolin-9-yl)oxy)methyl)phenoxy)meth yl)-1H-1,2,3-triazol-1-yl)hexanamido)-3,3'-dimethoxy-[1,1'-biphenyl]-4-yl)diazenyl) -5-hydroxynaphthalene-1,3-disulfonate disodium;
4-amino-6-((E)-(4'-(6-(4-((2-(((S)-4-ethyl-4-hydroxy-3,14-dioxy-3,4,12,1 4-tetrahydro-1H-pyrano[3',4':6,7]indolazino[1,2-b]quinolin-9-yl)oxy)methyl)-5-(((2S ,3R,4S,5S,6S)-3,4,5-trihydroxy-6-(methoxycarbonyl)tetrahydro-2H-pyran-2-yl)oxy)p henoxy)methyl)-1H-1,2,3-triazol-1-yl)hexanamido)-3,3'-dimethoxy-[1,1'-biphenyl]-4 -yl)diazenyl)-5-hydroxynaphthalene-1,3-disulfonate disodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-(((S)-10-((dimethylamino)methyl)-4-ethyl-4-hydroxy -3,14-dioxy-3,4,12,14-tetrahydro-1H-pyrano[3',4':6,7]indolazino[1,2-b]quinolin-9-yl)

oxy)methyl)phenoxy)-3,4,5-trihydroxytetrahydro-2H-pyran-2-carboxylate trisodium;

(2S,3S,4S,5R,6S)-6-(3-((1-(8-((4'-((E)-(8-amino-1-hydroxy-5,7-disulfoni enaphthalen-2-yl)diazenyl)-3,3'-dimethyl-[1,1'-biphenyl]-4-yl)amino)-8-oxooctyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;

4-amino-6-((E)-(4'-(8-(4-((5-(((2S,3R,4S,5S,6S)-6-carboxy-3,4,5-trihydr oxytetrahydro-2H-pyran-2-yl)oxy)-2-((((S)-4,11-diethyl-4-hydroxy-3,14-dioxy-3,4,12 ,14-tetrahydro-1H-pyrano[3',4':6,7]indolazino[1,2-b]quinolin-9-yl)oxy)methyl)pheno xy)methyl)-1H-1,2,3-triazol-1-yl)octanamido)-3,3'-dimethyl-[1,1'-biphenyl]-4-yl)dia zenyl)-5-hydroxynaphthalene-1,3-disulfonate disodium;

4-amino-6-((E)-(4'-(8-(4-((2-(((S)-4,11-diethyl-4-hydroxy-3,14-dioxy-3, 4,12,14-tetrahydro-1H-pyrano[3',4':6,7]indolazo[1,2-b]quinolin-9-yl)oxy)methyl)-5-( ((2S,3R,4S,5S,6S)-3,4,5-trihydroxy-6-(methoxycarbonyl)tetrahydro-2H-pyran-2-yl)o xy)phenoxy)methyl)-1H-1,2,3-triazol-1-yl)octanamido)-3,3'-dimethyl-[1,1'-biphenyl] -4-yl)diazenyl)-5-hydroxynaphthalene-1,3-disulfonate disodium;

(2S,3S,4S,5R,6S)-6-(3-((1-(8-((4'-((E)-(8-amino-1-hydroxy-5,7-disulfoni cnaphthalen-2-yl)diazenyl)-[1,1'-biphenyl]-4-yl)amino)-8-oxooctyl)-1H-1,2,3-triazol-4-yl)methoxy)-4-((((S)-4,11-diethyl-4-hydroxy-3,14-dioxo-3,4,12,14-tetrahydro-1H-p yrano[3',4':6,7]indolazino[1,2-b]quinolin-9-yl)oxy)methyl)phenoxy)-3,4,5-trihydroxy tetrahydro-2H-pyran-2-carboxylate trisodium;

(2S,3S,4S,5R,6S)-6-(3-((1-(8-((4'-((E)-(8-amino-1-hydroxy-5,7-disulfoni cnonaphthalen-2-yl)diazenyl)-3,3'-dimethyl-[1,1'-biphenyl]-4-yl)amino)-8-oxooctyl)-1H-1,2,3-triazol-4-yl)methoxy)-4-((((S)-4,11-diethyl-4-hydroxy-3,14-dioxy-3,4,12,14 -tetrahydro-1H-pyrano[3',4':6,7]indolazino[1,2-b]quinolin-9-yl)oxy)methyl)-2-methy lphenoxy)-3,4,5-trihydroxytetrahydro-2H-pyran-2-carboxylate trisodium;

(2S,3S,4S,5R,6S)-6-(3-((1-(2-(2-(2-((4'-((E)-(8-amino-1-hydroxy-5,7-dis ulfonicnaphthalen-2-yl)diazenyl)-3,3'-dimethyl-[1,1'-biphenyl]-4-yl)amino)-2-oxoeth oxy)ethoxy)ethyl)-1H-1,2,3-triazol-4-yl)methoxy)-4-(((S)-4,11-diethyl-4-hydroxy-3,1 4-dioxo-3,4,12,14-tetrahydro-1H-pyrano[3',4':6,7]indolazino[1,2-b]quinolin-9-yl)oxy )methyl)phenoxy)-3,4,5-trihydroxytetrahydro-2H-pyran-2-carboxylate trisodium;

4-amino-6-((E)-(4'-(2-(2-(2-(2-(2-((5-(((2S,3R,4S,5S,6S)-6-carboxy-3,4, 5-trihydroxytetrahydro-2H-pyran-2-yl)oxy)-2-((((S)-4,11-diethyl-4-hydroxy-3,14-dio xy-3,4,12,14-tetrahydro-1H-pyrano[3',4':6,7]indolazino[1,2-b]quinolin-9-yl)oxy)met hyl)phenoxy)methyl)-1H-1,2,3-triazol-1-yl)ethoxy)ethoxy)acetamido)-3,3'-dimethyl-[ 1,1'-biphenyl]-4-yl)diazenyl)-5-hydroxynaphthalene-1,3-disulfonate disodium;

4-amino-6-((E)-(4'-(2-(2-(2-((2-(((S)-4,11-diethyl-4-hydroxy-3,14-dioxy-3,4,12,14-tetrahydro-1H-pyrano[3',4':6,7]indolazo[1,2-b]quinolin-9-yl)oxy)methyl)-5 -(((2S,3R,4S,5S,6S)-3,4,5-trihydroxy-6-(methoxycarbonyl)tetrahydro-2H-pyran-2-yl) oxy)phenoxy)methyl)-1H-1,2,3-triazol-1-yl)ethoxy)acetamido)-3,3'-dimethyl-[1,1'-bi phenyl]-4-yl)diazenyl)-5-hydroxynaphthalene-1,3-disulfonate disodium;

(2S,3S,4S,5R,6S)-6-(3-((1-(2-(2-(2-((4'-((E)-(8-amino-1-hydroxy-5,7-dis ulfonicnaphthalen-2-yl)diazenyl)-3,3'-dimethoxy-[1,1'-biphenyl]-4-yl)amino)-2-oxoe thoxy)ethoxy)ethyl)-1H-1,2,3-triazol-4-yl)methoxy)-4-(((S)-4,11-diethyl-4-hydroxy-3 ,14-dioxo-3,4,12,14-tetrahydro-1H-pyrano[3',4':6,7]indolazino[1,2-b]quinolin-9-yl)o xy)methyl)phenoxy)-3,4,5-trihydroxytetrahydro-2H-pyran-2-carboxylate trisodium;

4-amino-6-((E)-(4'-(2-(2-(2-(2-(2-((5-(((2S,3R,4S,5S,6S)-6-carboxy-3,4, 5-trihydroxytetrahydro-2H-pyran-2-yl)oxy)-2-((((S)-4,11-diethyl-4-hydroxy-3,14-dio xy-3,4,12,14-tetrahydro-1H-pyrano[3',4':6,7]indolazino[1,2-b]quinolin-9-yl)oxy)met hyl)phenoxy)methyl)-1H-1,2,3-triazol-1-yl)ethoxy)ethoxy)acetamido)-3,3'-dimethox y-[1,1'-biphenyl]-4-yl)diazenyl)-5-hydroxynaphthalene-1,3-disulfonate disodium;

4-amino-6-((E)-(4'-(2-(2-(2-((2-(((S)-4,11-diethyl-4-hydroxy-3,14-dioxy-3,4,12,14-tetrahydro-1H-pyrano[3',4':6,7]indolazo[1,2-b]quinolin-9-yl)oxy)methyl)-5 -(((2S,3R,4S,5S,6S)-3,4,5-trihydroxy-6-(methoxycarbonyl)tetrahydro-2H-pyran-2-yl) oxy)phenoxy)methyl)-1H-1,2,3-triazol-1-yl)ethoxy)acetamido)-3,3'-dimethoxy-[1,1'-biphenyl]-4-yl)diazenyl)-5-hydroxynaphthalene-1,3-disulfonate disodium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethoxy-[1,1'-biphenyl]-4-yl)amino)-6-oxohexyl)-1H-1,2,3-triazol-4-yl)methoxy)-4-(((S)-4,11-diethyl-4-hydroxy-3,14-dioxy-3,4,12,14-tetrahydro-1H-pyrano[3',4':6,7]indolizino[1,2-b]quinolin-9-yl)oxy)methyl)phenoxy)-3,4,5-trihydroxytetrahydro-2H-pyran-2-carboxylate trisodium;

4-amino-6-((E)-(4'-(6-(4-((5-(((2S,3R,4S,5S,6S)-6-carboxy-3,4,5-trihydr oxytetrahydro-2H-pyran-2-yl)oxy)-2-((((S)-4,11-diethyl-4-hydroxy-3,14-dioxy-3,4,12 ,14-tetrahydro-1H-pyrano[3',4':6,7]indolazino[1,2-b]quinolin-9-yl)oxy)methyl)pheno xy)methyl)-1H-1,2,3-triazol-1-yl)hexanamido)-3,3'-dimethoxy-[1,1'-biphenyl]-4-yl)d iazenyl)-5-hydroxynaphthalene-1,3-disulfonate disodium;

4-amino-6-((E)-(4'-(6-(4-((2-(((S)-4,11-diethyl-4-hydroxy-3,14-dioxo-3, 4,12,14-tetrahydro-1H-pyrano[3',4':6,7]indolazo[1,2-b]quinolin-9-yl)oxy)methyl)-5-( ((2S,3R,4S,5S,6S)-3,4,5-trihydroxy-6-(methoxycarbonyl)tetrahydro-2H-pyran-2-yl)o xy)phenoxy)methyl)-1H-1,2,3-triazol-1-yl)hexanamido)-3,3'-dimethoxy-[1,1'-biphen yl]-4-yl)diazenyl)-5-hydroxynaphthalene-1,3-disulfonate disodium;

(2S,3S,4S,SR,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy)-3,4,5-trihydroxytetrahydro-2H-pyran-2-carboxylic acid;

1-amino-7-((E)-(4'-(6-(4-((5-(((2S,3R,4S,5S,6S)-6-carboxy-3,4,5-trihydr oxytetrahydro-2H-pyran-2-yl) oxy)-2-((((S)-4,11-diethyl-4-hydroxy-3,14-dioxy-3,4,12 ,14-tetrahydro-1H-pyrano[3',4':6,7]indolazino[1,2-b]quinolin-9-yl)oxy)methyl)pheno xy)methyl)-1H-1,2,3-triazol-1-yl)hexanamido)-3,3'-dimethyl-[1,1'-biphenyl]-4-yl)dia zenyl)-8-hydroxy-4-sulfonaphthalene-2-sulfonate sodium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy)-3,4,5-trihydroxytetrahydro-2H-pyran-2-carboxylate trilithium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy)-3,4,5-trihydroxytetrahydro-2H-pyran-2-carboxylate triammonium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy)-3,4,5-trihydroxytetrahydro-2H-pyran-2-carboxylate tripotassium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)phenoxy)-3 ,4,5-trihydroxytetrahydro-2H-pyran-2-carboxylate tricesium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-2-fluorop henoxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-2-chloro-4-((((S)-4,11-diethyl-4-hydroxy-3,14-dioxy-3 ,4,12,14-tetrahydro-1H-pyrano[3',4':6,7]indolazino[1,2-b]quinolin-9-yl)oxy)methyl)p henoxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-2-bromo-4-((((S)-4,11-diethyl-4-hydroxy-3,14-dioxy-3 ,4,12,14-tetrahydro-1H-pyrano[3',4':6,7]indolazino[1,2-b]quinolin-9-yl)oxy)methyl)p henoxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-2-methox yphenoxy)-3,4,5-trihydroxytetrahydro-2H-pyran-2-carboxylat trisodiume;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-2-ethylph enoxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-(1-(((S)-4,11-diethyl-4-hydroxy-3,14-dioxy-3,4,12,1 4-tetrahydro-1H-pyrano[3',4':6,7]indolizino[1,2-b]quinolin-9-yl)oxy)ethyl)phenoxy)-3,4,5-trihydroxytetrahydro-2H-pyran-2-carboxylate trisodium;

(2S,3S,4S,5R,6S)-6-((6-((1-((4'-((E)-(8-amino-1-hydroxy-5,7-disulfonicn aphthalen-2-yl)diazenyl)-3,3'-dimethyl-[1,1'-biphenyl]-4-yl)amino)-6-oxohexyl)-1H-1,2,3-triazol-4-yl)methoxy)-5-((((S)-4,11-diethyl-4-hydroxy-3,14-dioxy-3,4,12,14-tetr ahydro-1H-pyrano[3',4':6,7]indolizino[1,2-b]quinolin-9-yl)oxy)methyl)-[1,1'-biphen yl]-2-yl)oxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-2-nitroph enoxy)-3,4,5-trihydroxytetrahydro-2H-pyran-2-carboxylate trisodium;

(2S,3S,4S,5R,6S)-6-(2-amino-3-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxo hexyl)-1H-1,2,3-triazol-4-yl)methoxy)-4-((((S)-4,11-diethyl-4-hydroxy-3,14-dioxy-3, 4,12,14-tetrahydro-1H-pyrano[3',4':6,7]indolazino[1,2-b]quinolin-9-yl)oxy)methyl)p henoxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxohexyl)-1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)-2-(dimethylamino)phenoxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxohexyl)-1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)-5-fluorophenoxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxohexyl)-1H-1,2,3-triazol-4-yl)methoxy)-5-chloro-4-((((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxohexyl)-1H-1,2,3-triazol-4-yl)methoxy)-5-bromo-4-((((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxohexyl)-1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolizino[1,2-b]quinolin-9-yl)oxy)methyl)-5-methylphenoxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxohexyl)-1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)-5-nitrophenoxy)-3,4,5-trihydroxytetrahydro-2H-pyran-2-carboxylate trisodium;

(2S,3S,4S,5R,6S)-6-((5-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxohexyl)-1H-1,2,3-triazol-4-yl)methoxy)-6-((((S)-4,11-diethyl-4-hydroxy-3,14-dioxy-3,4,12,14-tetrahydro-1H-pyrano[3',4':6,7]indolizino[1,2-b]quinolin-9-yl)oxy)methyl)-[1,1'-biphenyl]-3-yl)oxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxohexyl)-1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)-5-ethylphenoxy)-3,4,5-trihydroxytetrahydro-2H-pyran-2-carboxylate trisodium;

(2S,3S,4S,SR,6S)-6-(3-amino-5-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxohexyl)-1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxohexyl)-1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)-5-(dimethylamino)phenoxy)-3,4,5-trihydroxytetrahydro-2H-pyran-2-carboxylate trisodium;

(2S,3S,4S,5R,6S)-6-(5-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxohexyl)-1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolizino[1,2-b]quinolin-9-yl)oxy)methyl)-2-fluorophenoxy)-3,4,5-trihydroxytetrahydro-2H-pyran-2-carboxylate trisodium;

(2S,3S,4S,5R,6S)-6-(5-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxohexyl)-1H-1,2,3-triazol-4-yl)methoxy)-2-chloro-4-((((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)phenoxy)-3,4,5-trihydroxytetrahydro-2H-pyran-2-carboxylat trisodiume;

(2S,3S,4S,5R,6S)-6-(5-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxohexyl)-1H-1,2,3-triazol-4-yl)methoxy)-2-bromo-4-((((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;

(2S,3S,4S,5R,6S)-6-(5-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxohexyl)-1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)-2-methox yphenoxy)-3,4,5-trihydroxytetrahydro-2H-pyran-2-carboxylate trisodium;

(2S,3S,4S,5R,6S)-6-(5-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)-2-nitrophe noxy)-3,4,5-trihydroxytetrahydro-2H-pyran-2-carboxylate trisodium;

(2S,3S,4S,5R,6S)-6-((4-((1-(6-((4'-((E)-(8-amino-1-hydroxy-5,7-disulfon icnaphthalen-2-yl)diazenyl)-3,3'-dimethyl-[1,1'-biphenyl]-4-yl)amino)-6-oxohexyl)-1 H-1,2,3-triazol-4-yl)methoxy)-5-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)-[1,1'-biph enyl]-2-yl)oxy)-3,4,5-trihydroxytetrahydro-2H-pyran-2-carboxylate trisodium;

(2S,3S,4S,5R,6S)-6-(5-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)-2-ethylphe noxy)-3,4,5-trihydroxytetrahydro-2H-pyran-2-carboxylate trisodium;

(2S,3S,4S,5R,6S)-6-(2-amino-5-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxo hexyl)-1H-1,2,3-triazol-4-yl)methoxy)-4-((((S)-4,11-diethyl-4-hydroxy-3,14-dioxy-3, 4,12,14-tetrahydro-1H-pyrano[3,4':6,7]indolazino[1,2-b]quinolin-9-yl)oxy)methyl)ph enoxy)-3,4,5-trihydroxytetrahydro-2H-pyran-2-carboxylate trisodium;

(2S,3S,4S,5R,6S)-6-(5-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)-2-(dimeth ylamino)phenoxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-6-fluoro-2 -methoxyphenoxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-6-chloro-4-((((S)-4,11-diethyl-4-hydroxy-3,14-dioxy-3 ,4,12,14-tetrahydro-1H-pyrano[3',4':6,7]indolazino[1,2-b]quinolin-9-yl)oxy)methyl)-5-fluoro-2-methylphenoxy)-3,4,5-trihydroxytetrahydro-2H-pyran-2-carboxylate trisodium;

(2S,3S,4S,SR,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-6-bromo-4-((((S)-4,11-diethyl-4-hydroxy-3,14-dioxy-3 ,4,12,14-tetrahydro-1H-pyrano[3',4':6,7]indolazino[1,2-b]quinolin-9-yl)oxy)methyl)-2-methoxyphenoxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-9-ethyl-9-hydroxy-10,13-dioxy-2,3,9,10,13,15 -tetrahydro-1H,7]benzo[de]pyrano[3',4':6,7]indolizino[1,2-b]quinolin-4-yl)oxy)meth yl)phenoxy)-3,4,5-trihydroxytetrahydro-2H-pyran-2-carboxylate trisodium;

(2S,3S,4S,5R,6S)-6-(4-(((S)-4-acetoxy-4-ethyl-3,14-dioxo-3,4,12,14-tetra hydro-1H-pyrano[3',4':6,7]indolazino [1,2-b]quinolin-9-yl)oxy)methyl)-3-((1-(6-((4'-((E)-(8-amino-1-hydroxy-5,7-disulfonicnaphthalen-2-yl)diazenyl)-3,3'-dimethyl-[1,1' -biphenyl]-4-yl)amino)-6-oxohexyl)-1H-1,2,3-triazol-4-yl)methoxy)phenoxy)-3,Triso dium4,5-trihydroxytetrahydro-2H-pyran-2-carboxylate;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4-ethyl-3,14-dioxo-4-(propionyloxy)-3,4,12,14 -tetrahydro-1H-pyrano[3',4':6,7]indolazino[1,2-b]quinolin-9-yl)oxy)methyl)phenoxy) -3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-(((S)-4-ethyl-4-(glycyloxy)-3,14-dioxy-3,4,12,14-tetr ahydro-1H-pyrano[3',4':6,7]indolizino[1,2-b]quinolin-9-yl)oxy)methyl)phenoxy)-3,4, 5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((R)-5-ethyl-5-hydroxy-3,15-dioxo-4,5,13,15-tetrah ydro-1H,3H-oxazepino[3',4':6,7]indolizino[1,2-b]quinolin-10-yl)oxy)methyl)phenox y)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-(((S)-4-ethyl-4-hydroxy-10-(methoxymethyl)-3,14-di oxy-3,4,12,14-tetrahydro-1H-pyrano[3',4':6,7]indolazino[1,2-b]quinolin-9-yl)oxy) me thyl)phenoxy)-3,4,5-trihydroxytetrahydro-2H-pyran-2-carboxylate trisodium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-(((S)-4-ethyl-4-hydroxy-10-((isopropylthio)methyl)-3,14-dioxy-3,4,12,14-tetrahydro-1H-pyrano[3',4':6,7]indolazino[1,2-b]quinolin-9-yl) oxy)methyl)

phenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4-ethyl-4-hydroxy-3-oxy-14-thio-3,4,12,14-tet rahydro-1H-pyrano[3',4':6,7]indolazino[1,2-b]quinolin-9-yl)oxy)methyl)phenoxy)-3, 4,5-trihydroxytetrahydro-2H-pyran-2-carboxylate trisodium;
(2S,3S,4S,SR,6S)-6-(3-((1-((2-((2-((2-((4'-(E)-(8-amino-1-hydroxy-5,7-d isulfonicnaphthalen-2-yl)diazenyl)-3,3'-dimethyl-[1,1'-biphenyl]-4-yl)amino)-2-oxoet hyl)(methyl)amino)-2-oxoethyl)(methyl)amino)-2-oxoethyl)-1H-1,2,3-triazol-4-yl)me thoxy)-4-((((S)-4,11-diethyl-4-hydroxy-3,14-dioxo-3,4,12,14-tetrahydro-1H-pyrano[3 ',4':6,7]indolazino[1,2-b]quinolin-9-yl)oxy)methyl)phenoxy)-3,4,5-trihydroxytetrahy dro-2H-pyran-2-carboxylate trisodium;
(2S,3S,4S,5R,6S)-6-(3-((1-((2-((2-((2-((4'-(E)-(8-amino-1-hydroxy-5,7-d isulfonicnaphthalen-2-yl)diazenyl)-3,3'-dimethyl-[1,1'-biphenyl]-4-yl)amino)-2-oxoet hyl)amino)-2-oxoethyl)amino)-2-oxoethyl)-1H-1,2,3-triazol-4-yl)methoxy)-4-((((S)-4 ,11-diethyl-4-hydroxy-3,14-dioxo-3,4,12,14-tetrahydro-1H-pyrano[3',4':6,7]indolazin o[1,2-b]quinolin-9-yl)oxy)methyl)phenoxy)-3,4,5-trihydroxytetrahydro-2H-pyran-2-c arboxylate trisodium;
(2S,3S,4S,5R,6S)-6-(3-((1-(2-((S)-1-((S)-1-((4'-((E)-(8-amino-1-hydroxy -5,7-disulfonicnaphthalen-2-yl)diazenyl)-3,3'-dimethyl-[1,1'-biphenyl]-4-yl)amino)-1 -oxopropan-2-yl)amino)-1-oxopropan-2-yl)amino)-2-oxoethyl)-1H-1,2,3-triazol-4-yl) methoxy)-4-((((S)-4,11-diethyl-4-hydroxy-3,14-dioxo-3,4,12,14-tetrahydro-1H-pyran o[3',4':6,7]indolazino[1,2-b]quinolin-9-yl)oxy)methyl)phenoxy)-3,4,5-trihydroxytetra hydro-2H-pyran-2-carboxylate trisodium;
(2S,3S,4S,SR,6S)-6-(3-((1-(2-((S)-1-((S)-1-((4'-((E)-(8-amino-1-hydroxy -5,7-disulfonicnaphthalen-2-yl)diazenyl)-3,3'-dimethyl-[1,1'-biphenyl]-4-yl)amino)-1 -oxo-3-phenylpropan-2-yl)amino)-1-oxopropan-2-yl)amino)-2-oxoethyl)-1H-1,2,3-tri azol-4-yl)methoxy)-4-((((S)-4,11-diethyl-4-hydroxy-3,14-dioxy-3,4,12,14-tetrahydro-1H-pyrano [3',4':6,7]indolazino[1,2-b]quinolin-9-yl)oxy)methyl)phenoxy)-3,4,5-trihy droxytetrahydro-2H-pyran-2-carboxylate trisodium;
(2S,3S,4S,5R,6S)-6-(3-((1-(2-(2-(2-(2-((4'-((E)-(8-amino-1-hydroxy-5,7-disulfonicnaphthalen-2-yl)diazenyl)-3,3'-dimethyl-[1,1'-biphenyl]-4-yl)amino)-2-oxo ethoxy)ethoxy)ethoxy)ethyl)-1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[1,2-b]quino lin-9-yl)oxy)methyl)phenoxy)-3,4,5-trihydroxytetrahydro-2H-pyran-2-carboxylate trisodium;
(2S,3S,4S,SR,6S)-6-(3-((1-((2-((2-((2-((2-((4'-(E)-(8-amino-1-hydroxy-5, 7-disulfonicnaphthalen-2-yl)diazenyl)-3,3'-dimethyl-[1,1'-biphenyl]-4-yl)amino)-2-o xoethyl)amino)ethyl)amino)ethyl)amino)ethyl)-1H-1,2,3-triazol-4-yl)methoxy)-4-(((( S)-4,11-diethyl-4-hydroxy-3,14-dioxy-3,4,12,14-tetrahydro-1H-pyrano[3',4':6,7]indo lazino[1,2-b]quinolin-9-yl)oxy)methyl)phenoxy)-3,4,5-trihydroxytetrahydro-2H-pyra n-2-carboxylate trisodium;
(2S,3S,4S,5R,6S)-6-(3-((1-(3-(3-(3-((4'-((E)-(8-amino-1-hydroxy-5,7-dis ulfonicnaphthalen-2-yl)diazenyl)-3,3'-dimethyl-[1,1'-biphenyl]-4-yl)amino)-3-oxopro poxy)propoxy)propyl)-1H-1,2,3-triazol-4-yl)methoxy)-4-(((S)-4,11-diethyl-4-hydroxy -3,14-dioxy-3,4,12,14-tetrahydro-1H-pyrano[3',4':6,7]indolazino[1,2-b]quinolin-9-yl) oxy)methyl)phenoxy)-3,4,5-trihydroxytetrahydro-2H-pyran-2-carboxylate trisodium;
(2S,3S,4S,5R,6S)-6-(3-((1-(4-((4'-((E)-(8-amino-1-hydroxy-5,7-disulfoni enaphthalen-2-yl)diazenyl)-3,3'-dimethyl-[1,1'-biphenyl]-4-yl)carbamoyl)phenethyl)-1H-1,2,3-triazol-4-yl)methoxy)-4-((((S)-4,11-diethyl-4-hydroxy-3,14-dioxy-3,4,12,14 -tetrahydro-1H-pyrano[3',4':6,7]indolazino[1,2-b]quinolin-9-yl)oxy)methyl)phenoxy) -3,4,5-trihydroxytetrahydro-2H-pyran-2-carboxylate trisodium;
(2S,3S,4S,5R,6S)-6-(3-((1-(2-(2-((4'-((E)-(8-amino-1-hydroxy-5,7-disulf onicnaphthalen-2-yl)diazenyl)-3,3'-dimethyl-[1,1'-biphenyl]-4-yl)carbamoyl)naphthal en-2-yl)ethyl)-1H-1,2,3-triazol-4-yl)methoxy)-4-((((S)-4,11-diethyl-4-hydroxy-3,14-d ioxy-3,4,12,14-tetrahydro-1H-pyrano[3',4':6,7]indolazino[1,2-b]quinolin-9-yl)oxy)m ethyl)phenoxy)-3,4,5-trihydroxytetrahydro-2H-pyran-2-carboxylate trisodium;
(2S,3S,4S,5R,6S)-6-(3-((1-(2-(4'-((4'-((E)-(8-amino-I-hydroxy-5,7-disul fonicnaphthalen-2-yl)diazenyl)-3,3'-dimethyl-[1,1'-biphenyl]-4-yl)carbamoyl)-[1,1'-b iphenyl]-4-yl)ethyl)-1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[1,2-b]quinolin-9-yl) oxy)methyl)phenoxy)-3,4,5-trihydroxytetrahydro-2H-pyran-2-carboxylate trisodium;
(2S,3S,4S,5R,6S)-6-(3-((1-(4-(4-((4'-((E)-(8-amino-1-hydroxy-5,7-disulf onicnaphthalen-2-yl)diazenyl)-3,3'-dimethyl-[1,1'-biphenyl]-4-yl)amino)-4-oxobutyl) phenethyl)-1H-1,2,3-triazol-4-yl)methoxy)-4-((((S)-4,11-diethyl-4-hydroxy-3,14-diox y-3,4,12,14-tetrahydro-1H-pyrano[3',4':6,7]indolazino[1,2-b]quinolin-9-yl)oxy)meth yl)phenoxy)-3,4,5-trihydroxytetrahydro-2H-pyran-2-carboxylate trisodium;
(2S,3S,4S,5R,6S)-6-(3-((1-(2-(2-(6-(4-((4'-((E)-(8-amino-1-hydroxy-5,7-disulfonicnaphthalen-2-yl)diazenyl)-3,3'-dimethyl-[1,1'-biphenyl]-4-yl)amino)-4-oxo butyl)pyridin-3-yl)ethyl)-1H-1,2,3-triazol-4-yl)methoxy)-4-((((S)-4,11-diethyl-4-hydr oxy-3,14-dioxy-3,4,12,14-tetrahydro-1H-pyrano[3',4':6,7]indolizino[1,2-b]quinolin-9 -yl)oxy)methyl)phenoxy)-3,4,5-trihydroxytetrahydro-2H-pyran-2-carboxylate trisodium;
(2S,3S,4S,5R,6S)-6-(3-((1-(2-(2-(5-(4-((4'-((E)-(8-amino-1-hydroxy-5,7-disulfonicnaphthalen-2-yl)diazenyl)-3,3'-dimethyl-[1,1'-biphenyl]-4-yl)amino)-4-oxo butyl)thiophen-3-yl)ethyl)-1H-1,2,3-triazol-4-yl)methoxy)-4-((((S)-4,11-diethyl-4-hy droxy-3,14-dioxy-3,4,12,14-tetrahydro-1H-pyrano[3',4':6,7]indolazino[1,2-b]quinoli n-9-yl)oxy)methyl)

phenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3',5,5'-tetramethyl-[1,1'-biphenyl]-4-yl)amino)-6-oxohe xyl)-1H-1,2,3-triazol-4-yl)methoxy)-4-((((S)-4,11-diethyl-4-hydroxy-3,14-dioxy-3,4,1 2,14-tetrahydro-1H-pyrano[3',4':6,7]indolazino[1,2-b]quinolin-9-yl)oxy)methyl)phen oxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-2,2',3,3',5,5',6,6'-octamethyl-[1,1'-biphenyl]-4-yl)amino) -6-oxohexyl)-1H-1,2,3-triazol-4-yl)methoxy)-4-((((S)-4,11-diethyl-4-hydroxy-3,14-di oxy-3,4,12,14-tetrahydro-1H-pyrano[3',4':6,7]indolazino[1,2-b]quinolin-9-yl)oxy)me thyl)phenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-2,2',3,3',5,5',6,6'-octamethoxy-[1,1'-biphenyl]-4-yl)amin o)-6-oxohexyl)-1H-1,2,3-triazol-4-yl)methoxy)-4-((((S)-4,11-diethyl-4-hydroxy-3,14-dioxy-3,4,12,14-tetrahydro-1H-pyrano[3',4' :6,7]indolazino[1,2-b]quinolin-9-yl)oxy) methyl)phenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3',5,5'-tetrachloro-[1,1'-biphenyl]-4-yl)amino)-6-oxohe xyl)-1H-1,2,3-triazol-4-yl)methoxy)-4-((((S)-4,11-diethyl-4-hydroxy-3,14-dioxy-3,4,1 2,14-tetrahydro-1H-pyrano[3',4':6,7]indolazino[1,2-b]quinolin-9-yl)oxy)methyl)phen oxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-2,2',3,3',5,5',6,6'-octafluoro-[1,1'-biphenyl]-4-yl)amino)-6-oxohexyl)-1H-1,2,3-triazol-4-yl)methoxy)-4-((((S)-4,11-diethyl-4-hydroxy-3,14-dio xy-3,4,12,14-tetrahydro-1H-pyrano[3',4':6,7]indolazino[1,2-b]quinolin-9-yl)oxy)met hyl)phenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3'-chloro-3-methoxy-5,5'-dimethyl-[1,1'-biphenyl]-4-yl)a mino)-6-oxohexyl)-1H-1,2,3-triazol-4-yl)methoxy)-4-((((S)-4,11-diethyl-4-hydroxy-3 ,14-dioxy-3,4,12,14-tetrahydro-1H-pyrano[3,4':6,7]indolazino[1,2-b]quinolin-9-yl)ox y)methyl) phenoxy)-3,4,5-trihydroxytetrahydro-2H-pyran-2-carboxylate trisodium;
(2S,3S,4S,5R,6S)-6-(3-((1-(6-((6'-((E)-(8-amino-1-hydroxy-5,7-disulfoni enaphthalen-2-yl)diazenyl)-[1,1':3',1'':3'',1'''-quaterphenyl]-4''-yl)-amino)-6-oxohe xyl)-1H-1,2,3-triazol-4-yl)methoxy)-4-(((((S)-4,11-diethyl-4-hydroxy-3,14-dioxy-3,4, 12,14-tetrahydro-1H-pyrano[3',4':6,7]indolizino[1,2-b]quinolin-9-yl)oxy)methyl) phe noxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni enaphthalen-2-yl)diazenyl)-3,3'-difluoro-[1,1'-biphenyl]-4-yl)amino)-6-oxohexyl)-1H -1,2,3-triazol-4-yl)methoxy)-4-(((S)-4,11-diethyl-4-hydroxy-3,14-dioxy-3,4,12,14-tetr ahydro-1H-pyrano[3',4':6,7]indolizino[1,2-b]quinolin-9-yl)oxy)methyl)phenoxy)-3,4, 5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dichloro-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)phenoxy)-3 ,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni enaphthalen-2-yl)diazenyl)-3,3'-dibromo-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;
(2S,3S,4S,SR,6S)-6-(3-((1-(5-((4'-((E)-(8-amino-1-hydroxy-5,7-disulfoni cnaphthalen-2-yl)diazenyl)-3,3'-dimethyl-[1,1'-biphenyl]-4-yl)amino)-5-oxopentyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)phenoxy)-3 ,4,5-trihydroxytetrahydro-2H-pyran-2-carboxylate trisodium;
(2S,3S,4S,5R,6S)-6-(3-((1-(2-((4'-((E)-(8-amino-1-hydroxy-5,7-disulfoni cnaphthalen-2-yl)diazenyl)-3,3'-dimethyl-[1,1'-biphenyl]-4-yl)amino)-2-oxoethyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;
(2S,3S,4S,5R,6S)-6-(3-((1-(3-(2-((4'-(E)-(8-amino-1-hydroxy-5,7-disulfo nicnaphthalen-2-yl)diazenyl)-3,3'-dimethyl-[1,1'-biphenyl]-4-yl)amino)-2-oxoethoxy )propyl)-1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[1,2-b]quinolin-9-yl)oxy)methyl) phenoxy)-3,4,5-trihydroxytetrahydro-2H-pyran-2-carboxylate trisodium; and
(2S,3S,4S,5R,6S)-6-(3-((1-(2-(2-((4'-((E)-(8-amino-1-hydroxy-5,7-disulf onicnaphthalen-2-yl)diazenyl)-3,3'-dimethyl-[1,1'-biphenyl]-4-yl)amino)-3 -oxopropo xy)ethyl)-1H-1,2,3-triazol-4-yl)methoxy)-4-((((S)-4,11-diethyl-4-hydroxy-3,14-dioxy -3,4,12,14-tetrahydro-1H-pyrano[3',4':6,7]indolazino[1,2-b]quinolin-9-yl)oxy)methyl )phenoxy)-3,4,5-trihydroxytetrahydro-2H-pyran-2-carboxylate trisodium.

In yet another aspect, the present disclosure relates to a process for preparing a compound of Formula (I):

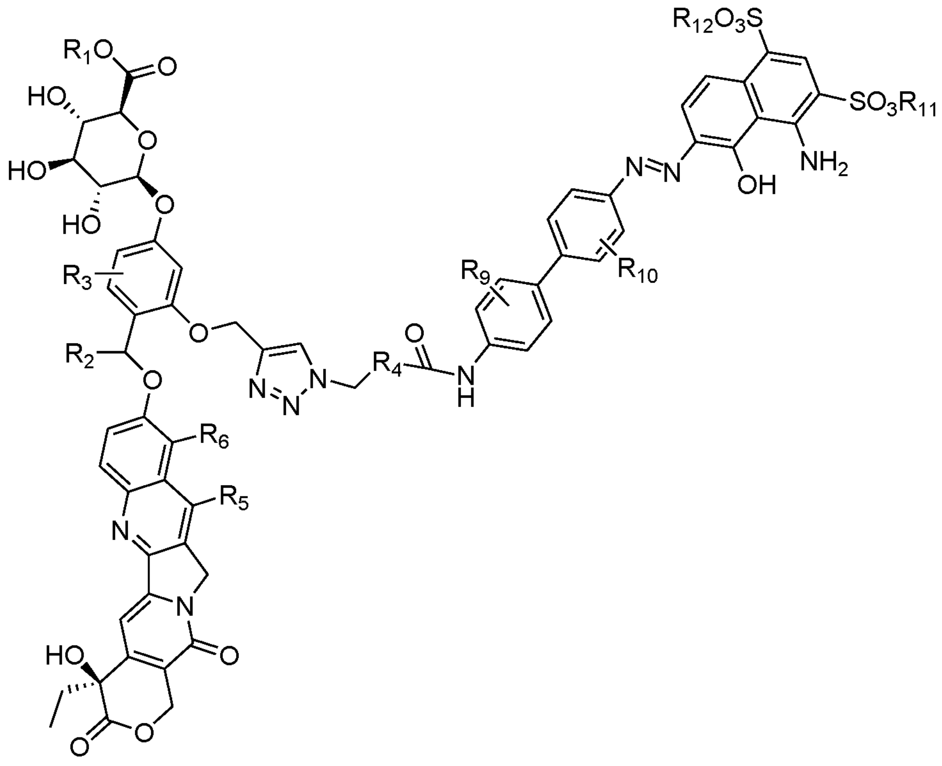

Formula (I)

wherein

$R_1$ is selected from the group consisting of hydrogen, $C_1$-$C_8$ hydrocarbyl, -Na, -Li, -K, -Cs, $-NH_4^+$, $-NH_2^+R_{13}R_{14}$, $-NH^+R_{13}R_{14}R_{15}$, $-NH^+{}_3R_{13}$, and natural or unnatural amino acids;
$R_2$ is selected from the group consisting of hydrogen, and $C_1$-$C_8$ hydrocarbyl;
$R_3$ is one or more substituents at any substitution position selected from the group consisting of hydrogen, halogen, $C_1$-$C_8$ hydrocarbyl, $C_1$-$C_8$ hydrocarbyloxy, cyano, nitro, amino, aryl, arylhydrocarbyl, and $C_1$-$C_4$ hydrocarbyl substituted amino;
$R_4$ is selected from the group consisting of $C_1$-$C_9$ alkylidene, $C_1$-$C_8$ alkylideneoxy, $C_1$-$C_8$ hydrocarbyl substituted or unsubstituted $C_3$-$C_8$ heteroaryl, $C_1$-$C_8$ hydrocarbyl substituted or unsubstituted $C_3$-$C_{15}$ aryl, $-(CH_2R_7)_n$, $-(R_8)_m$, $C_1$-$C_9$ alkylidene-arylidene, $C_1$-$C_9$ alkylidene-arylidene-$C_1$-$C_9$ alkyldiene, $C_1$-$C_9$ alkylidene-arylidene-arylidene, $C_1$-$C_9$ alkylidene-heteroaryliden-$C_1$-$C_9$ alkylidene, and amino acid peptide chain; wherein the amino acid peptide chain is composed of a combination of natural or unnatural amino acids, and the peptide chain length is 1 to100 peptides, preferably 1 to 50 peptides, and more preferably 1 to 20 peptides;
$R_5$ is selected from the group consisting of hydrogen, and $C_1$-$C_8$ hydrocarbyl;
$R_6$ is selected from the group consisting of hydrogen, $C_1$-$C_8$ hydrocarbyl, $C_1$-$C_8$ hydrocarbylidene amino ($C_1$-$C_4$ hydrocarbyl)$_2$, $C_1$-$C_8$ hydrocarbylidene thio $C_1$-$C_8$ hydrocarbyl, and $C_1$-$C_9$ alkylidene-oxy-$C_1$-$C_9$ alkyl;
$R_5$ and $R_6$ may form a ring, and if $R_5$ and $R_6$ form a ring, the ring may be a five-membered ring or a six-membered ring;
$R_7$ is selected from hydrogen, $C_1$-$C_8$ hydrocarbylideneoxy, amino $C_1$-$C_8$ hydrocarbylidene, thio $C_1$-$C_8$ hydrocarbylidene, and oxy $C_1$-$C_8$ hydrocarbylidene;
n is selected from the group consisting of 0, 1, 2, 3, 4, 5, 6, 7, 8, and 9; if n is 0, $(CH_2R_7)_n$ represents a covalent bond;
$R_8$ is carbonylamino, wherein amino may be substituted with $C_1$-$C_8$ hydrocarbyl or arylhydrocarbyl, $C_1$-$C_8$ hydrocarbylidene amino $C_1$-$C_8$ hydrocarbylidene, $C_1$-$C_8$ hydrocarbylidene thio $C_1$-$C_8$ hydrocarbylidene, or $C_1$-$C_8$ hydrocarbylidene oxy $C_1$-$C_8$ hydrocarbylidene;
m is selected from the group consisting of 0, 1, 2, 3, 4, 5, 6, 7, 8, and 9; if m is 0, $-(R_8)_m$ represents a covalent bond;
$R_9$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;
$R_{10}$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;
$R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;
$R_{13}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;
$R_{14}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;
$R_{15}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl; and
the natural or unnatural amino acids in $R_1$, $R_{11}$, and $R_{12}$ are selected from the group consisting of glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine;
wherein the process comprises:

(1) reacting the compound of Formula (A-I) with a compound of Formula (A-II) to obtain a compound of Formula (A-III),

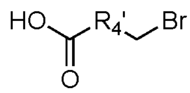

Formula (A-I)

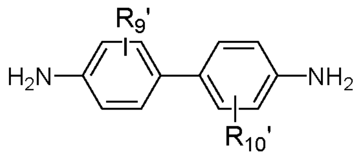

Formula (A-II)

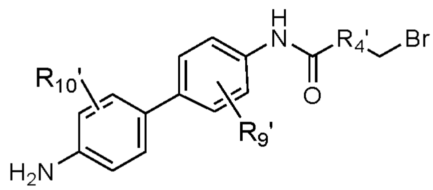

Formula (A-III)

wherein the groups represented by $R_4$', $R_9$', and $R_{10}$' in the Formula (A-I) and the Formula (A-II) are as defined for $R_4$, $R_9$ and $R_{10}$ in the Formula (I);
(2) reacting the compound of Formula (A-III) with sodium azide to obtain a compound of Formula (A-IV),

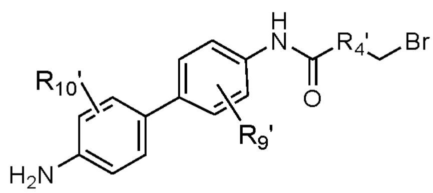

Formula (A-III)

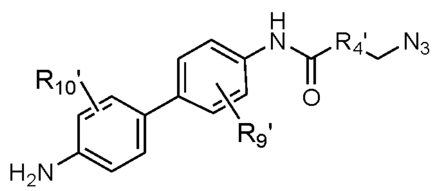

Formula (A-IV)

wherein the groups represented by $R_4$', $R_9$', and $R_{10}$' in the Formula (A-III) and the Formula (A-IV) are as defined for $R_4$, $R_9$ and $R_{10}$ in the Formula (I);

(3) reacting the compound of Formula (A-IV) with 4-amino-5-hydroxy-1,3-naphthalene disulfonic acid monosodium to obtain a compound of Formula (A-V),

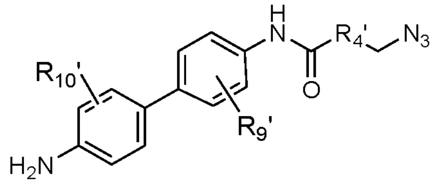

Formula (A-IV)

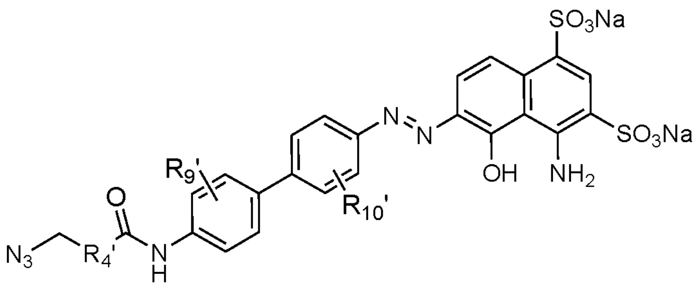

;

Formula (A-V)

wherein the groups represented by $R_4$', $R_9$', and $R_{10}$' in the Formula (A-IV) and the Formula (A-V) are as defined for $R_4$, $R_9$ and $R_{10}$ in the Formula (I);

(4) reacting the compound of Formula (B-I) with carbon tetrabromide to obtain a compound of Formula (B-II),

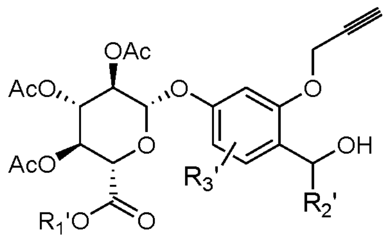

Formula (B-I)

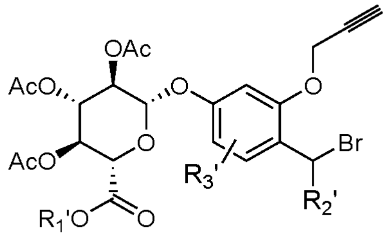

Formula (B-II)

wherein the groups represented by $R_1$', $R_2$', and $R_3$' in the Formula (B-I) and the Formula (B-II) are as defined for $R_1$, $R_2$ and $R_3$ in the Formula (I);

(5) reacting the compound of Formula (B-II) with a compound of Formula (B-III) to obtain a compound of Formula (B-IV)

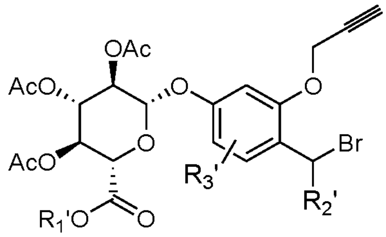

Formula (B-II)

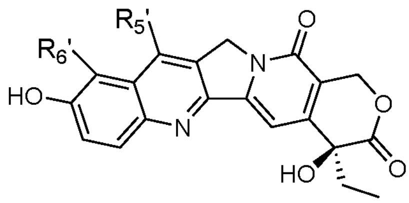

Formula (B-III)

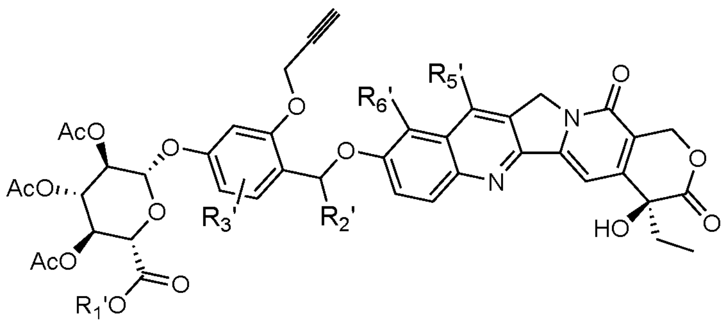

Formula (B-IV)

wherein the groups represented by $R_1$', $R_2$', $R_3$', $R_5$', and $R_6$' in the Formula (B-II), the Formula (B-III), and the Formula (B-IV) are as defined for $R_1$, $R_2$, $R_3$, $R_5$, and $R_6$ in the Formula (I);

(6) reacting the compound of Formula (B-IV) under basic conditions to obtain a compound of Formula (B-V),

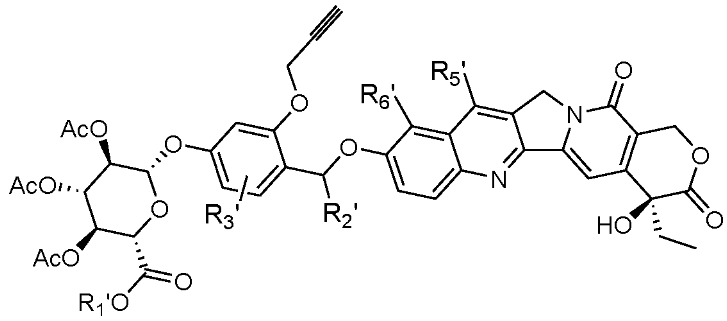

Formula (B-IV)

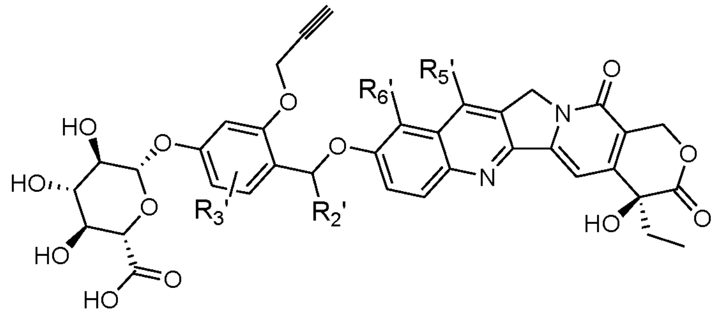

Formula (B-V)

wherein the groups represented by $R_1'$, $R_2'$, $R_3'$, $R_5'$, and $R_6'$ in the Formula (B-IV) and the Formula (B-V) are as defined for $R_1$, $R_2$, $R_3$, $R_5$ and $R_6$ in the Formula (I);

(7) reacting the compound of Formula (A-V) with the compound of Formula (B-V) to obtain the compound of Formula (I), or reacting a product obtained by reacting the compound of Formula (A-V) with the compound of Formula (B-V) with a corresponding acid or base to form a pharmaceutically acceptable salt thereof

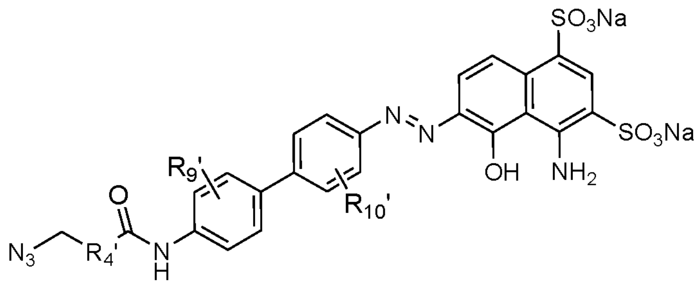

Formula (A-V)

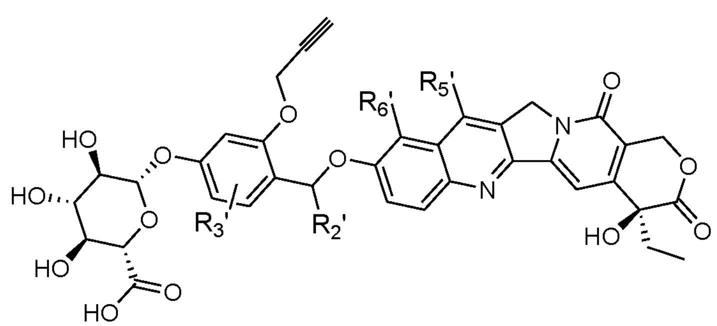

Formula (B-V)

wherein the groups represented by $R_2'$, $R_3'$, $R_4'$, $R_5'$, $R_6'$, $R_9'$, and $R_{10}'$ in the Formula (A-V) and the Formula (B-V) are defined as $R_2$, $R_3$, $R_4$, $R_5$, $R_6$, $R_9$, and $R_{10}$ in the Formula (I).

In still yet another aspect, the present disclosure relates to a compound of Formula (I), a stereoisomer, or a cis-trans isomer thereof for the manufacture of a medicament for the treatment of a cancer or tumor:

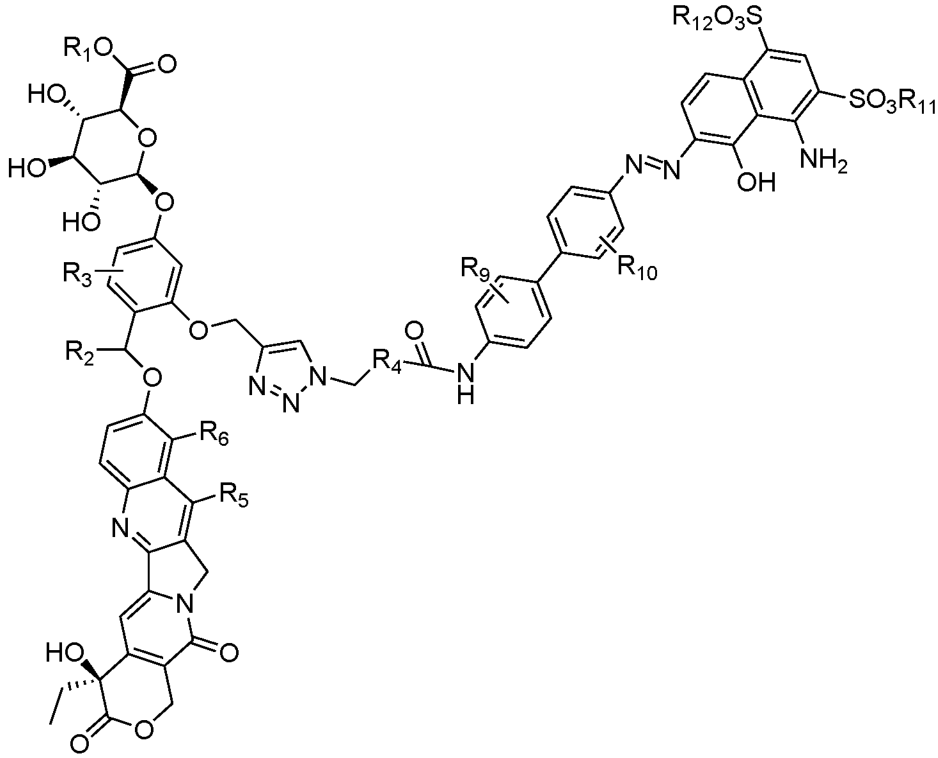

Formula (I)

wherein

$R_1$ is selected from the group consisting of hydrogen, $C_1$-$C_8$ hydrocarbyl, -Na, -Li, -K, -Cs, $-NH_4^+$, $-NH_2^+R_{13}R_{14}$, $-NH^+R_{13}R_{14}R_{15}$, $-NH^+{}_3R_{13}$, and natural or unnatural amino acids;

$R_2$ is selected from the group consisting of hydrogen, and $C_1$-$C_8$ hydrocarbyl;

$R_3$ is one or more substituents at any substitution position selected from the group consisting of hydrogen, halogen, $C_1$-$C_8$ hydrocarbyl, $C_1$-$C_8$ hydrocarbyloxy, cyano, nitro, amino, aryl, arylhydrocarbyl, and $C_1$-$C_4$ hydrocarbyl substituted amino;

$R_4$ is selected from the group consisting of $C_1$-$C_9$ alkylidene, $C_1$-$C_8$ alkylideneoxy, $C_1$-$C_8$ hydrocarbyl substituted or unsubstituted $C_3$-$C_8$ heteroaryl, $C_1$-$C_8$ hydrocarbyl substituted or unsubstituted $C_3$-$C_{15}$ aryl, $-(CH_2R_7)_n$, $-(R_8)_m$, $C_1$-$C_9$ alkylidene-arylidene, $C_1$-$C_9$ alkylidene-arylidene-$C_1$-$C_9$ alkyldiene, $C_1$-$C_9$ alkylidene-arylidene-arylidene, $C_1$-$C_9$ alkylidene-heteroaryliden-$C_1$-$C_9$ alkylidene, and amino acid peptide chain; wherein the amino acid peptide chain is composed of a combination of natural or unnatural amino acids, and the peptide chain length is 1 to100 peptides, preferably 1 to 50 peptides, and more preferably 1 to 20 peptides;

$R_5$ is selected from the group consisting of hydrogen, and $C_1$-$C_8$ hydrocarbyl;

$R_6$ is selected from the group consisting of hydrogen, $C_1$-$C_8$ hydrocarbyl, $C_1$-$C_8$ hydrocarbylidene amino ($C_1$-$C_4$ hydrocarbyl)$_2$, $C_1$-$C_8$ hydrocarbylidene thio $C_1$-$C_8$ hydrocarbyl, and $C_1$-$C_9$ alkylidene-oxy-$C_1$-$C_9$ alkyl;

$R_5$ and $R_6$ may form a ring, and if $R_5$ and $R_6$ form a ring, the ring may be a five-membered ring or a six-membered ring;

$R_7$ is selected from hydrogen, $C_1$-$C_8$ hydrocarbylideneoxy, amino $C_1$-$C_8$ hydrocarbylidene, thio $C_1$-$C_8$ hydrocarbylidene, and oxy $C_1$-$C_8$ hydrocarbylidene;

n is selected from the group consisting of 0, 1, 2, 3, 4, 5, 6, 7, 8, and 9; if n is 0, $(CH_2R_7)_n$ represents a covalent bond;

$R_8$ is carbonylamino, wherein amino may be substituted with $C_1$-$C_8$ hydrocarbyl or arylhydrocarbyl, $C_1$-$C_8$ hydrocarbylidene amino $C_1$-$C_8$ hydrocarbylidene, $C_1$-$C_8$ hydrocarbylidene thio $C_1$-$C_8$ hydrocarbylidene, or $C_1$-$C_8$ hydrocarbylidene oxy $C_1$-$C_8$ hydrocarbylidene;

m is selected from the group consisting of 0, 1, 2, 3, 4, 5, 6, 7, 8, and 9; if m is 0, $-(R_8)_m$ represents a covalent bond;

$R_9$ is one or more substituents at any position selected from the group consisting of hydrogen, halogen, cyano, $C_1$-$C_8$

hydrocarbyl, $C_1$-$C_8$ alkoxy, and $C_5$-$C_{12}$ aryl;

$R_{10}$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{13}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{14}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{15}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl; and

the natural or unnatural amino acids in $R_1$, $R_{11}$, and $R_{12}$ are selected from the group consisting of glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine.

In some embodiments, a compound of Formula (I), or a stereoisomer thereof, can be used in the manufacture of a medicament for the treatment of a cancer or tumor, wherein the cancer includes, but is not limited to, colorectal cancer, lung cancer, cervical cancer, ovarian cancer, gastric cancer, esophageal cancer, breast cancer, pancreatic cancer, bladder cancer, liver cancer, gastric cancer, colon cancer, head and neck cancer, uterine cancer, urothelial cancer, osteosarcoma, sarcoma, renal cancer, melanoma, prostate cancer, glioma, glioma, and leukemia.

**In** still another aspect, the present disclosure relates to a pharmaceutical composition, comprising a compound of Formula (I), a stereoisomer, or a cis-trans isomer thereof, and a pharmaceutically acceptable carrier:

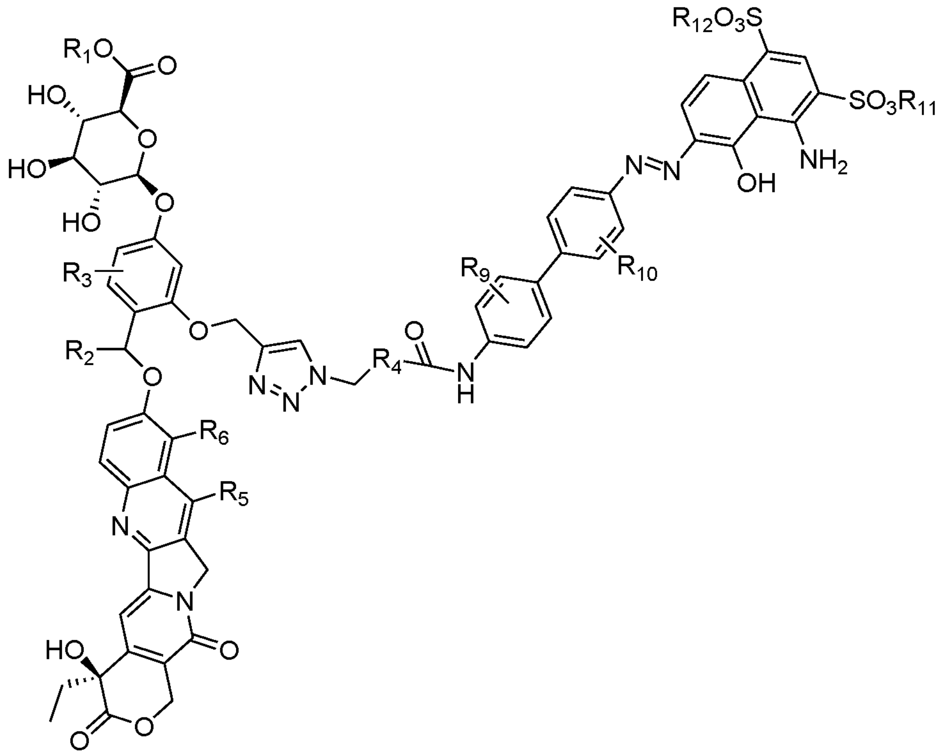

Formula (I)

wherein

$R_1$ is selected from the group consisting of hydrogen, $C_1$-$C_8$ hydrocarbyl, -Na, -Li, -K, -Cs, $-NH_4^+$, $-NH_2^+R_{13}R_{14}$, $-NH^+R_{13}R_{14}R_{15}$, $-NH^+_3R_{13}$, and natural or unnatural amino acids;

$R_2$ is selected from the group consisting of hydrogen, and $C_1$-$C_8$ hydrocarbyl;

$R_3$ is one or more substituents at any substitution position selected from the group consisting of hydrogen, halogen, $C_1$-$C_8$ hydrocarbyl, $C_1$-$C_8$ hydrocarbyloxy, cyano, nitro, amino, aryl, arylhydrocarbyl, and $C_1$-$C_4$ hydrocarbyl substituted amino;

$R_4$ is selected from the group consisting of $C_1$-$C_9$ alkylidene, $C_1$-$C_8$ alkylideneoxy, $C_1$-$C_8$ hydrocarbyl substituted or unsubstituted $C_3$-$C_8$ heteroaryl, $C_1$-$C_8$ hydrocarbyl substituted or unsubstituted $C_3$-$C_{15}$ aryl, $-(CH_2R_7)_n$, $-(R_8)_m$, $C_1$-$C_9$ alkylidene-arylidene, $C_1$-$C_9$ alkylidene-arylidene-$C_1$-$C_9$ alkyldiene, $C_1$-$C_9$ alkylidene-arylidene-arylidene, $C_1$-$C_9$ alkylidene-heteroaryliden-$C_1$-$C_9$ alkylidene, and amino acid peptide chain; wherein the amino acid peptide chain is composed of a combination of natural or unnatural amino acids, and the peptide chain length is 1 to100 peptides, preferably 1 to 50 peptides, and more preferably 1 to 20 peptides;

$R_5$ is selected from the group consisting of hydrogen, and $C_1$-$C_8$ hydrocarbyl;

$R_6$ is selected from the group consisting of hydrogen, $C_1$-$C_8$ hydrocarbyl, $C_1$-$C_8$ hydrocarbylidene amino ($C_1$-$C_4$ hydrocarbyl)$_2$, $C_1$-$C_8$ hydrocarbylidene thio $C_1$-$C_8$ hydrocarbyl, and $C_1$-$C_9$ alkylidene-oxy-$C_1$-$C_9$ alkyl;

$R_5$ and $R_6$ may form a ring, and if $R_5$ and $R_6$ form a ring, the ring may be a five-membered ring or a six-membered ring;

$R_7$ is selected from hydrogen, $C_1$-$C_8$ hydrocarbylideneoxy, amino $C_1$-$C_8$ hydrocarbylidene, thio $C_1$-$C_8$ hydrocarbylidene, and oxy $C_1$-$C_8$ hydrocarbylidene;

n is selected from the group consisting of 0, 1, 2, 3, 4, 5, 6, 7, 8, and 9; if n is 0, $(CH_2R_7)_n$ represents a covalent bond;

$R_8$ is carbonylamino, wherein amino may be substituted with $C_1$-$C_8$ hydrocarbyl or arylhydrocarbyl, $C_1$-$C_8$ hydrocarbylidene amino $C_1$-$C_8$ hydrocarbylidene, $C_1$-$C_8$ hydrocarbylidene thio $C_1$-$C_8$ hydrocarbylidene, or $C_1$-$C_8$ hydrocarbylidene oxy $C_1$-$C_8$ hydrocarbylidene;

m is selected from the group consisting of 0, 1, 2, 3, 4, 5, 6, 7, 8, and 9; if m is 0, $-(R_8)_m$ represents a covalent bond;

$R_9$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{10}$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{13}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{14}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{15}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl; and

the natural or unnatural amino acids in $R_1$, $R_{11}$, and $R_{12}$ are selected from the group consisting of glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine.

In yet another aspect, the present disclosure relates to a pharmaceutical composition, comprising a compound of Formula (I), a stereoisomer, or a cis-trans isomer thereof, and a pharmaceutically acceptable carrier, solvent, osmotic pressure modifier, pH modifier, solubilizer, cosolvent, antioxidant, bacteriostat, emulsifier, suspending agent, filler, complexing agent, and chelating agent:

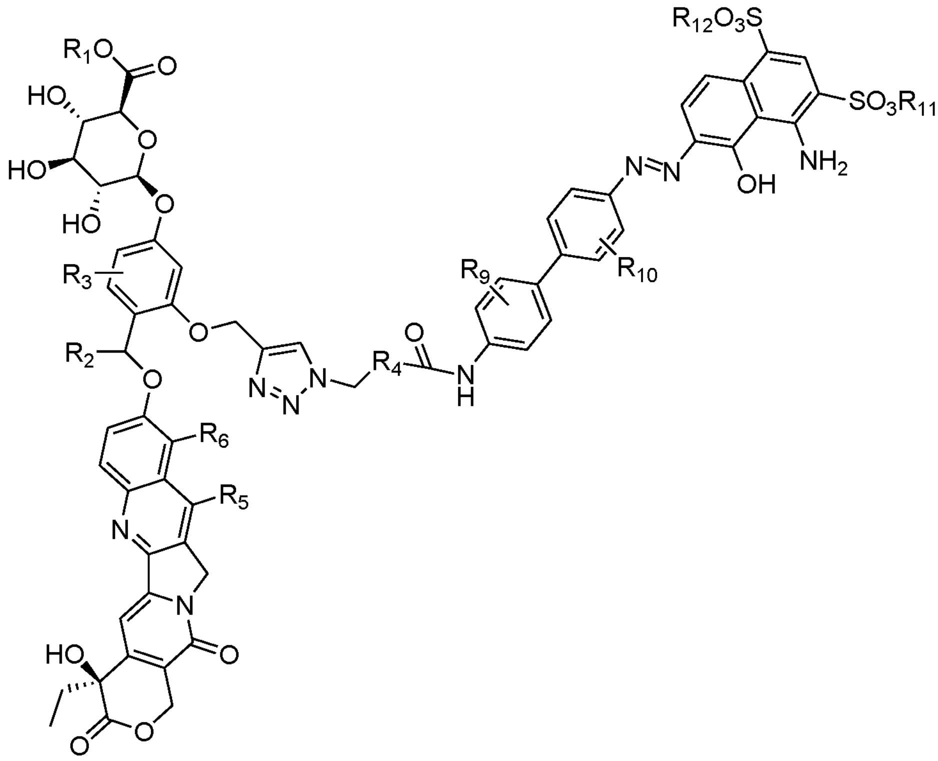

Formula (I)

wherein

$R_1$ is selected from the group consisting of hydrogen, $C_1$-$C_8$ hydrocarbyl, -Na, -Li, -K, -Cs, $-NH_4^+$, $-NH_2^+R_{13}R_{14}$, $-NH^+R_{13}R_{14}R_{15}$, $-NH^+{}_3R_{13}$, and natural or unnatural amino acids;

$R_2$ is selected from the group consisting of hydrogen, and $C_1$-$C_8$ hydrocarbyl;

$R_3$ is one or more substituents at any substitution position selected from the group consisting of hydrogen, halogen, $C_1$-$C_8$ hydrocarbyl, $C_1$-$C_8$ hydrocarbyloxy, cyano, nitro, amino, aryl, arylhydrocarbyl, and $C_1$-$C_4$ hydrocarbyl substituted amino;

$R_4$ is selected from the group consisting of $C_1$-$C_9$ alkylidene, $C_1$-$C_8$ alkylideneoxy, $C_1$-$C_8$ hydrocarbyl substituted or unsubstituted $C_3$-$C_8$ heteroaryl, $C_1$-$C_8$ hydrocarbyl substituted or unsubstituted $C_3$-$C_{15}$ aryl, $-(CH_2R_7)_n$, $-(R_8)_m$, $C_1$-$C_9$ alkylidene-arylidene, $C_1$-$C_9$ alkylidene-arylidene-$C_1$-$C_9$ alkyldiene, $C_1$-$C_9$ alkylidene-arylidene-arylidene, $C_1$-$C_9$ alkylidene-heteroaryliden-$C_1$-$C_9$ alkylidene, and amino acid peptide chain; wherein the amino acid peptide chain is composed of a combination of natural or unnatural amino acids, and the peptide chain length is 1 to100 peptides, preferably 1 to 50 peptides, and more preferably 1 to 20 peptides;

$R_5$ is selected from the group consisting of hydrogen, and $C_1$-$C_8$ hydrocarbyl;

$R_6$ is selected from the group consisting of hydrogen, $C_1$-$C_8$ hydrocarbyl, $C_1$-$C_8$ hydrocarbylidene amino $(C_1$-$C_4$ hydrocarbyl$)_2$, $C_1$-$C_8$ hydrocarbylidene thio $C_1$-$C_8$ hydrocarbyl, and $C_1$-$C_9$ alkylidene-oxy-$C_1$-$C_9$ alkyl;

$R_5$ and $R_6$ may form a ring, and if $R_5$ and $R_6$ form a ring, the ring may be a five-membered ring or a six-membered ring;

$R_7$ is selected from hydrogen, $C_1$-$C_8$ hydrocarbylideneoxy, amino $C_1$-$C_8$ hydrocarbylidene, thio $C_1$-$C_8$ hydrocarbylidene, and oxy $C_1$-$C_8$ hydrocarbylidene;

n is selected from the group consisting of 0, 1, 2, 3, 4, 5, 6, 7, 8, and 9; if n is 0, $(CH_2R_7)_n$ represents a covalent bond;

$R_8$ is carbonylamino, wherein amino may be substituted with $C_1$-$C_8$ hydrocarbyl or arylhydrocarbyl, $C_1$-$C_8$ hydrocarbylidene amino $C_1$-$C_8$ hydrocarbylidene, $C_1$-$C_8$ hydrocarbylidene thio $C_1$-$C_8$ hydrocarbylidene, or $C_1$-$C_8$ hydrocarbylidene oxy $C_1$-$C_8$ hydrocarbylidene;

m is selected from the group consisting of 0, 1, 2, 3, 4, 5, 6, 7, 8, and 9; if m is 0, $-(R_8)_m$ represents a covalent bond;

$R_9$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{10}$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{13}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{14}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{15}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl; and

the natural or unnatural amino acids in $R_1$, $R_{11}$, and $R_{12}$ are selected from the group consisting of glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine.

In still yet another aspect, the disclosure relates to a method for treating a tumor or cancer, comprising administering to a subject in need thereof a therapeutically effective amount of a compound of Formula (I), or a stereoisomer thereof, or a therapeutically effective amount of a pharmaceutical composition comprising a compound of Formula (I), or a stereoisomer thereof, wherein the cancer includes, but is not limited to, colorectal cancer, lung cancer, cervical cancer, ovarian cancer, gastric cancer, esophageal cancer, breast cancer, pancreatic cancer, bladder cancer, liver cancer, gastric cancer, intestinal cancer, head and neck cancer, uterine cancer, urothelial cancer, osteosarcoma, sarcoma, renal cancer, melanoma, prostate cancer, glioma, glioma, and leukemia:

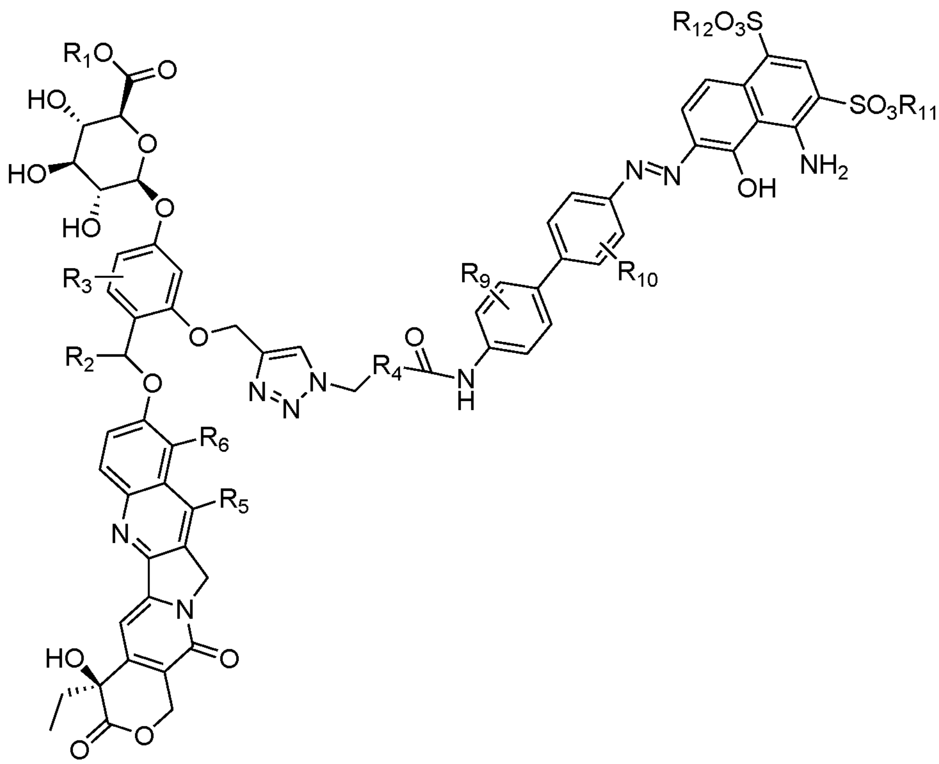

Formula (I)

wherein

$R_1$ is selected from the group consisting of hydrogen, $C_1$-$C_8$ hydrocarbyl, -Na, -Li, -K, -Cs, $-NH_4^+$, $-NH_2^+R_{13}R_{14}$, $-NH^+R_{13}R_{14}R_{15}$, $-NH^+{}_3R_{13}$, and natural or unnatural amino acids;

$R_2$ is selected from the group consisting of hydrogen, and $C_1$-$C_8$ hydrocarbyl;

$R_3$ is one or more substituents at any substitution position selected from the group consisting of hydrogen, halogen, $C_1$-$C_8$ hydrocarbyl, $C_1$-$C_8$ hydrocarbyloxy, cyano, nitro, amino, aryl, arylhydrocarbyl, and $C_1$-$C_4$ hydrocarbyl substituted amino;

$R_4$ is selected from the group consisting of $C_1$-$C_9$ alkylidene, $C_1$-$C_8$ alkylideneoxy, $C_1$-$C_8$ hydrocarbyl substituted or unsubstituted $C_3$-$C_8$ heteroaryl, $C_1$-$C_8$ hydrocarbyl substituted or unsubstituted $C_3$-$C_{15}$ aryl, $-(CH_2R_7)_n$, $-(R_8)_m$, $C_1$-$C_9$

alkylidene-arylidene, $C_1$-$C_9$ alkylidene-arylidene-$C_1$-$C_9$ alkyldiene, $C_1$-$C_9$ alkylidene-arylidene-arylidene, $C_1$-$C_9$ alkylidene-heteroaryliden-$C_1$-$C_9$ alkylidene, and amino acid peptide chain; wherein the amino acid peptide chain is composed of a combination of natural or unnatural amino acids, and the peptide chain length is 1 to100 peptides, preferably 1 to 50 peptides, and more preferably 1 to 20 peptides;

$R_5$ is selected from the group consisting of hydrogen, and $C_1$-$C_8$ hydrocarbyl;

$R_6$ is selected from the group consisting of hydrogen, $C_1$-$C_8$ hydrocarbyl, $C_1$-$C_8$ hydrocarbylidene amino ($C_1$-$C_4$ hydrocarbyl)$_2$, $C_1$-$C_8$ hydrocarbylidene thio $C_1$-$C_8$ hydrocarbyl, and $C_1$-$C_9$ alkylidene-oxy-$C_1$-$C_9$ alkyl;

$R_5$ and $R_6$ may form a ring, and if $R_5$ and $R_6$ form a ring, the ring may be a five-membered ring or a six-membered ring;

$R_7$ is selected from hydrogen, $C_1$-$C_8$ hydrocarbylideneoxy, amino $C_1$-$C_8$ hydrocarbylidene, thio $C_1$-$C_8$ hydrocarbylidene, and oxy $C_1$-$C_8$ hydrocarbylidene;

n is selected from the group consisting of 0, 1, 2, 3, 4, 5, 6, 7, 8, and 9; if n is 0, $(CH_2R_7)_n$ represents a covalent bond;

$R_8$ is carbonylamino, wherein amino may be substituted with $C_1$-$C_8$ hydrocarbyl or arylhydrocarbyl, $C_1$-$C_8$ hydrocarbylidene amino $C_1$-$C_8$ hydrocarbylidene, $C_1$-$C_8$ hydrocarbylidene thio $C_1$-$C_8$ hydrocarbylidene, or $C_1$-$C_8$ hydrocarbylidene oxy $C_1$-$C_8$ hydrocarbylidene;

m is selected from the group consisting of 0, 1, 2, 3, 4, 5, 6, 7, 8, and 9; if m is 0, $-(R_8)_m$ represents a covalent bond;

$R_9$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{10}$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;

$R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{13}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{14}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;

$R_{15}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl; and

the natural or unnatural amino acids in $R_1$, $R_{11}$, and $R_{12}$ are selected from the group consisting of glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine.

In some embodiments, a method for treating a tumor or cancer comprises administering to a subject in need thereof 10mg to 5g of a compound of the present disclosure, a stereoisomer, a cis-trans isomer, or a pharmaceutically acceptable salt thereof.

In some embodiments, a method for treating a tumor or cancer comprises administering to a subject in need thereof 10mg to 3000mg of a compound of the present disclosure, a stereoisomer, a cis-trans isomer, or a pharmaceutically acceptable salt thereof.

In some embodiments, a method for treating a tumor or cancer comprises administering to a subject in need thereof 50mg to 2000mg of a compound of the present disclosure, a stereoisomer, a cis-trans isomer, or a pharmaceutically acceptable salt thereof.

In some embodiments, a method for treating a tumor or cancer comprises administering to a subject in need thereof 50 mg, 100 mg, 200 mg, 250mg, 300 mg, 2000mg, 400 mg, 450mg, 500 mg, 550mg, 650mg, 700mg, 750mg, 800mg, 850mg, 900mg, 1000mg, 1100mg, 1200mg, 1300mg, 1400mg, 1500mg, 1600mg, 1900mg, 1800mg, 1900mg, or 2000mg of a compound of the present disclosure, a stereoisomer, a cis-trans isomer, or a pharmaceutically acceptable salt thereof.

## Pharmaceutical Composition

In some embodiments, the pharmaceutical composition comprises a compound of the present disclosure, a stereoisomer, a cis-trans isomer, or a pharmaceutically acceptable salt thereof, and a pharmaceutically acceptable carrier, diluent, or excipient.

In some embodiments, the administration route of a compound of the present disclosure, a stereoisomer, a cis-trans isomer thereof, or a pharmaceutically acceptable salt thereof for treating a tumor or cancer to a mammal can be gastrointestinal or parenteral.

The compound of the present disclosure may be obtained in any suitable form, such as an injectable preparation.

Exemplary examples of such injectable formulations include, but are not limited to, injection solutions, sterile powders for injection, and concentrated solutions for injection. Injection solutions include solution-type, emulsion-type (such as fat emulsion injection) or suspension-type injection solutions (such as some liposome injection solutions, cortisone acetate injection solutions, and the like), which can be used for intramuscular injection, intravenous injection, intravenous drip, and the like. The large-volume (generally not less than 100 ml, unless otherwise specified) injection solution for intravenous injection is also referred to as intravenous infusion. Ampoules are commonly referred to as small water needles and differ from large infusion solutions. Sterile powders for injection include lyophilized powder injection and sterile dispensing. Concentrated solutions for injection are sterile concentrated solutions diluted prior to use. Examples of pharmaceutically acceptable carriers that can be used in the pharmaceutical compositions of the present disclosure include, but are not limited to, any of the various forms of carriers, carriers, carrier materials, solvents, osmotic pressure modifiers, pH modifiers, solubilizers, co-solvents, antioxidants, bacteriostats, emulsifiers, suspending agents, fillers, complexing agents, chelating agents, and the like that have been approved by the United States Food and Drug Administration for use in humans or animals, without adverse effects on the composition of the pharmaceutical composition. Various forms of carriers that have no side effects on the composition of the pharmaceutical composition. Acceptable carriers for therapeutic use are well known in the pharmaceutical arts and are for example found in Remington's Pharmaceutical Sciences, 18th Ed., Mack Publishing Co., Easton, PA (1990). The description is hereby incorporated by reference in its entirety.

The pharmaceutical composition of the present disclosure can be administered by any method that achieves its intended purpose. The pharmaceutical composition of the present disclosure may be administered by injection administration, such as intradermal injection, subcutaneous injection, intramuscular injection, intravenous drip, intraarterial injection, intracardiac injection, intraarticular injection, subcutaneous infusion, and the like. The dosage administered will depend on the age, health and weight of the recipient, and, if concurrent, depends on the type of concurrent treatment, the frequency of treatment, and the nature of the desired effect.

Suitable dosage forms include, but are not limited to, injection solutions, sterile powders for injection, and concentrated solutions for injection. Injection solutions include solution-type, emulsion-type (such as fat emulsion injection) or suspension-type injection solutions (such as some liposome injection solutions, cortisone acetate injection solutions, and the like), which can be used for intramuscular injection, intravenous injection, intravenous drip, and the like. The injection for intravenous injection includes an intravenous infusion and a small water needle. Sterile powders for injection include lyophilized powder injection and sterile dispensing. Concentrated solutions for injection are sterile concentrated solutions diluted prior to use, which can be prepared according to methods known in the art.

Suitable carriers, solvents, osmotic pressure modifiers, pH modifiers, solubilizers, co-solvents, antioxidants, bacteriostats, emulsifiers, suspending agents, fillers, complexing agents and chelating agents may be provided as desired in the pharmaceutical composition. For example, water for injection, vegetable oils such as soybean oil for injection, ethanol, propylene glycol, polyethylene glycol, and the like may be added as a solvent; sodium chloride, glucose, and the like may be added as an osmotic pressure regulator; inorganic acid or base, organic acid or base, such as hydrochloric acid, sodium hydroxide, sodium citrate, and citric acid, and the like may be added as a pH regulators; polyoxyethylene castor oil, polysorbate, cyclodextrin, such as hydroxypropyl-β-cyclodextrin, and the like may be added as a solubilizing agent; organic acid and its sodium salt, such as sodium benzoate, may be added as a solubilizing agent; amides and amines such as meglumine may be added as a co-solvent; ascorbic acid, sodium sulfite, sodium bisulfite, sodium metabisulfite, and the like may be added as an antioxidant; phenol, cresol, trichlorobutanol, and thimerosal may be added as an antibacterial agent; lecithin, poloxamer, and the like may be added as an emulsifier; polyvinylpyrrolidone, methyl cellulose, and the like may be added as a suspending agent; mannitol, glucose, and the like may be added as a filler; ethylenediamine tetraacetate, and the like may be added as a complexing agent, a chelating agent and the like.

Suitable routes of administration may, for example, include oral administration, rectal administration, transmembrane administration, parenteral delivery, transdermal administration, topical administration or enteral administration; parenteral delivery includes intramuscular injection, subcutaneous injection, Intravenous, intramedullary, and intrathecal, direct intraventricular, intraperitoneal, intranasal, or intraocular. The compounds can also be extended at a predetermined rate and/or in a sustained release or controlled release dosage form including depot injections, osmotic pumps, pills, transdermal (including electromigration) patches, and the like. Timing, pulsed administration.

The pharmaceutical compositions of the present disclosure can be produced in a known manner, for example, by conventional methods of mixing, formulating, filtering, encapsulating, sterilizing, and the like.

Thus, according to the present disclosure, the pharmaceutical composition used can be formulated in a conventional manner using one or more physiologically acceptable carriers comprising adjuvants, which facilitate the treatment of the active compound into a pharmaceutically acceptable formulation. Suitable formulations will depend on the route of administration chosen. Any of the well-known techniques, carriers, and excipients can be used as appropriate and understood in the art.

The injection can be prepared in the following conventional form: as a solution or suspension, a solid dosage form suitable as a solution or suspension prior to injection, or as an emulsion. Suitable excipients are, for example, water, saline,

dextrose, mannitol, lactose, lecithin, albumin, sodium glutamate, cysteine hydrochloride and the like. Additionally, if desired, the injectable pharmaceutical compositions may contain minor amounts of non-toxic adjuvants such as wetting agents, pH buffering agents and the like. Physiologically suitable buffers include, but are not limited to, Hank's solution, Ringer's solution, or physiological saline buffer. Absorption enhancing formulations (e.g., liposomes) can be used if desired.

In some embodiments, the pharmaceutical composition of the present disclosure may comprise 0.1% to 95% of a compound of the present disclosure, a stereoisomer, or a pharmaceutically acceptable salt thereof.

In some embodiments, the pharmaceutical composition of the present disclosure may comprise 1% to 70% of a compound of the present disclosure, a stereoisomer, or a pharmaceutically acceptable salt thereof.

In any event, the composition or formulation to be administered will contain an amount of a compound of the present disclosure, a stereoisomer, or a pharmaceutically acceptable salt thereof.

In any event, administration will contain a compound of the present disclosure, a stereoisomer, or a pharmaceutically acceptable salt thereof in an amount effective to treat the disease/condition of the subject being treated.

Administration Method

The at least one compound described in the present disclosure, a stereoisomer, or a pharmaceutically acceptable salt thereof, or a pharmaceutical composition comprising at least one compound described in the present disclosure, a stereoisomer, or a pharmaceutically acceptable salt thereof, can be subjected to any suitable systemic and/or topical delivery of any of the compounds described in the present disclosure, a stereoisomer, or a pharmaceutically acceptable salt thereof. The method is administered to the patient. Non-limiting examples of methods of administration include (a) administration by the oral route, including administration in the form of capsules, tablets, granules, sprays, syrups, or the like; (b) by non-oral administration, for example, rectal, vaginal, intraurethral, intraocular, intranasal or intraaural, the administration includes an aqueous suspension, an oily preparation, or the like, or in the form of drops, sprays, suppositories, ointments, ointments, and the like; (c) administration by subcutaneous injection, intraperitoneal injection, intravenous injection, intramuscular injection, intradermal injection, intraorbital injection, intracapsular injection, intraspinal injection, intrathoracic injection, etc., including infusion pump delivery; (d) local administration such as injection directly into the kidney region or cardiac region, such as by depot implantation; and (e) topical administration; as is known to those skilled in the art a suitable mode of administration is considered to be contact with living tissue with the compound described in this disclosure.

The most suitable route depends on the nature and severity of the condition being treated. Those skilled in the art are also familiar with determining the method of administration (oral, intravenous, inhalation, subcutaneous, rectal, etc.), dosage form, appropriate pharmaceutical excipients, and other matters relating to delivery of the compound to a subject in need thereof.

Pharmaceutical compositions suitable for administration include those in which an effective amount of the active ingredient is included in such compositions. The dose required for a therapeutically effective amount of the pharmaceutical composition described in this disclosure depends on the route of administration, the type of animal being treated, including the human, and the physical characteristics of the particular animal in question. The dosage can be adjusted to achieve the desired effect, but this will depend on factors such as body weight, diet, concurrent medical therapy, and other factors recognized by those skilled in the medical arts. More specifically, a therapeutically effective amount refers to an amount of a compound that is effective to prevent, alleviate or ameliorate the symptoms of the disease, or to prolong the life of the individual being treated. The actual ability of a person skilled in the art can well determine a therapeutically effective amount, particularly in accordance with the detailed disclosure provided by the present disclosure.

As will be apparent to those skilled in the art, the dosage and specific mode of administration for in vivo administration will vary depending on the age, weight and type of mammal being treated, the particular compound employed, and the compound employed. Specific use. Those skilled in the art will be able to achieve the objective of determining an effective dosage level, i.e., the dosage level necessary to achieve the desired effect, using conventional pharmacological methods. Typically, human clinical application of the product begins at a lower dosage level as the dosage level increases until the desired effect is achieved. Alternatively, an established in vitro study can be used to establish an effective dosage and route of administration of the compositions identified by the methods using established pharmacological methods.

In non-human animal studies, the application of potential products begins at a higher dose level, as the dose is reduced until the desired effect is no longer achieved or the undesirable side effects disappear. The dosage range can be broader depending on the desired effect and therapeutic indication. Generally, the dosage can be from about 10 $\mu$g/kg body weight to 1000 mg/kg body weight, and in certain embodiments from about 100 $\mu$g/kg body weight to 300 mg/kg body weight. Alternatively, as will be understood by those skilled in the art, the dosage can be based on and calculated from the body surface area of the patient.

Each physician can select the exact formulation, route of administration and dosage of the pharmaceutical

compositions described in this disclosure based on the condition of the patient. Generally, the dosage of the composition administered to the patient can range from about 0.05 mg/kg to 3000 mg/kg of patient body weight. The dose may be administered once or two or more times during one or several days depending on the needs of the patient. Where the human dosage of the compound is established by at least some of the conditions, the present disclosure will use those same dosages, or dosages ranging from about 0.1% to 500% of the determined human dose, in certain embodiments the dosage. The range of 25% to 250% of the determined human dose. In the absence of a determined human dose In the case of newly discovered drug compounds, suitable human doses can be inferred from half effective doses or median values of infectious doses, or other suitable values from in vitro or in vivo studies, as in animals. Quantitative by the Institute for Toxicity Research and Performance.

It should be noted that due to toxicity and organ dysfunction, the attending physician will know how and when to terminate, interrupt or adjust the administration. Conversely, if the clinical response is inadequate (excluding toxicity), the attending physician will also know to adjust the treatment to a higher level. The size of the administered dose in the treatment of the condition of interest will vary with the severity of the condition being treated and the route of administration. The severity of the disease state can be assessed, for example, in part by standard prognostic evaluation methods. In addition, the dosage and possible dosage frequency will also vary depending on the age, weight, and response of the individual patient. A protocol comparable to the above discussed protocol can be used in veterinary medicine.

Although the exact dose can be determined based on drug-by-drug, in most cases, some generalizations can be made with respect to the agent. The daily dosing regimen for an adult patient is, for example, an oral dose of from 0.1 mg to 2000 mg of each active ingredient, and in certain embodiments from 1 mg to 2000 mg of each active ingredient, for example from 5 mg to 1500 mg of each active ingredient. In other embodiments, the intravenous, subcutaneous or intramuscular dose of each active ingredient used is from 0.01 mg to 1000 mg, and in certain embodiments from 0.1 mg to 1000 mg, such as from 1 mg to 800 mg. In the case of administration of a pharmaceutically acceptable salt, the dosage can be calculated as the free base. In certain embodiments, the composition is administered from 1 to 4 times daily. Alternatively, the compositions described in this disclosure may be administered by continuous intravenous infusion, in certain embodiments at doses up to 2000 mg of each active ingredient per day. As will be appreciated by those skilled in the art, in certain instances, in order to effectively and rapidly treat a rapidly developing disease or infection, it is necessary to administer a compound described in the present disclosure in an amount exceeding or far exceeding the above dosage range. In certain embodiments, the compound is administered during continuous treatment, such as one week or weeks, or months or years.

The dose and dosing interval can be adjusted individually to provide a plasma level sufficient to maintain an adjustment effect or an active fraction of the lowest effective concentration (MEC). The MEC of each compound is different, but the MEC can be evaluated from in vitro data. The dosage required to achieve MEC will depend on the individual characteristics and route of administration. However, plasma concentration can be determined using HPLC (High Performance Liquid Chromatography) assay or bioassay.

The medication interval can also be determined using the MEC value. Treatments that maintain plasma levels above the MEC over a period of 10 to 90% of the time, in some embodiments, 30 to 90% of the time, and in some embodiments, 50 to 90% of the time should be used. The regimen is administered to the composition.

In the case of topical administration or selective absorption, the effective local concentration of the drug is independent of plasma concentration.

Of course, the amount of the composition to be administered depends on the individual to be treated, depending on the weight of the individual, the severity of the pain, the mode of administration, and the judgment of the prescribing physician.

The potency and toxicity of the compounds described in this disclosure can be assessed using known methods. For example, the toxicology of a particular compound or a subset of such compounds that share certain chemical moieties, such as a mammalian cell line and, in certain embodiments, a human cell line, can be established by measuring the toxicity of the cell line in vitro. The results of such studies are generally predictive of toxicity in animals such as mammals, or more specifically, toxicity in humans. Alternatively, the toxicity of a particular compound in an animal model such as a mouse, rat, rabbit or monkey can be determined using known methods. The efficacy of a particular compound can be determined using a number of accepted methods, such as in vitro methods, animal models, or human clinical trials. There are well-recognized in vitro models for almost every type of disease state, including but not limited to cancer, cardiovascular disease, and a variety of immune dysfunctions. Similarly, acceptable animal models can be used to determine the efficacy of a chemical that treats these disease states. When selecting a model to measure potency, the skilled artisan can select the appropriate model, dosage and route of administration, and treatment regimen under the guidance of the prior art in the art. Of course, human clinical trials can also be used to determine the potency of a compound in the human body.

If desired, the composition can be placed into a pack or dispenser device, which can contain one or more unit dosage forms containing the active ingredient. The package may for example comprise a metal or plastic foil, such as a blister pack. The pack or dispenser device can be provided with instructions for administration. The packaging or dispensing device may also carry precautions associated with the container, the precautions being prescribed by a

government agency that manages the manufacture, use or sale of the drug, the precaution reflecting that the drug form has been Approved for human or veterinary administration. Such considerations, for example, may be labels for prescription drugs approved by the National Food and Drug Administration or the U.S. Food and Drug Administration, or approved product specifications. Compositions comprising a compound of the invention may also be prepared in a suitable container, placed in a compatible pharmaceutical carrier, and labeled for treatment in a defined disease state.

Hereinafter, the present disclosure will be explained in detail by the following examples in order to better understand various aspects of the present application and advantages thereof. However, it is to be understood that the following examples are non-limiting and are intended to illustrate only certain embodiments of the present application.

Examples

Abbreviations:

aq: aqueous solution
ml: milliliter
g: gram
V: volume/weight
eq: equivalent ratio
L: liter
M: mol/L
mg: milligram
μl: microliters
EDCI: 1-(3-dimethylaminopropyl)-3-ethylcarbodiimide hydrochloride
HOBt: 1-hydroxybenzotriazole
DIEA: diisopropylethylamine
DCM: dichloromethane
PE: petroleum ether
EA: ethyl acetate
DMSO: dimethyl sulfoxide
ACN: acetonitrile
MeOH: methanol
DMF: N,N-dimethylformamide
$PPh_3$: triphenylphosphine
THF: tetrahydrofuran
VCNa: sodium ascorbate
NaH: sodium hydride
$CuSO_4$: anhydrous copper sulfate
HCl: hydrochloric acid
NaOH: sodium hydroxide
$CBr_4$: carbon tetrabromide
AcOH: glacial acetic acid
Ar: argon
$NaN_3$: sodium azide
NaCl: sodium chloride
$AlCl_3$: aluminum chloride
$K_2CO_3$: potassium carbonate
$Na_2SO_4$: sodium sulfate
$CHCl_3$: chloroform
$NaBH_4$: sodium borohydride
IPA: isopropanol
TFA: trifluoroacetic acid
MTBE: methyl tert-butyl ether
HATU: 2-(7-azabenzotriazole)-N,N,N',N'-tetramethyluronium hexafluorophosphate
$NH_4Cl$: ammonium chloride
MeONa: sodium methoxide
$Ag_2O$: silver oxide

$Boc_2O$: ditertbutyl dicarbonate
$CDCl_3$: deuterochloroform
HPLC: High Performance Liquid Chromatography
TLC: Thin Layer Chromatography
DMF: N,N-dimethylformamide
EDTA: ethylenediaminetetraacetic acid
mM: mmol/L
$mm^3$: cubic centimeter
$^1H$ NMR: Nuclear Magnetic Resonance Spectrum

Example 1

N-(4'-amino-3,3'-dimethyl-[1,1'-biphenyl]-4-yl)-6-bromohexanamide

To 60ml of DCM were added ortho-toluidine 2.177g, EDCI 2.359g, HOBt 1.663g and DIEA 1.590g. The mixture was stirred under argon until completely dissolved and cooled to 0 to 5°C. 2g 6-bromohexanoic acid was dissolved in 40ml DCM. The resultant was added dropwise to the reaction system at 0 to 5°C. After completion of the dropwise addition, the reaction was carried out overnight at room temperature. To the system was added 20 ml of saturated aqueous sodium bicarbonate solution, stirred and filtered by suction. The filter cake was stirred in DCM, filtered by suction. The filtrate was retained. The above steps were repeated three times. The DCM phases were combined, dried, filtered by suction. After concentration and drying, the resultant was purified by column chromatography and concentrated to give the product 2.047g.MS (m/e): 389.20$[M+H]^+$.

Example 2

N-(4'-amino-3,3'-dimethyl-[1,1'-biphenyl]-4-yl)-6-azidohexanamide

To a reaction flask were added 0.73g of the compound of Example 1 and 5.1ml of DMSO. The mixture was stirred at room temperature for complete dissolution. 0.15g of $NaN_3$ was added to give an orange-red suspension and stirred at room temperature overnight. DCM (20 ml) and water (20 ml) were added. The mixture was were extracted to separate. The DCM phase was retained and the aqueous phase was extracted again with DCM (20 ml) to separate. The combined DCM phase was washed with saturated aqueous NaCl solution, dried over anhydrous sodium sulfate, filtered by suction, concentrated and purified by column chromatography to give the product 0.28g. MS (m/e): 352.24$[M+H]^+$.

Example 3

(E)-4-amino-6-((4'-(6-azidohexanamide)-3,3'-dimethyl-[1,1'-biphenyl]-4-yl)diazenyl )-5-hydroxynaphthalene-1,3-disulfonate disodium

Step 1: To 4 ml of acetonitrile was added 0.25g of the compound of Example 2. The mixture was cooled in an ice salt bath and stirred under argon for complete dissolution. The ice salt bath was cooled to 0 to 5°C and HCl solution (0.18ml of 12mol/L hydrochloric acid in 6ml deionized water) was added dropwise. After stirring for 5 minutes, sodium nitrite solution (0.1 g of sodium nitrite in 300 ml of deionized water) was added dropwise for about 20 minutes. The addition was complete with stirring for 30 minutes.

Step 2: To a reaction flask were added 0.24g of 4-amino-5-hydroxy-1,3-naphthalene disulfonate monosodium and 5 ml of deionized water. The mixture was cooled in an ice salt bath and stirred and dissolved completely under the protection of Ar. The ice salt bath was cooled to 0 to 5°C. Sodium bicarbonate solution (0.24g of sodium bicarbonate in 5 ml deionized water) was added dropwise for about 20 minutes.

The ice salt bath was maintained at a temperature of 0 to 5°C. The reaction solution of step 1 was slowly added to the reaction solution of step 2. The resultant mixture was stirred in an ice salt bath for 3 hours. An aqueous solution of 19mL of 20% NaCl was added. The mixture was stirred at room temperature overnight. The reaction solution was centrifuged to give about 1.2g of a purple solid. The solid was dissolved with $H_2O$: ACN=10:1 (total of 13.2mL). The resultant mixture was stirred at room temperature overnight. The reaction solution was centrifuged to give a crude purple solution. The crude purple solution was purified by medium pressure. The preparation solution was concentrated to remove acetonitrile and lyophilized to give 0.41g of the product. $^1H$ NMR (DMSO-$d_6$, 600MHz) $\delta$: 15.954 (s, 1H), 9.643 (s, 1H), 9.277 (s, 1H), 8.362 (s, 1H), 8.042-8.025 (d, 1H), 7.902-7.887 (d, 1H), 7.652-7.650 (m, 2H), 7.572 (s, 1H), 1.661-1.581 (s, 2H), 7.003-6.987 (d, 1H), 3.366-3.344 (t, 2H), 2.277 (s, 3H), 2.385-2.361 (t, 2H), 2.277 (s, 3H), 1.661-1.581 (m, 4H), 1.422-1.397 (m, 2H).

Example 4

(2S,3R,4S,5S,6S)-2-(4-(bromomethyl)-3-(prop-2-ynyloxy)phenoxy)-6-(methoxycarbo nyl)tetrahydro-2H-pyran-3,4,5-triacetate

To 7mL of DCM were added 1g of (2S,3R,4S,5S,6S)-2-(4-(hydroxymethyl)-3-(prop-2-ynyloxy)phenoxy)-6-(methoxycar bonyl)tetrahydro-2H-pyran-3,4,5-triacetate (cf. the preparation process in PCT Int. Appl., 2012153193, 15 Nov 2012) and 1.6g of carbon tetrabromide. The mixture was stirred until dissolved. The resultant was cooled in an ice salt bath and stirred. The reaction system was reduced to 6 to 8°C. 0.8g of triphenylphosphine was weighed and added in portions to the reaction flask. The mixture was stirred in an ice salt bath for 5 minutes. The system was cooled to about 10°C. The ice salt bath was removed and the reaction was stirred at room temperature for 2 hours. Silica gel was added directly to the reaction solution and 0.74g of the product was obtained by column chromatography. $^1$H NMR ($CDCl_3$, 400MHz) $\delta$: 7.277-7.263 (d, 1H), 6.752-6.721 (d, 1H), 6.640-6.586 (dd, 1H), 2.051 (m, 2H), 5.292-5.263 (m, 1H), 5.159-5.146 (d, 1H), 4.758-4.754 (d, 2H), 4.542-4.506 (d, 2H), 4.189-4.173 (m, 1H), 3.730 (s, 3H), 2.043 (t, 1H), 2.064 (s, 3H), 2.051 (s, 3H), 2.043 (s, 3H). MS (m/e): 579.65[M+Na]$^+$.

Example 5

(2S,3R,4S,5S,65)-2-(4-(((S)-4,11-diethyl-4-hydroxy-3,14-dioxy-3,4,12,14-tetrahydro-1H-pyrano[3',4':6,7]indolazino [1,2-b]quinolin-9-yl)oxy)methyl)-3-(prop-2-ynyloxy)p henoxy)-6-(methoxycarbonyl)tetrahydro-2H-pyran-3,4,5-triacetate

To a reaction flask were added 0.74g of (2S,3R,4S,5S,6S)-2-(4-(bromomethyl)-3-(prop-2-ynyloxy)phenoxy)-6-(methoxycarbo nyl)tetrahydro-2H-pyran-3,4,5-triacetate and 43.3g of (S)-4,11-diethyl-4,9-dihydroxy-1,12-dihydro-14H-pyrano[3',4':6,7]indolizino[1,2-b]q uinoline-3,14(4H)-dione. The mixture was protected from light and dissolved in 600mL of DMF. The resultant mixture was stirred in an ice salt bath for 2 minutes. 0.04. g of sodium hydride was added slowly and portionwise. The resultant was stirred under argon for 1 minute. The ice salt bath was removed and after 20 minutes the system was dissolved and clear and stirred at room temperature for 1 hour. To the reaction was added 0.06ml of AcOH. The mixture was stirred for 1 minute. DMF was removed by concentration over silica gel. 1.2g of a product was obtained by column chromatography. 12ml of EA was added. The mixture was sonicated, stirred at room temperature for 16 hours and filtered by suction. The filter cake was washed with EA to give 0.73g of the product. HPLC purity 96.5%. $^1$H NMR (DMSO-$d_6$, 400MHz) $\delta$: 8.076-8.053 (d, 1H), 7.561-7.468 (m, 3H), 3.634 (s, 1H), 2.010 (s, 1H), 3.586 (d, 1H), 2.028 (s, 1H), 5.731-5.711 (d, 1H), 2.000 (t, 1H), 3.178-3.160 (s, 2H), 0.895-0.859 (s, 2H), 1.883-1.845 (s, 2H), 5.136-5.050 (m, 2H), 4.925 (s, 2H), s (d, 1H), 3.634 (s, 3H), 3.586 (s, 1H), 3.178-3.160 (d, 2H), 2.028 (s, 3H), 2.010 (s, 3H), 2.000 (s, 3H), 1.883-1.845 (m, 2H), 1.297-1.260 (t, 3H), 0.895-0.859 (t, 3H). MS (m/e): 869.98 [M+H]$^+$.

Example 6

(2S,3S,4S,5R,6S)-6-(4-(((S)-4,11-diethyl-4-hydroxy-3,14-dioxy-3,4,12,14-tetrahydro-1H-pyrano[3',4':6,7]indolazino [1,2-b]quinolin-9-yl)oxy)methyl)-3-(prop-2-ynyloxy)p henoxy)-3,4,5-trihydroxytetrahydro-2H-pyran-2-carboxylic acid

To a reaction flask were added 0.72g of (2S,3R,4S,5S,65)-2-(4-(((S)-4,11-diethyl-4-hydroxy-3,14-dioxy-3,4,12,14-tetrahydro-1H-pyrano[3',4':6,7]indolazo[1,2-b]quinolin-9-yl)oxy)methyl)-3-(prop-2-ynyloxy)ph enoxy)-6-(methoxycarbonyl)tetrahydro-2H-pyran-3,4,5-triacetate and 7.2ml of THF. The mixture was stirred and cooled in a bath of glacial ethanol. The mixture was stirred for about 30 minutes. 0.2 g of sodium hydroxide was dissolved in 7.2ml of deionized water. The solution was added dropwise to the mixture. The sample was gradually dissolved. The glacial ethanol was removed. The resultant mixture was stirred at room temperature for 1 hour and was cooled by glacial ethanol for about 10 minutes. 5ml of 1N·HCl was added dropwise for about 20 minutes. THF was removed by concentration. The resultant was filtered under reduced pressure by water pump. 0.57g of the product was obtained by column chromatography. $^1$H NMR (DMSO-$d_6$, 400MHz) $\delta$: 8.050-8.035 (d, 1H), 3.164-3.153 (s, 1H), 7.519-7.503 (d, 1H), t (d, 1H), 3.310-3.256 (s, 1H), 6.863 (s, 1H), 6.720-6.707 (d, 1H), 6.495 (s, 1H), 1.301-1.276 (m, 0.890-0.865), 4.997-4.985 (d, 1H), 1.905-1.833 (s, 2H), 3.686 (s, 1H), 3.585 (s, 1H), 3.310-3.256 (m, 3H), 3.164-3.153 (d, 2H), 1.905-1.833 (m, 2H), 1.301-1.276 (t, 3H), 0.890-0.865 (t, 3H). MS (m/e): 729.74 [M+H]$^+$.

Example 7

(25,35,4S,5R,65)-6-(3-((1-(6-((4'-((E)-(8-amino-1-hydroxy-5,7-disulfonicnaphthalen -2-yl)diazenyl)-3,3'-dimethyl-[1,1'-biphenyl]-4-yl)amino)-6-oxohexyl)-1H-1,2,3-triaz ol-4-yl)methoxy)-4-((((S)-4,11-diethyl-4-hydroxy-3,14-dioxy-3,4,12,14-tetrahydro-1 H-pyrano[3',4':6,7]indolazino[1,2-b]quinolin-9-yl)oxy)methyl)phenoxy)-3,4,5-trihyd roxytetrahydro-2H-pyran-2-carboxylate trisodium

Under room temperature conditions, to a reaction flask were added 0.54g of the compound of Example 6, 0.5g of (E)-4-amino-6-((4'-(6-azidohexanamide)-3,3'-dimethyl-[1,1'-biphenyl]-4-yl)diazenyl )-5-hydroxynaphthalene-1,3-disulfonate disodium. The mixture was protected from light and dissolved in 11ml of THF under stirring. 0.02g of $CuSO_4$ was dissolved with 0.3ml of deionized water and 0.06g of VCNa was dissolved with 0.3ml of deionized water. To the system were added successively the above solutions and 10 ml of deionized water under protection by argon. The mixture was stirred at room temperature for 1 hour and filtered by suction to give a purple filtrate. THF was removed by concentration at 38°C. After concentration, water was added to 10 ml and stirred for 5 to 10 min. An aqueous solution of 4ml of 20% NaCl was added and the crude product was prepared as an aqueous solution of about 5% NaCl and salted out at room temperature overnight. The reaction was centrifuged and separated to give a solid. The solid was dissolved in 210ml of deionized water and mechanically stirred at room temperature for 1 hour. An aqueous solution of 4ml of 20%NaCl was added and the crude was prepared as an aqueous solution of 3% NaCl and salted out at room temperature overnight. The reaction was centrifuged and separated to give a solid. The solid was dissolved with 15 ml of deionized water and stirred at room temperature overnight. The resultant was filtered by suction to give a purple filtrate. The filtrate was purified by medium pressure. The preparation solution was concentrated to remove acetonitrile and lyophilized and crushed to give 0.73g of the product. $^1H$ NMR (DMSO-$d_6$, 400MHz) δ: 15.907 (s, 1H), 9.638 (s, 1H), 9.378 (s, 1H), 8.413 (s, 1H), 8.281 (s, 1H), 8.029-8.013 (d, 1H), 7.984-7.970 (d, 1H), 7.816-7.804 (d, 1H), 7.580 (s, 2H), 7.493-7.432 (m, 5H), 7.372-7.358 (d, 1H), 7.243 (s, 1H), 7.033 (s, 1H), 6.979-6.963 (d, 1H), 6.681-6.668 (d, 1H), 6.506 (s, 1H), 5.424-5.184 (m, 10H), 4.980-4.970 (d, 1H), 4.348 (s, 2H), 3.623 (s, 1H), 3.337-3.247 (m, 3H), 3.073 (s, 2H), 2.455 (s, 3H), 2.315 (s, 2H), 2.211 (s, 3H), 1.821-1.810 (m, 4H), 1.578 (s, 2H), 1.233-1.223 (m, 5H), 0.884-0.860 (t, 3H).

Example 8

(2S,3R,4S,5S,6S)-2-(4-formyl-3-hydroxy-2-methylphenoxy)-6-(methoxycarbonyl)tetr ahydro-2H-pyran-3,4,5-triacetate

1.686g of 2,4-hydroxy-3-methylbenzaldehyde, 4g of (2S,3R,4S,5S,6S)-2-bromo-6-(methoxycarbonyl)tetrahydro-2H-pyran-3,4,5-triacetate, 1.292g of silver oxide, and 1.6g of 4A molecular sieves were weighed and added into a reaction flask successively. To the reaction flask was added 80ml of acetonitrile. The mixture was protected with argon and stirred at room temperature for 1 hour. The resultant was filtered by suction. Diatomite was added. The filter cake was washed by dichloromethane. The filtrate was concentrated to dryness. 2.935g of the product was obtained after purification by column chromatography. MS (m/e): 491.11 $[M+Na]^+$. $^1H$ NMR (DMSO-$d_6$, 600MHz) δ: 11.348 (s, 1H), 9.895 (s, 1H), 7.674-7.660 (d, 1H), 6.806-6.792 (d, 1H), 5.772-5.759 (d, 1H), 5.531-5.499 (t, 1H), 5.222-5.193 (dd, 1H), 5.120-5.087 (t, 1H), 4.796-4.779 (d, 1H), 3.649 (s, 3H), 2.034-2.020 (t, 9H), 1.950 (s, 3H).

Example 9

(2S,3R,4S,5S,6S)-2-(4-formyl-2-methyl-3-(prop-2-ynyloxy)phenoxy)-6-(methoxycar bonyl)tetrahydro-2H-pyran-3,4,5-triacetate

2.83g of (2S,3R,4S,5S,6S)-2-(4-formyl-3-hydroxy-2-methylphenoxy)-6-(methoxycarbonyl)tetr ahydro-2H-pyran-3,4,5-triacetate was weighed and was added into 28ml of dried DMF. The mixture was stirred at room temperature until completely dissolved. Potassium carbonate (2.505g) and bromopropyne (1.078g) were sequentially weighed and added to the reaction system slowly in portions. The reaction was carried out at room temperature for 4 hours. The system was extracted with ethyl acetate. The organic phase was washed twice with saturated ammonium chloride solution and washed with saturated sodium chloride and dried over anhydrous sodium sulfate. The filtrate was concentrated to dryness by suction. 2.588g of the product was obtained after purification by column chromatography. MS (m/e): 507.15 $[M+H]^+$. $^1H$ NMR (6H, 600MHz) δ: 10.183 (s, 1H), 7.691-7.677 (d, 1H), 7.066-7.051 (d, 1H), 5.782-5.769 (d, 1H), 2.027-2.021 (t, 1H), 5.225-5.196 (dd, 1H), 5.120-5.087 (t, 1H), 4.795-4.779 (m, 3H), 3.660-3.652 (t, 1H), 3.611 (s, 3H), 2.060 (s, 3H), 2.047 (s, 3H), 2.027-2.021 (d, 6H).

Example 10

(2S,3R,4S,5S,6S)-2-(4-hydroxymethyl)-2-methyl-3-(prop-2-ynyloxy)phenoxy)-6-(me thoxycarbonyl)tetrahydro-2H-pyran-3,4,5-triacetate

2.367g of (2S,3R,4S,5S,6S)-2-(4-formyl-2-methyl-3-(prop-2-ynyloxy)phenoxy)-6-(methoxycar bonyl)tetrahydro-2H-pyran-3,4,5-triacetate was weighed and added into 23.7ml of chloroform. Th mixture was stirred until completely dissolved and cooled by 0-5°C in an ice-salt bath. 11.835g of silica gel and 4.7ml of isopropanol were added sequentially. 442mg of sodium borohydride was weighed and added in portions. The reaction was held at 0-5°C for 1 hour. The reaction was quenched by addition of 1 ml of glacial acetic acid. The resultant was filtered by suction. Diatomite was added. The filter cake was washed with dichloromethane. The filtrate was concentrated to dryness. 2.038g of the product was obtained after purification by column chromatography. MS (m/e): 531.20 $[M+Na]^+$. $^1$H NMR (DMSO-$d_6$, 600MHz) δ: 7.223-7.209 (2.025-2.010, 1H), 6.868-6.854 (2.025-2.010, 1H), 5.556-5.543 (2.025-2.010, 1H), 5.504-5.472 (t, 1H), 5.159-5.130 (dd, 1H), 5.091-5.058 (t, 1H), 5.027-5.009 (t, 1H), 4.727-4.711 (2.025-2.010, 1H), 4.569-4.565 (2.025-2.010, 2H), 4.501-4.491 (2.025-2.010, 2H), 3.652 (s, 3H), 3.571-5.562 (t, 1H), 2.040 (s, 3H), 2.025-2.010 (t, 9H).

Example 11

(2S,3R,4S,5S,6S)-2-(4-(bromomethyl)-2-methyl-3-(prop-2-ynyloxy)phenoxy)-6-(met hoxycarbonyl)tetrahydro-2H-pyran-3,4,5-triacetate

1.99g of the product was prepared with (2S,3R,4S,5S,6S)-2-(4-hydroxymethyl)-2-methyl-3-(prop-2-ynyloxy) phenoxy)-6-(me thoxycarbonyl)tetrahydro-2H-pyran-3,4,5-triacetate by the preparation method of Example 4. $^1$H NMR (DMSO-$d_6$, 600MHz) δ: 7.248-7.234 (d, 1H), 6.839-6.825 (d, 1H), 5.375-5.359 (m, 3H), 5.141-5.129 (dd, 1H), 4.676-4.659 (m, 3H), 4.614-4.596 (d, 1H), 4.216-4.201 (dd, 1H), 3.756 (s, 3H), 2.585-2.577 (t, 1H), 2.163 (s, 3H), 2.088-2.070 (t, 9H).

Example 12

(2S,3R,4S,5S,6S)-2-(4-(((S)-4,11-diethyl-4-hydroxy-3,14-dioxy-3,4,12,14-tetrahydro-1H-pyrano[3',4':6,7]indolazino [1,2-b]quinolin-9-yl)oxy)methyl)-2-methyl-3-(prop-2-ynyloxy)phenoxy)-6-(methoxycarbonyl)tetrahydro-2H-pyran-3,4,5-triacetate

2.6g of product (2S,3R,4S,5S,6S)-2-(4-(((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)-2-methyl-3-(prop-2-ynyloxy)phenoxy)-6-(methoxycarbonyl)tetrahydro-2H-pyran-3,4,5-triacetate was prepared with (2S,3R,4S,5S,6S)-2-(4-(bromomethyl)-2-methyl-3-(prop-2-ynyloxy) phenoxy)-6-(met hoxycarbonyl)tetrahydro-2H-pyran-3,4,5-triacetate and compound (S)-4,11-diethyl-4,9-dihydroxy-1,12-dihydro-14H-pyrano[3',4':6,7]indolazino[1,2-b] quinoline-3,14(4H)-dione by the preparation method of Example 5. MS (m/e): 883.49 $[M+H]^+$. $^1$H NMR (DMSO-d6, 600MHz) δ: 8.086-8.071 (d, 1H), 7.603-7.598 (d, 1H), 7.541-7.521 (dd, 1H), 7.421-7.406 (d, 1H), 7.272 (s, 1H), 6.937-6.923 (d, 1H), 6.502 (s, 1H), 5.610-5.597 (d, 1H), 5.507-5.460 (t, 1H), 5.432 (s, 2H), 5.350-5.323 (d, 2H), 5.297 (s, 2H), 5.179-5.150 (q, 1H), 5.097-5.059 (t, 1H), 4.745-4.734 (d, 1H), 4.700-4.696 (d, 2H), 3.652-3.635 (s, 3H), 3.609-3.601 (t, 1H), 3.196-3.159 (dd, 2H), 2.086 (s, 3H), 2.039 (s, 3H), 2.013-2.011 (d, 6H), 1.915-1.829 (m, 2H), 1.294-1.268 (t, 3H), 0.896-0.872 (t, 3H).

Example 13

(2S,3S,4S,5R,6S)-6-(4-(((S)-4,11-diethyl-4-hydroxy-3,14-dioxy-3,4,12,14-tetrahydro-1H-pyrano[3',4':6,7]indolazino [1,2-b]quinolin-9-yl)oxy)methyl)-2-methyl-3-(prop-2-ynyloxy)phenoxy)-3,4,5-trihydroxytetrahydro-2H-pyran-2-carboxylic acid

1.5 g of the product was prepared with (2S,3R,4S,5S,6S)-2-(4-((((S)-4,11-diethyl-4-hydroxy-3,14-dioxy-3,4,12,14-tetrahydro -1H-pyrano[3',4':6,7]indolizino[1,2-b]quinolin-9-yl)oxy)methyl)-2-methyl-3-(prop-2-ynyloxy) phenoxy)-6-(methoxycarbonyl)tetrahydro-2H-pyran-3,4,5-triacetate by the preparation method of Example 6. MS (m/e): 743.38 $[M+H]^+$. $^1$H NMR (DMSO-$d_6$, 600MHz) δ: 8.064-8.049 (d, 1H), 7.608 (s, 1H), 1.913-1.830 (d, 1H), 1.315-1.290 (d, 1H), 7.265 (s, 1H), 6.969-6.955 (d, 1H), 6.497 (s, 1H), 5.454-5.398 (t, 2H), 3.345-3.291 (m, 6H), 5.134 (s, 1H), 0.897-0.873 (d, 1H), 4.680-4.677 (d, 2H), 3.591-3.583 (t, 2H), 3.345-3.291 (d, 3H), s (d, 2H), 2.209 (s, 3H), 1.913-1.830 (m, 2H), 1.315-1.290 (t, 3H), 0.897-0.873 (t, 3H).

Example 14

(25,35,4S,5R,65)-6-(3-((1-(6-((4'-((E)-(8-amino-1-hydroxy-5,7-disulfonicnaphthalen -2-yl)diazenyl)-3,3'-dimethyl-[1,1'-biphenyl]-4-yl)amino)-6-oxohexyl)-1H-1,2,3-triaz ol-4-yl)methoxy)-4-((((S)-4,11-diethyl-4-hydroxy-3,14-dioxy-3,4,12,14-tetrahydro-1 H-pyrano[3',4':6,7]indolazino[1,2-b]quinolin-9-yl)oxy)methyl)2-methylphenoxy)-3,4 ,5-trihydroxytetrahydro-2H-pyran-2-carboxylate trisodium

248mg of the product was prepared with (2S,3S,4S,5R,6S)-6-(4-(((S)-4,11-diethyl-4-hydroxy-3,14-dioxy-3,4,12,14-tetrahydro-1H-pyrano[3',4':6,7]indolizino[1,2-b]quinolin-9-yl)oxy)methyl)-2-methyl-3-(prop-2-ynyloxy) phenoxy)-3,4,5-trihydroxytetrahydro-2H-pyran-2-carboxylic acid and (E)-4-amino-6-((4'-(6-azidohexanamide)-3,3'-dimethyl-[1,1'-biphenyl]-4-yl)diazenyl )-5-hydroxynaphthalene-1,3-disulfonate disodium by the preparation method of Example 7. MS (m/e): 1424.70 $[M-3Na+4H]^+$. $^1H$ NMR (DMSO-$d_6$, 600MHz) $\delta$: 15.915 (s, 1H), 9.253 (s, 1H), 8.370 (s, 1H), 8.186(s, 1H), 8.063-8.032 (t, 2H), 7.887-7.872 (d, 1H), 7.629-7.619 (d, 3H), 7.535-7.504 (m, 2H), 7.469 (s, 2H), 7.331-7.317 (d, 1H), 7.268 (s, 1H), 7.008-6.956 (dd, 2H), 6.483 (s, 1H), 5.419-5.414 (d, 2H), 5.292 (s, 2H), 5.253-5.180 (m, 3H), 5.055-5.016 (q, 2H), 4.979 (s, 1H), 4.817-4.805 (d, 1H), 4.342-4.319 (t, 2H), 3.402-3.385 (d, 1H), 3.271-3.255 (m, 2H), 3.171-3.148 (t, 3H), 2.511-2.502 (m, 3H), 2.311-2.288 (t, 2H), 2.243-2.235 (d, 6H), 1.893-1.823 (m, 2H), 1.811-1.772 (m, 2H), 1.588-1.564 (m, 2H), 1.264-1.251 (m, 5H), 0.885-0.860 (t, 3H).

Example 15

4-amino-6-((E)-(4'-(6-(4-((3-(((2S,3R,4S,5S,6S)-6-carboxy-3,4,5-trihydroxytetrahydr o-2H-pyran-2-yl) oxy)-6-((((S)-4,11-diethyl-4-hydroxy-3,14-dioxy-3,4,12,14-tetrahydr o-1H-pyrano[3',4':6,7]indolazino[1,2-b]quinolin-9-yl)oxy)methyl)-2-tolyloxy)methyl )-1H-1,2,3-triazol-1-yl)hexanamido)-3,3'-dimethyl-[1,1'-biphenyl]-4-yl)diazenyl)-5-h ydroxynaphthalene-1,3-disulfonate disodium

At room temperature, 49mg of the compound of Example 14 was dissolved in 1 ml of ionic water. To the mixture was added 0.33ml of 0.1M hydrochloric acid with stirring. The resultant was stirred at room temperature for 1 hour. A purple reaction solution was prepared and purified under medium pressure. The preparation solution was concentrated to remove acetonitrile and lyophilized to give 10 mg of the product. MS (m/e): 1424.53 $[M-2Na+3H]^+$.

Example 16

methyl (2S,3S,4S,SR,6S)-6-(4-(((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)-2-methyl-3-(prop-2-ynyloxy)phenoxy)-3,4,5-trihydroxytetrahydro-2H-pyran-2-carboxylate

126mg of the product was prepared with(2S,3R,4S,5S,6S)-2-(4-((((S)-4,11-diethyl-4-hydroxy-3,14-dioxy-3,4,12,14-tetrah ydro-1H-pyrano[3',4':6,7]indolizino[1,2-b]quinolin-9-yl)oxy)methyl)-2-methyl-3-(pr op-2-ynyloxy) phenoxy)-6-(methoxycarbonyl)tetrahydro-2H-pyran-3,4,5-triacetate by the preparation method of Example 20. MS (m/e): 757.58 $[M+H]^+$.

Example 17

4-amino-6-((E)-(4'-(6-(4-((6-(((S)-4,11-diethyl-4-hydroxy-3,14-dioxy-3,4,12,14-tetra hydro-1H-pyrano[3',4':6,7]indolazo [1,2-b]quinolin-9-yl)oxy)methyl)-2-methyl-3-(((( 2S,3R,4S,5S,6S)-3,4,5-trihydroxy-6-(methoxycarbonyl)tetrahydro-2H-pyran-2-yl)oxy )phenoxy)methyl)-1H-1,2,3-triazol-1-yl)hexanamido)-3,3'-dimethyl-[1,1'-biphenyl]-4 -yl)diazenyl)-5-hydroxynaphthalene-1,3-disulfonate disodium

24mg of product was prepared with methyl (2S,3S,4S,5R,6S)-6-(4-(((S)-4,11-diethyl-4-hydroxy-3,14-dioxy-3,4,12,14-tetrahydro-1H-pyrano[3',4':6,7]indolazo[1,2-b]quinolin-9-yl)oxy)methyl)-2-methyl-3-(prop-2-y nyloxy) phenoxy)-3,4,5-trihydroxytetrahydro-2H-pyran-2-carboxylate and (E)-4-amino-6-((4'-(6-azidohexanamide)-3,3'-dimethyl-[1,1'-biphenyl]-4-yl)diazenyl )-5-hydroxynaphthalene-1,3-disulfonate disodium by the preparation method of Example 7. MS (m/e): 1438.57 $[M-2Na+3H]^+$.

Example18

(25,35,4S,5R,65)-6-(3-((1-(6-((4'-((E)-(8-amino-1-hydroxy-5,7-disulfonicnaphthalen -2-yl)diazenyl)-[1,1'-biphenyl]-4-yl) amino)-6-oxohexyl)-1H-1,2,3-triazol-4-yl)metho xy)-4-((((S)-4,11-diethyl-4-hydroxy-3,14-dioxo-3,4,12,14-tetrahydro-1H-pyrano[3',4' :6,7]indolazino[1,7]quinolin-9-yl)oxy)methyl)-2-methylphenoxy)-3,4,5-trihydroxytetr ahydro-2H-pyran-2-carboxylate trisodium

135mg of product was prepared with (2S,3S,4S,5R,6S)-6-(4-(((S)-4,11-diethyl-4-hydroxy-3,14-dioxy-3,4,12,14-tetrahydro-1H-pyrano[3',4':6,7]indolazo[1,2-b]quinolin-9-yl)oxy)methyl)-2-methyl-3-(prop-2-y nyloxy)phenoxy)-3,4,5-trihydroxytetrahydro-2H-pyran-2-carboxylic acid and (E)-4-amino-6-((4'-(6-azidohexanamide)-[1,1'-biphenyl]-4-yl)diazenyl)-5-hydroxyna phthalene-1,3-disulfonate disodium by the preparation method of Example 7. MS (m/e): 1397.04 $[M-3Na+4H]^+$. $^1H$ NMR (DMSO-$d_6$, 600MHz) $\delta$: 15.644 (s, 1H), 10.036 (s, 1H), 8.353 (s, 1H), 8.150 (s, 1H), 8.061-8.046 (d, 1H), 8.009-7.992 (d, 1H), 7.719-7.705 (d, 2H), 7.669-7.649 (dd, 4H), 7.626-7.611 (d, 3H), 7.514-7.495 (d, 1H), 7.286-7.262 (t, 2H), 6.960-6.928 (t, 2H), 6.472 (s, 1H), 5.438-5.378 (q, 2H), 5.296 (s, 2H), 5.230-5.211 (d, 2H), 5.161-5.142 (d, 1H), 5.052-5.002 (q, 2H), 4.969-4.963 (d, 1H), 4.808 (d, 1H), 4.332-4.309 (t, 2H), 3.403-3.388 (d, 1H), 3.259 (s, 2H), 3.166-3.154 (d, 3H), 2.270-2.246 (t, 2H), 2.211 (s, 3H), 1.874-1.797 (m, 2H), 1.775-1.727 (m, 2H), 1.555-1.531 (m, 2H), 1.282-1.256 (t, 3H), 1.192 (m, 2H), 0.878-0.853 (t, 3H).

Example 19

4-amino-6-((E)-(4'-(6-(4-((5-(((2S,3R,4S,5S,6S)-6-carboxy-3,4,5-trihydroxytetrahydr o-2H-pyran-2-yl) oxy)-2-((((S)-4,11-diethyl-4-hydroxy-3,14-dioxy-3,4,12,14-tetrahydr o-1H-pyrano[3',4':6,7]indolazino[1,2-b]quinolin-9-yl)oxy)methyl)phenoxy)methyl)-1 H-1,2,3-triazol-1-yl)hexanamido)-3,3'-dimethyl-[1,1'-biphenyl]-4-yl)diazenyl)-5-hyd roxynaphthalene-1,3-disulfonate disodium

15mg of the product was prepared with (2S,3S,4S,5R,6S)-6-(3-((1-(6-((4'-((E)-(8-amino-1-hydroxy-5,7-disulfonicnaphthalen -2-yl)diazenyl)-3,3'-dimethyl-[1,1'-biphenyl]-4-yl)amino)-6-oxohexyl)-1H-1,2,3-triaz ol-4-yl)methoxy)-4-(((S)-4,11-diethyl-4-hydroxy-3,14-dioxo-3,4,12,14-tetrahydro-1H -pyrano[3',4':6,7]indolazino[1,2-b]quinolin-9-yl)oxy)methyl)phenoxy)-3,4,5-trihydro xytetrahydro-2H-pyran-2-carboxylate trisodium by the preparation method of Example 15. MS (m/e): 1410.57 $[M-2Na+3H]^+$.

Example 20

methyl (25,35,4S,5R,65)-6-(4-(((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)-3-(prop-2-ynyloxy)p henoxy)-3,4,5-trihydroxytetrahydro-2H-pyran-2-carboxylate

1 g of (2S,3R,4S,5S,6S)-2-(4-(((S)-4,11-diethyl-4-hydroxy-3,14-dioxo-3,4,12,14-tetrahydro-1H-pyrano [3',4':6,7]indolazo[1,2-b]quinolin-9-yl)oxy)methyl)-3-(prop-2-ynyloxy)ph enoxy)-6-(methoxycarbonyl)tetrahydro-2H-pyran-3,4,5-triacetate, 120ml of methanol, 120ml of tetrahydrofuran are dissolved under stirring at room temperature. 180mg of methoxide sodium was added. The mixture was stirred at room temperature for 1 hour. Five drops of glacial acetic acid were added to the reaction. The mixture was evaporated to dryness. The resultant was dissolved in methanol. The mixture was stirred until clear. 670mg of the product was obtained after purification by column chromatography. MS (m/e): 743.34 $[M+H]^+$. $^1H$ NMR (DMSO-$d_6$, 600MHz) $\delta$: 8.083-8.068 (m, 1H), 0.899-0.848 (m, 1H), 4.091-4.068 (1.310-1.284, 1H), 7.443-7.429 (m, 1H), 3.661 (s, 1H), 4.918-4.914 (m, 1H), 3.572-3.564 (1.310-1.284, 1H), 1.915-1.832 (s, 1H), 5.495-5.487 (m, 1H), 5.430-5.421 (m, 3H), 5.301 (s, 2H), t (m, 1H), 5.235 (s, 2H), 5.170-5.158 (m, 1H), 4.918-4.914 (m, 2H), 4.091-4.068 (1.310-1.284, 1H), 3.661 (s, 3H), 3.572-3.564 (t, 1H), 3.437-3.305 (m, 3H), 3.190-3.165 (m, 2H), 1.915-1.832 (m, 2H), 1.310-1.284 (t, 3H), 0.899-0.848 (m, 3H).

Example 21

4-amino-6-((E)-(4'-(6-(4-((2-(((S)-4,11-diethyl-4-hydroxy-3,14-dioxy-3,4,12,14-tetra hydro-1H-pyrano[3',4':6,7]indolazo [1,2-b]quinolin-9-yl)oxy)methyl)-5-(((2S,3R,4S,5 S,6S)-3,4,5-trihydroxy-6-(methoxycarbonyl)tetrahydro-2H-pyran-2-yl) oxy)phenoxy) methyl)-1H-1,2,3-triazol-1-yl)hexanamido)-3,3'-dimethyl-[1,1'-biphenyl]-4-yl)diazen yl)-5-hydroxynaphthalene-1,3-disulfonate disodium

223mg of the product was prepared with methyl (2S,3S,4S,SR,6S)-6-(4-(((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)-3-(prop-2-ynyloxy)p henoxy)-3,4,5-trihydroxytetrahydro-2H-pyran-2-carboxylate and (E)-4-amino-6-((4'-(6-azidohexanamide)-3,3'-dimethyl-[1,1'-biphenyl]-4-yl)diazenyl )-5-hydroxynaphthalene-1,3-disulfonate disodium by the preparation method of Example 7. MS (m/e): 1424.91 $[M-2Na+3H]^+$. $^1H$ NMR (DMSO-d6, 600MHz) δ:15.945 (s, 1H), 9.217 (s, 1H), 8.368 (s, 1H), 8.182 (s, 1H), 8.065-8.050 (d, 1H), 8.040-8.024 (d, 1H), 7.881-7.866 (d, 1H), 7.625-7.616 (d, 2H), 7.533 (s, 3H), 7.465 (s, 2H), 7.413-7.398 (d, 1H), 7.264 (s, 1H), 7.005-6.978 (m, 2H), 6.688-6.670 (dd, 1H), 6.504 (s, 1H), 5.533-5.525 (d, 1H), 5.474-5.465 (d, 1H), 5.415 (s, 2H), 5.329-5.320 (d, 1H), 5.292-5.272 (t, 3H), 5.225-5.195 (t, 4H), 4.354-4.330 (t, 2H), 4.132-4.116 (d, 1H), 3.654 (s, 3H), 3.438-3.398 (m, 1H), 3.328 (s, 2H), 3.139-3.127 (dd, 2H), 2.514-2.505 (s, 3H), 2.306-2.284 (t, 2H), 2.226 (s, 3H), 1.876-1.792 (m, 4H), 1.593-1.569 (m, 2H), 1.266-1.219 (m, 5H), 0.883-0.858 (t, 3H).

Example 22

(2S,3R,4S,5S,6S)-2-(4-(((S)-4-ethyl-4-hydroxy-3,14-dioxo-3,4,12,14-tetrahydro-1H-p yrano[3',4':6,7]indolizino[1,2-b]quinolin-9-yl)oxy)methyl)-3-(prop-2-ynyloxy)pheno xy)-6-(methoxycarbonyl)tetrahydro-2H-pyran-3,4,5-triacetate

700mg of the product was prepared with (2S,3R,4S,5S,6S)-2-(4-(bromomethyl)-3-(prop-2-ynyloxy)phenoxy)-6-(methoxycarbo nyl)tetrahydro-2H-pyran-3,4,5-triacetate and (S)-4-ethyl-4,9-dihydroxy-1,12-dihydro-14H-pyrano [3',4':6,7]indolazino[1,2-b]quino line-3,14(4H)-dione by the preparation method of Example 5. MS (m/e): 841.88 $[M+H]^+$.

Example 23

(2S,3S,4S,5R,6S)-6-(4-(((S)-4-ethyl-4-hydroxy-3,14-dioxo-3,4,12,14-tetrahydro-1H-p yrano[3',4':6,7]indolazino[1,2-b]quinolin-9-yl)oxy)methyl)-3-(prop-2-ynyloxy)pheno xy)-3,4,5-trihydroxytetrahydro-2H-pyran-2-carboxylic acid

220mg of the product was prepared with (2S,3R,4S,5S,6S)-2-(4-((((S)-4-ethyl-4-hydroxy-3,14-dioxy-3,4,12,14-tetrahydro-1H-pyrano[3',4':6,7]indolazo[1,2-b]quinolin-9-yl)oxy)methyl)-3-(prop-2-ynyloxy)pheno xy)-6-(methoxycarbonyl)tetrahydro-2H-pyran-3,4,5-triacetate by the preparation method of Example 6. MS (m/e): 701.51 $[M+H]^+$. $^1H$ NMR (DMSO-$d_6$, 600MHz) δ: 8.551 (s, 1H), 8.075-8.059 (1.917-1.822, 1H), m (s, 1H), 7.554-7.538 (1.917-1.822, 1H), 7.418-7.404 (1.917-1.822, 1H), 0.802-0.878 (s, 1H), 6.891 (s, 1H), 3.185-3.154 (1.917-1.822, 1H), 5.450-5.394 (t, 2H), 5.273 (s, 2H), 5.182 (s, 2H), 4.889 (s, 3H), 3.596 (s, 1H), 3.446-3.430 (1.917-1.822, 1H), 3.296-3.227 (m, 2H), 3.185-3.154 (t, 1H), 1.917-1.822 (m, 2H), 0.802-0.878 (t, 3H).

Example 24

(25,35,4S,5R,65)-6-(3-((1-(6-((4'-((E)-(8-amino-1-hydroxy-5,7-disulfonicnaphthalen -2-yl)diazenyl)-3,3'-dimethyl-[1,1'-biphenyl]-4-yl)amino)-6-oxohexyl)-1H-1,2,3-triaz ol-4-yl)methoxy)-4-(((S)-4-ethyl-4-hydroxy-3,14-dioxy-3,4,12,14-tetrahydro-1H-pyra no[3',4':6,7]indolazino[1,2-b]quinolin-9-yl)oxy)methyl)phenoxy)-3,4,5-trihydroxytet rahydro-2H-pyran-2-carboxylate trisodium

68mg of the product was prepared with (2S,3S,4S,5R,6S)-6-(4-(((S)-4-ethyl-4-hydroxy-3,14-dioxy-3,4,12,14-tetrahydro-1H-p yrano[3',4':6,7]indolazo[1,2-b]quinolin-9-yl)oxy)methyl)-3-(prop-2-ynyloxy)phenox y)-3,4,5-trihydroxytetrahydro-2H-pyran-2-carboxylicacid and (E)-4-amino-6-((4'-(6-azidohexanamide)-3,3'-dimethyl-[1,1'-biphenyl]-4-yl) diazenyl )-5-hydroxynaphthalene-1,3-disulfonate disodium by the preparation method of Example 7. MS (m/e): 1380.59 $[M-3Na+2H]^-$. $^1H$ NMR (DMSO-d6, 600MHz) δ: 15.956 (s, 1H), 9.529 (s, 1H), 8.526 (s, 1H), 8.371-8.357 (d, 2H), 8.062-8.027 (t, 2H), 7.889-7.871 (d, 1H), 7.631 (s, 2H), 7.583-7.579 (d, 1H), 7.531-7.513 (dd, 2H), 7.480-7.446 (t, 2H), 7.368-7.355 (d, 1H), 7.239 (s, 1H), 7.041 (s, 1H), 7.011-6.991 (d, 1H), 6.646 (s, 1H), 6.501 (s, 1H), 5.441-5.385 (t, 2H), 5.343-5.322 (d, 1H), 5.262 (s, 4H), 5.142 (t, 2H), 5.038 (d, 1H), 4.962 (s, 1H), 4.375-4.367 (d, 2H), 3.582 (s, 1H), 3.295-3.189 (m, 3H), 2.511-2.502 (m, 3H), 2.352 (t, 2H), 2.234 (s, 3H), 1.896-1.815 (m, 4H), 1.606 (s, 2H), 1.281-1.260 (t, 2H), 0.891-0.867 (t, 3H).

Example 25

4-amino-6-((E)-(4'-(6-(4-((5-(((2S,3R,4S,5S,6S)-6-carboxy-3,4,5-trihydroxytetrahydr o-2H-pyran-2-yl)oxy)-2-((((S)-4-ethyl-4-hydroxy-3,14-dioxo-3,4,12,14-tetrahydro-1H -pyrano[3',4':6,7]indolazino[1,2-b]quinolin-9-yl)oxy)methyl)phenoxy)methyl)-1H-1, 2,3-triazol-1-ylhexanamido)-3,3'-dimethyl-[1,1'-biphenyl]-4-yl)diazenyl)-5-hydroxyn aphthalene-1,3-disulfonate disodium

12mg of product was prepared with (2S,3S,4S,5R,6S)-6-(3-((1-(6-((4'-((E)-(8-amino-1-hydroxy-5,7-disulfonicnaphthalen -2-yl)diazenyl)-3,3'-dimethyl-[1,1'-biphenyl]-4-yl)amino)-6-oxohexyl)-1H-1,2,3-triaz ol-4-yl)methoxy)-4-(((S)-4-ethyl-4-hydroxy-3,14-dioxo-3,4,12,14-tetrahydro-1H-pyra no[3',4':6,7]indolazino[1,2-b]quinolin-9-yl)oxy)

methyl)phenoxy)-3,4,5-trihydroxytet rahydro-2H-pyran-2-carboxylate trisodium by the preparation process of Example 15. MS (m/e): 1382.44 [M-2Na+3H]+.

Example 26

methyl (25,3S,4S,5R,6S)-6-(4-(((S)-4-ethyl-4-hydroxy-3,14-dioxo-3,4,12,14-tetrahydro-1H-p yrano[3',4':6,7]indolazino [1,2-b]quinolin-9-yl)oxy)methyl)-3-(prop-2-ynyloxy)pheno xy)-3,4,5-trihydroxytetrahydro-2H-pyran-2-carboxylate

108mg of the product was prepared with (2S,3R,4S,5S,6S)-2-(4-((((S)-4-ethyl-4-hydroxy-3,14-dioxy-3,4,12,14-tetrahydro-1H-pyrano[3',4':6,7]indolazo[1,2-b]quinolin-9-yl)oxy)methyl)-3-(prop-2-ynyloxy)pheno xy)-6-(methoxycarbonyl)tetrahydro-2H-pyran-3,4,5-triacetate by the preparation method of Example 20. MS (m/e): 715.23 [M+H]+.

Example 27

4-amino-6-((E)-(4'-(6-(4-((2-(((S)-4-ethyl-4-hydroxy-3,14-dioxo-3,4,12,14-tetrahydro -1H-pyrano[3',4':6,7]indolizino [1,2-b]quinolin-9-yl)oxy)methyl)-5-(((2S,3R,4S,5S,6 S)-3,4,5-trihydroxy-6-(methoxycarbonyl)tetrahydro-2H-pyran-2-yl) oxy)phenoxy)met hyl)-1H-1,2,3-triazol-1-yl)hexanamido)-3,3'-dimethyl-[1,1'-biphenyl]-4-yl)diazenyl)-5-hydroxynaphthalene-1,3-disulfonate disodium

19mg of the product was prepared with methyl (2S,3S,4S,5R,6S)-6-(4-(((S)-4-ethyl-4-hydroxy-3,14-dioxy-3,4,12,14-tetrahydro-1H-p yrano[3',4':6,7]indolazo[1,2-b]quinolin-9-yl)oxy)methyl)-3-(prop-2-ynyloxy)phenox y)-3,4,5-trihydroxytetrahydro-2H-pyran-2-carboxylate and (E)-4-amino-6-((4'-(6-azidohexanamide)-3,3'-dimethyl-[1,1'-biphenyl]-4-yl)diazenyl )-5-hydroxynaphthalene-1,3-disulfonate disodium by the preparation method of Example 7. MS (m/e): 1396.37 [M-2Na+3H]+.

Example 28

(25,35,4S,5R,65)-6-(3-((1-(6-((4'-((E)-(8-amino-1-hydroxy-5,7-disulfonicnaphthalen -2-yl)diazenyl)-[1,1'-biphenyl]-4-yl) amino)-6-oxohexyl)-1H-1,2,3-triazol-4-yl)metho xy)-4-((((S)-4,11-diethyl-4-hydroxy-3,14-dioxo-3,4,12,14-tetrahydro-1H-pyrano[3',4' :6,7]indolazino[1,2-b]quinolin-9-yl)oxy)methyl)-5-methoxyphenoxy)-3,4,5-trihydrox ytetrahydro-2H-pyran-2-carboxylate trisodium

7mg of the product was prepared with (2S,3S,4S,5R,6S)-6-(4-(((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)-3-methoxy-5-(prop-2-ynyloxy) phenoxy)-3,4,5-trihydroxytetrahydro-2H-pyran-2-carboxylic acid and (E)-4-amino-6-((4'-(6-azidohexanamide)-[1,1'-biphenyl]-4-yl)diazenyl)-5-hydroxyna phthalene-1,3-disulfonate disodium by the prepared method of Example 7. MS (m/e): 1412.11 [M-3Na+4H]+.

Example 29

(2S,3S,4S,5R,6S)-6-(3-((1-(6-((4'-((E)-(8-amino-1-hydroxy-5,7-disulfonicnaphthalen -2-yl)diazenyl)-[1,1'-biphenyl]-4-yl)amino)-6-oxohexyl)-1H-1,2,3-triazol-4-yl)metho xy)-4-((((S)-4-ethyl-4-hydroxy-3,14-dioxo-3,4,12,14-tetrahydro-1H-pyrano[3',4':6,7] indolazino[1,2-b]quinolin-9-yl)oxy)methyl)phenoxy)-3,4,5-trihydroxytetrahydro-2H-pyran-2-carboxylate trisodium

82mg of the product was prepared with (2S,3S,4S,5R,6S)-6-(4-(((S)-4-ethyl-4-hydroxy-3,14-dioxy-3,4,12,14-tetrahydro-1H-p yrano[3',4':6,7]indolazo[1,2-b]quinolin-9-yl)oxy)methyl)-3-(prop-2-ynyloxy)phenox y)-3,4,5-trihydroxytetrahydro-2H-pyran-2-carboxylic acid and (E)-4-amino-6-((4'-(6-azidohexanamide)-[1,1'-biphenyl]-4-yl)diazenyl)-5-hydroxyna phthalene-1,3-disulfonate disodium by the preparation method of Example 7. MS (m/e): 1354.57 [M-3Na+4H]+.

Example 30

(2S,3S,4S,5R,6S)-6-(3-((1-(6-((4'-((E)-(8-amino-1-hydroxy-5,7-disulfonicnaphthalen -2-yl)diazenyl)-3,3'-dimethyl-[1,1'-biphenyl]-4-yl)amino)-6-oxohexyl)-1H-1,2,3-triaz ol-4-yl)methoxy)-4-((((S)-4,11-diethyl-4-hydroxy-3,14-dioxy-3,4,12,14-tetrahydro-1 H-pyrano[3',4':6,7]indolazino[1,2-b]quinolin-9-yl)oxy)methyl)-5-methoxyphenoxy)-3,4,5-trihydroxytetrahydro-2H-pyran-2-carboxylate trisodium

5 mg of the product was prepared with (2S,3S,4S,5R,6S)-6-(4-(((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)-3-methoxy-5-(prop-2-ynyloxy) phenoxy)-3,4,5-trihydroxytetrahydro-2H-pyran-2-carboxylic acid and (E)-4-amino-6-((4'-(6-azidohexanamide)-3,3'-dimethyl-[1,1'-biphenyl]-4-yl)diazenyl )-5-hydroxynaphthalene-1,3-disulfonate disodium by the preparation method of Example 7. MS (m/e): 1440.2 [M-3Na+4H]+.

Example 31

(2S,3S,4S,5R,6S)-6-(3-((1-(6-((4'-((E)-(8-amino-1-hydroxy-5,7-disulfonicnaphthalen -2-yl)diazenyl)-3,3'-dimethoxy-[1,1' -biphenyl]-4-yl)amino)-6-oxohexyl)-1H-1,2,3-tri azol-4-yl)methoxy)-4-(((S)-4-ethyl-4-hydroxy-3,14-dioxy-3,4,12,14-tetrahydro-1H-py rano[3',4':6,7]indolazino[1,2-b]quinolin-9-yl)oxy)methyl)phenoxy)-3,4,5-trihydroxyt etrahydro-2H-pyran-2-carboxylate trisodium

82mg of the product was prepared with (2S,3S,4S,5R,6S)-6-(4-(((S)-4-ethyl-4-hydroxy-3,14-dioxy-3,4,12,14-tetrahydro-1H-p yrano[3',4':6,7]indolazo[1,2-b]quinolin-9-yl)oxy)methyl)-3-(prop-2-ynyloxy)phenox y)-3,4,5-trihydroxytetrahydro-2H-pyran-2-carboxylic acid and (E)-4-amino-6-((4'-(6-azidohexanamide)-3,3'-dimethoxy-[1,1'-biphenyl]-4-yl) diazen yl)-5-hydroxynaphthalene-1,3-disulfonate disodium by the preparation method of Example 7. MS (m/e): 1414.80 [M-3Na+4H]+.

Example 32

4-amino-6-((E)-(4'-(6-(4-((5-(((2S,3R,4S,5S,6S)-6-carboxy-3,4,5-trihydroxytetrahydr o-2H-pyran-2-yl)oxy)-2-((((S)-4-ethyl-4-hydroxy-3,14-dioxo-3,4,12,14-tetrahydro-1H -pyrano[3',4':6,7]indolazino[1,2-b]quinolin-9-yl)oxy)methyl)phenoxy)methyl)-1H-1, 2,3-triazol-1-yl)hexanamido)-3,3'-dimethoxy-[1,1'-biphenyl]-4-yl)diazenyl)-5-hydrox ynaphthalene-1,3-disulfonate disodium

15mg of the product was prepared with (2S,3S,4S,5R,6S)-6-(3-((1-(6-((4'-((E)-(8-amino-1-hydroxy-5,7-disulfonicnaphthalen -2-yl)diazenyl)-3,3'-dimethoxy-[1,1'-biphenyl]-4-yl)amino)-6-oxohexyl)-1H-1,2,3-tri azol-4-yl)methoxy)-4-(((S)-4-ethyl-4-hydroxy-3,14-dioxo-3,4,12,14-tetrahydro-1H-py rano[3',4':6,7]indolazino[1,2-b]quinolin-9-yl)oxy) methyl)phenoxy)-3,4,5-trihydroxyt etrahydro-2H-pyran-2-carboxylate trisodium by the preparation method of Example 15. MS (m/e): 1414.56 [M-2Na+3H]+.

Example 33

4-amino-6-((E)-(4'-(6-(4-((2-(((S)-4-ethyl-4-hydroxy-3,14-dioxo-3,4,12,14-tetrahydro -1H-pyrano[3',4':6,7]indolizino [1,2-b]quinolin-9-yl)oxy)methyl)-5-(((2S,3R,4S,5S,6 S)-3,4,5-trihydroxy-6-(methoxycarbonyl)tetrahydro-2H-pyran-2-yl) oxy)phenoxy)met hyl)-1H-1,2,3-triazol-1-yl)hexanamido)-3,3'-dimethoxy-[1,1'-biphenyl]-4-yl)diazenyl )-5-hydroxynaphthalene-1,3-disulfonate disodium

11mg of the product was prepared with (2S,3S,4S,5R,6S)-6-(4-(((S)-4-ethyl-4-hydroxy-3,14-dioxy-3,4,12,14-tetrahydro-1H-p yrano[3',4':6,7]indolazo[1,2-b]quinolin-9-yl)oxy)methyl)-3-(prop-2-ynyloxy)phenox y)-3,4,5-trihydroxytetrahydro-2H-pyran-2-carboxylicacidmethylesterand(E)-4-amino-6-((4'-(6-azidohexanamide)-3,3'-dimethoxy-[1,1'-biphenyl]-4-yl)diazinyl)-5-hydroxy naphthalene-1,3-disulfonate disodium by the preparation method of Example 7. MS (m/e): 1428.33 [M-2Na+3H]+.

Example 34

(2S,3S,4S,5R,6S)-6-(3-((1-(6-((4'-((E)-(8-amino-1-hydroxy-5,7-disulfonicnaphthalen -2-yl)diazenyl)-3,3'-dimethyl-[1,1'-biphenyl]-4-yl)amino)-6-oxohexyl)-1H-1,2,3-triaz ol-4-yl)methoxy)-4-(((S)-10-((dimethylamino)methyl)-4-ethyl-4-hydroxy-3,14-dioxy-3,4,12,14-tetrahydro-1H-pyrano[3',4':6,7]indolazino[1,2-b]quinolin-9-yl)oxy)methyl) phenoxy)-3,4,5-trihydroxytetrahydro-2H-pyran-2-carboxylate trisodium

5 mg of the product was prepared with (2S,3S,4S,5R,6S)-6-(4-(((S)-10-((dimethylamino)methyl)-4-ethyl-4-hydroxy-3,14-dio xo-3,4,12,14-tetrahydro-1H-pyrano[3',4':6,7]indolizino[1,2-b]quinolin-9-yl)oxy)meth yl)-3-(prop-2-ynyloxy)phenoxy)-3,4,5-trihydroxytetrahydro-2H-pyran-2-carboxylic acid and (E)-4-amino-6-((4'-(6-azidohexanamide)-3,3'-dimethyl-[1,1'-biphenyl]-4-yl)diazenyl )-5-hydroxynaphthalene-1,3-disulfonate disodium by the preparation method of Example 7. MS (m/e): 1439.21 [M-3Na+4H]+.

Example 35

tert-butyl (4'-amino-3,3'-dimethyl-[1,1'-biphenyl]-4-yl)carbamate

5g of 3,3'-dimethyl-[1,1'-biphenyl]-4,4'-diamine was weighed and added into 75ml of 1,4-dioxane. The mixture was stirred under argon at room temperature. 5.14g of $Boc_2O$ was weighed and diluted with 25ml of 1,4-dioxane. The resultant was added dropwise to the reaction solution. The resultant mixture was stirred at room temperature overnight. The resultant was concentrated to dryness. 4.485g of product was obtained after purification by column chromatography.

Example 36

tert-butyl (4'-(8-bromooctanamide)-3,3'-dimethyl-[1,1'-biphenyl]-4-yl)carbamate

2.4g of tert-butyl (4'-amino-3,3'-dimethyl-[1,1'-biphenyl]-4-yl)carbamate and 2g pf 8-bromooctanoic acid were weighed and added into 40 ml of dichloromethane to dissolve. To the mixture were added 2.1g of EDCI and 1g of HOBT successively. 1.3ml of DIEA was added dropwise. The mixture was stirred under argon at room temperature overnight and concentrated to dryness by water. 1.247g of the product was obtained after purification by column chromatography. MS (m/e): 517.25 [M+H]+.

Example 37

N-(4'-amino-3,3'-dimethyl-[1,1'-biphenyl]-4-yl)-8-bromooctanamide

1. 147g of tert-butyl (4'-(8-bromooctanamide)-3,3'-dimethyl-[1,1'-biphenyl]-4-yl)carbamate was weighed and dissolved in 14ml of dichloromethane. To the mixture was added 1.4ml of trifluoroacetic acid dropwise at room temperature for 2 hours. The resultant was dried and purified by column chromatography to give 1.159g of the product. MS (m/e): 417.24 $[M+H]^+$.

Example 38

N-(4'-amino-3,3'-dimethyl-[1,1'-biphenyl]-4-yl)-8-azocinamide

1.159g of N-(4'-amino-3,3'-dimethyl-[1,1'-biphenyl]-4-yl)-8-bromooctanamide was weighed and added into a reaction flask. To the reaction flask were added 8ml of dimethyl sulfoxide to resolve and 271mg sodium azide. The reaction was transferred to an oil bath and stirred for 6 hours. The reaction was quenched and stirred with 40 ml of water, stirred for 10 minutes, adjusted to pH 7 to 8 with saturated aqueous sodium bicarbonate. The system was extracted 5 times with dichloromethane. The organic phase was washed with saturated sodium chloride, dried over anhydrous sodium sulfate. The filtrate was concentrated to dryness by suction. 640mg of the product was obtained after purification by column chromatography. MS (m/e): 380.30 $[M+H]^+$.

Example 39

(E)Disodium4-amino-6-((4'-(8-azidooctanamide)-3,3'-dimethyl-[1,1'-biphenyl]-4-yl) diazenyl)-5-hydroxynaphthalene-1,3-disulfonate

212mg of the product was prepared with N-(4'-amino-3,3'-dimethyl-[1,1'-biphenyl]-4-yl)-8-azidoctanamide and 4-amino-5-hydroxy-1,3-naphthalene disulfonic acid monosodium by the preparation method of Example 3. MS (m/e): 710.28 $[M-2Na+3H]^+$. $^1H$ NMR (DMSO-$d_6$, 600MHz) $\delta$: 15.962 (s, 1H), 9.278 (s, 1H), 6H (s, 1H), 8.031 (d, 1H), 7.896 (d, 1H), 7.654 (s, 2H), 7.578 (s, 1H), 1.352 (t, 2H), 7.013 (d, 1H), 3.337 (s, 2H), 2.528-2.508 (s, 3H), 1.556 (t, 2H), 2.281 (s, 3H), 1.630 (s, 2H), 1.556 (t, 2H), 1.352 (s, 6H).

Example 40

(2S,3S,4S,5R,6S)-6-(3-((1-(8-((4'-((E)-(8-amino-1-hydroxy-5,7-disulfonicnaphthalen -2-yl)diazenyl)-3,3'-dimethyl-[1,1'-biphenyl]-4-yl)amino)-8-oxooctyl)-1H-1,2,3-triaz ol-4-yl)methoxy)-4-((((S)-4,11-diethyl-4-hydroxy-3,14-dioxy-3,4,12,14-tetrahydro-1 H-pyrano[3',4':6,7]indolizino[1,2-b]quinolin-9-yl)oxy)methyl)phenoxy)-3,4,5-trihydr oxytetrahydro-2H-pyran-2-carboxylate trisodium

103mg of the product was prepared with (E)-4-amino-6-((4'-(8-azidoctanamide)-3,3'-dimethyl-[1,1'-biphenyl]-4-yl)diazenyl)-5-hydroxynaphthalene-1,3-disulfonate disodium and (2S,3S,4S,5R,6S)-6-(4-(((S)-4,11-diethyl-4-hydroxy-3,14-dioxy-3,4,12,14-tetrahydro-1H-pyrano[3',4':6,7]indolazino[1,7]quinolin-9-yl)oxy)methyl)-3-(prop-2-ynyloxy)ph enoxy)-3,4,5-trihydroxytetrahydro-2H-pyran-2-carboxylic acid by the preparation method of Example 7. MS (m/e): 1438.40 $[M-3Na+4H]^+$.

Example 41

4-amino-6-((E)-(4'-(8-(4-((5-(((2S,3R,4S,5S,6S)-6-carboxy-3,4,5-trihydroxytetrahydr o-2H-pyran-2-yl) oxy)-2-((((S)-4,11-diethyl-4-hydroxy-3,14-dioxy-3,4,12,14-tetrahydr o-1H-pyrano[3',4':6,7]indolazino[1,2-b]quinolin-9-yl)oxy)methyl)phenoxy)methyl)-1 H-1,2,3-triazol-1-yl)octanamido)-3,3'-dimethyl-[1,1'-biphenyl]-4-yl)diazenyl)-5-hydr oxynaphthalene-1,3-disulfonate disodium

18mg of the product was prepared with (2S,3S,4S,5R,6S)-6-(3-((1-(8-((4'-((E)-(8-amino-1-hydroxy-5,7-disulfonicnaphthalen -2-yl)diazenyl)-3,3'-dimethyl-[1,1'-biphenyl]-4-yl)amino)-8-oxooctyl)-1H-1,2,3-triaz ol-4-yl)methoxy)-4-(((S)-4,11-diethyl-4-hydroxy-3,14-dioxy-3,4,12,14-tetrahydro-1H -pyrano[3',4':6,7]indolazino[1,7]quinolin-9-yl) oxy)methyl)phenoxy)-3,4,5-trihydroxy tetrahydro-2H-pyran-2-carboxylate trisodium by the preparation method of Example 15. MS (m/e): 1438.32 $[M-2Na+3H]^+$.

Example 42

4-amino-6-((E)-(4'-(8-(4-((2-(((S)-4,11-diethyl-4-hydroxy-3,14-dioxo-3,4,12,14-tetra hydro-1H-pyrano[3',4':6,7]indolazo [1,2-b]quinolin-9-yl)oxy)methyl)-5-(((2S,3R,4S,5 S,6S)-3,4,5-trihydroxy-6-(methoxycarbonyl)tetrahydro-2H-pyran-2-yl) oxy)phenoxy) methyl)-1H-1,2,3-triazol-1-yl)octanamido)-3,3'-dimethyl-[1,1'-biphenyl]-4-yl)diazen yl)-5-hydroxynaphthalene-1,3-disulfonate disodium

9mg of the product was prepared with methyl (2S,3S,4S,5R,6S)-6-(4-(((S)-4,11-diethyl-4-hydroxy-3,14-dioxy-3,4,12,14-tetrahydro-1H-pyrano[3',4':6,7]indolizino[1,2-b]quinolin-9-yl)oxy)methyl)-3-(prop-2-ynyloxy)p henoxy)-3,4,5-trihydroxytetrahydro-2H-pyran-2-carboxylate and (E)-4-amino-6-((4'-(8-azidoctanamide)-3,3'-dimethyl-[1,1'-biphenyl]-4-yl)diazenyl)-5-hydroxynaphthalene-1,3-disulfonate disodium by the prparation method of Example 7. MS (m/e): 1452.61 $[M-2Na+3H]^+$.

Example 43

tert-butyl (4'-(8-bromooctanamide)-[1,1'-biphenyl]-4-yl)carbamate

942mg of 8-bromooctanoic acid, 1g of tert-butyl (4'-amino-[1,1'-biphenyl]-4-yl)carbamate and 1.605g of HATU were weighed and dissolved in 20ml of DMF. To the mixture was added 697μl of DIEA dropwise. The resultant was stirred at room temperature for 1 hour. The reaction was dried and 20 ml of dichloromethane was added to the slurry overnight at room temperature. The solid was filtered by suction and dried to give 1.43g of the product tert-butyl (4'-(8-bromooctanamide)-[1,1'-biphenyl]-4-yl)carbamate. MS (m/e): 489.25 $[M+H]^+$.

Example 44

N-(4'-amino-[1,1'-biphenyl]-4-yl)-8-bromooctanamide

714mg of tert-butyl (4'-(8-bromooctanamide)-[1,1'-biphenyl]-4-yl)carbamate was weighed and dissolved in 8.6ml of dichloromethane. The mixture was stirred overnight at room temperature and 860μl trifluoroacetic acid was added dropwise. The reaction was dried and 10 ml of methyl tert-butyl ether was added at room temperature overnight. The solid was filtered by suction and dried to give 709mg of the product. MS (m/e): 389.15 $[M+H]^+$. $^1H$ NMR (DMSO-$d_6$, 600MHz) δ: 9.948 (s, 1H), 7.677-7.662 (d, 2H), 7.634-7.620 (d, 2H), 7.581-7.567 (d, 2H), 7.176-7.163 (d, 2H), 3.541-3.518

(t, 2H), 2.330-2.306 (t, 2H), 1.810-1.786 (m, 2H), 1.612-1.588 (t, 2H), 1.394-1.381 (m, 2H), 1.323-1.312 (t, 4H).

Example 45

N-(4'-amino-[1,1'-biphenyl]-4-yl)-8-azocinamide

140mg ofN-(4'-amino-[1,1'-biphenyl]-4-yl)-8-bromooctanamide was prepared with N-(4'-amino-[1,1'-biphenyl]-4-yl)-8-bromooctanamide by the preparation method of Example 2. MS (m/e): 352.22 $[M+H]^+$.

Example 46

(E)-4-amino-6-((4'-(8-azidoctanamide)-[1,1'-biphenyl]-4-yl)diazenyl)-5-hydroxynaph thalene-1,3-disulfonate disodium

170mg of the product (E)-4-amino-6-((4'-(8-azidooctanamide)-[1,1'-biphenyl]-4-yl)diazidoalkenyl)-5-hydr oxynaphthalene-1,3-disulfonate disodium was prepared with N-(4'-amino-[1,1'-biphenyl]-4-yl)-8-azidooctanamide and 4-amino-5-hydroxy-1,3-naphthalene disulfonate monosodium by the preparation method of Example 3. MS (m/e): 682.31 [M-2Na+3H]+. $^1H$ NMR (DMSO-$d_6$, 600MHz) δ: 15.656 (s, 1H), 9.966 (s, 1H), 8.351 (s, 1H), 8.015-7.998 (d, 1H), 7.752-7.738 (d, 2H), 7.705-7.656 (m, 6H), 6.953-6.937 (d, 1H), 3.341-3.330 (d, 2H), 2.346-2.321 (t, 2H), 1.629-1.605 (t, 2H), 1.557-1.534 (t, 2H), 1.339-1.333 (d, 6H).

Example 47

(2S,3S,4S,5R,6S)-6-(3-((1-(8-((4'-((E)-(8-amino-1-hydroxy-5,7-disulfonicnaphthalen -2-yl)diazenyl)-[1,1'-biphenyl]-4-yl)amino)-8-oxooctyl)-1H-1,2,3-triazol-4-yl)methox y)-4-((((S)-4,11-diethyl-4-hydroxy-3,14-dioxo-3,4,12,14-tetrahydro-1H-pyrano[3',4': 6,7]indolazino[1,2-b]quinolin-9-yl)oxy)methyl)phenoxy)-3,4,5-trihydroxytetrahydro-2H-pyran-2-carboxylate trisodium

95mg of the product (2S,3S,4S,5R,6S)-6-(3-((1-(8-((4'-((E)-(8-amino-1-hydroxy-5,7-disulfonicnaphthalen -2-yl) diazenyl)-[1,1'-biphenyl]-4-yl)amino)-8-oxooctyl)-1H-1,2,3-triazol-4-yl)methox y)-4-((((S)-4,11-diethyl-4-hydroxy-3,14-dioxy-3,4,12,14-tetrahydro-1H-pyrano[3',4': 6,7]indolazino[1,2-b]quinolin-9-yl)oxy)methyl)phenoxy)-3,4,5-trihydroxytetrahydro-2H-pyran-2-carboxylate trisodium was prepared with (E)-4-amino-6-((4 ' -(8-azidoctanamide)-[1,1 ' -biphenyl]-4-yl)diazenyl)-5-hydroxynaphthalene-1,3-disulfonate disodium and (2S,3S,4S,5R,6S)-6-4-((((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)-3-(prop-2-ynyloxy)p henoxy)-3,4,5-trihydroxytetrahydro-2H-pyran-2-carboxylic acid by the preparation method of Example 7. MS (m/e): 1410.01 $[M-3Na+4H]^+$.

Example 48

(2S,3S,4S,5R,6S)-6-(3-((1-(8-((4'-((E)-(8-amino-1-hydroxy-5,7-disulfonicnaphthalen -2-yl)diazenyl)-3,3'-dimethyl-[1,1'-biphenyl]-4-yl)amino)-8-oxooctyl)-1H-1,2,3-triaz ol-4-yl)methoxy)-4-((((S)-4,11-diethyl-4-hydroxy-3,14-dioxy-3,4,12,14-tetrahydro-1 H-pyrano[3',4':6,7]indolazino[1,2-b]quinolin-9-yl)oxy)methyl)-2-methylphenoxy)-3, 4,5-trihydroxytetrahydro-2H-pyran-2-carboxylate trisodium

125mg of the product was prepared with (2S,3S,4S,5R,6S)-6-(4-(((S)-4,11-diethyl-4-hydroxy-3,14-dioxy-3,4,12,14-tetrahydro-1H-pyrano[3',4':6,7]indolazo[1,2-b]quinolin-9-yl)oxy)methyl)-2-methyl-3-(prop-2-y nyloxy) phenoxy)-3,4,5-trihydroxytetrahydro-2H-pyran-2-carboxylic acid and (E)-4-amino-6-((4'-(8-azidoctylamide)-3,3'-dimethyl-[1,1'-biphenyl]-4-yl)diazenyl)-5 -hydroxynaphthalene-1,3-disulfonate disodium by the preparation method of Example 7. MS (m/e): 1452.97 $[M-3Na+4H]^+$.

Example 49

2-(2-(2-bromoethoxy)ethoxy)acetic acid

400mg of tert-butyl 2-(2-(2-bromoethoxy)ethoxy)acetate was weighed and added into a reaction flask. To the reaction flask were added 8ml of dichloromethane and 480μl of trifluoroacetic acid dropwise. The mixture was stirred at room temperature overnight. The resultant was concentrated to dryness. The solid was dissolved with dichloromethane and the mixture was concentrated to dryness. The above procedures were carried out three times. The solid was dissolved with ethyl acetate and the mixture was concentrated to dryness. The above procedures were carried out three times. 10 ml

of methyl tert-butyl ether was added and the mixture stirred at room temperature for 3 hours. The reaction solution was concentrated to dryness to obtain the compound with a yield of 100%.

Example 50

tert-butyl (4'-(2-(2-(2-bromoethoxy)ethoxy)acetamido)-3,3'-dimethyl-[1,1'-biphenyl]-4-yl)carb amate

321mg of tert-butyl 2-(2-(2-bromoethoxy)ethoxy)acetic acid, 368mg of (4'-amino-3,3'-dimethyl-[1,1'-biphenyl]-4-yl)carbamate , and 538mg of HATU were weighed and dissolved in 6.5ml of DMF. The mixture was stirred at room temperature overnight. The reaction was quenched by addition of 80ml of water and the system was extracted three times with dichloromethane. The organic phase was washed with saturated sodium chloride, dried over anhydrous sodium sulfate and the filtrate was concentrated to dryness by suction. 412mg of the product was obtained after purification by column chromatography. MS (m/e): 521.20 $[M+H]^+$. $^1$H NMR (DMSO-$d_6$, 600MHz) $\delta$: 9.073 (s, 1H), 8.553 (s, 1H), 7.636-7.622 (d, 1H), 7.527-7.524 (d, 1H), 7.476-7.439 (m, 2H), 7.425-7.406 (m, 2H), 4.146 (s, 2H), 1.482 (t, 2H), 3.748-3.734 (m, 2H), 3.705-3.688 (m, 2H), 3.618-3.599 (t, 2H), 2.286-2.266 (d, 9H), 1.482 (s, 9H).

Example 51

N-(4'-amino-3,3'-dimethyl-[1,1'-biphenyl]-4-yl)-2-(2-(2-bromoethoxy)ethoxy)acetam ide

389mg of the product of N-(4'-amino-3,3'-dimethyl-[1,1'-biphenyl]-4-yl)-2-(2-(2-bromoethoxy)ethoxy)acetam ide was prepared with tert-butyl (4'-(2-(2-(2-bromoethoxy)ethoxy)acetamido)-3,3'-dimethyl-[1,1'-biphenyl]-4-yl)carb amate by the preparation method of Example 37. MS (m/e): 421.23 [M+H]+.

Example 52

N-(4'-amino-3,3'-dimethyl-[1,1'-biphenyl]-4-yl)-2-(2-(2-azidoethoxy)ethoxy)acetami de

216mg of the product N-(4'-amino-3,3'-dimethyl-[1,1'-biphenyl]-4-yl)-2-(2-(2-bromoethoxy)ethoxy)acetam ide was prepared with N-(4'-amino-3,3'-dimethyl-[1,1'-biphenyl]-4-yl)acetamide by the preparation method of Example 2. MS (m/e): 384.24 $[M+H]^+$. $^1$H NMR (DMSO-$d_6$, 400MHz) $\delta$: 9.001 (s, 1H), 7.530-7.509 (d, 1H), 7.415-7.411 (d, 1H), 7.360-7.335 (dd, 1H), 7.262-7.205 (m, 2H), 2.123 (d, 1H), 4.947 (s, 2H), 4.124 (s, 2H), 3.748-3.726 (t, 2H), 3.695-3.645 (m, 4H), 3.429-3.440 (t, 2H), 2.250 (s, 3H), 2.123 (s, 3H).

Example 53

(E)-4-amino-6-((4'-(2-(2-(2-azidoethoxy)ethoxy)acetamide)-3,3'-dimethyl-[1,1'-biph enyl]-4-yl)diazenyl)-5-hydroxynaphthalene-1,3-disulfonate disodium

137mg of the product was prepared with N-(4'-amino-3,3'-dimethyl-[1,1'-biphenyl]-4-yl)-2-(2-(2-azidoethoxy)ethoxy)acetami de and 4-amino-5-hydroxy-1,3-naphthalene disulfonate monosodium by the preparation method of Example 3. MS (m/e): 714.24 $[M-2Na+3H]^+$.

Example 54

(2S,3S,4S,5R,6S)-6-(3-((1-(2-(2-(2-((4'-((E)-(8-amino-1-hydroxy-5,7-disulfonicnapht halen-2-yl)diazenyl)-3,3'-dimethyl-[1,1'-biphenyl]-4-yl)amino)-2-oxoethoxy)ethoxy) ethyl)-1H-1,2,3-triazol-4-yl)methoxy)-4-(((S)-4,11-diethyl-4-hydroxy-3,14-dioxo-3,4, 12,14-tetrahydro-1H-pyrano[3',4':6,7]indolazino[1,2-b]quinolin-9-yl)oxy)methyl)phe noxy)-3,4,5-trihydroxytetrahydro-2H-pyran-2-carboxylate trisodium

130mg of the product was prepared with(E)-4-amino-6-((4'-(2-(2-(2-azidoethoxy)ethoxy)acetamide)-3,3'-dimethyl-[1,1'-biphenyl]-4-yl)diazenyl)-5-hydroxynaphthalene-1,3-disulfonate disodium and (2S, 3S, 4S,5R,6S)-6-(4-((((S)-4,11-diethyl-4-hydroxy-3,14-dioxo-3,4,12,14-tetrahydro-1H-py rano[3',4':6,7]indolazino[1,2-b] quinolin-9-yl)oxy)methyl)-3-(prop-2-ynyloxy)phenox y)-3,4,5-trihydroxytetrahydro-2H-pyran-2-carboxylic acid by the preparation method of Example 7. MS (m/e): 1442.45 $[M-3Na+4H]^+$.

Example 55

4-amino-6-((E)-(4'-(2-(2-(2-(2-((5-(((2S,3R,4S,5S,6S)-6-carboxy-3,4,5-trihydroxytetr ahydro-2H-pyran-2-yl) oxy)-2-((((S)-4,11-diethyl-4-hydroxy-3,14-dioxy-3,4,12,14-tetr ahydro-1H-pyrano[3',4':6,7]indolazino[1,2-b]quinolin-9-yl)oxy)methyl)phenoxy)met hyl)-1H-1,2,3-triazol-1-yl)ethoxy)acetamido)-3,3'-dimethyl-[1,1'-biphenyl]-4-yl)diaz enyl)-5-hydroxynaphthalene-1,3-disulfonate disodium

12mg of the product was prepared with (2S,3S,4S,5R,6S)-6-(3-((2-(2-(2-((4'-(E)-(8-amino-1-hydroxy-5,7-disulfonicnaphthal en-2-yl)diazenyl)-3,3'-dimethyl-[1,1'-biphenyl]-4-yl)amino)-2-oxoethoxy)ethoxy)eth yl)-1H-1,2,3-triazol-4-yl)methoxy)-4-(((S)-4,11-diethyl-4-hydroxy-3,14-dioxy-3,4,12, 14-tetrahydro-1H-pyrano[3',4':6,7]indolazino[1,2-b]quinolin-9-yl)oxy)methyl)pheno xy)-3,4,5-trihydroxytetrahydro-2H-pyran-2-carboxylate trisodium by the preparation method of Example 15. MS (m/e): 1442.39 $[M-2Na+3H]^+$.

Example 56

4-amino-6-((E)-(4'-(2-(2-(2-((2-(((S)-4,11-diethyl-4-hydroxy-3,14-dioxy-3,4,12,14-te trahydro-1H-pyrano[3',4':6,7]indolazo[1,2-b]quinolin-9-yl)oxy)methyl)-5-(((2S,3R,4 S,5S,6S)-3,4,5-trihydroxy-6-(methoxycarbonyl)tetrahydro-2H-pyran-2-yl)oxy)phenox y)methyl)-1H-1,2,3-triazol-1-yl)ethoxy)acetamido)-3,3'-dimethyl-[1,1'-biphenyl]-4-y l)diazenyl)-5-hydroxynaphthalene-1,3-disulfonate disodium

20 mg of the product was prepared with methyl (2S,3S,4S,5R,6S)-6-(4-(((S)-4,11-diethyl-4-hydroxy-3,14-dioxo-3,4,12,14-tetrahydro-1H-pyrano[3',4':6,7]indolizino[1,2-b]quinolin-9-yl)oxy)methyl)-3-(prop-2-ynyloxy)p henoxy)-3,4,5-trihydroxytetrahydro-2H-pyran-2-carboxylate and (E)-4-amino-6-((4'-(2-(2-(2-azidoethoxy)ethoxy)acetamide)-3,3'-dimethyl-[1,1'-biph enyl]-4-yl)diazinyl)-5-hydroxynaphthalene-1,3-disulfonate disodium by the preparation method of Example 7. MS (m/e): 1456.53 $[M-2Na+3H]^+$.

Example 57

tert-butyl (4'-(2-(2-(2-bromoethoxy)ethoxy)acetamido)-3,3'-dimethoxy-[1,1'-biphenyl]-4-yl)car bamate

458mg of the product was prepared with 2-(2-(2-bromoethoxy)ethoxy)acetic acid and the compound tert-butyl (4'-amino-3,3'-dimethoxy-[1,1'-biphenyl]-4-yl)carbamate by the preparation method of Example 36. MS (m/e): 553.21 $[M+H]^+$. $^1H$ NMR (DMSO-$d_6$, 600MHz) δ: 9.002 (s, 1H), 8.235-8.221 (d, 1H), 7.936 (s, 1H), 7.780-7.767 (d, 1H), 7.323-7.319 (d, 1H), 7.272-7.228 (m, 3H), 1.479 (s, 2H), 3.980 (s, 3H), 3.920 (s, 3H), 3.813-3.793 (t, 2H), 3.748-3.731 (m, 2H), 9H (m, 2H), 3.627-3.608 (t, 2H), 1.479 (s, 9H).

Example 58

N-(4'-amino-3,3'-dimethoxy-[1,1'-biphenyl]-4-yl)-2-(2-(2-bromoethoxy)ethoxy)aceta mide

432mg of the product was prepared with tert-butyl (4'-(2-(2-(2-bromoethoxy)ethoxy)acetamido)-3,3'-dimethoxy-[1,1'-biphenyl]-4-yl)car bamate by the preparation method of Example 37. MS (m/e): 453.20 $[M+H]^+$. $^1H$ NMR (DMSO-$d_6$, 600MHz) δ: 9.000 (s, 1H), 8.227-8.213 (d, 1H), 7.306-7.292 (dd, 2H), 7.257-7.240 (dd, 1H), 7.224-7.207 (dd, 1H), 7.078-7.065 (d, 1H), 4.144 (s, 2H), 3.973-3.934 (d, 3.607-3.608), 3.811-3.792 (t, 2H), 3.746-3.729 (m, 2H), 3.704-3.687 (m, 2H), 3.607-3.608 (t, 2H).

Example 59

N-(4'-amino-3,3'-dimethoxy-[1,1'-biphenyl]-4-yl)-2-(2-(2-azidoethoxy)ethoxy)aceta mide

252mg of the product was prepared with N-(4'-amino-3,3'-dimethoxy-[1,1'-biphenyl]-4-yl)-2-(2-(2-bromoethoxy) ethoxy)aceta mide by the preparation method of Example 2. MS (m/e): 416.27 $[M+H]^+$. $^1H$ NMR (DMSO-$d_6$, 600MHz) δ: 8.951 (s, 1H), 8.169-8.155 (d, 1H), 7.230-7.226 (d, 1H), 7.171-7.154 (m, 1H), 7.102-7.099 (d, 1H), 7.057-7.041 (m, 1H), 6.709-6.696 (d, 1H), 4.853 (s, 2H), 3.432-3.416 (s, 2H), 3.956 (s, 3H), 3.865 (s, 3H), t (q, 2H), 3.692-3.662 (m, 4H), 3.432-3.416 (t, 2H).

Example 60

(E)-4-amino-6-((4'-(2-(2-(2-azidoethoxy)ethoxy)acetamide)-3,3'-dimethoxy-[1,1'-bip henyl]-4-yl)diazenyl)-5-hydroxynaphthalene-1,3-disulfonate disodium

147mg of the product (E)-4-amino-6-((4'-(2-(2-(2-azidoethoxy)ethoxy)acetamide)-3,3'-dimethoxy-[1,1'-bip henyl]-4-yl)diazenyl)-5-hydroxynaphthalene-1,3-disulfonate disodium was prepared with N-(4'-amino-3,3'-dimethoxy-[1,1'-biphenyl]-4-yl)-2-(2-(2-azidoethoxy)ethoxy)aceta mide and 4-amino-5-hydroxy-1,3-naphthalene disulfonate monosodium by the preparation method of Example 3. MS (m/e): 746.40 $[M-2Na+3H]^+$. $^1$H NMR (DMSO-$d_6$, 600MHz) $\delta$: 15.697 (s, 1H), 9.636 (s, 1H), 9.042 (s, 1H), t (s, 1H), 3.441-3.425 (4.012, 1H), 8.015-7.998 (4.012, 1H), 7.860-7.846 (4.012, 1H), 3.702-3.670 (s, 1H), q (m, 3H), 7.365-7.348 (3.757-3.742, 1H), 6.979-6.962 (4.012, 1H), 4.153 (s, 2H), 4.121 (s, 3H), 4.012 (s, 3H), 3.757-3.742 (q, 2H), 3.702-3.670 (m, 4H), 3.441-3.425 (t, 2H).

Example 61

(2S,3S,4S,5R,6S)-6-(3-((1-(2-(2-(2-((4'-((E)-(8-amino-1-hydroxy-5,7-disulfononapht halen-2-yl)diazenyl)-3,3'-dimethoxy-[1,1'-biphenyl]-4-yl)amino)-2-oxoethoxy)ethox y)ethyl)-1H-1,2,3-triazol-4-yl)methoxy)-4-(((S)-4,11-diethyl-4-hydroxy-3,14-dioxo-3, 4,12,14-tetrahydro-1H-pyrano[3',4':6,7]indolazino[1,2-b]quinolin-9-yl)oxy)methyl)p henoxy)-3,4,5-trihydroxytetrahydro-2H-pyran-2-carboxylate trisodium

128mg of the product was prepared with (E)-4-amino-6-((4'-(2-(2-(2-azidoethoxy)ethoxy)acetamide)-3,3'-dimethoxy-[1,1'-bip henyl]-4-yl)diazenyl)-5-hydroxynaphthalene-1,3-disulfonate disodium and (2S,3S,4S,5R,6S)-6-(4-((((S)-4,11-diethyl-4-hydroxy-3,14-dioxo-3,4,12,14-tetrahydro -1H-pyrano[3',4':6,7]indolazino [1,2-b]quinolin-9-yl)oxy)methyl)-3-(prop-2-ynyloxy) phenoxy)-3,4,5-trihydroxytetrahydro-2H-pyran-2-carboxylic acid by the preparation method of Example 7. MS (m/e): 1474.63 $[M-3Na+4H]^+$. $^1$H NMR (DMSO-$d_6$, 600MHz) $\delta$: 15.697 (s, 1H), 9.636 (s, 1H), 9.042 (s, 1H), 8.352 (s, 1H), 8.278-8.264 (d, 1H), 8.015-7.998 (d, 1H), 7.860-7.846 (d, 1H), 7.651 (s, 1H), 7.456-7.411 (m, 3H), 7.365-7.348 (dd, 1H), 6.979-6.962 (d, 1H), 4.153 (s, 2H), 4.121 (s, 3H), 4.012 (s, 3H), 3.757-3.742 (q, 2H), 3.702-3.670 (m, 4H), 3.441-3.425 (t, 2H).

Example 62

4-amino-6-((E)-(4'-(2-(2-(2-(2-(4-((5-(((2S,3R,4S,5S,6S)-6-carboxy-3,4,5-trihydroxyt etrahydro-2H-pyran-2-yl) oxy)-2-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy) methyl)-1H-1,2,3-triazol-1-yl)ethoxy)ethoxy)acetamido)-3,3'-dimethoxy-[1,1'-biphen yl]-4-yl) diazenyl)-5-hydroxynaphthalene-1,3-disulfonate disodium

20 mg of the product was prepared with (2S,3S,4S,5R,6S)-6-(3-((2-(2-(2-((4'-(E)-(8-amino-1-hydroxy-5,7-disulfonicnaphthal en-2-yl)diazenyl)-3,3'-dimethoxy-[1,1'-biphenyl]-4-yl)amino)-2-oxoethoxy)ethoxy)et hyl)-1H-1,2,3-triazol-4-yl)methoxy)-4-(((S)-4,11-diethyl-4-hydroxy-3,14-dioxy-3,4,1 2,14-tetrahydro-1H-pyrano[3',4':6,7]indolazino [1,2-b]quinolin-9-yl)oxy)methyl)phen oxy)-3,4,5-trihydroxytetrahydro-2H-pyran-2-carboxylate trisodium by the preparation method of Example 15. MS (m/e): 1474.55 $[M-2Na+3H]^+$.

Example 63

4-amino-6-((E)-(4'-(2-(2-(2-((2-(((S)-4,11-diethyl-4-hydroxy-3,14-dioxy-3,4,12,14-te trahydro-1H-pyrano[3',4':6,7]indolazo[1,2-b]quinolin-9-yl)oxy)methyl)-5-(((2S,3R,4 S,5S,6S)-3,4,5-trihydroxy-6-(methoxycarbonyl)tetrahydro-2H-pyran-2-yl)oxy)phenox y)methyl)-1H-1,2,3-triazol-1-yl)ethoxy)acetamido)-3,3'-dimethoxy-[1,1'-biphenyl]-4-yl)diazenyl)-5-hydroxynaphthalene-1,3-disulfonate disodium

16mg of the product was prepared with (2S,3S,4S,5R,6S)-6-(4-(((S)-4,11-diethyl-4-hydroxy-3,14-dioxo-3,4,12,14-tetrahydro-1H-pyrano[3',4':6,7]indolizino[1,2-b]quinolin-9-yl)oxy)methyl)-3-(prop-2-ynyloxy)p henoxy)-3,4,5-trihydroxytetrahydro-2H-pyran-2-carboxylicacidmethylesterand(E)-4-a mino-6-((4'-(2-(2-(2-(2-azidoethoxy)ethoxy)acetamide)-3,3'-dimethoxy-[1,1'-biphen yl]-4-yl)diazinyl)-5-hydroxynaphthalene-1,3-disulfonate disodium by the preparation method of Example 7. MS (m/e): 1488.69 $[M-2Na+3H]^+$.

Example 64

tert-butyl (4'-amino-3,3'-dimethoxy-[1,1'-biphenyl]-4-yl)carbamate

4.879g of the product tert-butyl (4'-amino-3,3'-dimethoxy-[1,1'-biphenyl]-4-yl)carbamate was prepared with 3,3'-dimethoxy-[1,1'biphenyl]-4,4'-diamineby the preparation method of Example 35. MS (m/e): 345.19 $[M+H]^+$. $^1H$ NMR (DMSO-$d_6$, 400MHz) $\delta$: 7.835 (s, 1H), 7.688-7.668 (d, 1H), 7.153-7.010 (m, 4H), 6.706-6.686 (d, 1H), 4.809 (s, 2H), 3.892-3.860 (d, 6H), 1.473 (s, 9H).

Example 65

tert-butyl (4'-(6-bromohexanamide)-3,3'-dimethoxy-[1,1'-biphenyl]-4-yl)carbamate

1.735g of the product tert-butyl (4'-(6-bromohexanamide)-3,3'-dimethoxy-[1,1'-biphenyl]-4-yl)carbamate was prepared with tert-butyl (4'-amino-3,3'-dimethoxy-[1,1'-biphenyl]-4-yl)carbamate and 6-bromohexanoic acid by the preparation method of Example 50. MS (m/e): 521.24 $[M+H]^+$. $^1H$ NMR (DMSO-$d_6$, 600MHz) $\delta$: 9.108 (s, 1H), 8.034-8.020 (d, 1H), 7.931 (s, 1H), 7.774-7.761 (d, 1H), 7.272-7.259 (dd, 2H), 7.233-7.204 (1.478, 2H), 3.939-3.917 (d, 6H), 9H (t, 2H), 1.265-1.240 (t, 2H), 1.859-1.823 (t, 2H), 1.630-1.592 (t, 2H), 1.478 (s, 9H), 1.265-1.240 (t, 2H).

Example 66

N-(4'-amino-3,3'-dimethoxy-[1,1'-biphenyl]-4-yl)-6-bromohexanamide

1.412g of the product N-(4'-amino-3,3'-dimethoxy-[1,1'-biphenyl]-4-yl)-6-bromohexanamide was prepared with tert-butyl (4'-(6-bromohexanamide)-3,3'-dimethoxy-[1,1'-biphenyl]-4-yl)carbamate by the preparation method of Example 37. MS (m/e): 421.23 $[M+H]^+$. $^1H$ NMR (DMSO-$d_6$, 400MHz) $\delta$: 9.090 (s, 1H), 8.022-8.001 (d, 1H), 7.275-7.186 (dd, 4H), 7.075-7.057 (m, 1H), 3.947 (s, 6H), 3.570-3.537 (t, 2H), 2.436-4.400 (t, 2H), 1.883-1.812 (m, 2H), 1.637-1.581 (m, 2H), 1.477-1.402 (m, 2H).

Example 67

N-(4'-amino-3,3'-dimethoxy-[1,1'-biphenyl]-4-yl)-6-azidohexanamide

959mg of the product was prepared with N-(4'-amino-3,3'-dimethoxy-[1,1'-biphenyl]-4-yl)-6-bromohexanamide by the preparation method of Example 2. MS (m/e): 384.24 $[M+H]^+$. $^1H$ NMR (DMSO-$d_6$, 600MHz) $\delta$: 1.385-1.360 (m, 1H), 7.943-7.929 (d, 1H), 7.179-7.176 (d, 1H), 7.121-7.104 (dd, 1H), 7.089-7.086 (d, 1H), 7.044-7.027 (dd, 1H), 6.705-6.692 (d, 1H), 4.832 (m, 2H), 3.913 (m, 3H), 3.862 (m, 3H), 3.355-3.332 (t, 2H), 2.415-4.390 (t, 2H), 1.624-1.566 (m, 4H), 1.385-1.360 (t, 2H).

Example 68

(E)4-amino-6-((4'-(6-azidohexanamide)-3,3'-dimethoxy-[1,1'-biphenyl]-4-yl)diazeny l)-5-hydroxynaphthalene-1,3-disulfonate disodium

171mg of the product was prepared with N-(4'-amino-3,3'-dimethoxy-[1,1'-biphenyl]-4-yl)-6-azidohexanamide and 4-amino-5-hydroxy-1,3-naphthalene disulfonate monosodium by the preparation method of Example 3. MS (m/e): 714.25 $[M-2Na+3H]^+$. $^1H$ NMR (DMSO-$d_6$, 600MHz) $\delta$: 15.707 (s, 1H), 9.638 (s, 1H), 9.154 (s, 1H), 8.355 (s, 1H), 8.087-8.073 (d, 1H), 8.013-7.997 (d, 1H), 7.855-7.842 (d, 1H), 7.648 (s, 1H), dt (d, 2H), 7.361-7.358 (d, 1H), 1.391-1.366 (3.384-3.344, 1H), m (d, 1H), 4.118 (s, 3H), t (s, 3H), 3.384-3.344 (t, 2H), 2.449-2.425 (t, 2H), 1.634-1.571 (dt, 4H), 1.391-1.366 (m, 2H).

Example 69

(2S,3S,4S,5R,6S)-6-(3-((1-(6-((4'-((E)-(8-amino-1-hydroxy-5,7-disulfonicnaphthalen -2-yl)diazenyl)-3,3'-dimethoxy-[1,1'-biphenyl]-4-yl)amino)-6-oxohexyl)-1H-1,2,3-tri azol-4-yl)methoxy)-4-(((S)-4,11-diethyl-4-hydroxy-3,14-dioxy-3,4,12,14-tetrahydro-1 H-pyrano[3',4':6,7]indolizino[1,7]quinolin-9-yl)oxy)methyl)phenoxy)-3,4,5-trihydro xytetrahydro-2H-pyran-2-carboxylate trisodium

129mg of the product was prepared with (E)-4-amino-6-((4'-(6-azidohexanamide)-3,3'-dimethoxy-[1,1'-biphenyl]-4-yl)diazen yl)-5-hydroxynaphthalene-1,3-disulfonate disodium and (2S,3S,4S,5R,6S)-6-(4-(((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)-3-(prop-2-ynyloxy)p henoxy)-3,4,5-trihydroxytetrahydro-2H-pyran-2-carboxylic acid by the preparation method of Example 7. MS (m/e): 1442.04 $[M-3Na+4H]^+$. $^1H$ NMR (DMSO-$d_6$, 600MHz) δ: 15.692 (s, 1H), 9.638 (s, 1H), 9.153 (s, 1H), 8.355 (s, 1H), 8.309 (s, 1H), 8.069-8.001 (m, 3H), 7.838-7.824 (d, 1H), 7.646 (s, 1H), 7.569-7.565 (d, 1H), 7.539-7.520 (dd, 1H), 7.423-7.403 (m, 2H), 7.379-7.365 (d, 1H), 7.321-7.318 (d, 1H), 7.270-7.256 (t, 2H), 7.035-7.032 (d, 1H), 6.973-6.957 (d, 1H), 6.661-6.644 (dd, 1H), 6.482 (s, 1H), 5.413-5.407 (d, 2H), 5.317-5.265 (q, 4H), 5.244-5.188 (m, 4H), 4.995 (d, 1H), 4.933-4.921 (d, 1H), 4.359-4.336 (t, 2H), 4.111 (s, 3H), 3.934 (s, 3H), 3.528-3.511 (d, 1H), 3.286-3.233 (m, 2H), 3.168-3.148 (m, 3H), 2.392-2.362 (t, 2H), 1.894-1.782 (m, 4H), 1.560-1.535 (t, 2H), 1.279-1.219 (m, 5H), 0.887-0.863 (t, 3H).

Example 70

4-amino-6-((E)-(4'-(6-(4-((5-(((2S,3R,4S,5S,6S)-6-carboxy-3,4,5-trihydroxytetrahydr o-2H-pyran-2-yl)oxy)-2-((((S)-4,11-diethyl-4-hydroxy-3,14-dioxy-3,4,12,14-tetrahydr o-1H-pyrano[3',4':6,7]indolazino[1,2-b]quinolin-9-yl)oxy)methyl)phenoxy)methyl)-1 H-1,2,3-triazol-1-yl)hexanamido)-3,3'-dimethoxy-[1,1'-biphenyl]-4-yl)diazenyl)-5-hy droxynaphthalene-1,3-disulfonate disodium

10 mg of the product was prepared with (2S,3S,4S,5R,6S)-6-(3-((1-(6-((4'-((E)-(8-amino-1-hydroxy-5,7-disulfonicnaphthalen -2-yl)diazenyl)-3,3'-dimethoxy-[1,1'-biphenyl]-4-yl)amino)-6-oxohexyl)-1H-1,2,3-tri azol-4-yl)methoxy)-4-((((S)-4,11-diethyl-4-hydroxy-3,14-dioxy-3,4,12,14-tetrahydro-1H-pyrano[3,4':6,7]indolazino[1,7]quinolin-9-yl) oxy)methyl)phenoxy)-3,4,5-trihydro xytetrahydro-2H-pyran-2-carboxylate trisodium by the preparation method of Example 15. MS (m/e): 1442.69 $[M-2Na+3H]^+$.

Example 71

4-amino-6-((E)-(4'-(6-(4-((2-(((S)-4,11-diethyl-4-hydroxy-3,14-dioxo-3,4,12,14-tetra hydro-1H-pyrano[3',4':6,7]indolazo [1,2-b]quinolin-9-yl)oxy)methyl)-5-(((2S,3R,4S,5 S,6S)-3,4,5-trihydroxy-6-(methoxycarbonyl)tetrahydro-2H-pyran-2-yl) oxy)phenoxy) methyl)-1H-1,2,3-triazol-1-yl)hexanamido)-3,3'-dimethoxy-[1,1'-biphenyl]-4-yl)diaz enyl)-5-hydroxynaphthalene-1,3-disulfonate disodium

12mg of the product was prepared with methyl (2S,3S,4S,5R,6S)-6-(4-(((S)-4,11-diethyl-4-hydroxy-3,14-dioxy-3,4,12,14-tetrahydro-1H-pyrano[3',4':6,7]indolizino[1,2-b]quinolin-9-yl)oxy)methyl)-3-(prop-2-ynyloxy)p henoxy)-3,4,5-trihydroxytetrahydro-2H-pyran-2-carboxylate and (E)-4-amino-6-((4'-(6-azidohexanamide)-3,3'-dimethoxy-[1,1'-biphenyl]-4-yl)diazen yl)-5-hydroxynaphthalene-1,3-disulfonate disodium by the preparation method of Example 7. MS (m/e): 1456.63 $[M-2Na+3H]^+$.

Example 72

6-bromohexanoyl chloride

10g of 6-bromohexanoic acid was weighed and dissolved in 100ml of DCM with stirring. 1 drop of DMF was added and 8.75ml of oxalyl chloride was added dropwise. The mixture was stirred at room temperature for 1 h under argon. 11.09g of the product was obtained after concentration to dryness. The yield was calculated as 100%.

Example 73

tert-butyl (4'-amino-[1,1'-biphenyl]-4-yl)carbamate

6.5g of the product tert-butyl (4'-amino-[1,1'-biphenyl]-4-yl)carbamate was prepared with p-diaminobiphenyl by the preparation method of Example 35.

Example 74

tert-butyl(4'-(6-bromohexanamide)-[1,1'-biphenyl]-4-yl)carbamate

4.7g of tert-butyl (4'-amino-[1,1'-biphenyl]-4-yl)carbamatewas weighed and added into a reaction flask. To the reaction flask were added 50 ml of dichloromethane and 4.7g of DIEA. The mixture was stirred at room temperature and cooled to 0°C with glacial ethanol. 4.587g of 6-bromohexanoyl chloride in 10 ml of dichloromethane was added to a reaction flask. The reaction became a white suspension. The glacial ethanol was removed and the reaction was stirred at room temperature for 2 hours. The reaction was quenched with 20 ml of water and stirred for 10 minutes, filtered by suction and dried. 5.524g of the product was obtained. MS (m/e): 461.23 $[M+H]^+$. $^1$H NMR (DMSO-$d_6$, 600MHz) $\delta$: 9.936 (s, 1H), 9.406 (s, 1H), 7.662-7.847 (d, 2H), 7.568-7.511 (m, 6H), 3.565-3.542 (t, 2H), 2.346-2.321 (t, 2H), 1.868-1.820 (t, 2H), 1.658-1.608 (t, 2H), 1.493 (s, 9H), 1.451-1.426 (t, 2H).

Example 75

N-(4'-amino-[1,1'-biphenyl]-4-yl)-6-bromohexanamide

5.5g of tert-butyl (4'-(6-bromohexanamide)-[1,1'-biphenyl]-4-yl)carbamate was weighed and added into a reaction flask. To the reaction flash were added 66ml of dichloromethane and 6.6ml of trifluoroacetic acid. The mixture was stirred at room temperature for 6 hours and concentrated to dryness. The solid was dissolved in dichloromethane and the mixture was concentrated to dryness. The above procedures were repeated three times. The solid was dissolved in ethyl acetate and the mixture was concentrated to dryness. The above procedures were repeated twice. 30 ml of methyl tert-butyl ether was added. The mixture was stirred at room temperature overnight and filtered by suction. The solid was washed with methyl tert-butyl ether to obtain a white filter cake. The filtrate cake was dried to obtain 5.624g of the product. $^1$H NMR (DMSO-$d_6$, 600MHz) $\delta$: 9.991 (s, 1H), 7.686-7.672 (d, 2H), 7.631-7.617 (d, 2H), 7.586-7.572 (d, 2H), 7.174-7.160 (d, 2H), 3.562-3.539 (t, 2H), 2.352-2.328 (t, 2H), 1.854-1.829 (t, 2H), 1.646-1.621 (t, 2H), 1.450-1.425 (t, 2H).

Example 76

N-(4'-amino-[1,1'-biphenyl]-4-yl)-6-azidohexanamide

1.352g of the product was prepared with N-(4'-amino-[1,1'-biphenyl]-4-yl)-6-bromohexanamide by the preparation method of Example 2. MS (m/e): 324.22 $[M+H]^+$. $^1$H NMR (DMSO-$d_6$, 600MHz) $\delta$: 9.853 (s, 1H), 7.597-7.582 (d, 2H), 7.463-7.449 (d, 2H), 7.324-7.310 (d, 2H), 6.626-6.612 (d, 2H), 5.158 (s, 2H), 3.357-3.334 (t, 2H), 2.330-2.306 (t, 2H), 1.637-1.612 (t, 2H), 1.587-1.562 (t, 2H), 1.386-1.357 (t, 2H).

Example 77

(E)-4-amino-6-((4'-(6-azidohexanamide)-[1,1'-biphenyl]-4-yl)diazenyl)-5-hydroxyna phthalene-1,3-disulfonate disodium

206mg of the product was prepared with N-(4'-amino-[1,1'-biphenyl]-4-yl)-6-azidohexanamide and 4-amino-5-hydroxy-1,3-naphthalene disulfonate monosodium by the preparation method of Example 3. MS (m/e): 654.25 $[M-2Na+3H]^+$. $^1$H NMR (DMSO-$d_6$, 600MHz) $\delta$: 15.658 (s, 1H), 9.986 (s, 1H), 8.354 (s, 1H), 8.014-7.998 (d, 1H), 7.754-7.739 (d, 2H), 7.708-7.659 (m, 6H), 6.957-6.940 (d, 1H), 3.365-3.342 (t, 2H), 2.362-2.337 (t, 2H), 1.655-1.630 (t, 2H), 1.597-1.572 (t, 2H), 1.385-1.370 (t, 2H).

Example 78

(2S,3S,4S,5R,6S)-6-(3-((1-(6-((4'-((E)-(8-amino-1-hydroxy-5,7-disulfonicnaphthalen -2-yl)diazenyl)-[1,1'-biphenyl]-4-yl)amino)-6-oxohexyl)-1H-1,2,3-triazol-4-yl)metho xy)-4-((((S)-4,14-diethyl-4-hydroxy-3,14-dioxo-3,4,12,14-tetrahydro-1H-pyrano[3',4' :6,7]indolazino[1,2-b]quinolin-9-yl)oxy)methyl)phenoxy)-3,4,5-trihydroxytetrahydro-2H-pyran-2-carboxylate trisodium

150 mg of the product was prepared with (E)-4-amino-6-((4'-(6-azidohexanamide)-[1,1'-biphenyl]-4-yl)diazenyl)-5-hydroxyna phthalene-1,3-disulfonate disodium and (2S,3S,4S,5R,6S)-6-(4-((((S)-4,11-diethyl-4-hydroxy-3,14-dioxy-3,4,12,14-tetrahydro -1H-pyrano[3',4':6,7]indolazino[1,2-b]quinolin-9-yl)oxy)methyl)-3-(prop-2-ynyloxy) phenoxy)-3,4,5-trihydroxytetrahydro-2H-pyran-2-carboxylic acid by the preparation method of Example 7. MS (m/e):

1382.48 [M-3Na+4H]+. $^1$H NMR (DMSO-$d_6$, 600MHz) δ: 15.664 (s, 1H), 10.332 (s, 1H), 8.361 (s, 2H), 8.065-8.050 (d, 1H), 8.008-7.994 (d, 1H), 7.733-7.718 (d, 2H), 7.690-7.662 (t, 4H), , 7.626-7.612 (d, 2H), 7.564-7.560 (d, 1H), 7.531-7.511 (dd, 1H), 7.366-7.352 (d, 1H), 7.272 (s, 1H), 7.048 (s, 1H), 6.962-6.944 (d, 1H), 6.644-6.632 (d, 1H), 6.485 (s, 1H), 5.415-5.408 (d, 2H), 5.381-5.343 (d, 1H), 5.289 (s, 2H), 5.264-5.256 (d, 2H), 5.234-5.228 (d, 1H), 5.209-5.175 (t, 2H), 5.033-5.025 (d, 1H), 4.966-4.956 (d, 1H), 4.381-4.314 (m, 2H), 3.617-3.600 (d, 1H), 3.290-3.203 (s, 3H), 3.158-3.145 (m, 2H), 2.355-2.279 (m, 2H), 1.897-1.774 (m, 4H), 1.599-1.566 (m, 2H), 1.279-1.231 (m, 5H), 0.890-0.865 (t, 3H).

Example 79

4-amino-6-((E)-(4'-(6-(4-((5-(((2S,3R,4S,5S,6S)-6-carboxy-3,4,5-trihydroxytetrahydr o-2H-pyran-2-yl) oxy)-2-((((S)-4,11-diethyl-4-hydroxy-3,14-dioxy-3,4,12,14-tetrahydr o-1H-pyrano[3',4':6,7]indolazino[1,2-b]quinolin-9-yl)oxy)methyl)phenoxy)methyl)-1 H-1,2,3-triazol-1-yl)hexanamido)-[1,1'-biphenyl]-4-yl)diazenyl)-5-hydroxynaphthale ne-1,3-disulfonate disodium

18mg of the product was prepared with (2S,3S,4S,5R,6S)-6-(3-((1-(6-((4'-((E)-(8-amino-1-hydroxy-5,7-disulfonatonaphthale n-2-yl)diazenyl)-[1,1'-biphenyl]-4-yl)amino)-6-oxohexyl)-1H-1,2,3-triazol-4-yl)meth oxy)-4-((((S)-4,11-diethyl-4-hydroxy-3,14-dioxy-3,4,12,14-tetrahydro-1H-pyrano[3', 4':6,7]indolazino[1,7]quinolin-9-yl)oxy)methyl)phenoxy)-3,4,5-trihydroxytetrahydro-2H-pyran-2-carboxylate trisodium by the preparation method of Example 15. MS (m/e): 1382.42 [M-2Na+3H]+.

Example 80

4-amino-6-((E)-(4'-(6-(4-((2-(((S)-4,11-diethyl-4-hydroxy-3,14-dioxy-3,4,12,14-tetra hydro-1H-pyrano[3',4':6,7]indolazino[1,2-b]quinolin-9-yl)oxy)methyl)-5-(((2S,3R,4S ,5S,6S)-3,4,5-trihydroxy-6-(methoxycarbonyl)tetrahydro-2H-pyran-2-yl)oxy)phenoxy )methyl)-1H-1,2,3-triazol-1-yl)hexanamido)-[1,1'-biphenyl]-4-yl)diazenyl)-5-hydrox ynaphthalene-1,3-disulfonate disodium

19mg of the product was prepared with (2S,3S,4S,5R,6S)-6-(4-(((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)-3-(prop-2-ynyloxy)p henoxy)-3,4,5-trihydroxytetrahydro-2H-pyran-2-carboxylicacidmethylesterand(E)-4-a mino-6-((4'-(6-azidohexanamide)-[1,1'-biphenyl]-4-yl)diazenyl)-5-hydroxynaphthale ne-1,3-disulfonate disodium by the preparation method of Example 7. MS (m/e): 1396.10 [M-2Na+3H]+.

Example 81

tert-Butyl (4'-(5-bromopentanamide)-3,3'-dimethyl-[1,1'-biphenyl]-4-yl)carbamate

The product tert-butyl (4'-(5-bromopentanamide)-3,3'-dimethyl-[1,1'-biphenyl]-4-yl)carbamate was prepared with the compound tert-butyl (4'-amino-3,3'-dimethyl-[1,1'-biphenyl]-4-yl)carbamate and 5-bromopentanoyl chloride by the preparation method of Example 74 as a white solid 763mg. MS (m/e): 475.52 [M+H]+.

Example 82

N-(4'-amino-3,3'-dimethyl-[1,1'-biphenyl]-4-yl)-5-bromopentanamide

The product N-(4'-amino-3,3'-dimethyl-[1,1'-biphenyl]-4-yl)-5-bromopentanamide was prepared with tert-butyl (4'-(5-bromopentanamide)-3,3'-dimethyl-[1,1'-biphenyl]-4-yl)carbamate by the preparation method of Example 75 as a brown solid 665mg. MS (m/e): 375.20 [M+H]+.

Example 83

N-(4'-amino-3,3'-dimethyl-[1,1'-biphenyl]-4-yl)-5-azidopentanamide

The product N-(4'-amino-3,3'-dimethyl-[1,1'-biphenyl]-4-yl)-5-bromopentanamide was prepared with the compound N-(4'-amino-3,3'-dimethyl-[1,1'-biphenyl]-4-yl)-5-bromopentanamide by the preparation method of Example 2 as a yellow solid 347mg. MS (m/e): 338.31 [M+H]+.

Example 84

(E)-4-amino-6-((4'-(5-azidopentanamide)-3,3'-dimethyl-[1,1'-biphenyl]-4-yl)diazenyl )-5-hydroxynaphthalene-1,3-disulfonate disodium

The title compound (E)-4-amino-6-((4'-(5-azidopentanamide)-3,3'-dimethyl-[1,1'-biphenyl]-4-yl)-5-hydr oxynaphthalene-1,3-disulfoniate disodium was prepared with the compound N-(4'-amino-3,3'-dimethyl-[1,1'-biphenyl]-4-yl)-5-hydroxynaphthalene-1,3-disulfona te disodium and the compound 4-amino-5-hydroxy-1,3-naphthalene disulfonate monosodium by the preparation method of Example 3 as a purple solid 233mg. MS (m/e): 668.46 $[M-2Na+3H]^+$.

Example 85

(2S,3S,4S,5R,6S)-6-(3-((1-(5-((4'-((E)-(8-amino-1-hydroxy-5,7-disulfonicnaphthalen -2-yl)diazenyl)-3,3'-dimethyl-[1,1'-biphenyl]-4-yl)amino)-5-oxopentyl)-1H-1,2,3-tria zol-4-yl)methoxy)-4-((((S)-4,11-diethyl-4-hydroxy-3,14-dioxy-3,4,12,14-tetrahydro-1 H-pyrano[3',4':6,7]indolazino[1,2-b]quinolin-9-yl)oxy)methyl)phenoxy)-3,4,5-trihyd roxytetrahydro-2H-pyran-2-carboxylate trisodium

70mg of (2S,3S,4S,5R,6S)-6-(3-((1-(5-((4'-((E)-(8-amino-1-hydroxy-5,7-disulfonicnaphthalen -2-yl)diazenyl)-3,3'-dimethyl-[1,1'-biphenyl]-4-yl)amino)-5-oxopentyl)-1H-1,2,3-tria zol-4-yl)methoxy)-4-((((S)-4,11-diethyl-4-hydroxy-3,14-dioxy-3,4,12,14-tetrahydro-1 H-pyrano[3',4':6,7]indolazino[1,2-b]quinolin-9-yl)oxy)methyl)phenoxy)-3,4,5-trihyd roxytetrahydro-2H-pyran-2-carboxylate trisodium was prepared with the compound (E)-4-amino-6-((4'-(5-azidopentanamide)-3,3'-dimethyl-[1,1'-biphenyl]-4-yl)diazenyl )-5-hydroxynaphthalene-1,3-disulfonate disodium and the compound (2S,3S,4S,5R,6S)-6-(4-(((S)-4,11-diethyl-4-hydroxy-3,14-dioxy-3,4,12,14-tetrahydro-1H-pyrano[3',4':6,7]indolazino[1,7]quinolin-9-yl)oxy)methyl)-3-(prop-2-ynyloxy)ph enoxy)-3,4,5-trihydroxytetrahydro-2H-pyran-2-carboxylic acid. MS (m/e): 1396.84 $[M-3Na+4H]^+$.

Example 86

tert-Butyl (4'-(2-bromoacetamide)-3,3'-dimethyl-[1,1'-biphenyl]-4-yl)carbamate

The product tert-butyl (4'-(2-bromoacetamide)-3,3'-dimethyl-[1,1'-biphenyl]-4-yl)carbamate was prepared with the compound tert-butyl 4'-amino-3,3'-dimethyl-[1,1'-biphenyl]-4-yl)carbamateand2-bromoacetyl chloride by the preparation method of example 74 as a white solid 390mg. MS (m/e): 433.10 $[M+H]^+$.

Example 87

N-(4'-amino-3,3'-dimethyl-[1,1'-biphenyl]-4-yl)-2-bromoacetamide

The product N-(4'-amino-3,3'-dimethyl-[1,1'-biphenyl]-4-yl)-2-bromoacetamide was prepared with tert-butyl (4'-(2-bromoacetamide)-3,3'-dimethyl-[1,1'-biphenyl]-4-yl)carbamate by the preparation method of Example 75 as a yellow solid 263mg. MS (m/e): 333.18 $[M+H]^+$.

Example 88

N-(4'-amino-3,3'-dimethyl-[1,1'-biphenyl]-4-yl)-2-azidoacetamide

The product N-(4'-amino-3,3'-dimethyl-[1,1'-biphenyl]-4-yl)-2-bromoacetamide was prepared with the compound N-(4'-amino-3,3'-dimethyl-[1,1'-biphenyl]-4-yl)-2-azidoacetamide by the preparation method of Example 2 as a yellow solid 180mg. MS (m/e): 296.24 $[M+H]^+$.

Example 89

(E)-4-amino-6-((4'-(2-azidoacetamide)-3,3'-dimethyl-[1,1'-biphenyl]-4-yl)diazenyl)-5-hydroxynaphthalene-1,3-disulfonate disodium

The title compound (E)-4-amino-6-((4'-(2-azidoacetamide)-3,3'-dimethyl-[1,1'-biphenyl]-4-yl)-5-hydrox ynaphthalene-1,3-disulfonate disodium was prepared with the compound N-(4'-amino-3,3'-dimethyl-[1,1'-biphenyl]-4-yl)-2-azidoacetamide and the compound 4-amino-5-hydroxy-1,3-naphthalene disulfonate disodium by the preparation

method of Example 3 as a purple solid 271mg. MS (m/e): 626.57 [M-2Na+3H]$^+$.

Example 90

(2S,3S,4S,5R,6S)-6-(3-((1-(2-((4'-((E)-(8-amino-1-hydroxy-5,7-disulfonicnaphthalen -2-yl)diazenyl)-3,3'-dimethyl-[1,1'-biphenyl]-4-yl)amino)-2-oxoethyl)-1H-1,2,3-triaz ol-4-yl)methoxy)-4-((((S)-4,11-diethyl-4-hydroxy-3,14-dioxy-3,4,12,14-tetrahydro-1 H-pyrano[3',4':6,7]indolazino[1,2-b]quinolin-9-yl)oxy)methyl)phenoxy)-3,4,5-trihyd roxytetrahydro-2H-pyran-2-carboxylate trisodium

61mg of (2S,3S,4S,5R,6S)-6-(3-((1-((2-((4'-((E)-(8-amino-1-hydroxy-5,7-disulfonicnaphthale n-2-yl)diazenyl)-3,3'-dimethyl-[1,1'-biphenyl]-4-yl)amino)-2-oxoethyl)-1H-1,2,3-tria zol-4-yl)methoxy)-4-((((S)-4,11-diethyl-4-hydroxy-3,14-dioxo-3,4,12,14-tetrahydro-1 H-pyrano[3',4':6,7]indolazino[1,2-b]quinolin-9-yl)oxy)methyl)phenoxy)-3,4,5-trihyd roxytetrahydro-2H-pyran-2-carboxylate trisodium was prepared with the compound (E)-4-amino-6-((4'-(2-azidoacetamide)-3,3'-dimethyl-[1,1'-biphenyl]-4-yl)diazenyl)-5-hydroxynaphthalene-1,3-disulfonate disodium and the compound (2S,3S,4S,5R,6S)-6-(4-(((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)-3-(prop-2-ynyloxy)p henoxy)-3,4,5-trihydroxytetrahydro-2H-pyran-2-carboxylic acid by the preparation method of Example 7. MS (m/e): 1354.73 [M-3Na+4H]$^+$.

Example 91

2-(3-chloropropoxy)acetic acid

The compound ethyl 2-(3-chloropropoxy)acetate (1.0 g) was dissolved in tetrahydrofuran (10.0ml). The mixture was stirred at room temperature. The sodium hydroxide (266mg) was dissolved in water (10.0ml). The solution was added dropwise. The mixture was stirred at room temperature for 1.5 hours. The tetrahydrofuran was concentrated to dryness. 1mol/L of hydrochloric acid (6.64ml) was added dropwise to adjust the pH. The system was extracted with ethyl acetate three times. The organic phase was washed with saturated aqueous sodium chloride, dried over anhydrous sodium sulfate and filtered by suction. The filtrate was concentrated to dryness to give the product 2-(3-chloropropoxy)acetic acid was obtained as a yellow oil 723mg. MS (m/e): 153.75[M+H]+.

Example 92

tert-butyl (4'-(2-(3-chloropropoxy)acetamide)-3,3'-dimethyl-[1,1'-biphenyl]-4-yl)carbamate

The product tert-butyl (4'-(2-(3-chloropropoxy)acetamide)-3,3'-dimethyl-[1,1'-biphenyl]-4-yl)carbamate was prepared with the compounds tert-butyl 4'-amino-3,3'-dimethyl-[1,1'-biphenyl]-4-yl)carbamate and 2-(3-chloropropoxy)acetic acid by the preparation method of Example 50 as a white solid 1.61g. MS (m/e): 447.40 [M+H]$^+$.

Example 93

N-(4'-amino-3,3'-dimethyl-[1,1'-biphenyl]-4-yl)-2-(3-chloropropoxy)acetamide

The product N-(4'-amino-3,3'-dimethyl-[1,1'-biphenyl]-4-yl)-2-(3-chloropropoxy)acetamide was prepared with tert-butyl (4'-(2-(3-chloropropoxy)acetamide)-3,3'-dimethyl-[1,1'-biphenyl]-4-yl)carbamate by the preparation method of Example 37 as a yellow solid 1.315g. MS (m/e): 347.32 [M+H]$^+$.

Example 94

N-(4'-amino-3,3'-dimethyl-[1,1'-biphenyl]-4-yl)-2-(3-azidopropoxy)acetamide

The starting material N-(4'-amino-3,3'-dimethyl-[1,1'-biphenyl]-4-yl)-2-(3-chloropropoxy)acetamide (1.315g) was weighed and added into dimethyl sulfoxide (10 ml). The mixture was stirred at room temperature and dissolved completely. Sodium azide (287mg) and sodium iodide (441mg) were added. The reaction was carried out at 80 °C for 17 hours. The reaction was quenched with water and extracted with dichloromethane (200 ml). The aqueous phase was extracted with dichloromethane (200 ml) and separated. The dichloromethane phases were combined and washed four times with saturated aqueous sodium chloride (100 ml). The organic phase was dried over anhydrous sodium sulfate and filtered by suction. The filtrate was concentrated to give the crude product. The crude product was subject to column chromatography (eluent: dichloromethane: ethyl acetate = 10:1) to give a yellow oil 914mg. MS (m/e): 354.32 [M+H]$^+$.

Example 95

(E)-4-amino-6-((4'-(2-(3-azidopropoxy)acetamide)-3,3'-dimethyl-[1,1'-biphenyl]-4-y l)diazenyl)-5-hydroxynaphthalene-1,3-disulfonate disodium

345mg of the product was prepared with the compound N-(4'-amino-3,3'-dimethyl-[1,1'-biphenyl]-4-yl)-2-(3-azidopropoxy)acetamide and the compound 4-amino-5-hydroxy-1,3-naphthalene disulfonate disodium by the preparation method of Example 3. MS (m/e): 684.39 $[M-2Na+3H]^+$.

Example 96

(2S,3S,4S,5R,6S)-6-(3-((1-(3-(2-((4'-(E)-(8-amino-1-hydroxy-5,7-disulfonicnaphthal en-2-yl)diazenyl)-3,3'-dimethyl-[1,1'-biphenyl]-4-yl)amino)-2-oxoethoxy)propyl)-1H -1,2,3-triazol-4-yl)methoxy)-4-((((S)-4,11-diethyl-4-hydroxy-3,14-dioxy-3,4,12,14-tet rahydro-1H-pyrano[3',4':6,7]indolazino[1,7]quinolin-9-yl)oxy)methyl)phenoxy)-3,4, 5-trihydroxytetrahydro-2H-pyran-2-carboxylate trisodium

234mg of the product was prepared with the compound (E)-4-amino-6-((4'-(2-(3-azidopropoxy)acetamide)-3,3'-dimethyl-[1,1'-biphenyl]-4-y l)diazenyl)-5-hydroxynaphthalene-1,3-disulfonate disodium and the compound (2S,3S,4S,5R,6S)-6-(4-(((S)-4,11-diethyl-4-hydroxy-3,14-dioxy-3,4,12,14-tetrahydro-1H-pyrano[3',4':6,7]indolazino [1,2-b]quinolin-9-yl)oxy)methyl)-3-(prop-2-ynyloxy)p henoxy)-3,4,5-trihydroxytetrahydro-2H-pyran-2-carboxylic acid by the preparation method of Example 7. MS (m/e): 1412.27 $[M-3Na+4H]^+$.

Example 97

tert-butyl (4'-(3-(2-bromoethoxy)propionamide)-3,3'-dimethyl-[1,1'-biphenyl]-4-yl)carbamate

1.6g of the product was prepared with the compound tert-butyl 4'-amino-3,3'-dimethyl-[1,1'-biphenyl]-4-yl) carbamate and 3-(2-bromoethoxy)propionic acid by the preparation method of Example 50. MS (m/e): 491.35 $[M+H]^+$.

Example 98

N-(4'-amino-3,3'-dimethyl-[1,1'-biphenyl]-4-yl)-3-(2-bromoethoxy)propanamide

1.2g of the product was prepared with the compound tert-butyl (4'-(3-(2-bromoethoxy)propanamide)-3,3'-dimethyl-[1,1'-biphenyl]-4-yl)carbamate by the preparation method of Example 37. MS (m/e): 391.30 $[M+H]^+$.

Example 99

N-(4'-amino-3,3'-dimethyl-[1,1'-biphenyl]-4-yl)-3-(2-azidoethoxy)propanamide

770mg of the product was prepared with the compound N-(4'-amino-3,3'-dimethyl-[1,1'-biphenyl]-4-yl)-3-(2-bromoethoxy)propanamide by the preparation method of Example 2. MS (m/e): 354.32 $[M+H]^+$.

Example 100

(E)-4-amino-6-((4'-(3-(2-azidoethoxy)propanamide)-3,3'-dimethyl-[1,1'-biphenyl]-4-yl)diazenyl)-5-hydroxynaphthalene-1,3-disulfonate disodium

379mg of the product was prepared with the compound N-(4'-amino-3,3'-dimethyl-[1,1'-biphenyl]-4-yl)-3-(2-azidoethoxy)propanamide and the compound 4-amino-5-hydroxy-1,3-naphthalene disulfonate monosodium by the preparation method of Example 3. MS (m/e): 684.63 $[M-2Na+3H]^+$.

Example 101

(2S,3S,4S,5R,6S)-6-(3-((1-(2-(2-((4'-(E)-(8-amino-1-hydroxy-5,7-disulfonicnaphthal en-2-yl)diazenyl)-3,3'-dimethyl-[1,1'-biphenyl]-4-yl)amino)-3-oxopropoxy)ethyl)-1H -1,2,3-triazol-4-yl)methoxy)-4-((((S)-4,11-diethyl-4-hydroxy-3,14-dioxy-3,4,12,14-tet rahydro-1H-pyrano[3',4':6,7]indolazino[1,2-b]quinolin-9-yl)oxy)methyl)phenoxy)-3, 4,5-trihydroxytetrahydro-2H-pyran-2-carboxylate trisodium

129mg of the product was prepared with the compound (E)-4-amino-6-((4'-(3-(2-azidoethoxy)propanamide)-3,3'-dimethyl-[1,1'-biphenyl]-4-yl)diazenyl)-5-hydroxynaphthalene-1,3-disulfonate disodium and the compound (2S,3S,4S,5R,6S)-6-(4-(((S)-4,11-diethyl-4-hydroxy-3,14-dioxo-3,4,12,14-tetrahydro-1H-pyrano[3',4':6,7]indolizino [1,2-b]quinolin-9-yl)oxy)methyl)-3-(prop-2-ynyloxy)p henoxy)-3,4,5-trihydroxytetrahydro-2H-pyran-2-carboxylic acid by the preparation method of Example 7. MS (m/e): 1412.55 $[M-3Na+4H]^+$.

Biological Examples

Biological Example 1

In Vitro Inhibition Study Tests on Human Tumor Cells

1.1 Test Methods

1.1.1 Additives in cell medium formulations (complete medium) were generally adjusted according to the formulation provided by the Cell Bank of the Chinese Academy of Sciences and the ATCC (American Type Culture Collection) in combination with the formulation of the basal medium.

1.1.2 Cell culture method: Cells were cultured in complete medium, 37 °C, 5% CO2, saturated humidity to log growth phase (adherent cells generally attended to complete fusion), and cells were collected for subsequent experiments (adherent cells were first digested with trypsin/ethylene diamine tetraacetic acid (EDTA) to remove adherent).

1.1.3 96-well cell culture plates (hereinafter referred to as 96-well plates) were inoculated with a certain number of cells (see Table 1 for details). The cells were allowed to adhere overnight. Different concentrations of compounds were added, and co-cultured for a period of time (typically 3 days). Finally, the total amount of cell protein in the assay wells was determined by the SRB method, or the cell viability was determined by the MTT method:

1.1.3.1 The total amount of cell protein was measured by SRB method: The culture medium was aspirated from the wells and the cells were fixed for 1 hour or more by adding 10% trichloroacetic acid. Trichloroacetic acid was removed. The resultant was washed with $H_2O$ and stained with 0.4% SRB for 15 to 30 minutes. Excess SRB was removed. The resultant was washed with 1% acetic acid. The protein-bound SRB was dissolved by adding 10mM of 100μl Tris (trishydroxymethylaminomethane). Tests were carried out at the wavelength of 570nm.

1.1.3.2 Cell viability was determined by MTT assay:

Adherent cells: Culture medium was aspirated and 100μl/wells of basal culture (generally refers to medium without fetal bovine serum (FBS) and other additives) containing 0.5mg/ml MTT were added. The cells were cultured for 3 hours. MTT-containing basal medium was aspirated and 100μl/wells of DMSO were added to dissolve formazan. The tests were carried out at the wavelength of 490nm.

Unstably adherent cells: Unstably adherent cells were directly added to the MTT solution to achieve the concentration of 0.5mg/ml and cultured for 3 hours. The ternary solution in an equal volume to the system was added to the system to dissolve the formazan. The tests were carried out at the wavelength of 570nm.

1.1.3.3 Basis for Selection of SRB and MTT methods: The SRB method and the MTT method revealed the number of cells in the assay wells from different points and overall better revealed the activity of the reacting compounds to cell inhibition. The SRB method had a higher sensitivity and required fewer cells, while the MTT method was simpler. However, the MTT reduction capacity of different cells was quite different. The main principle for the selection of the SRB method and the MTT method was that the MTT method was preferentially selected if the cells had sufficient MTT reduction capacity, such as an OD value (optical density) of the positive solvent control group of 0.4 or more after the end of co-culture.

1.1.4 Ddilution and addition of β-glucuronidase: 1 to 2mg of β-glucuronidase was weighed and dissolved in PBS to give a 2mg/ml of stock solution. 12.5μg/ml of enzyme working solution was diluted with complete medium. 80μl of enzyme working solution was added to the enzyme-containing assay wells. The amount of β-glucuronidase was 1μg/wells. No enzyme-containing assay wells were added to the 80μl of complete medium.

1.1.5 Dilution and addition of compound: 1 to 3mg of the test compound was weighed and dissolved in DMSO to give a 20mM of stock solution. The initial concentrations were adjusted according to the test requirements, then diluted with a 3-fold gradient of DMSO and then diluted 100-fold with RPMI 1640 base medium to give a total of 8 concentrations of

10× working solution. The assay wells were charged with 20μl of 10× compound working solution. The final volume was 200μl per well. The DMSO concentration was 0.1%.

1.1.6 Compound inhibition activity calculation: The test included a compound test group, a compound-free positive solvent control group (PC) and a compound-free negative solvent control group (NC). The inhibition rate was [1-(test value - NC average)/(PC average- NC average)]×100%. The average inhibition rate and the standard deviation (SD) were obtained from the repeated test wells. The average inhibition rate was plotted on the basis of the compound concentration as an abscissa and distributed in the form of logarithms. The curve was fitted to the Logistic 4 parameter equation. The compound concentration corresponding to the 50% inhibition rate on the curve was the $IC_{50}$ value. The test was generally repeated to obtain 3 valid results.

Table 1. Medium Formulation of Human Tumor Cell Line and Cell Inoculation Concentration

| Cell Line | Culture Medium Formulation | Cell Inoculation Concentration (per well) |
|---|---|---|
| HCT116 | 90% McCoy '5A, 10% FBS | $5\times10^3$ |
| SW620 | 90% L-15, 10% FBS | $5\times10^3$ |
| LoVo | 90% F-12K, 10% FBS | $7\times10^3$ |
| NCI-N87 | 90% RPMI-1640, 10%FBS, 1mM sodium pyruvate, 2.5g/L glucose | $1.5\times10^4$ |
| MDA-MB-468 | 90% L-15, 10% FBS | $8\times10^3$ |
| BxPC-3 | 90% RPMI-1640, 10% FBS, 1mM sodium pyruvate, 2.5g/L glucose | $8\times10^3$ |
| NCI-H460 | 90% RPMI-1640, 10% FBS | $3\times10^3$ |
| BEL-7404 | 90% RPMI-1640, 10% FBS | $3\times10^3$ |
| Fadu | 90% EMEM, 10% FBS, 1mM sodium pyruvate | $7\times10^3$ |
| 22RV1 | 90% RPMI-1640, 10% FBS, 1mM sodium pyruvate, 2.5g/L glucose | $7\times10^3$ |
| A375 | A375 medium 90% DMEM + 10% serum | $8\times10^3$ |

1.2 Test results

$IC_{50}$ values for inhibition of various tumor cell lines (see Tables 2 to 4).

Table 2. Inhibitory activity of compounds against various tumor cells ($IC_{50}$: NM)

| Cell Line Test Method | HCT116 MTT | | SW620 SRB | | LoVo SRB | |
|---|---|---|---|---|---|---|
| | No βGU | Adding βGU | No βGU | Adding βGU | No βGU | Adding βGU |
| Example 40 | 218 | 6.1 | 244 | 6.9 | 302 | 23 |
| Example 78 | 42 | 2.4 | 329 | 7.3 | 301 | 11 |
| Example 47 | 183 | 12 | 329 | 6.7 | 310 | 22 |
| Example 24 | 339 | 14 | 915 | 6.5 | 764 | 18 |
| Example 21 | 244 | 35 | 764 | 23 | 720 | 45 |
| Example 69 | 181 | 8.2 | 361 | 7.7 | 592 | 11 |
| Example 61 | 220 | 5.4 | 283 | 2.6 | 244 | 5.9 |
| Example 14 | 175 | 3.3 | 151 | 6.3 | 204 | 10 |
| Example 54 | 259 | 13 | 549 | 15 | 275 | 9.3 |
| Example 18 | 186 | 6.5 | 204 | 6.8 | 812 | 19 |
| Example 29 | 1353 | 17 | 350 | 9.6 | 1200 | 55 |
| Example 48 | 119 | 5.9 | 230 | 10 | 372 | 28 |
| Example 31 | 339 | 9.3 | 220 | 10 | 699 | 32 |
| Example 7 | 147 | 3.9 | 306 | 6.9 | 253 | 19 |

Note:
BGU: β-glucuronidase
No βGU: Direct effects of compound on cells
Adding βGU: Effects of compounds on cells after adding β-glucuronidase

Table 3. Inhibitory activity of compounds against human tumor cells ($IC_{50}$: NM)

| Cell Line Test Method | NCI-N87 MTT | | MDA-MB-468 SRB | |
|---|---|---|---|---|
| | No βGU | Adding βGU | No βGU | Adding βGU |
| Example 40 | 1572 | 30 | 217 | 6.9 |
| Example 78 | 170 | 26 | 28 | 3.3 |
| Example 47 | 1481 | 62 | 172 | 14 |
| Example 24 | 2μM about 34.4% | 61 | 171 | 11 |
| Example 21 | - | - | 177 | 42 |
| Example 69 | 350 | 32 | 88 | 10 |
| Example 61 | 395 | 33 | 212 | 6.5 |
| Example 14 | 1481 | 26 | 106 | 4.4 |
| Example 54 | 2μM about 46.1% | 38 | 217 | 6.8 |
| Example 18 | 2μM about 49.3% | 29 | 110 | 5.5 |
| Example 29 | 2μM about 31.0% | 64 | 788 | 14 |
| Example 48 | 1200 | 52 | 94 | 7.3 |
| Example 31 | 1774 | 62 | 329 | 11 |
| Example 7 | 1138 | 24 | 70 | 6.8 |
| Example 101 | 1237 | 51 | 372 | 11 |
| Example 96 | 964 | 30 | 213 | 7.5 |
| Example 90 | 839 | 23 | 79 | 4.3 |
| Example 85 | 178 | 31 | 71 | 9.7 |

Table 4. Inhibitory Activity of Compounds on Human Tumor Cells ($IC_{50}$: NM)

| Cell | BxPC-3 | | NCI-H460 | |
|---|---|---|---|---|
| Test Method | MTT | | MTT | |
| | No βGU | Adding βGU | No βGU | Adding βGU |

| | | | | |
|---|---|---|---|---|
| Example 40 | 658 | 41 | 943 | 15 |
| Example 78 | 610 | 13 | 530 | 9.6 |
| Example 47 | 698 | 27 | 350 | 17 |
| Example 24 | 1274 | 19 | 1720 | 39 |
| Example 21 | 688 | 62 | 770 | 80 |
| Example 69 | 720 | 16 | 566 | 13 |
| Example 61 | 915 | 7.1 | 518 | 8.6 |
| Example 14 | 406 | 16 | 533 | 8.7 |
| Example 54 | 638 | 8.8 | 360 | 11 |
| Example 18 | 1620 | 17 | 533 | 7.6 |
| Example 29 | 916 | 38 | 2μM about 43.9% | 30 |
| Example 48 | 432 | 28 | 419 | 6.8 |
| Example 31 | 518 | 21 | 540 | 19 |
| Example 7 | 1106 | 27 | 219 | 14 |

| Cell | BEL-7404 | | Fadu | |
|---|---|---|---|---|
| Test Method | SRB | | MTT | |
| | No βGU | Adding βGU | No βGU | Adding βGU |
| Example 61 | 565 | 40 | 498 | 15 |
| Example 78 | 458 | 35 | 346 | 6.9 |
| Example 85 | 721 | 50 | 706 | 15 |
| Example 21 | 439 | 28 | 298 | 9.8 |
| Example 7 | 514 | 33 | 242 | 8.5 |

| Cell | 22RV1 | | A375 | |
|---|---|---|---|---|
| Test Method | SRB | | SRB | |
| | No βGU | Adding βGU | No βGU | Adding βGU |
| Example 61 | 225 | 8.4 | 782 | 12.5 |
| Example 78 | 267 | 7.6 | 984 | 22 |
| Example 85 | 248 | 9 | 864 | 19 |
| Example 21 | 399 | 14.5 | 556 | 8.8 |

| | | | | |
|---|---|---|---|---|
| Example 7 | 147 | 5.2 | 600 | 10 |

Biological Example 2

Efficacy Evaluation Experiment

Effects on Growth of Human Colon Cancer LoVo in Allogeneic Transplantation Model

2.1 Test Method

An allogeneic transplantation model of human colon cancer LoVo was established in nude mice subcutaneously using a tumor block inoculation method. When the tumor volume of the allogeneic transplantation was about 100 to 300mm$^3$ and kept stable, the experimental animals were randomly divided into groups according to the tumor volume (day 0) by using a remainder method. The tumor volume was measured 2 to 3 times a week. At the end of the dosage period, the experimental animals were sacrificed. The subcutaneous tumors were peeled and weighed. The anti-tumor activity of the drug against the model was evaluated based on tumor volume and tumor weight-related data.

2.1.1 Grouping and dosage regimen

Table 5. Experimental animal grouping and dosage regimen regarding allogeneic transplantation model on growth of human colon cancer LoVo

| Group | | Administration dose (mg/kg) | Solvent | Administration volume (ml/kg) | Administration concentration (mg/ml) | Route of administration | Administration period | Number |
|---|---|---|---|---|---|---|---|---|
| Model control group | G1 | - | - | 10 | - | - | - | 6 |
| Example 78 | G2 | 30 | 0.9% sodium chloride injection | | 3 | i.v. | qw×2 | |
| Example 21 | G3 | 30 | | | 3 | | | |
| Example 7 | G4 | 30 | | | 3 | | | |
| Note: "-" means not filled in or no valid data. | | | | | | | | |

2.2 Evaluation index and statistical method

The calculation formula of the tumor volume was: Volume =0.5 × long diameter × short diameter × short diameter.

$$\text{Relative tumor volume (RTV): RTV} = Vt/V0.$$

V0 was the resulting tumor volume measured at the time of dosing in groups (i.e., d0) and Vt is the tumor volume at each measurement.
Relative tumor proliferation rate T/C: T/C = TRTV/CRTV×100%.
T was the subject and C was the model control.
TRTV was the average RTV of the subject and CRTV was the average RTV of the model control group.

Tumor inhibition rate (%) = (average tumor weight of model control group - average tumor weight of treatment group)/ average tumor weight of model control group ×100%.

$$\text{Percent change in body weight} = Wn/W0 \times 100\%.$$

Wn: average body weight of each group of experimental animals on day n, and W0: average body weight of each group of experimental animals on day 0.

2.3 Experimental results

Table 6. Effects of compounds on growth of human colon cancer LoVo in allogeneic transplantation model

| | Group | Host (nude mouse) Response | | Tumor Response | |
|---|---|---|---|---|---|
| | | Number of animals | Final body weight change (%) | Tumor inhibition rate (%) | T/C (%) |
| | | Start/end | | | |
| Model control group | G1 | 6/6 | 2.5 | - | 100 |
| Example 78 | G2 | 6/6 | 3.8 | 88.2 | 15.8 |
| Example 21 | G3 | 6/6 | -1.6 | 93.1 | 9.8 |
| Example 7 | G4 | 6/6 | 0.0 | 91.1 | 12.3 |
| Note: "-" means no valid data or not filled in. | | | | | |

Biological Example 3

Efficacy Evaluation Experiment

Effects on Growth of Human Colon Cancer SW620 in Allogeneic Transplantation Model

3.1 Test Method

An allogeneic transplantation model of human colon cancer SW620 was established in nude mice subcutaneously using a tumor block inoculation method. When the tumor volume of the allogeneic transplantation was about 100 to 300mm$^3$ and remained stable, the experimental animals were randomly divided into groups according to the size of the tumor volume (day 0) by using a remainder method. The tumor volume was measured 2 to 3 times a week. At the end of the dosage period, the experimental animals were sacrificed. The subcutaneous tumors were peeled and weighed. The antitumor activity of the drug against the model was evaluated based on tumor volume and tumor weight-related data.

3.1.1 Grouping and dosage regimen

Table 7. Experimental animal grouping and dosage regimen regarding allogeneic transplantation model on growth of human colon cancer SW620

| Group | | Administration dose (mg/kg) | Solvent | Administration volume (ml/kg) | Administration concentration (mg/ml) | Route of administration | Administration period | Number |
|---|---|---|---|---|---|---|---|---|
| Model control group 1 | G1 | - | - | - | - | - | - | 6 |
| Example 40 | G2 | 30 | 0.9% sodium chloride injection | 10 | 3 | i.v. | qw×2 | |
| Example 78 | G3 | 30 | | | | | | |
| Example 21 | G4 | 30 | | | | | | |
| Example 7 | G5 | 30 | | | | | | |
| Example 47 | G6 | 30 | | | | | | |
| Example 69 | G7 | 30 | | | | | | |
| Note: "-" means not filled in or no valid data. | | | | | | | | |

3.2 Evaluation index and statistical method

The calculation formula of the tumor volume was: Volume = 0.5 × long diameter × short diameter × short diameter.

$$\text{Relative tumor volume (RTV): RTV} = Vt/V0.$$

V0 was the resulting tumor volume measured at the time of dosing in groups (i.e., d0) and Vt was the tumor volume at each measurement.

Relative tumor proliferation rate T/C: T/C = TRTV/CRTV×100%.

T was the subject and C is the model control.

TRTV was the average value of RTV of the subject, and CRTV was the average RTV of the model control group.

Tumor inhibition rate (%) = (average tumor weight of the model control group - average tumor weight of treatment group)/average tumor weight of the model control group × 100%.

$$\text{Percent change in body weight} = Wn/W0 \times 100\%.$$

Wn: average body weight of each group of experimental animals on day n, and W0: average body weight of each group of experimental animals on day 0. 3.3 Experimental results

Table 8. Effects of compounds on growth of human colon cancer SW620 in allogeneic transplantation model

| Group | Host (nude mouse) Response | | Tumor Response | |
|---|---|---|---|---|
| | Number of animals Start/end | Final body weight change (%) | Tumor inhibition rate (%) | T/C (%) |
| G1 | 6/6 | 3.9 | - | 100 |
| G2 | 6/6 | 8.5 | 97.2 | 2.9 |
| G3 | 6/6 | 7.1 | 99.4 | 0.6 |
| G4 | 6/6 | 6.9 | 99.8 | 0.5 |
| G5 | 6/6 | -4.6 | 99.7 | 0.6 |
| G6 | 6/6 | 9.7 | 96.2 | 3.5 |
| G7 | 6/6 | 5.8 | 99.1 | 1.1 |

Biological Example 4

Efficacy Evaluation Experiment

Effects on Growth of Human Colon Cancer HCT116 in Allogeneic Transplantation Model

4.1 Test Method

An allogeneic transplantation model of human colon cancer HCT116 was established in nude mice subcutaneously using a tumor block inoculation method. When the tumor volume of the allogeneic transplantation was about 100 to 300mm$^3$ and remained stable, the experimental animals were randomly divided into groups according to the size of the tumor volume (day 0) by using a remainder method. The tumor volume was measured 2 to 3 times a week. At the end of the dosage period, the experimental animals were sacrificed. The subcutaneous tumors were peeled and weighed. The anti-tumor activity of the drug against the model was evaluated based on tumor volume and tumor weight-related data.

4.1.1 Grouping and dosing schedule

Table 9. Experimental animal grouping and dosage regimen regarding allogeneic transplantation model on growth of human colon cancer HCT116

| Group | | Administration dose (mg/kg) | Solvent | Administration volume (ml/kg) | Administration concentration (mg/ml) | Route of administration | Administration period | Number |
|---|---|---|---|---|---|---|---|---|
| Model control group | G1 | - | - | 10 | - | - | - | 5 |
| Example 61 | G2 | 10 | 0.9% sodium chloride injection | | 1 | i.v. | qw×2 | |
| Example 54 | G3 | 10 | | | 1 | | | |
| Example 90 | G4 | 10 | | | 1 | | | |
| Example 7 | G5 | 10 | | | 1 | | | |
| irinotecan | G6 | 100 | sterilized injection water | | 10 | i.p. | | |
| Note: "-" means not filled in or no valid data. | | | | | | | | |

4.2 Evaluation index and statistical method

The calculation formula of the tumor volume was: Volume = 0.5 × long diameter × short diameter × short diameter.

$$\text{Relative tumor volume (RTV): RTV} = Vt/V0.$$

V0 was the resulting tumor volume measured at the time of dosing in groups (i.e., d0) and Vt was the tumor volume at each measurement.
Relative tumor proliferation rate T/C: T/C = TRTV/CRTV × 100%.
T was the subject and C is the model control.
TRTV was the average RTV of the subject, and CRTV was the average RTV of the model control group.

Tumor inhibition rate (%) = (average tumor weight of model control group - average tumor weight of treatment group)/ average tumor weight of model control group ×100%.

$$\text{Percent change in body weight} = Wn/W0 \times 100\%.$$

Wn: average body weight of each group of experimental animals on day n, and W0: average body weight of each group of experimental animals on day 0. 4.3 Experimental results

Table 10. Effects of compounds on growth of human colon cancer HCT116 in allogeneic transplantation model

| Host (nude mouse) Response | | | Tumor Response | |
|---|---|---|---|---|
| Group | Number of animals Start/end | Final body weight change (%) | Tumor inhibition rate (%) | T/C (%) |
| G1 | 5/5 | -0.2 | - | 100 |
| G2 | 5/5 | -4.9 | 76.8 | 27.2 |
| G3 | 5/5 | -12.5 | 74.4 | 27.0 |
| G4 | 5/5 | -9.5 | 81.7 | 23.7 |
| G5 | 5/5 | -12.8 | 90.0 | 13.3 |
| G6 | 5/5 | -10.5 | 71.6 | 37.1 |

Biological Example 5

Efficacy Evaluation Experiment

Effects on Growth of Human Pancreatic Cancer BxPC-36 in Allogeneic Transplantation Model

5.1 Test Method

An allogeneic transplantation model of human pancreatic cancer BxPC-3 was established in nude mice subcutaneously using a tumor block inoculation method. When the tumor volume of the allogeneic transplantation was about 100 to 300mm$^3$ and remained stable, the experimental animals were randomly divided into groups according to the size of the tumor volume (day 0) by using a remainder method. The tumor volume was measured 2 to 3 times a week. At the end of the dosage period, the experimental animals were sacrificed. The subcutaneous tumors were peeled and weighed. The anti-tumor activity of the drug against the model was evaluated based on tumor volume and tumor weight-related data.

5.1.1 Grouping and Dosage Regimen

Table 11. Experimental animal grouping and dosage regimen regarding allogeneic transplantation model on growth of human pancreatic cancer BxPC-3

| Group | Compound | Number | Dosage Regimen |
|---|---|---|---|
| G1 | Model control | 5 | - |
| G2 | Example 24 | | 25mg/kg i.v. qw×2 |
| G3 | Example 85 | | 25mg/kg i.v. qw×2 |
| G4 | Example 7 | | 25mg/kg i.v. qw×2 |
| G5 | Irinotecan | 12 | 100mg/kg i.p. qw×2 |
| Secondary grouping of animals in G5 | | | |
| G6.1 | Model control group | 2 | - |
| G6.2 | Example 24 | 3 | 30mg/kg i.v. qw×2 |
| G6.3 | Example 85 | 3 | 30mg/kg i.v. qw×2 |
| G6.4 | Example 7 | 2 | 30mg/kg i.v. qw×2 |
| Note: "-" means not filled in or no valid data. | | | |

After two administrations, the animals in group G5 were redivided into four groups, namely, a model control group (G6.1) and three experimental groups (G6.2, G6.3, G6.4), after the tumor volume of the animals in group G5 was continuously increased to an average tumor volume of more than 600mm$^3$, and the small 8# and 18# experimental animals were removed.

5.2 Evaluation Index and Statistical Method

The calculation formula of the tumor volume was: Volume = 0.5 × long diameter × short diameter × short diameter.

$$\text{Relative tumor volume (RTV): RTV} = Vt/V0.$$

V0 was the resulting tumor volume measured at the time of dosing in groups (i.e., d0) and Vt was the tumor volume at each measurement.

$$\text{Relative tumor proliferation rate T/C: T/C} = \text{TRTV/CRTV} \times 100\%.$$

T was the subject and C was the model control.
TRTV was the average RTV of the subject, and CRTV was the average RTV of the model control group.

Tumor inhibition rate (%) = (average tumor weight of model control group - average tumor weight of treatment group)/ average tumor weight of model control group × 100%.

$$\text{Percent change in body weight} = Wn/W0 \times 100\%.$$

Wn: average body weight of each group of experimental animals on day n, and W0: average body weight of each group of experimental animals on day 0.
On D17 (day 17) was administered to the G5 group in two groups. Therefore, the end-of-test data of the G5 group

in the main test were d17 data. The tumor was not stripped in the G5 group. Therefore, there was no tumor inhibition rate data. In the large tumor efficacy test after the second grouping of the G5 group, since the number of samples in each group of G6.1 and G6.4 was less than three, the relevant data of the large tumor efficacy test were statistically analyzed among groups using the T-Test method.

5.3 Experimental results

Table 12. Effects of compounds on growth of human pancreatic cancer BxPC-3 in allogeneic transplantation model

| | Host (nude mouse) Response | | Tumor Response | | | |
|---|---|---|---|---|---|---|
| Group | Number of animals Start/end | Final body weight change (%) | Tumor weight (g, $\overline{X}$ ±S.D.) | Tumor inhibition rate (%) | Relative volume RTV ($\overline{X}$±S.D.) | T/C (%) |
| G1 | 5/5 | 6.3 | 1.679±0.9 68 | - | 4.75±1.01 | 100 |
| G2 | 5/5 | -0.6 | 0.134±0.0 80 | 92.0 | 0.46±0.12 | 9.6 |
| G3 | 5/5 | 4.0 | 0.047±0.0 27 | 97.2 | 0.23±0.05 | 4.8 |
| G4 | 5/5 | 2.9 | 0.055±0.0 37 | 96.7 | 0.23±0.08 | 4.9 |
| G5a | 12/12 | 6.4 | - | - | 2.16±0.57 | 52.7 |

a: Since the d17 was administered to the G5 group in two groups, the end-of-test data of the G5 group in the main test were d17 data, and the tumor was not peeled in this group, so that there was no tumor weight and tumor inhibition rate data.

Table 13. Effects of irinotecan on growth of human pancreatic cancer BxPC-3 in allogeneic transplantation model

| | Host (nude mouse) Response | | Tumor Response | | | |
|---|---|---|---|---|---|---|
| Group | Number of animals Start/end | Final body weight change (%) | Tumor weight (g, $\overline{X}$ ±S.D.) | Tumor inhibition rate (%) | Relative volume RTV ($\overline{X}$±S.D.) | T/C (%) |
| G6.1 | 2/2 | 4.9 | 3.000±0.631 | - | 3.72±0.49 | 100 |
| G6.2 | 3/3 | 5.8 | 0.742±0.267 | 75.3 | 1.04±0.07 | 25.5 |
| G6.3 | 3/3 | 0.4 | 0.301±0.051 | 90.0 | 0.48±0.11 | 13.7 |
| G6.4 | 2/2 | -0.3 | 0.174±0.164 | 94.2 | 0.28±0.03 | 7.7 |

Biological Example 6

Efficacy Evaluation Experiment

Effects on Growth of Human Small Cell Lung Cancer NCI-H446 in Allogeneic Transplantation Model

6.1 Test Method

An allogeneic transplantation model of human small cell lung cancer NCI-H446 was established in nude mice subcutaneously using a tumor block inoculation method. When the tumor volume of the allogeneic transplantation was about 100 to 300mm$^3$ and remained stable, the experimental animals were randomly divided into groups according to the size of the tumor volume (day 0, i.e., d0) using a remainder method. The tumor volume was measured 2 to 3 times a week. At the end of the dosage period, the experimental animals were sacrificed. The subcutaneous tumors were peeled and weighed. The anti-tumor activity of the drug against the model was evaluated based on tumor volume and tumor weight-related data.

6.1.1 Grouping and Dosage Regimen

Table 14. Experimental animal grouping and dosage regimen regarding allogeneic transplantation model on growth of human small cell lung cancer NCI-H446

| Group | | Administration dose (mg/kg) | Solvent | Administration volume (ml/kg) | Administration concentration (mg/ml) | Route of administration | Administration period | Number |
|---|---|---|---|---|---|---|---|---|
| Model control group | G1 | - | - | - | - | - | - | 5 |
| Example 61 | G2 | 20 | 0.9% sodium chloride injection | 10 | 2 | i.v. | qw×2 | |
| Example 14 | G3 | 20 | | | 2 | | | |
| Example 54 | G4 | 20 | | | 2 | | | |
| Example 7 | G5 | 20 | | | 2 | | | |
| Note: "-" means not filled in or no valid data. | | | | | | | | |

6.2 Evaluation Index and Statistical Method

The calculation formula of the tumor volume was: Volume = 0.5 × long diameter × short diameter × short diameter.

$$\text{Relative tumor volume (RTV): RTV} = Vt/V0.$$

V0 was the resulting tumor volume measured at the time of dosing in groups (i.e., d0) and Vt was the tumor volume at each measurement.

$$\text{Relative tumor proliferation rate T/C: T/C} = \text{TRTV/CRTV} \times 100\%.$$

T was the subject and C was the model control.

TRTV was the average RTV of the subject, and CRTV was the average RTV of the model control group.

Tumor inhibition rate (%) = (average tumor weight of model control group - average tumor weight of treatment group)/ average tumor weight of model control group × 100%.

$$\text{Percent change in body weight} = Wn/W0 \times 100\%.$$

Wn: average body weight of each group of experimental animals on day n, and W0: average body weight of each group of experimental animals on day 0. 6.3 Experimental results

Table 15. Effects of compounds on growth of human small cell lung cancer NCI-H446 in allogeneic transplantation model

| | Host (nude mouse) Response | | Tumor Response | | | |
|---|---|---|---|---|---|---|
| Group | Number of animals Start/end | Final body weight change (%) | Tumor weight (g, $\overline{X}$±S.D.) | Tumor inhibition rate (%) | Relative volume RTV ($\overline{X}$ ±S.D.) | T/C (%) |
| Model control group | 5/5 | 5.2 | 0.974±0. 243 | - | 6.82±2.83 | 100.0 |
| Example 61 (20 mg/kg) | 5/5 | 5.4 | 0.039±0. 049 | 96.0 | 0.25±0.25 | 3.7 |
| Example 14 (20 mg/kg) | 5/5 | 2.4 | 0.085±0. 075 | 91.3 | 0.69±0.45 | 10.2 |
| Example 54 (20 mg/kg) | 5/5 | 5.8 | 0.093±0. 080 | 90.5 | 0.61±0.48 | 8.9 |
| Example 7 (20 mg/kg) | 5/5 | 4.1 | 0.011±0. 005 | 98.9 | 0.11±0.02 | 1.5 |

Biological Example 7

Efficacy Evaluation Experiment

Effects on Growth of Human Gastric Cancer NCI-N87 in Allogeneic Transplantation Model

7.1 Test Method

An allogeneic transplantation model of human gastric cancer NCI-N87 was established in nude mice subcutaneously using a tumor block inoculation method. When the tumor volume of the allogeneic transplantation was about 100 to 300mm$^3$ and remained stable, the experimental animals were randomly divided into groups according to the size of the tumor volume using a remainder method (day 0, i.e., d0) and administered. The tumor volume was measured 2 to 3 times per week. At the end of the dosage period, the experimental animals were sacrificed. The subcutaneous tumors were peeled and weighed. The anti-tumor activity of the drug against the model was evaluated based on tumor volume and

tumor weight-related data.

7.1.1 Grouping and Dosage Regimen

Table 16. Experimental animal grouping and dosage regimen regarding allogeneic transplantation model on growth of human gastric cancer NCI-N87

| Group | | Administration dose (mg/kg) | Solvent | Administration volume (ml/kg) | Administration concentration (mg/ml) | Route of administration | Administration period | Number |
|---|---|---|---|---|---|---|---|---|
| Model control group | G1 | - | - | 10 | - | - | - | 7 |
| Example 90 | G2 | 20 | 0.9% sodium chloride injection | | 2 | i.v. | qw×2 | |
| Example 85 | G3 | 20 | | | 2 | | | |
| Example 7 | G4 | 20 | | | 2 | | | |
| Note: "-" means not filled in or no valid data. | | | | | | | | |

7.2 Evaluation Index and Statistical Method

The calculation formula of the tumor volume : Volume = 0.5 × long diameter × short diameter × short diameter.

$$\text{Relative tumor volume (RTV): RTV} = \text{Vt/V0}.$$

V0 was the resulting tumor volume measured at the time of dosing in groups (i.e., d0) and Vt was the tumor volume at each measurement.

$$\text{Relative tumor proliferation rate T/C: T/C} = \text{TRTV/CRTV} \times 100\%.$$

T was the subject and C was the model control.
TRTV was the average RTV of the subject, and CRTV was the average RTV of the model control group.

Tumor inhibition rate (%) = (average tumor weight of model control group - average tumor weight of treatment group)/ average tumor weight of model control group × 100%.

$$\text{Percent change in body weight} = \text{Wn/W0} \times 100\%.$$

Wn: average body weight of each group of experimental animals on day n, and W0: average body weight of each group of experimental animals on day 0. 7.3 Experimental results

Table 17. Effects of compounds on growth of human gastric cancer NCI-N87 in allogeneic transplantation model

| Group | Host (nude mouse) | | Tumor Response | | | |
|---|---|---|---|---|---|---|
| | Response | | | | | |
| | Number of animals Start/end | Final body weight change (%) | Tumor weight (g, $\overline{X}$ ±S.D.) | Tumor inhibition rate (%) | Relative volume RTV ($\overline{X}$±S.D.) | T/C (%) |
| G1 | 7/7 | 18.5 | 1.192±0.401 | - | 5.13±1.66 | 100 |
| G2 | 7/7 | 9.0 | 0.197±0.085 | 83.5 | 1.30±0.36 | 25.3 |
| G3 | 7/7 | 10.0 | 0.076±0.014 | 93.6 | 0.67±0.24 | 13.0 |
| G4 | 7/7 | 13.6 | 0.069±0.009 | 94.2 | 0.69±0.15 | 13.6 |

Biological Example 8

Efficacy Evaluation Experiment

Effects of irinotecan on Growth of Human Colon Cancer HCT116 in Allogeneic Transplantation Model

8.1 Test Methods

An allogeneic transplantation model of human colon cancer HCT116 was established in nude mice subcutaneously using a tumor block inoculation method. When the tumor volume of the allogeneic transplantation was about 100 to 300mm$^3$ and stable growth was maintained, 15 experimental animals were selected to administer irinotecan (100mg/kg qw×2). When the tumor volume was more than 600mm$^3$ and stable growth was maintained, the experimental animals were randomly divided by the remainder method and administered according to the size of the tumor volume. The tumor volume was measured 2 to 3 times a week. At the end of the dosage period, the experimental animals were sacrificed. The subcutaneous tumors were stripped and weighed. The anti-tumor activity of the drug against large tumors after irinotecan treatment of the model was evaluated based on tumor volume and tumor weight-related data.

8.1.1 Grouping and Dosing Regimens

Table 18. Experimental animal grouping and dosage regimen regarding allogeneic transplantation model on growth of human colon cancer SW620 after irinotecan treatment

| Before grouping | | | | After grouping | | | |
|---|---|---|---|---|---|---|---|
| Compound | Number | Dosage regimen | Solvent | Compound group | Number | Dosage regimen | Solvent |
| Irinotecan | 15 | 100mg/kg i.p. qw×2 | Sterilized water for injection | G1 - model control group | 3 | - | 0.9% sodium chloride injection |
| | | | | G2 - Example 90 | | 30mg/kg i.v. qw×2 | |
| | | | | G3 - Example 101 | | 30mg/kg i.v. qw×2 | |
| | | | | G4 - Example 96 | | 30mg/kg i.v. qw×2 | |
| | | | | G5 - Example 7 | | 30mg/kg i.v. qw×2 | |
| Note: "-" means not filled in or no valid data. | | | | | | | |

8.2 Evaluation index and statistical method

The calculation formula of the tumor volume was: Volume = 0.5 × long diameter × short diameter × short diameter.

$$\text{Relative tumor volume (RTV): RTV} = \text{Vt/V0}.$$

V0 was the resulting tumor volume measured at the time of dosing in groups (i.e., d0) and Vt was the tumor volume at each measurement.

$$\text{Relative tumor proliferation rate T/C: T/C} = \text{TRTV/CRTV} \times 100\%.$$

T was the subject and C is the model control.

TRTV was the average RTV of the subject, and CRTV was the average RTV of the model control group.

Tumor inhibition rate (%) = (average tumor weight of model control group - average tumor weight of treatment group)/ average tumor weight of model control group × 100%.

$$\text{Percent change in body weight} = \text{Wn/W0} \times 100\%.$$

Wn: average body weight of each group of experimental animals on day n, and W0: average body weight of each group of experimental animals on day 0.

8.3 Experimental results

8.3.1 Model tumor growth curve before grouping

D18 (Day 18) after modeling, 15 experimental animals with tumor volumes ranging from 90.36mm$^3$ to 187.06mm$^3$ (average 142.55mm$^3$, SD 31.36) were selected for administration of irinotecan (100mg/kg qw×2) after tumor volume measurement. Tumor volume growth was observed on a regular basis after completion of dosing. The tumor

growth curve was shown in Fig. 1. At d53 after modeling (day 53), the model tumor volume rebound significantly after irinotecan treatment, with an average of 689.82mm$^3$. Therefore, the experimental animals was grouped and the compounds of the present application were administered to continue treatment.

8.3.2 Effects on growth of human colon cancer HCT116 large tumor in allogeneic transplantation model after irinotecan treatment

Table 19. Effects on growth of human colon cancer HCT116 large tumor in allogeneic transplantation model after irinotecan treatment

| Group | Host (nude mouse) Response | | Tumor Response | | | |
|---|---|---|---|---|---|---|
| | Number of animals Start/end | Final body weight change (%) | Tumor weight (g, $\overline{X}\pm$S.D.) | Tumor inhibition rate (%) | Relative volume RTV ($\overline{X}\pm$S.D.) | T/C (%) |
| G1 | 3/3 | 4.7 | 2.723±1.748 | - | 3.72±1.21 | 100 |
| G2 | 3/3 | -3.0 | 0.627±0.408 | 77.0 | 0.89±0.10 | 23.9 |
| G3 | 3/3 | -1.9 | 0.501±0.081 | 81.6 | 0.90±0.20 | 24.2 |
| G4 | 3/3 | -3.7 | 0.425±0.091 | 84.4 | 0.78±0.03 | 21.0 |
| G5 | 3/3 | -10.7 | 0.720±0.492 | 73.6 | 1.10±0.16 | 29.6 |

Biological Example 9

Toxicity Evaluation Experiment in Mice After multiple Administrations

9.1 Test Method

Experimental animals were randomized in the order of body weight. The corresponding doses of the compounds were administered to the test animals via tail vein (i.v.) at the time of the experiment. The grouping and the dosage regimen were detailed in Table 2. The dosage regimen may be adjusted appropriately during the course of the test according to animal toxicity. After adminstration, the performance of the experimental animals was observed periodically and the weight and death anatomy of each group of experimental animals were recorded. After the end of the observation period, each group of animals was sacrificed and dissected. The main organ changes were observed. The toxicity effect of the compounds and toxicity recovery were evaluated.

9.1.1 Grouping and Dosage Regimen

Table 20. Test Protocol on toxicity in mice after multiple administration

| Group | | Administration dose mg/kg | Administration volume (ml/kg) | Administration concentration (mg/ml) | Route of administration | Administration period | Number (♀/♂) |
|---|---|---|---|---|---|---|---|
| G1 | Blank control group | - | - | - | - | - | 3/3 |
| G2 | Example 40 | 150 | 10 | 15 | I.v. | qw×2 | |
| G3 | Example 78 | | | | | | |
| G4 | Example 47 | | | | | | |
| G5 | Example 21 | | | | | | |
| G6 | Example 69 | | | | | | |
| G7 | Example 61 | | | | | | |
| G8 | Example 14 | | | | | | |
| G9 | Example 54 | | | | | | |
| Note: "-" means not filled in or no valid data | | | | | | | |

9.2 Evaluation index and statistical method

$$\text{Percent change in body weight} = Wn/W0 \times 100\%$$

(Wn: average body weight of each group of experimental animals on day n, W0: average body weight of each group of experimental animals on day 0)

Animal weight-related data were statistically analyzed using a general linear model test in the SPSS 22.0 software. Firstly, a spherical test was performed using a repeated measurement variance analysis method. If P>0.05, it was considered that there was no correlation among the repeated measurement data, and a one-way variance analysis (One-Way Anova test) was used to perform a statistical analysis among groups. If P≤0.05, it was considered that there was a correlation among the repeated measurement data, and a multivariate analysis of variance (Multivariate) was used for the inter-group statistical analysis.

9.3 Experimental results

By the end of the test, no death or moribundity occurred in each treatment group. The maximum tolerated dose (MTD) of a single dose of the compound of the present application was greater than 150 mg/kg and the maximum tolerated dose (MTD) of the total administered amount was greater than 300 mg/kg. It was suggested that the compounds of the present application have good safety and good tolerance.

In the present disclosure, relational terms, such as first and second and the like, are used solely to distinguish one entity or operation from another entity or operation without necessarily requiring or implying any such actual relationship or order between such entities or operations.

From the foregoing it will be appreciated that, although specific embodiments of the present disclosure have been described herein for illustrative purposes, various modifications or improvements may be made by those skilled in the art without departing from the spirit and scope of the present disclosure. Such variations or modifications are intended to fall

within the scope of the claims appended hereto.

**Claims**

1. A compound of Formula (I), a stereoisomer, and a cis-trans isomer thereof,

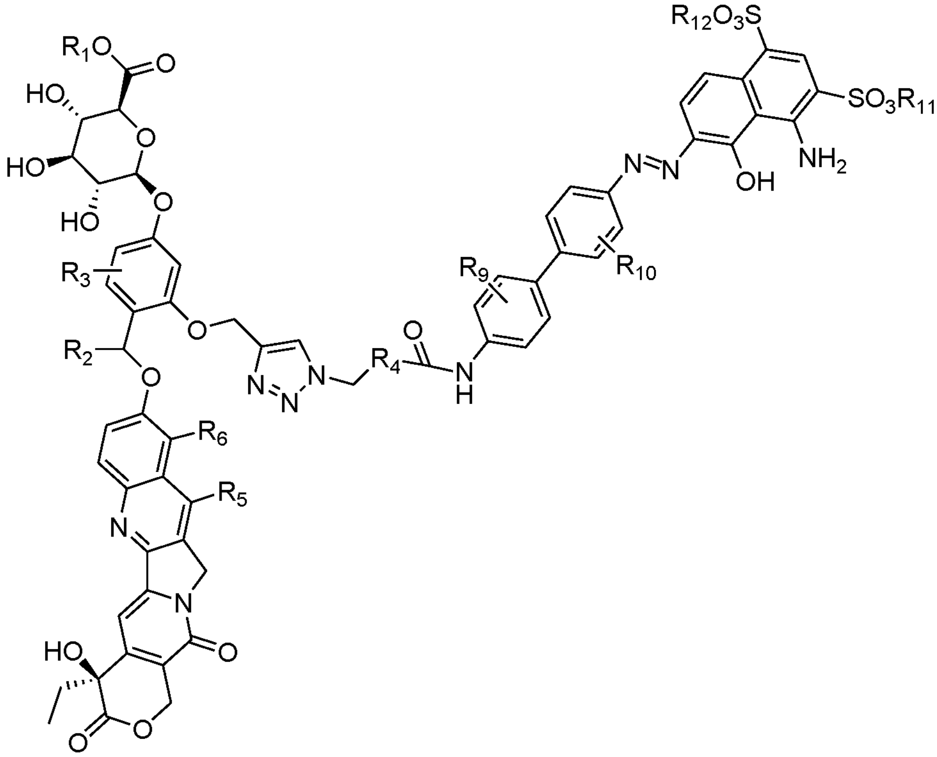

Formula (I)

wherein

$R_1$ is selected from the group consisting of hydrogen, $C_1$-$C_8$ hydrocarbyl, -Na, -Li, -K, -Cs, $-NH_4^+$, $-NH_2^+R_{13}R_{14}$, $-NH^+R_{13}R_{14}R_{15}$, $-NH^+{}_3R_{13}$, and natural or unnatural amino acids;
$R_2$ is selected from the group consisting of hydrogen, and $C_1$-$C_8$ hydrocarbyl;
$R_3$ is one or more substituents at any substitution position selected from the group consisting of hydrogen, halogen, $C_1$-$C_8$ hydrocarbyl, $C_1$-$C_8$ hydrocarbyloxy, cyano, nitro, amino, aryl, arylhydrocarbyl, and $C_1$-$C_4$ hydrocarbyl substituted amino;
$R_4$ is selected from the group consisting of $C_1$-$C_9$ alkylidene, $C_1$-$C_8$ alkylideneoxy, $C_1$-$C_8$ hydrocarbyl substituted or unsubstituted $C_3$-$C_8$ heteroaryl, $C_1$-$C_8$ hydrocarbyl substituted or unsubstituted $C_3$-$C_{15}$ aryl, $-(CH_2R_7)_n$, $-(R_8)_m$, $C_1$-$C_9$ alkylidene-arylidene, $C_1$-$C_9$ alkylidene-arylidene-$C_1$-$C_9$ alkyldiene, $C_1$-$C_9$ alkylidene-arylidene-arylidene, $C_1$-$C_9$ alkylidene-heteroaryliden-$C_1$-$C_9$ alkylidene, and amino acid peptide chain; wherein the amino acid peptide chain is composed of a combination of natural or unnatural amino acids, and the peptide chain length is 1 to100 peptides, preferably 1 to 50 peptides, and more preferably 1 to 20 peptides;
$R_5$ is selected from the group consisting of hydrogen, and $C_1$-$C_8$ hydrocarbyl;
$R_6$ is selected from the group consisting of hydrogen, $C_1$-$C_8$ hydrocarbyl, $C_1$-$C_8$ hydrocarbylidene amino ($C_1$-$C_4$ hydrocarbyl)$_2$, $C_1$-$C_8$ hydrocarbylidene thio $C_1$-$C_8$ hydrocarbyl, and $C_1$-$C_9$ alkylidene-oxy-$C_1$-$C_9$ alkyl;
$R_5$ and $R_6$ may form a ring, and if $R_5$ and $R_6$ form a ring, the ring may be a five-membered ring or a six-membered ring;
$R_7$ is selected from hydrogen, $C_1$-$C_8$ hydrocarbylideneoxy, amino $C_1$-$C_8$ hydrocarbylidene, thio $C_1$-$C_8$ hydrocarbylidene, and oxy $C_1$-$C_8$ hydrocarbylidene;
n is selected from the group consisting of 0, 1, 2, 3, 4, 5, 6, 7, 8, and 9; if n is 0, $(CH_2R_7)_n$ represents a covalent bond;

$R_8$ is carbonylamino, wherein amino may be substituted with $C_1$-$C_8$ hydrocarbyl or arylhydrocarbyl, $C_1$-$C_8$ hydrocarbylidene amino $C_1$-$C_8$ hydrocarbylidene, $C_1$-$C_8$ hydrocarbylidene thio $C_1$-$C_8$ hydrocarbylidene, or $C_1$-$C_8$ hydrocarbylidene oxy $C_1$-$C_8$ hydrocarbylidene;
m is selected from the group consisting of 0, 1, 2, 3, 4, 5, 6, 7, 8, and 9; if m is 0, -$(R_8)_m$ represents a covalent bond;
$R_9$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;
$R_{10}$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;
$R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;
$R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;
$R_{13}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;
$R_{14}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;
$R_{15}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl; and
the natural or unnatural amino acids in $R_1$, $R_{11}$, and $R_{12}$ are selected from the group consisting of glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine.

2. The compound of Formula (I), the stereoisomer, and the cis-trans isomer thereof of claim 1,

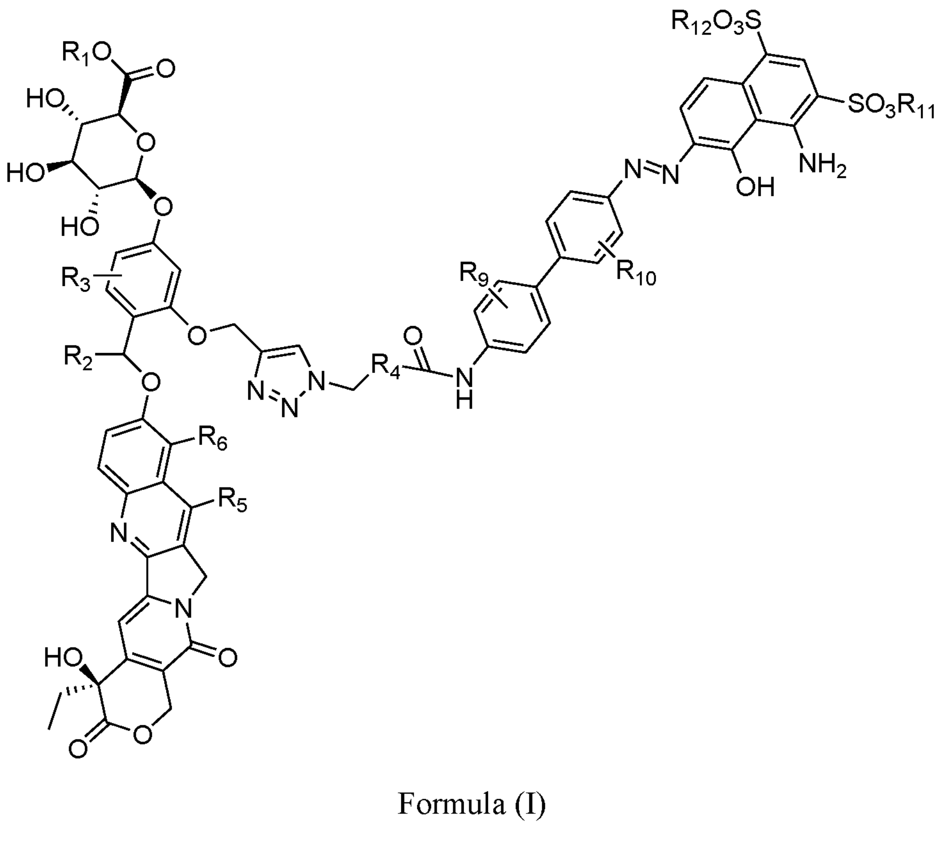

Formula (I)

wherein

$R_1$ is selected from the group consisting of hydrogen, $C_1$-$C_4$ hydrocarbyl, -Na, -Li, -K, -Cs, $-NH_4^+$, $-NH_2^+R_{13}R_{14}$, $-NH^+R_{13}R_{14}R_{15}$, $-NH^+_3R_{13}$, and natural or unnatural amino acids;
$R_2$ is selected from the group consisting of hydrogen, and $C_1$-$C_4$ hydrocarbyl;

$R_3$ is one or more substituents at any position selected from the group consisting of hydrogen, halogen, $C_1$-$C_4$ hydrocarbyl, $C_1$-$C_4$ hydrocarbyloxy, cyano, nitro, amino, aryl, arylhydrocarbyl, and $C_1$-$C_4$ hydrocarbyl substituted amino;

$R_4$ is selected from the group consisting of $C_1$-$C_6$ alkylidene, $C_1$-$C_6$ alkylideneoxy, $C_1$-$C_6$ hydrocarbyl substituted or unsubstituted $C_3$-$C_8$ heteroaryl, $C_1$-$C_8$ hydrocarbyl substituted or unsubstituted $C_3$-$C_{15}$ aryl, $-(CH_2R_7)_n$, $-(R_8)_m$, $C_1$-$C_4$ alkylidene-arylidene, $C_1$-$C_4$ alkylidene-aryliden-$C_1$-$C_4$ alkylidene, $C_1$-$C_4$ alkylidene-arylidene-arylidene, $C_1$-$C_4$ alkylidene-heteroarylidene-$C_1$-$C_4$ alkylidene, and amino acid peptide chain; wherein the amino acid peptide chain is composed of a combination of natural or unnatural amino acids, and the peptide chain length is 1 to 100 peptides, preferably 1 to 50 peptides, and more preferably 1 to 20 peptides;

$R_5$ is selected from the group consisting of hydrogen, and $C_1$-$C_4$ hydrocarbyl;

$R_6$ is selected from the group consisting of hydrogen, $C_1$-$C_4$ hydrocarbyl, $C_1$-$C_4$ hydrocarbylidene amino ($C_1$-$C_4$ hydrocarbyl)$_2$, $C_1$-$C_4$ hydrocarbylidene thio $C_1$-$C_4$ hydrocarbyl, and $C_1$-$C_4$ alkylidene-oxy-$C_1$-$C_4$ alkyl;

$R_5$ and $R_6$ may form a ring, and if $R_5$ and $R_6$ form a ring, the entire ring may be a five-membered ring or a six-membered ring;

$R_7$ is selected from the group consisting of hydrogen, $C_1$-$C_4$ alkylideneoxy, amino $C_1$-$C_4$-alkylidene, thio $C_1$-$C_4$-alkylidene, and oxy $C_1$-$C_4$ alkylidene;

n is selected from the group consisting of 0, 1, 2, 3, 4, 5, 6, 7, 8, and 9; if n is 0, $-(CH_2R_7)_n$ represents a covalent bond;

$R_8$ is carbonylamino, wherein amino may be substituted with $C_1$-$C_4$ hydrocarbyl or arylhydrocarbyl, $C_1$-$C_4$ hydrocarbylidene amino $C_1$-$C_4$ hydrocarbylidene, $C_1$-$C_4$ hydrocarbylidene thio $C_1$-$C_4$ hydrocarbylidene, or $C_1$-$C_4$ hydrocarbylidene oxy $C_1$-$C_4$ hydrocarbylidene;

m is selected from the group consisting of 0, 1, 2, 3, 4, 5, 6, 7, 8, and 9; if m is 0, $-(R_8)_m$ represents a covalent bond;

$R_9$ is one or more substituents at any position selected from the group consisting of hydrogen, halogen, cyano, $C_1$-$C_4$ hydrocarbyl, $C_1$-$C_4$ alkoxy, and $C_5$-$C_{12}$ aryl;

$R_{10}$ is one or more substituents at any position selected from the group consisting of hydrogen, halogen, cyano, $C_1$-$C_4$ hydrocarbyl, $C_1$-$C_4$ alkoxy, and $C_5$-$C_{12}$ aryl;

$R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;

$R_{13}$ is selected from the group consisting of hydrogen, $C_1$-$C_4$ alkyl, $C_1$-$C_8$ cycloalkyl, $C_5$-$C_{18}$ aryl, and $C_5$-$C_{18}$ heteroaryl;

$R_{14}$ is selected from the group consisting of hydrogen, $C_1$-$C_4$ alkyl, $C_1$-$C_8$ cycloalkyl, $C_5$-$C_{18}$ aryl, and $C_5$-$C_{18}$ heteroaryl;

$R_{15}$ is selected from the group consisting of hydrogen, $C_1$-$C_4$ alkyl, $C_1$-$C_8$ cycloalkyl, $C_5$-$C_{18}$ aryl, and $C_5$-$C_{18}$ heteroaryl; and

the natural or unnatural amino acid in $R_1$, $R_{11}$, and $R_{12}$ are selected from the group consisting of glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine.

**3.** The compound of Formula (I), the stereoisomer, and the cis-trans isomers thereof of claim 1 or 2,

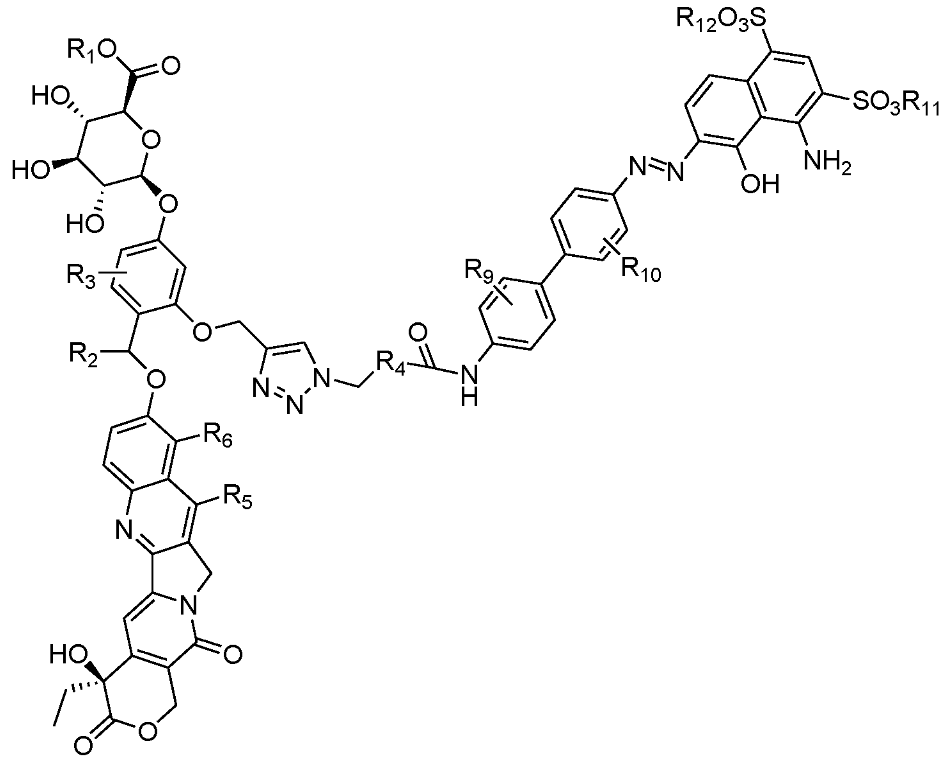

Formula (I)

wherein

$R_1$ is selected from the group consisting of hydrogen, $-CH_3$, $-CH_2CH_3$, -Na, -Li, -K, -Cs, $-NH_4^+$, $-NH_2^+R_{13}R_{14}$, $-NH^+R_{13}R_{14}R_{15}$, $-NH^+{}_3R_{13}$, and natural or unnatural amino acids;

$R_2$ is selected from the group consisting of hydrogen, $-CH_3$, and $-CH_2CH_3$;

$R_3$ is selected from one or more substituents substituted at any position selected from hydrogen, -F-, -Cl, -Br, -CN, $-CH_3$, $-CH_2CH_3$, $-CH_2CH_2CH_3$, $-CH_2CH_2CH_2CH_3$, $-OCH_3$, $-OCH_2CH_3$, $-OCH_2CH_2CH_3$, $-OCH_2CH_2CH_2CH_3$, phenyl, benzyl, $-NO_2$, $-NH_2$, and $-N(CH_3)_2$;

$R_4$ is selected from the group consisting of $-CH_2-$, $-CH_2CH_2-$, $-CH_2CH_2CH_2-$, $-CH_2CH_2CH_2CH_2-$, $-CH_2CH_2CH_2CH_2CH_2-$, $-CH_2CH_2CH_2CH_2CH_2CH_2-$, $-CH_2CH_2CH_2CH_2CH_2CH_2CH_2-$, $-CH_2CH_2CH_2CH_2CH_2CH_2CH_2CH_2-$, $-CH_2CH_2CH_2CH_2CH_2CH_2CH_2CH_2CH_2-$, $-CH_2OCH_2-$, $-CH_2OCH_2CH_2-$, $-CH_2OCH_2CH_2OCH_2-$, $-CH_2OCH_2CH_2OCH_2CH_2OCH_2-$, $-CH_2OCH_2CH_2OCH_2CH_2OCH_2CH_2OCH_2-$, $-CH_2CH_2OCH_2CH_2CH_2OCH_2-$, $-CH_2CH_2OCH_2CH_2CH_2OCH_2CH_2CH_2OCH_2-$, $-CH_2CH_2OCH_2CH_2CH_2OCH_2CH_2CH_2OCH_2CH_2CH_2OCH_2-$, $-CH_2CH_2OCH_2-$, $-CH_2CH_2OCH_2CH_2CH_2OCH_2CH_2-$, $-CH_2CH_2OCH_2CH_2CH_2OCH_2-$, $-CH_2NHCH_2CH_2NHCH_2CH_2NHCH_2-$,

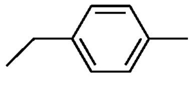 , 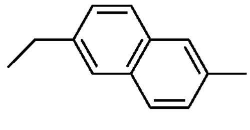 ,

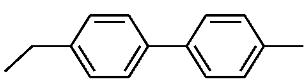 , 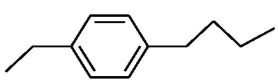 , 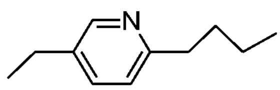 ,

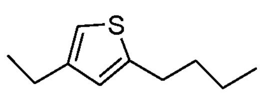 ,

and peptides, wherein the peptides are composed of a combination of natural or unnatural amino acids, such as

glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, homoalanine, or any combination thereof; the peptide chain length is 1 to 100 peptides, preferably 1 to 50 peptides, and more preferably 1 to 20 peptides;

$R_5$ is selected from the group consisting of hydrogen, $-CH_3$, and $-CH_2CH_3$;

$R_6$ is selected from the group consisting of hydrogen, $-CH_3$, $-CH_2OCH_3$, $-CH_2N(CH_3)_2$, and $-CH_2SCH(CH_3)_2$; if $R_5$ and $R_6$ form a ring, the ring may be a five-membered ring or a six-membered ring;

$R_7$ is selected from the group consisting of -H, $-OCH_2-$, $-OCH_2CH_2-$, $-OCH_2CH_2CH_2-$, $-OCH_2CH_2CH_2CH_2-$, $-NHCH_2-$, $-NHCH_2CH_2-$, $-NHCH_2CH_2CH_2-$, $-NHCH_2CH_2CH_2CH_2-$, $-SCH_2-$, $-SCH_2CH_2-$, $-SCH_2CH_2CH_2-$, $-SCH_2CH_2CH_2CH_2-$, $-CH_2O-$, $-CH_2CH_2O-$, $-CH_2CH_2CH_2O-$, and $-CH_2CH_2CH_2CH_2O-$;

n is selected from the group consisting of 0, 1, 2, 3, 4, 5, 6, 7, 8, and 9; if n is 0, $-(CH_2R_7)_n$ represents a covalent bond;

$R_8$ is selected from the group consisting of $-CH_2OCH_2CH_2-$, $-CH_2CH_2OCH_2-$, $-CH_2CH_2OCH_2CH_2-$, $-CH_2CH_2OCH_2CH_2CH_2-$, $-CH_2CH_2CH_2OCH_2CH_2-$, $-CH_2CH_2CH_2OCH_2CH_2CH_2-$, $-CH_2SCH_2CH_2-$, $-CH_2CH_2SCH_2-$, $-CH_2CH_2SCH_2CH_2-$, $-CH_2CH_2SCH_2CH_2CH_2-$, $-CH_2CH_2CH_2SCH_2CH_2-$, $-CH_2CH_2CH_2SCH_2CH_2CH_2-$, $-CH_2NHCH_2CH_2-$, $-CH_2CH_2NHCH_2-$, $-CH_2CH_2NHCH_2CH_2-$, $-CH_2CH_2NHCH_2CH_2CH_2-$, $-CH_2CH_2CH_2NHCH_2CH_2-$, $-CH_2CH_2CH_2NHCH_2CH_2CH_2-$, $-CONHCH_2-$, $-CONHCH_2CH_2-$, $-CONHCH_2CH_2CH_2-$, $-CON(CH_3)CH_2-$, $-CON(CH_3)CH_2CH_2-$, $-CON(CH_3)CH_2CH_2CH_2-$, $-CONHCH(CH_3)-$, $-CONHCH(CH_3)CH_2-$, $-CONHCH(CH_3)CH_2CH_2-$, $-CONHCH_2CH(CH_3)CH_2-$, $-CONHCH(CH_3)CONHCH(CH_3)CH_2C_6H_5-$;

m is selected from the group consisting of 0, 1, 2, 3, 4, 5, 6, 7, 8, and 9; if m is 0, $-(R_8)_m$ represents a covalent bond;

$R_9$ is one or more substituents at any position selected from the group consisting of hydrogen, -F-, -Cl, -Br, -CN, $-CH_3$, $-CH_2CH_3$, $-OCH_3$, $-OCH_2CH_3$, and phenyl;

$R_{10}$ is one or more substituents at any position selected from the group consisting of hydrogen, -F-, -Cl, -Br, -CN, $-CH_3$, $-CH_2CH_3$, $-OCH_3$, $-OCH_2CH_3$, and phenyl;

$R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine;

$R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine;

$R_{13}$ is selected from the group consisting of hydrogen, $-CH_3$, $-CH_2CH_3$, $-CH(CH_3)_2$, $-CH_2CH_2CH_3$, $-CH_2CH_2CH_2CH_2CH_3$, cyclohexyl, phenyl, pyridyl, thienyl, imidazolyl, indolyl, quinolinyl, pyridoimidazolyl, pyridoindolyl, imidazoquinolinyl, imidazoquinoxalinyl, imidazopyridyl, and benzoxazinyl;

$R_{14}$ is selected from the group consisting of hydrogen, $-CH_3$, $-CH_2CH_3$, $-CH(CH_3)_2$, $-CH_2CH_2CH_3$, $-CH_2CH_2CH_2CH_2CH_3$, cyclohexyl, phenyl, pyridyl, thienyl, imidazolyl, indolyl, quinolinyl, pyridoimidazolyl, pyridoindolyl, imidazoquinolinyl, imidazoquinoxalinyl, imidazopyridyl, and benzoxazinyl; and

$R_{15}$ is selected from the group consisting of hydrogen, $-CH_3$, $-CH_2CH_3$, $-CH(CH_3)_2$, $-CH_2CH_2CH_3$, $-CH_2CH_2CH_2CH_2CH_3$, cyclohexyl, phenyl, pyridyl, thienyl, imidazolyl, indolyl, quinolinyl, pyridoimidazolyl, pyridoindolyl, imidazoquinolinyl, imidazoquinoxalinyl, imidazopyridyl, and benzoxazinyl.

**4.** A compound, a stereoisomer, and a cis-transisomer thereof, wherein the compound is selected from the group consisting of

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)2-methylp henoxy)-3,4,5-trihydroxytetrahydro-2H-pyran-2-carboxylate trisodium;

4-amino-6-((E)-(4'-(6-(4-((3-(((2S,3R,4S,5S,6S)-6-carboxy-3,4,5-trihydr oxytetrahydro-2H-pyran-2-yl)oxy)-6-((((S)-4,11-diethyl-4-hydroxy-3,14-dioxy-3,4,12, 14-tetrahydro-1H-pyrano[3',4':6,7]indolazino[1,2-b]quinolin-9-yl)oxy)methyl)-2-tol yloxy)methyl)-1H-1,2,3-triazol-1-yl)hexanamido)-3,3'-dimethyl-[1,1'-biphenyl]-4-yl)diazenyl)-5-hydroxynaphthalene-1,3-disulfonate disodium;

4-amino-6-((E)-(4'-(6-(4-((6-(((S)-4,11-diethyl-4-hydroxy-3,14-dioxy-3, 4,12,14-tetrahydro-1H-pyrano[3',4':6,7]indolazo[1,2-b]quinolin-9-yl)oxy)methyl)-2-methyl-3-(((2S,3R,4S,5S,6S)-3,4,5-trihydroxy-6-(methoxycarbonyl)tetrahydro-2H-py ran-2-yl)oxy)phenoxy)methyl)-1H-1,2,3-triazol-1-yl)hexanamido)-3,3'-dimethyl-[1,1' -biphenyl]-4-yl)diazenyl)-5-hydroxynaphthalene-1,3-disulfonate disodium;

(2S,3S,4S,5R,6S)-6-(3-((1-(6-((4'-((E)-(8-amino-1-hydroxy-5,7-disulfoni cnaphthalen-2-yl)diazenyl)-[1,1'-biphenyl]-4-yl)amino)-6-oxohexyl)-1H-1,2,3-triazol -4-yl)methoxy)-4-((((S)-4,11-diethyl-4-hydroxy-3,14-dioxo-3,4,12,14-tetrahydro-1H-pyrano[3',4':6,7]indolazino[1,2-b]quinolin-9-yl)oxy)methyl)-2-methylphenoxy)-3,4,5 -trihydroxytetrahydro-2H-pyran-2-carboxylate trisodium;
4-amino-6-((E)-(4'-(6-(4-((5-(((2S,3R,4S,5S,6S)-6-carboxy-3,4,5-trihydr oxytetrahydro-2H-pyran-2-yl)oxy)-2-((((S)-4,11-diethyl-4-hydroxy-3,14-dioxy-3,4,12, 14-tetrahydro-1H-pyrano[3',4':6,7]indolazino[1,2-b]quinolin-9-yl)oxy)methyl)pheno xy)methyl)-1H-1,2,3-triazol-1-yl)hexanamido)-3,3'-dimethyl-[1,1'-biphenyl]-4-yl)dia zenyl)-5-hydroxynaphthalene-1,3-disulfonate disodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;
4-amino-6-((E)-(4'-(6-(4-((2-(((S)-4,11-diethyl-4-hydroxy-3,14-dioxy-3, 4,12,14-tetrahydro-1H-pyrano[3',4':6,7]indolazino[1,2-b]quinolin-9-yl)oxy)methyl)-5 -(((2S,3R,4S,5S,6S)-3,4,5-trihydroxy-6-(methoxycarbonyl)tetrahydro-2H-pyran-2-yl) oxy)phenoxy)methyl)-1H-1,2,3-triazol-1-yl)hexanamido)-3,3'-dimethyl-[1,1'-bipheny l]-4-yl)diazenyl)-5-hydroxynaphthalene-1,3-disulfonate disodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-(((S)-4-ethyl-4-hydroxy-3,14-dioxy-3,4,12,14-tetrah ydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;
4-amino-6-((E)-(4'-(6-(4-((5-(((2S,3R,4S,5S,6S)-6-carboxy-3,4,5-trihydr oxytetrahydro-2H-pyran-2-yl)oxy)-2-((((S)-4-ethyl-4-hydroxy-3,14-dioxo-3,4,12,14-t etrahydro-1H-pyrano[3',4':6,7]indolazino[1,2-b]quinolin-9-yl)oxy)methyl)phenoxy) methyl)-1H-1,2,3-triazol-1-yl)hexanamido)-3,3'-dimethyl-[1,1'-biphenyl]-4-yl)diazen yl)-5-hydroxynaphthalene-1,3-disulfonate disodium;
4-amino-6-((E)-(4'-(6-(4-((2-(((S)-4-ethyl-4-hydroxy-3,14-dioxo-3,4,12,1 4-tetrahydro-1H-pyrano[3',4':6,7]indolizino[1,2-b]quinolin-9-yl)oxy)methyl)-5-(((2S, 3R,4S,5S,6S)-3,4,5-trihydroxy-6-(methoxycarbonyl)tetrahydro-2H-pyran-2-yl)oxy)ph enoxy)methyl)-1H-1,2,3-triazol-1-yl)hexanamido)-3,3'-dimethyl-[1,1'-biphenyl]-4-yl )diazenyl)-5-hydroxynaphthalene-1,3-disulfonate disodium;
(2S,3S,4S,5R,6S)-6-(3-((1-(6-((4'-((E)-(8-amino-1-hydroxy-5,7-disulfoni cnaphthalen-2-yl)diazenyl)-[1,1'-biphenyl]-4-yl)amino)-6-oxohexyl)-1H-1,2,3-triazol -4-yl)methoxy)-4-((((S)-4,11-diethyl-4-hydroxy-3,14-dioxo-3,4,12,14-tetrahydro-1H-pyrano[3',4':6,7]indolazino[1,2-b]quinolin-9-yl)oxy)methyl)-5-methoxyphenoxy)-3,4, 5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-[1,1'-biphenyl]-4-yl)amino)-6-oxohexyl)-1H-1,2,3-triazol -4-yl)methoxy)-4-((((S)-4-ethyl-4-hydroxy-3,14-dioxy-3,4,12,14-tetrahydro-1H-pyran o[3',4':6,7]indolazino[1,2-b]quinolin-9-yl)oxy)methyl)phenoxy)-3,4,5-trihydroxytetra hydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-5-methox yphenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethoxy-[1,1'-biphenyl]-4-yl)amino)-6-oxohexyl)-1H-1,2,3-triazol-4-yl)methoxy)-4-(((S)-4-ethyl-4-hydroxy-3,14-dioxy-3,4,12,14-tetra hydro-1H-pyrano[3',4':6,7]indolazino[1,2-b]quinolin-9-yl)oxy)methyl)phenoxy)-3,4, 5-trihydroxytetrahydro-2H-pyran-2-carboxylate trisodium;
4-amino-6-((E)-(4'-(4-((5-(((2S,3R,4S,5S,6S)-6-carboxy-3,4,5-trihydroxy tetrahydro-2H-pyran-2-yl)oxy)-2-((((S)-4-ethyl-4-hydroxy-3,14-dioxo-3,4,12,14-tetra hydro-1H-pyrano[3',4':6,7]indolazino[1,2-b]quinolin-9-yl)oxy)methyl)phenoxy)meth yl)-1H-1,2,3-triazol-1-yl)hexanamido)-3,3'-dimethoxy-[1,1'-biphenyl]-4-yl)diazenyl) -5-hydroxynaphthalene-1,3-disulfonate disodium;
4-amino-6-((E)-(4'-(6-(4-((2-(((S)-4-ethyl-4-hydroxy-3,14-dioxy-3,4,12,1 4-tetrahydro-1H-pyrano[3',4':6,7]indolazino[1,2-b]quinolin-9-yl)oxy)methyl)-5-(((2S, 3R,4S,5S,6S)-3,4,5-trihydroxy-6-(methoxycarbonyl)tetrahydro-2H-pyran-2-yl)oxy)p henoxy)methyl)-1H-1,2,3-triazol-1-yl)hexanamido)-3,3'-dimethoxy-[1,1'-biphenyl]-4 -yl)diazenyl)-5-hydroxynaphthalene-1,3-disulfonate disodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-(((S)-10-((dimethylamino)methyl)-4-ethyl-4-hydroxy -3,14-dioxy-3,4,12,14-tetrahydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;
(2S,3S,4S,5R,6S)-6-(3-((1-(8-((4'-((E)-(8-amino-1-hydroxy-5,7-disulfoni cnaphthalen-2-yl)diazenyl)-3,3'-dimethyl-[1,1'-biphenyl]-4-yl)amino)-8-oxooctyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;

4-amino-6-((E)-(4'-(8-(4-((5-(((2S,3R,4S,5S,6S)-6-carboxy-3,4,5-trihydr oxytetrahydro-2H-pyran-2-yl)oxy)-2-((((S)-4,11-diethyl-4-hydroxy-3,14-dioxy-3,4,12 14-tetrahydro-1H-pyrano[3',4':6,7]indolazino[1,2-b]quinolin-9-yl)oxy)methyl)pheno xy)methyl)-1H-1,2,3-triazol-1-yl)octanamido)-3,3'-dimethyl-[1,1'-biphenyl]-4-yl)dia zenyl)-5-hydroxynaphthalene-1,3-disulfonate disodium;

4-amino-6-((E)-(4'-(8-(4-((2-(((S)-4,11-diethyl-4-hydroxy-3,14-dioxy-3, 4,12,14-tetrahydro-1H-pyrano[3',4':6,7]indolazo[1,2-b]quinolin-9-yl)oxy)methyl)-5-( ((2S,3R,4S,5S,6S)-3,4,5-trihydroxy-6-(methoxycarbonyl)tetrahydro-2H-pyran-2-yl)o xy)phenoxy)methyl)-1H-1,2,3-triazol-1-yl)octanamido)-3,3'-dimethyl-[1,1'-biphenyl] -4-yl)diazenyl)-5-hydroxynaphthalene-1,3-disulfonate disodium;

(2S,3S,4S,5R,6S)-6-(3-((1-(8-((4'-((E)-(8-amino-1-hydroxy-5,7-disulfoni cnaphthalen-2-yl)diazenyl)-[1,1'-biphenyl]-4-yl)amino)-8-oxooctyl)-1H-1,2,3-triazol-4-yl)methoxy)-4-((((S)-4,11-diethyl-4-hydroxy-3,14-dioxo-3,4,12,14-tetrahydro-1H-p yrano[3',4':6,7]indolazino[1,2-b]quinolin-9-yl)oxy)methyl)phenoxy)-3,4,5-trihydroxy tetrahydro-2H-pyran-2-carboxylate trisodium;

(2S,3S,4S,5R,6S)-6-(3-((1-(8-((4'-((E)-(8-amino-1-hydroxy-5,7-disulfoni cnonaphthalen-2-yl)diazenyl)-3,3'-dimethyl-[1,1'-biphenyl]-4-yl)amino)-8-oxooctyl)-1H-1,2,3-triazol-4-yl)methoxy)-4-((((S)-4,11-diethyl-4-hydroxy-3,14-dioxy-3,4,12,14 -tetrahydro-1H-pyrano[3',4':6,7]indolazino[1,2-b]quinolin-9-yl)oxy)methyl)-2-methy lphenoxy)-3,4,5-trihydroxytetrahydro-2H-pyran-2-carboxylate trisodium;

(2S,3S,4S,5R,6S)-6-(3-((1-(2-(2-(2-((4'-((E)-(8-amino-1-hydroxy-5,7-dis ulfonicnaphthalen-2-yl)diazenyl)-3,3'-dimethyl-[1,1'-biphenyl]-4-yl)amino)-2-oxoeth oxy)ethoxy)ethyl)-1H-1,2,3-triazol-4-yl)methoxy)-4-(((S)-4,11-diethyl-4-hydroxy-3,1 4-dioxo-3,4,12,14-tetrahydro-1H-pyrano[3',4':6,7]indolazino[1,2-b]quinolin-9-yl)oxy )methyl)phenoxy)-3,4,5-trihydroxytetrahydro-2H-pyran-2-carboxylate trisodium;

4-amino-6-((E)-(4'-(2-(2-(2-(2-(2-((5-(((2S,3R,4S,5S,6S)-6-carboxy-3,4, 5-trihydroxytetrahydro-2H-pyran-2-yl)oxy)-2-((((S)-4,11-diethyl-4-hydroxy-3,14-dio xy-3,4,12,14-tetrahydro-1H-pyrano[3',4':6,7]indolazino[1,2-b]quinolin-9-yl)oxy)met hyl)phenoxy)methyl)-1H-1,2,3-triazol-1-yl)ethoxy)ethoxy)acetamido)-3,3'-dimethyl-[ 1,1'-biphenyl]-4-yl)diazenyl)-5-hydroxynaphthalene-1,3-disulfonate disodium;

4-amino-6-((E)-(4'-(2-(2-(2-((2-(((S)-4,11-diethyl-4-hydroxy-3,14-dioxy-3,4,12,14-tetrahydro-1H-pyrano [3',4':6,7]indolazo[1,2-b]quinolin-9-yl)oxy)methyl)-5 -(((2S,3R,4S,5S,6S)-3,4,5-trihydroxy-6-(methoxycarbonyl) tetrahydro-2H-pyran-2-yl) oxy)phenoxy)methyl)-1H-1,2,3-triazol-1-yl)ethoxy)acetamido)-3,3'-dimethyl-[1,1'-bi phenyl]-4-yl)diazenyl)-5-hydroxynaphthalene-1,3-disulfonate disodium;

(2S,3S,4S,5R,6S)-6-(3-((1-(2-(2-(2-((4'-((E)-(8-amino-1-hydroxy-5,7-dis ulfonicnaphthalen-2-yl)diazenyl)-3,3'-dimethoxy-[1,1'-biphenyl]-4-yl)amino)-2-oxoe thoxy)ethoxy)ethyl)-1H-1,2,3-triazol-4-yl)methoxy)-4-(((S)-4,11-diethyl-4-hydroxy-3, 14-dioxo-3,4,12,14-tetrahydro-1H-pyrano[3',4':6,7]indolazino[1,2-b]quinolin-9-yl)o xy)methyl)phenoxy)-3,4,5-trihydroxytetrahydro-2H-pyran-2-carboxylate trisodium;

4-amino-6-((E)-(4'-(2-(2-(2-(2-(2-((5-(((2S,3R,4S,5S,6S)-6-carboxy-3,4, 5-trihydroxytetrahydro-2H-pyran-2-yl)oxy)-2-((((S)-4,11-diethyl-4-hydroxy-3,14-dio xy-3,4,12,14-tetrahydro-1H-pyrano[3',4':6,7]indolazino[1,2-b]quinolin-9-yl)oxy)met hyl)phenoxy)methyl)-1H-1,2,3-triazol-1-yl)ethoxy)ethoxy)acetamido)-3,3'-dimethox y-[1,1'-biphenyl]-4-yl)diazenyl)-5-hydroxynaphthalene-1,3-disulfonate disodium;

4-amino-6-((E)-(4'-(2-(2-(2-((2-(((S)-4,11-diethyl-4-hydroxy-3,14-dioxy-3,4,12,14-tetrahydro-1H-pyrano [3',4':6,7]indolazo[1,2-b]quinolin-9-yl)oxy)methyl)-5 -(((2S,3R,4S,5S,6S)-3,4,5-trihydroxy-6-(methoxycarbonyl) tetrahydro-2H-pyran-2-yl) oxy)phenoxy)methyl)-1H-1,2,3-triazol-1-yl)ethoxy)acetamido)-3,3'-dimethoxy-[1,1'-biphenyl]-4-yl)diazenyl)-5-hydroxynaphthalene-1,3-disulfonate disodium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethoxy-[1,1'-biphenyl]-4-yl)amino)-6-oxohexyl)-1H-1,2,3-triazol-4-yl)methoxy)-4-(((S)-4,11-diethyl-4-hydroxy-3,14-dioxy-3,4,12,14-tetrahydro-1H-pyrano[3',4':6,7]indolizino[1,2-b]quinolin-9-yl)oxy)methyl)phenoxy)-3,4,5-trihydroxytetrahydro-2H-pyran-2-carboxylate trisodium;

4-amino-6-((E)-(4'-(6-(4-((5-(((2S,3R,4S,5S,6S)-6-carboxy-3,4,5-trihydr oxytetrahydro-2H-pyran-2-yl)oxy)-2-((((S)-4,11-diethyl-4-hydroxy-3,14-dioxy-3,4,12 14-tetrahydro-1H-pyrano[3',4':6,7]indolazino[1,2-b]quinolin-9-yl)oxy)methyl)pheno xy)methyl)-1H-1,2,3-triazol-1-yl)hexanamido)-3,3'-dimethoxy-[1,1'-biphenyl]-4-yl)d iazenyl)-5-hydroxynaphthalene-1,3-disulfonate disodium;

4-amino-6-((E)-(4'-(6-(4-((2-(((S)-4,11-diethyl-4-hydroxy-3,14-dioxo-3, 4,12,14-tetrahydro-1H-pyrano[3',4':6,7]indolazo[1,2-b]quinolin-9-yl)oxy)methyl)-5-( ((2S,3R,4S,5S,6S)-3,4,5-trihydroxy-6-(methoxycarbonyl)tetrahydro-2H-pyran-2-yl)o xy)phenoxy)methyl)-1H-1,2,3-triazol-1-yl)hexanamido)-3,3'-dimethoxy-[1,1'-biphen yl]-4-yl)diazenyl)-5-hydroxynaphthalene-1,3-disulfonate disodium;

(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy)-3,4,5-trihydroxytetrahydro-2H-pyran-2-carboxylic acid;

1-amino-7-((E)-(4'-(6-(4-((5-(((2S,3R,4S,5S,6S)-6-carboxy-3,4,5-trihydr oxytetrahydro-2H-pyran-2-yl) oxy)-2-((((S)-4,11-diethyl-4-hydroxy-3,14-dioxy-3,4,12 14-tetrahydro-1H-pyrano[3',4':6,7]indolazino[1,2-b]quinolin-9-yl)oxy)methyl)pheno xy)methyl)-1H-1,2,3-triazol-1-yl)hexanamido)-3,3'-dimethyl-[1,1'-biphenyl]-4-yl) dia zenyl)-8-hydroxy-4-sulfonaphthalene-2-sulfonate sodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy)-3,4,5-trihydroxytetrahydro-2H-pyran-2-carboxylate trilithium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy)-3,4,5-trihydroxytetrahydro-2H-pyran-2-carboxylate triammonium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy)-3,4,5-trihydroxytetrahydro-2H-pyran-2-carboxylate tripotassium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)phenoxy)-3,4,5-trihydroxytetrahydro-2H-pyran-2-carboxylate tricesium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-2-fluorop henoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-2-chloro-4-((((S)-4,11-diethyl-4-hydroxy-3,14-dioxy-3, 4,12,14-tetrahydro-1H-pyrano[3',4':6,7]indolazino[1,2-b]quinolin-9-yl)oxy) methyl)p henoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-2-bromo-4-((((S)-4,11-diethyl-4-hydroxy-3,14-dioxy-3, 4,12,14-tetrahydro-1H-pyrano[3',4':6,7]indolazino[1,2-b]quinolin-9-yl)oxy) methyl)p henoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-2-methox yphenoxy)-3,4,5-trihydroxytetrahydro-2H-pyran-2-carboxylat trisodiume;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-2-ethylph enoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-(1-(((S)-4,11-diethyl-4-hydroxy-3,14-dioxy-3,4,12,1 4-tetrahydro-1H-pyrano[3',4':6,7]indolizino[1,2-b]quinolin-9-yl)oxy)ethyl)phenoxy)-3,4,5-trihydroxytetrahydro-2H-pyran-2-carboxylate trisodium;
(2S,3S,4S,SR,6S)-6-((6-((1-((4'-((E)-(8-amino-1-hydroxy-5,7-disulfonicn aphthalen-2-yl)diazenyl)-3,3'-dimethyl-[1,1'-biphenyl]-4-yl)amino)-6-oxohexyl)-1H-1,2,3-triazol-4-yl)methoxy)-5-((((S)-4,11-diethyl-4-hydroxy-3,14-dioxy-3,4,12,14-tetr ahydro-1H-pyrano[3',4':6,7]indolizino[1,2-b]quinolin-9-yl)oxy)methyl)-[1,1'-biphen yl]-2-yl)oxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-2-nitroph enoxy)-3,4,5-trihydroxytetrahydro-2H-pyran-2-carboxylate trisodium;
(2S,3S,4S,5R,6S)-6-(2-amino-3-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxo hexyl)-1H-1,2,3-triazol-4-yl)methoxy)-4-((((S)-4,11-diethyl-4-hydroxy-3,14-dioxy-3, 4,12,14-tetrahydro-1H-pyrano[3',4':6,7]indolazino[1,2-b]quinolin-9-yl)oxy) methyl)p henoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-

xy-3,14-dioxy-3,4,12,14-t etrahydro-1H-pyrano[3',4':6,7]indolazino[1,2-b]quinolin-9-yl)oxy)methyl)-2-(dimeth ylamino)phenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-5-fluorop henoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-5-chloro-4-((((S)-4,11-diethyl-4-hydroxy-3,14-dioxy-3, 4,12,14-tetrahydro-1H-pyrano[3',4':6,7]indolazino[1,2-b]quinolin-9-yl)oxy)methyl)p henoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-5-bromo-4-((((S)-4,11-diethyl-4-hydroxy-3,14-dioxy-3, 4,12,14-tetrahydro-1H-pyrano[3',4':6,7]indolazino[1,2-b]quinolin-9-yl)oxy)methyl)p henoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)-5-methylp henoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-5-nitroph enoxy)-3,4,5-trihydroxytetrahydro-2H-pyran-2-carboxylate trisodium;
(2S,3S,4S,5R,6S)-6-((5-((1-(6-((4'-((E)-(8-amino-1-hydroxy-5,7-disulfon icnaphthalen-2-yl)diazenyl)-3,3'-dimethyl-[1,1'-biphenyl]-4-yl)amino)-6-oxohexyl)-1 H-1,2,3-triazol-4-yl)methoxy)-6-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)-[1,1'-biph enyl]-3-yl)oxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-5-ethylph enoxy)-3,4,5-trihydroxytetrahydro-2H-pyran-2-carboxylate trisodium;
(2S,3S,4S,5R,6S)-6-(3-amino-5-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxo hexyl)-1H-1,2,3-triazol-4-yl)methoxy)-4-((((S)-4,11-diethyl-4-hydroxy-3,14-dioxy-3, 4,12,14-tetrahydro-1H-pyrano[3',4':6,7]indolazino[1,2-b]quinolin-9-yl)oxy)methyl)p henoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-5-(dimeth ylamino)phenoxy)-3,4,5-trihydroxytetrahydro-2H-pyran-2-carboxylate trisodium;
(2S,3S,4S,5R,6S)-6-(5-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)-2-fluoroph enoxy)-3,4,5-trihydroxytetrahydro-2H-pyran-2-carboxylate trisodium;
(2S,3S,4S,5R,6S)-6-(5-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-2-chloro-4-((((S)-4,11-diethyl-4-hydroxy-3,14-dioxy-3 ,4,12,14-tetrahydro-1H-pyrano[3',4':6,7]indolazino[1,2-b]quinolin-9-yl)oxy)methyl)p henoxy)-3,4,5-trihydroxytetrahydro-2H-pyran-2-carboxylat trisodiume;
(2S,3S,4S,5R,6S)-6-(S-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-2-bromo-4-((((S)-4,11-diethyl-4-hydroxy-3,14-dioxy-3, 4,12,14-tetrahydro-1H-pyrano[3',4':6,7]indolazino[1,2-b]quinolin-9-yl)oxy)methyl)p henoxy)-3,4,5-trihydroxytetrahydro-2H-pyran-2-carboxylate trisodium;
(2S,3S,4S,5R,6S)-6-(5-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-2-methox yphenoxy)-3,4,5-trihydroxytetrahydro-2H-pyran-2-carboxylate trisodium;
(2S,3S,4S,5R,6S)-6-(5-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)-2-nitrophe noxy)-3,4,5-trihydroxytetrahydro-2H-pyran-2-carboxylate trisodium;

(2S,3S,4S,5R,6S)-6-((4-((1-(6-((4'-((E)-(8-amino-1-hydroxy-5,7-disulfon icnaphthalen-2-yl)diazenyl)-3,3'-dimethyl-[1,1'-biphenyl]-4-yl)amino)-6-oxohexyl)-1 H-1,2,3-triazol-4-yl)methoxy)-5-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)-[1,1'-biph enyl]-2-yl)oxy)-3,4,5-trihydroxytetrahydro-2H-pyran-2-carboxylate trisodium;
(2S,3S,4S,5R,6S)-6-(5-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)-2-ethylphe noxy)-3,4,5-trihydroxytetrahydro-2H-pyran-2-carboxylate trisodium;
(2S,3S,4S,5R,6S)-6-(2-amino-5-((1-(6-((4'-((E)-(8-amino-1-hydroxy-5,7-disulfonicnaphthalen-2-yl)diazenyl)-3,3'-dimethyl-[1,1'-biphenyl]-4-yl)amino)-6-oxo hexyl)-1H-1,2,3-triazol-4-yl)methoxy)-4-((((S)-4,11-diethyl-4-hydroxy-3,14-dioxy-3, 4,12,14-tetrahydro-1H-pyrano[3,4':6,7]indolazino[1,2-b]quinolin-9-yl)oxy) methyl)ph enoxy)-3,4,5-trihydroxytetrahydro-2H-pyran-2-carboxylate trisodium;
(2S,3S,4S,5R,6S)-6-(5-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)-2-(dimeth ylamino)phenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)-6-fluoro-2 -methoxyphenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-6-chloro-4-((((S)-4,11-diethyl-4-hydroxy-3,14-dioxy-3, 4,12,14-tetrahydro-1H-pyrano[3',4':6,7]indolazino[1,2-b]quinolin-9-yl)oxy) methyl)-5-fluoro-2-methylphenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-6-bromo-4-((((S)-4,11-diethyl-4-hydroxy-3,14-dioxy-3, 4,12,14-tetrahydro-1H-pyrano[3',4':6,7]indolazino[1,2-b]quinolin-9-yl)oxy) methyl)-2-methoxyphenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-9-ethyl-9-hydroxy-10,13-dioxy-2,3,9,10,13,15 -tetrahydro-1H,7]benzo[de]pyrano[3',4':6,7]indolizino[1,2-b]quinolin-4-yl)oxy) meth yl)phenoxy)-3,4,5-trihydroxytetrahydro-2H-pyran-2-carboxylate trisodium;
(2S,3S,4S,5R,6S)-6-(4-(((S)-4-acetoxy-4-ethyl-3,14-dioxo-3,4,12,14-tetra hydro-1H-pyrano[3',4':6,7]indolazino[1,2-b]quinolin-9-yl)oxy)methyl)-3-((1-(6-((4'-((E)-(8-amino-1-hydroxy-5,7-disulfonicnaphthalen-2-yl)diazenyl)-3,3'-dimethyl-[1,1' -biphenyl]-4-yl)amino)-6-oxohexyl)-1H-1,2,3-triazol-4-yl)methoxy)phenoxy)-3,Triso dium4,5-trihydroxytetrahydro-2H-pyran-2-carboxylate;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4-ethyl-3,14-dioxo-4-(propionyloxy)-3,4,12,14 -tetrahydro-1H-pyrano[3',4':6,7]indolazino[1,2-b]quinolin-9-yl)oxy)methyl)phenoxy) -3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1'-biphenyl]-4-yl)amino)-6-oxohexyl)-1 H-1,2,3-triazol-4-yl)methoxy)-4-(((S)-4-ethyl-4-(glycyloxy)-3,14-dioxy-3,4,12,14-tetr ahydro-1H-pyrano[3',4':6,7]indolizino[1,2-b]quinolin-9-yl)oxy)methyl)phenoxy)-3,4, 5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((R)-5-ethyl-5-hydroxy-3,15-dioxo-4,5,13,15-tetrah ydro-1H,3H-oxazepino[3',4':6,7]indolizino[1,2-b]quinolin-10-yl)oxy)methyl)phenox y)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-(((S)-4-ethyl-4-hydroxy-10-(methoxymethyl)-3,14-di oxy-3,4,12,14-tetrahydro-1H-pyrano[3',4':6,7]indolazino[1,2-b]quinolin-9-yl) oxy)me thyl)phenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-(((S)-4-ethyl-4-hydroxy-10-((isopropylthio)methyl)-3,14-dioxy-3,4,12,14-tetrahydro-1H-pyrano[3',4':6,7]indolazino[1,2-b]quinolin-9-yl) oxy)methyl)phenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dimethyl-[1,1' -biphenyl]-4-yl)amino)-6-oxohexyl)-1 H-1,2,3-triazol-4-yl)methoxy)-4-((((S)-4-ethyl-4-hydroxy-3-oxy-14-thio-3,4,12,14-tet rahydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;
(2S,3S,4S,5R,6S)-6-(3-((1-((2-((2-((2-((4'-(E)-(8-amino-1-hydroxy-5,7-d isulfonicnaphthalen-2-yl)diazenyl)-3,3'-dimethyl-[1,1'-biphenyl]-4-yl)amino)-2-oxoet hyl)(methyl)amino)-2-oxoethyl)(methyl)amino)-2-oxoethyl)-1H-1,2,3-triazol-4-yl)me thoxy)-4-((((S)-4,11-diethyl-4-hydroxy-3,14-dioxo-3,4,12,14-tetrahydro-1H-pyrano[3', 4':6,7]indolazino[1,2-b]quinolin-9-yl)oxy)methyl)phenoxy)-3,4,5-trihydroxytetrahy dro-2H-pyran-2-carboxylate trisodium;
(2S,3S,4S,5R,6S)-6-(3-((1-((2-((2-((2-((4'-(E)-(8-amino-1-hydroxy-5,7-d isulfonicnaphthalen-2-yl)diazenyl)-3,3'-dimethyl-[1,1'-biphenyl]-4-yl)amino)-2-oxoet hyl)amino)-2-oxoethyl)amino)-2-oxoethyl)-1H-1,2,3-triazol-4-yl)methoxy)-4-((((S)-4 ,11-diethyl-4-hydroxy-3,14-dioxo-3,4,12,14-tetrahydro-1H-pyrano[3',4':6,7]indolazin o[1,2-b]quinolin-9-yl)oxy)methyl)phenoxy)-3,4,5-trihydroxytetrahydro-2H-pyran-2-c arboxylate trisodium;
(2S,3S,4S,5R,6S)-6-(3-((1-(2-((S)-1-((S)-1-((4'-((E)-(8-amino-1-hydroxy -5,7-disulfonicnaphthalen-2-yl)diazenyl)-3,3'-dimethyl-[1,1'-biphenyl]-4-yl)amino)-1 -oxopropan-2-yl)amino)-1-oxopropan-2-yl)amino)-2-oxoethyl)-1H-1,2,3-triazol-4-yl) methoxy)-4-((((S)-4,11-diethyl-4-hydroxy-3,14-dioxo-3,4,12,14-tetrahydro-1H-pyran o[3',4':6,7]indolazino[1,2-b]quinolin-9-yl)oxy)methyl)phenoxy)-3,4,5-trihydroxytetra hydro-2H-pyran-2-carboxylate trisodium;
(2S,3S,4S,5R,6S)-6-(3-((1-(2-((S)-1-((S)-1-((4'-((E)-(8-amino-1-hydroxy -5,7-disulfonicnaphthalen-2-yl)diazenyl)-3,3'-dimethyl-[1,1'-biphenyl]-4-yl)amino)-1 -oxo-3-phenylpropan-2-yl)amino)-1-oxopropan-2-yl)amino)-2-oxoethyl)-1H-1,2,3-tri azol-4-yl)methoxy)-4-((((S)-4,11-diethyl-4-hydroxy-3,14-dioxy-3,4,12,14-tetrahydro-1H-pyrano[3',4':6,7]indolazino[1,2-b]quinolin-9-yl)oxy)methyl)phenoxy)-3,4,5-trihy droxytetrahydro-2H-pyran-2-carboxylate trisodium;
(2S,3S,4S,5R,6S)-6-(3-((1-(2-(2-(2-(2-((4'-((E)-(8-amino-1-hydroxy-5,7-disulfonicnaphthalen-2-yl)diazenyl)-3,3'-dimethyl-[1,1'-biphenyl]-4-yl)amino)-2-oxo ethoxy)ethoxy)ethoxy)ethyl)-1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[1,2-b]quino lin-9-yl)oxy)methyl)phenoxy)-3,4,5-trihydroxytetrahydro-2H-pyran-2-carboxylate trisodium;
(2S,3S,4S,5R,6S)-6-(3-((1-((2-((2-((2-((2-((4'-(E)-(8-amino-1-hydroxy-5, 7-disulfonicnaphthalen-2-yl)diazenyl)-3,3'-dimethyl-[1,1'-biphenyl]-4-yl)amino)-2-o xoethyl)amino)ethyl)amino)ethyl)amino)ethyl)-1H-1,2,3-triazol-4-yl)methoxy)-4-(((( S)-4,11-diethyl-4-hydroxy-3,14-dioxy-3,4,12,14-tetrahydro-1H-pyrano[3',4':6,7]indo lazino[1,2-b]quinolin-9-yl)oxy)methyl)phenoxy)-3,4,5-trihydroxytetrahydro-2H-pyra n-2-carboxylate trisodium;
(2S,3S,4S,5R,6S)-6-(3-((1-(3-(3-(3-((4'-((E)-(8-amino-1-hydroxy-5,7-dis ulfonicnaphthalen-2-yl)diazenyl)-3,3'-dimethyl-[1,1'-biphenyl]-4-yl)amino)-3-oxopro poxy)propoxy)propyl)-1H-1,2,3-triazol-4-yl)methoxy)-4-(((S)-4,11-diethyl-4-hydroxy -3,14-dioxy-3,4,12,14-tetrahydro-1H-pyrano[3',4':6,7]indolazino[1,2-b]quinolin-9-yl) oxy)methyl)phenoxy)-3,4,5-trihydroxytetrahydro-2H-pyran-2-carboxylate trisodium;
(2S,3S,4S,5R,6S)-6-(3-((1-(4-((4'-((E)-(8-amino-1-hydroxy-5,7-disulfoni cnaphthalen-2-yl)diazenyl)-3,3'-dimethyl-[1,1'-biphenyl]-4-yl)carbamoyl)phenethyl)-1H-1,2,3-triazol-4-yl)methoxy)-4-((((S)-4,11-diethyl-4-hydroxy-3,14-dioxy-3,4,12,14 -tetrahydro-1H-pyrano[3',4':6,7]indolazino[1,2-b]quinolin-9-yl)oxy)methyl)phenoxy) -3,4,5-trihydroxytetrahydro-2H-pyran-2-carboxylate trisodium;
(2S,3S,4S,5R,6S)-6-(3-((1-(2-(2-((4'-((E)-(8-amino-1-hydroxy-5,7-disulf onicnaphthalen-2-yl)diazenyl)-3,3'-dimethyl-[1,1'-biphenyl]-4-yl)carbamoyl)naphthal en-2-yl)ethyl)-1H-1,2,3-triazol-4-yl)methoxy)-4-((((S)-4,11-diethyl-4-hydroxy-3,14-d ioxy-3,4,12,14-tetrahydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;
(2S,3S,4S,5R,6S)-6-(3-((1-(2-(4'-((4'-((E)-(8-amino-1-hydroxy-5,7-disul fonicnaphthalen-2-yl)diazenyl)-3,3'-dimethyl-[1,1'-biphenyl]-4-yl)carbamoyl)-[1,1'-b iphenyl]-4-yl)ethyl)-1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[1,2-b]quinolin-9-yl) oxy) methyl)phenoxy)-3,4,5-trihydroxytetrahydro-2H-pyran-2-carboxylate trisodium;
(2S,3S,4S,5R,6S)-6-(3-((1-(4-(4-((4'-((E)-(8-amino-1-hydroxy-5,7-disulf onicnaphthalen-2-yl)diazenyl)-3,3'-dimethyl-[1,1'-biphenyl]-4-yl)amino)-4-oxobutyl) phenethyl)-1H-1,2,3-triazol-4-yl)methoxy)-4-((((S)-4,11-diethyl-4-hydroxy-3,14-diox y-3,4,12,14-tetrahydro-1H-pyrano[3',4':6,7]indolazino[1,2-b]quinolin-9-yl)oxy)meth yl)phenoxy)-3,4,5-trihydroxytetrahydro-2H-pyran-2-carboxylate trisodium;
(2S,3S,4S,5R,6S)-6-(3-((1-(2-(2-(6-(4-((4'-((E)-(8-amino-1-hydroxy-5,7-disulfonicnaphthalen-2-yl)diazenyl)-3,3'-dimethyl-[1,1'-biphenyl]-4-yl)amino)-4-oxo butyl)pyridin-3-yl)ethyl)-1H-1,2,3-triazol-4-yl)methoxy)-4-((((S)-4,11-diethyl-4-hydr oxy-3,14-dioxy-3,4,12,14-tetrahydro-1H-pyrano[3',4':6,7]indolizino[1,2-b]quinolin-9 -yl)oxy)methyl)phenoxy)-3,4,5-trihydroxytetrahydro-2H-pyran-2-carboxylate trisodium;
(2S,3S,4S,5R,6S)-6-(3-((1-(2-(2-(5-(4-((4'-((E)-(8-amino-1-hydroxy-5,7-disulfonicnaphthalen-2-yl)diazenyl)-3,3'-dimethyl-[1,1'-biphenyl]-4-yl)amino)-4-oxo butyl)thiophen-3-yl)ethyl)-1H-1,2,3-triazol-4-yl)methoxy)-4-((((S)-4,11-diethyl-4-hy droxy-3,14-dioxy-3,4,12,14-tetrahydro-1H-pyrano[3',4':6,7]indolazino[1,2-b]quinoli n-9-yl)oxy)methyl)phenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3',5,5'-tetramethyl-[1,1'-biphenyl]-4-yl)amino)-6-oxohe xyl)-1H-1,2,3-triazol-4-yl)methoxy)-4-((((S)-4,11-diethyl-4-hydroxy-3,14-dioxy-3,4,1 2,14-tetrahydro-1H-pyrano[3',4':6,7]indolazino[1,2-b]quinolin-9-yl)oxy)methyl)phen oxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-2,2',3,3',5,5',6,6'-octamethyl-[1,1'-biphenyl]-4-yl)amino) -6-oxohexyl)-1H-1,2,3-triazol-4-yl)methoxy)-4-((((S)-4,11-diethyl-4-hydroxy-3,14-di oxy-3,4,12,14-tetrahydro-1H-pyrano[3',4':6,7]indolazino[1,2-b]quinolin-9-yl)oxy)me thyl)phenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-2,2',3,3',5,5',6,6'-octamethoxy-[1,1'-biphenyl]-4-yl)amin o)-6-oxohexyl)-1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[1,2-b]quinolin-9-yl)oxy) methyl)phenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3',5,5'-tetrachloro-[1,1'-biphenyl]-4-yl)amino)-6-oxohe xyl)-1H-1,2,3-triazol-4-yl)methoxy)-4-((((S)-4,11-diethyl-4-hydroxy-3,14-dioxy-3,4,1 2,14-tetrahydro-1H-pyrano[3',4':6,7]indolazino[1,2-b]quinolin-9-yl)oxy)methyl)phen oxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-2,2',3,3',5,5',6,6'-octafluoro-[1,1'-biphenyl]-4-yl)amino)-6-oxohexyl)-1H-1,2,3-triazol-4-yl)methoxy)-4-((((S)-4,11-diethyl-4-hydroxy-3,14-dio xy-3,4,12,14-tetrahydro-1H-pyrano[3',4':6,7]indolazino[1,2-b]quinolin-9-yl)oxy)met hyl)phenoxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3'-chloro-3-methoxy-5,5'-dimethyl-[1,1'-biphenyl]-4-yl)a mino)-6-oxohexyl)-1H-1,2,3-triazol-4-yl)methoxy)-4-((((S)-4,11-diethyl-4-hydroxy-3, 14-dioxy-3,4,12,14-tetrahydro-1H-pyrano[3,4':6,7]indolazino[1,2-b]quinolin-9-yl)ox y)methyl)phenoxy)-3,4,5-trihydroxytetrahydro-2H-pyran-2-carboxylate trisodium;
(2S,3S,4S,5R,6S)-6-(3-((1-(6-((6'-((E)-(8-amino-1-hydroxy-5,7-disulfoni cnaphthalen-2-yl)diazenyl)-[1,1':3',1'':3'',1'''-quaterphenyl]-4''-yl)-amino)-6-oxohe xyl)-1H-1,2,3-triazol-4-yl)methoxy)-4-(((((S)-4,11-diethyl-4-hydroxy-3,14-dioxy-3,4, 12,14-tetrahydro-1H-pyrano[3',4':6,7]indolizino[1,2-b]quinolin-9-yl)oxy) methyl)phe noxy)-3,4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-difluoro-[1,1'-biphenyl]-4-yl)amino)-6-oxohexyl)-1H -1,2,3-triazol-4-yl)methoxy)-4-(((S)-4,11-diethyl-4-hydroxy-3,14-dioxy-3,4,12,14-tetr ahydro-1H-pyrano[3',4':6,7]indolizino[1,2-b]quinolin-9-yl)oxy)methyl)phenoxy)-3,4, 5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni cnaphthalen-2-yl)diazenyl)-3,3'-dichloro-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)phenoxy)-3 4,5-trihydroxytetrahydro-2H-pyran-2-carboxylate trisodium;
(2S,3S,4S,5R,6S)-6-(3-((1-(6-((4'-((E)-(8-amino-1-hydroxy-5,7-disulfoni enaphthalen-2-yl)diazenyl)-3,3'-dibromo-[1,1'-biphenyl]-4-yl)amino)-6-oxohexyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;
(2S,3S,4S,5R,6S)-6-(3-((1-(5-((4'-((E)-(8-amino-1-hydroxy-5,7-disulfoni cnaphthalen-2-yl)diazenyl)-3,3'-dimethyl-[1,1'-biphenyl]-4-yl)amino)-5-oxopentyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolizino[1,2-b]quinolin-9-yl)oxy)methyl)phenoxy)-3 4,5-trihydroxytetrahydro-2H-pyran-2-carboxylate trisodium;
(2S,3S,4S,5R,6S)-6-(3-((1-(2-((4'-((E)-(8-amino-1-hydroxy-5,7-disulfoni cnaphthalen-2-yl)diazenyl)-3,3'-dimethyl-[1,1'-biphenyl]-4-yl)amino)-2-oxoethyl)-1 H-1,2,3-triazol-4-yl)methoxy)-4-((((S)-4,11-diethyl-4-hydroxy-3,14-dioxy-3,4,12,14-t etrahydro-1H-pyrano[3',4':6,7]indolazino[1,2-b]quinolin-9-yl)oxy)methyl)phenoxy)-3,4,5-trihydroxytetrahydro-2H-pyran-2-carboxylate trisodium;
(2S,3S,4S,5R,6S)-6-(3-((1-(3-(2-((4'-(E)-(8-amino-1-hydroxy-5,7-disulfo nicnaphthalen-2-yl)diazenyl)-3,3'-dimethyl-[1,1'-biphenyl]-4-yl)amino)-2-oxoethoxy )propyl)-1H-1,2,3-triazol-4-yl)methoxy)-4-((((S)-4,11-diethyl-4-hydroxy-3,14-dioxy-3,4,12,14-tetrahydro-1H-pyrano[3',4':6,7]indolazino[1,2-b]quinolin-9-yl)oxy)methyl) phenoxy)-3,4,5-trihydroxytetrahydro-2H-pyran-2-carboxylate trisodium; and
(2S,3S,4S,5R,6S)-6-(3-((1-(2-(2-((4'-((E)-(8-amino-1-hydroxy-5,7-disulf onicnaphthalen-2-yl)diazenyl)-3,3'-dimethyl-[1,1'-biphenyl]-4-yl)amino)-3-oxopropo xy)ethyl)-1H-1,2,3-triazol-4-yl)methoxy)-4-((((S)-4,11-diethyl-4-hydroxy-3,14-dioxy -3,4,12,14-tetrahydro-1H-pyrano[3',4':6,7]indolazino[1,2-b]quinolin-9-yl)oxy)methyl )phenoxy)-3,4,5-trihydroxytetrahydro-2H-pyran-2-carboxylate trisodium.

**5.** A pharmaceutical composition, comprising the compound, the stereoisomer or the cis-trans isomer thereof of any one of claims 1 to 4, and a pharmaceutically acceptable carrier.

**6.** The pharmaceutical composition of claim 5, which is prepared as an injectable preparation.

**7.** The pharmaceutical composition of claim 6, wherein the injectable preparation is an injection, a sterile powder for injection or a concentrated solution for injection.

**8.** A method for treating a tumor or cancer, comprising administering to a subject in need thereof a therapeutically effective amount of the compound, the stereoisomer or the cis-trans isomer thereof of any one of claims 1 to 4, or the pharmaceutical composition of any one of claims 5 to 7.

**9.** The method of claim 8, wherein the tumor or cancer is selected from the group consisting of colorectal cancer, lung cancer, cervical cancer, ovarian cancer, gastric cancer, esophageal cancer, breast cancer, pancreatic cancer, bladder cancer, liver cancer, gastric cancer, colon cancer, head and neck cancer, uterine cancer, urothelial cancer, osteosarcoma, sarcoma, renal cancer, melanoma, prostate cancer, glioma, glioma and leukemia.

**10.** A process for preparing a compound of Formula (I), a stereoisomer, a cis-trans isomer or a pharmaceutically acceptable salt of thereof of claim 1:

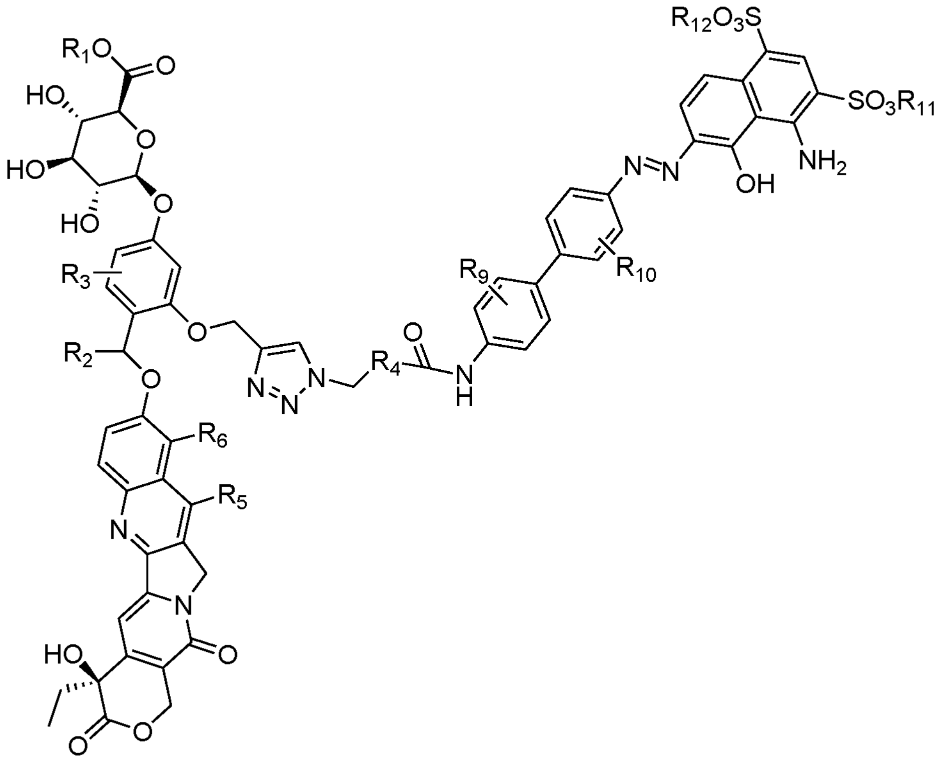

Formula (I)

wherein

$R_1$ is selected from the group consisting of hydrogen, $C_1$-$C_8$ hydrocarbyl, -Na, -Li, -K, -Cs, $-NH_4^+$, $-NH_2^+R_{13}R_{14}$, $-NH^+R_{13}R_{14}R_{15}$, $-NH^+{}_3R_{13}$, and natural or unnatural amino acids;
$R_2$ is selected from the group consisting of hydrogen, and $C_1$-$C_8$ hydrocarbyl;
$R_3$ is one or more substituents at any substitution position selected from the group consisting of hydrogen, halogen, $C_1$-$C_8$ hydrocarbyl, $C_1$-$C_8$ hydrocarbyloxy, cyano, nitro, amino, aryl, arylhydrocarbyl, and $C_1$-$C_4$ hydrocarbyl substituted amino;
$R_4$ is selected from the group consisting of $C_1$-$C_9$ alkylidene, $C_1$-$C_8$ alkylideneoxy, $C_1$-$C_8$ hydrocarbyl substituted or unsubstituted $C_3$-$C_8$ heteroaryl, $C_1$-$C_8$ hydrocarbyl substituted or unsubstituted $C_3$-$C_{15}$ aryl, $-(CH_2R_7)_n$, $-(R_8)_m$, $C_1$-$C_9$ alkylidene-arylidene, $C_1$-$C_9$ alkylidene-arylidene-$C_1$-$C_9$ alkyldiene, $C_1$-$C_9$ alkylidene-arylidene-arylidene, $C_1$-$C_9$ alkylidene-heteroaryliden-$C_1$-$C_9$ alkylidene, and amino acid peptide chain; wherein the amino acid peptide chain is composed of a combination of natural or unnatural amino acids, and the peptide chain length

is 1 to100 peptides, preferably 1 to 50 peptides, and more preferably 1 to 20 peptides;
$R_5$ is selected from the group consisting of hydrogen, and $C_1$-$C_8$ hydrocarbyl;
$R_6$ is selected from the group consisting of hydrogen, $C_1$-$C_8$ hydrocarbyl, $C_1$-$C_8$ hydrocarbylidene amino ($C_1$-$C_4$ hydrocarbyl)$_2$, $C_1$-$C_8$ hydrocarbylidene thio $C_1$-$C_8$ hydrocarbyl, and $C_1$-$C_9$ alkylidene-oxy-$C_1$-$C_9$ alkyl;
$R_5$ and $R_6$ may form a ring, and if $R_5$ and $R_6$ form a ring, the ring may be a five-membered ring or a six-membered ring;
$R_7$ is selected from hydrogen, $C_1$-$C_8$ hydrocarbylideneoxy, amino $C_1$-$C_8$ hydrocarbylidene, thio $C_1$-$C_8$ hydrocarbylidene, and oxy $C_1$-$C_8$ hydrocarbylidene;
n is selected from the group consisting of 0, 1, 2, 3, 4, 5, 6, 7, 8, and 9; if n is 0, $(CH_2R_7)_n$ represents a covalent bond;
$R_8$ is carbonylamino, wherein amino may be substituted with $C_1$-$C_8$ hydrocarbyl or arylhydrocarbyl, $C_1$-$C_8$ hydrocarbylidene amino $C_1$-$C_8$ hydrocarbylidene, $C_1$-$C_8$ hydrocarbylidene thio $C_1$-$C_8$ hydrocarbylidene, or $C_1$-$C_8$ hydrocarbylidene oxy $C_1$-$C_8$ hydrocarbylidene;
m is selected from the group consisting of 0, 1, 2, 3, 4, 5, 6, 7, 8, and 9; if m is 0, $-(R_8)_m$ represents a covalent bond;
$R_9$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;
$R_{10}$ is one or more substituents at any position selected from the group consisting of hydrogen, halogen, cyano, $C_1$-$C_8$ hydrocarbyl, $C_1$-$C_8$ alkoxy, and $C_5$-$C_{12}$ aryl;
$R_{11}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;
$R_{12}$ is selected from the group consisting of hydrogen, -Na, -Li, -K, -Cs, $-NH_4^+$, $-NH_2^+R_{13}R_{14}$, $-NH^+R_{13}R_{14}R_{15}$, $-NH_3^+R_{13}$, and natural or unnatural amino acids;
$R_{13}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;
$R_{14}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl;
$R_{15}$ is selected from the group consisting of hydrogen, $C_1$-$C_8$ hydrocarbyl, $C_1$-$C_8$ cyclohydrocarbyl, $C_5$-$C_{18}$ aryl, and $C_5$-$C_{18}$ heteroaryl; and
the natural or unnatural amino acids in $R_1$, $R_{11}$, and $R_{12}$ are selected from the group consisting of glycine, alanine, valine, leucine, isoleucine, methionine (methionine), proline, tryptophan, serine, tyrosine, cysteine, phenylalanine, asparagine, glutamine, threonine, aspartic acid, glutamic acid, lysine, arginine, histidine, selenocysteine, sarcosine, pyrrolysine, and homoalanine;
wherein the process comprises:

(1) reacting the compound of Formula (A-I) with a compound of Formula (A-II) to obtain a compound of Formula (A-III),

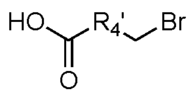

Formula (A-I)

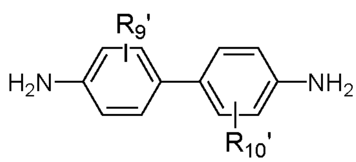

Formula (A-II)

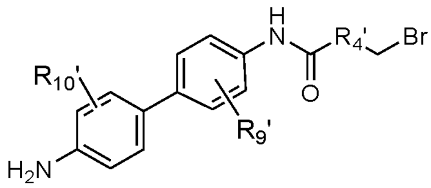

Formula (A-III)

wherein the groups represented by $R_4$', $R_9$', and $R_{10}$' in the Formula (A-I) and the Formula (A-II) are as defined for $R_4$, $R_9$ and $R_{10}$ in the Formula (I);

(2) reacting the compound of Formula (A-III) with sodium azide to obtain a compound of Formula (A-IV),

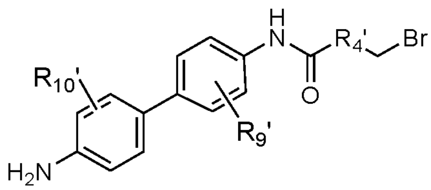

Formula (A-III)

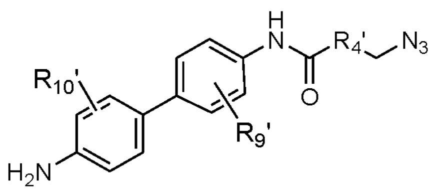

Formula (A-IV)

wherein the groups represented by $R_4$', $R_9$', and $R_{10}$' in the Formula (A-III) and the Formula (A-IV) are as defined for $R_4$, $R_9$ and $R_{10}$ in the Formula (I);

(3) reacting the compound of Formula (A-IV) with 4-amino-5-hydroxy-1,3-naphthalene disulfonic acid monosodium to obtain a compound of Formula (A-V),

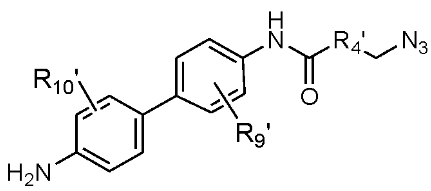

Formula (A-IV)

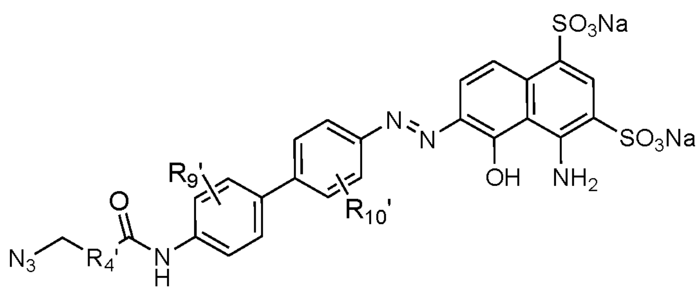

;

Formula (A-V)

wherein the groups represented by $R_4$', $R_9$', and $R_{10}$' in the Formula (A-IV) and the Formula (A-V) are as defined for $R_4$, $R_9$ and $R_{10}$ in the Formula (I);

(4) reacting the compound of Formula (B-I) with carbon tetrabromide to obtain a compound of Formula (B-II),

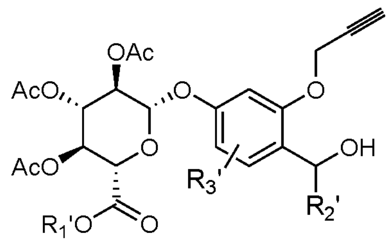

Formula (B-I)

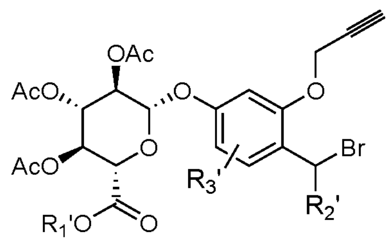

Formula (B-II)

wherein the groups represented by $R_1$', $R_2$', and $R_3$' in the Formula (B-I) and the Formula (B-II) are as defined for $R_1$, $R_2$ and $R_3$ in the Formula (I);

(5) reacting the compound of Formula (B-II) with a compound of Formula (B-III) to obtain a compound of Formula (B-IV)

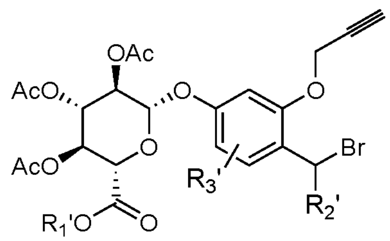

Formula (B-II)

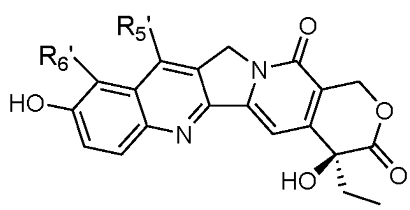

Formula (B-III)

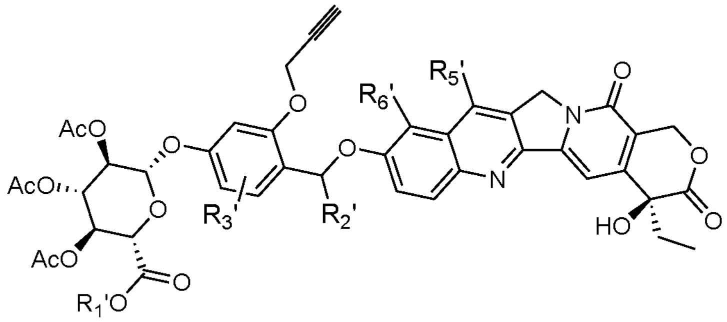

Formula (B-IV)

wherein the groups represented by $R_1'$, $R_2'$, $R_3'$, $R_5'$, and $R_6'$ in the Formula (B-II), the Formula (B-III), and the Formula (B-IV) are as defined for $R_1$, $R_2$, $R_3$, $R_5$, and $R_6$ in the Formula (I);

(6) reacting the compound of Formula (B-IV) under basic conditions to obtain a compound of Formula (B-V),

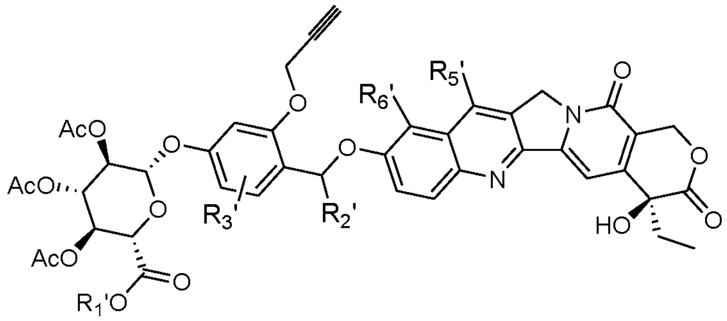

Formula (B-IV)

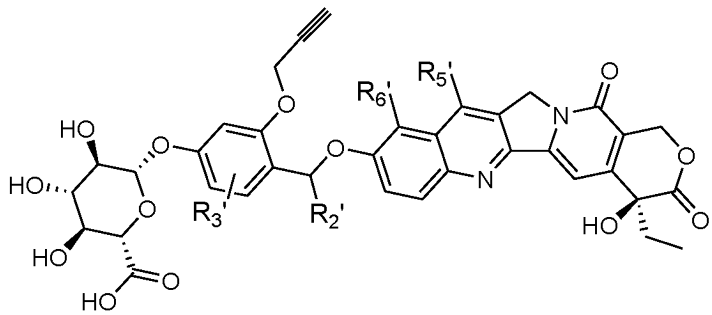

Formula (B-V)

wherein the groups represented by $R_1'$, $R_2'$, $R_3'$, $R_5'$, and $R_6'$ in the Formula (B-IV) and the Formula (B-V) are as defined for $R_1$, $R_2$, $R_3$, $R_5$ and $R_6$ in the Formula (I);

(7) reacting the compound of Formula (A-V) with the compound of Formula (B-V) to obtain the compound of Formula (I), or reacting a product obtained by reacting the compound of Formula (A-V) with the compound of Formula (B-V) with a corresponding acid or base to form a pharmaceutically acceptable salt thereof

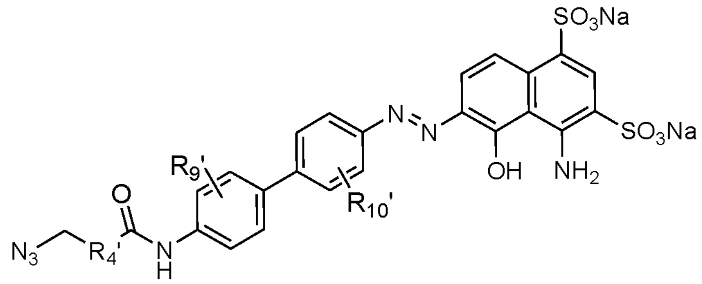

Formula (A-V)

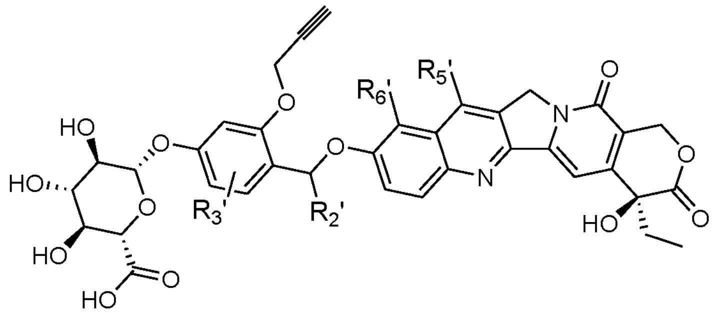

Formula (B-V)

wherein the groups represented by $R_2$', $R_3$', $R_4$', $R_5$', $R_6$', $R_9$', and $R_{10}$' in the Formula (A-V) and the Formula (B-V) are defined as $R_2$, $R_3$, $R_4$, $R_5$, $R_6$, $R_9$, and $R_{10}$ in the Formula (I).

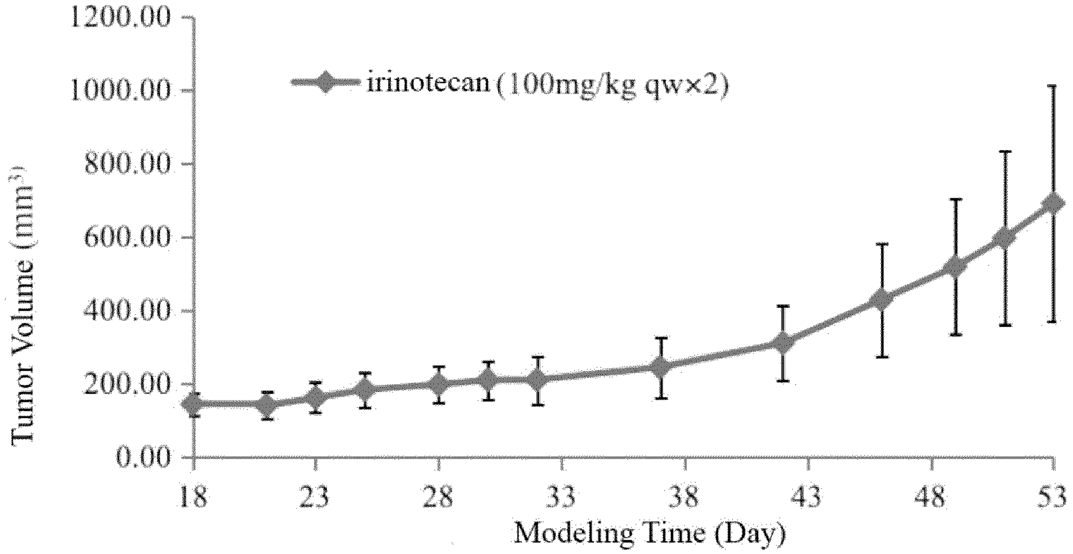
1200.00
1000.00
800.00
600.00
400.00
200.00
0.00
Tumor Volume (mm³)
irinotecan (100mg/kg qw×2)
18
23
28
33
38
43
48
53
Modeling Time (Day)

Fig. 1

**INTERNATIONAL SEARCH REPORT**

International application No.
**PCT/CN2023/097277**

**A. CLASSIFICATION OF SUBJECT MATTER**

C07H15/203(2006.01)i； A61K31/706(2006.01)i； A61K38/14(2006.01)i； A61P35/00(2006.01)i

According to International Patent Classification (IPC) or to both national classification and IPC

**B. FIELDS SEARCHED**

Minimum documentation searched (classification system followed by classification symbols)

IPC：C07H15/203，A61K31/706，A61K38/14，A61P35/-

Documentation searched other than minimum documentation to the extent that such documents are included in the fields searched

Electronic data base consulted during the international search (name of data base and, where practicable, search terms used)

CNTXT, VEN, OETXT, ENTXT, CNKI, REGISTRY(STN), CAPLUS(STN): 喜树碱, 糖苷, 联苯, 三唑, 萘, camptothecin, 根据通式(I)的结构式检索, structural formula search according to general formula (I)

**C. DOCUMENTS CONSIDERED TO BE RELEVANT**

| Category* | Citation of document, with indication, where appropriate, of the relevant passages | Relevant to claim No. |
|---|---|---|
| A | CN 111362990 A (LEVENA (SUZHOU) BIOPHARMA CO., LTD.) 03 July 2020 (2020-07-03)<br>entire document | 1-10 |
| A | WO 2011066418 A1 (ACADEMIA SINICA; PRIJOVIC ZELJKO M.; LEU YU-LIN; ROFFLER STEVE R.;) 03 June 2011 (2011-06-03)<br>entire document | 1-10 |
| A | PRIJOVICH, Zeljko M. et al. "Synthesis and Antitumor Properties of BQC-Glucuronide, a Camptothecin Prodrug for Selective Tumor Activation"<br>*Molecular Pharmaceutics,* Vol. 13, No. (4), 29 January 2016 (2016-01-29),<br>pages 1242-1250 | 1-10 |
| A | LEU, Yu-Ling et al. "Benzyl Ether-Linked Glucuronide Derivative of 10-Hydroxycamptothecin Designed for Selective Camptothecin-Based Anticancer Therapy"<br>*Journal of Medicinal Chemistry,* Vol. 51, No. (6), 05 March 2008 (2008-03-05),<br>pages 1740-1746 | 1-10 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 September 2023** | **19 September 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2023/097277**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **8-9**
because they relate to subject matter not required to be searched by this Authority, namely:

Although claims 8-9 relate to a treatment method implemented on the human or animal body (PCT Rule 39.1(iv)), a search is still carried out on the basis of the pharmaceutical use of the compound/pharmaceutical composition.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/097277**

| Patent document cited in search report | Publication date (day/month/year) | Patent family member(s) | Publication date (day/month/year) |
|---|---|---|---|
| CN 111362990 A | 03 July 2020 | None | |
| WO 2011066418 A1 | 03 June 2011 | US 2013012467 A1 | 10 January 2013 |
| | | US 9353140 B2 | 31 May 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210602187
- WO 2012153193 A


### Non-patent literature cited in the description


- Remington's Pharmaceutical Sciences. Mack Publishing Co., 1990